(19) Europäisches Patentamt
European Patent Office
Office européen des brevets

(11) **EP 4 684 780 A1**

(12) **EUROPEAN PATENT APPLICATION**
published in accordance with Art. 153(4) EPC


(43) Date of publication:
**28.01.2026 Bulletin 2026/05**

(21) Application number: **24774093.9**

(22) Date of filing: **19.03.2024**

(51) International Patent Classification (IPC):
***A61K 31/145*** *(2006.01)* ***A61K 31/196*** *(2006.01)*
***A61P 25/00*** *(2006.01)* ***A61P 13/12*** *(2006.01)*
***A61P 1/00*** *(2006.01)* ***A61P 29/00*** *(2006.01)*
***A61P 9/00*** *(2006.01)* ***A61P 1/16*** *(2006.01)*

(52) Cooperative Patent Classification (CPC):
**A61K 31/145; A61K 31/167; A61K 31/18; A61K 31/196; A61K 31/337; A61K 31/381; A61K 31/40; A61K 31/41; A61K 31/4192; A61K 31/4196; A61K 31/433; A61K 31/549; A61K 31/664; A61P 1/00; A61P 1/04;** (Cont.)

(86) International application number:
**PCT/CN2024/082356**

(87) International publication number:
**WO 2024/193528 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.03.2023 CN 202310299839**
**30.06.2023 CN 202310802971**
**20.11.2023 CN 202311550119**



(71) Applicants:
- **Hangzhou Phecdamed Co., Ltd.**
  **Hangzhou, Zhejiang 311100 (CN)**
- **Nanjing Phecdamed Co., Ltd.**
  **Nanjing, Jiangsu 211122 (CN)**

(72) Inventor: **LIU, Dong**
**Hangzhou, Zhejiang 311100 (CN)**

(74) Representative: **Viering, Jentschura & Partner mbB**
**Patent- und Rechtsanwälte**
**Am Brauhaus 8**
**01099 Dresden (DE)**


(54) **USE OF SMALL-MOLECULE NAPHTHALENE-RING COMPOUND**


(57) The present application discloses uses of naphthalene ring-based small molecule compounds. In the present application, a compound of Formula I is first found exhibiting excellent therapeutic effects on various refractory diseases comprising reproductive system diseases, autoimmune diseases, skin diseases, eye diseases, muscular or skeletal degenerative diseases, neurological diseases, anemia, adrenal diseases, chronic gastritis, myocarditis, periodontitis, hypothyroidism, alopecia, liver injury, tinnitus, chronic obstructive pulmonary disease and the like. Therefore, the compound has significant application value in the prevention and treatment of the aforementioned diseases.


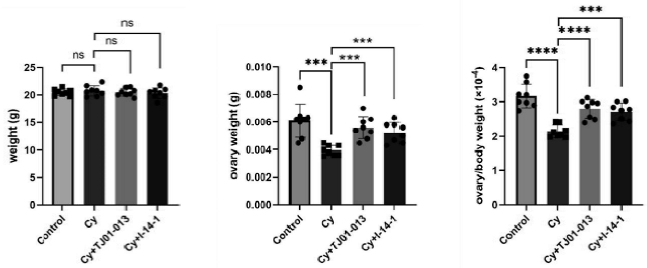


FIG. 1

EP 4 684 780 A1

(52) Cooperative Patent Classification (CPC): (Cont.)
**A61P 1/16; A61P 7/06; A61P 9/00; A61P 9/10;
A61P 13/12; A61P 15/08; A61P 15/10;
A61P 17/00; A61P 17/06; A61P 19/02;
A61P 19/08; A61P 21/00; A61P 25/00;
A61P 25/08; A61P 27/02; A61P 27/12;
A61P 29/00; A61P 37/02**

## Description

### CROSS REFERENCE TO RELATED APPLICATIONS

This patent application claims priority to Chinese Patent Application No. 2023102998399, filed on March 20, 2023 and entitled "USES OF SMALL MOLECULE COMPOUNDS OF NAPHTHYLAMINE STRUCTURE", the disclosure of which is incorporated herein by reference in its entirety.
This patent application claims priority to Chinese Patent Application No. 2023108029717, filed on June 30, 2023 and entitled "NAPHTHALENE RING-BASED SMALL MOLECULE COMPOUNDS", the disclosure of which is incorporated herein by reference in its entirety.
This patent application claims priority to Chinese Patent Application No. 2023115501191, filed on November 20, 2023 and entitled "NAPHTHALENE RING-BASED SMALL MOLECULE COMPOUNDS", the disclosure of which is incorporated herein by reference in its entirety.

### TECHNICAL FIELD

The present invention relates to the field of biomedicine, and in particular to uses of naphthalene ring-based small molecule compounds.

### BACKGROUND ART

Premature ovarian failure (POF) is a major cause of female infertility, affecting at least 1%-5% of women under the age of 40, but there is still no effective therapeutic method available. Women with POF suffer from infertility, a range of menopausal symptoms and other serious complications, such as psychological distress, osteoporosis, autoimmune diseases, ischemic heart disease, and even an increased risk of death. The clinical care for POF women includes a hormone replacement therapy plus psychological support. However, this therapy has significant side effects and limited curative efficacy.
Systemic lupus erythematosus is a prototypical autoimmune disease characterized by loss of immune tolerance. Lupus nephritis is the primary cause of morbidity and mortality in systemic lupus erythematosus. Most patients with lupus nephritis eventually progress to the end-stage renal disease. Currently, there are no specific drugs for the treatment of lupus nephritis. In clinical practice, main therapies include a hormone pulse therapy and an immunosuppressant therapy, in which, however, the use of two primary drugs is accompanied by various severe side effects and exacerbated renal damage.
Sarcopenia is a syndrome characterized by reduced skeletal muscle mass, decreased muscle strength, and impaired muscle function during the aging process. Sarcopenia is the early clinical manifestation of frailty syndrome, and current research on it is still in its infancy. Resistance exercise is the primary method for treating sarcopenia. Sarcopenia has a higher incidence in middle-aged and elderly individuals and is a major trigger for stroke and falls, severely reducing the quality of life in the elderly and increasing the incidence and mortality of diseases. There is currently no cure or symptomatic medication available in the prior art.
Liver injury has numerous causes, with common examples including alcoholic steatohepatitis or drug-induced liver injury. The alcoholic steatohepatitis is a liver disease caused by long-term excessive alcohol consumption, and it is a subtype of alcoholic liver disease. The alcoholic liver disease is the most prevalent type of chronic liver disease worldwide. The alcoholic liver disease may progress from the alcoholic steatohepatitis to the alcoholic steatohepatitis, and the chronic alcoholic steatohepatitis leads to fibrosis and cirrhosis. Drug-induced liver injury is an adverse reaction to drugs or other xenobiotic factors, and the exposure to the toxic doses of some compounds may be harmful to the liver of some susceptible individuals. These risk factors may alter the liver metabolism, leading to cellular stress, cell death, activation of adaptive immune responses, reduced adaptive capacity, and progression to overt liver injury. The progression of liver injury to liver fibrosis and cirrhosis is often chronic and difficult to reverse. Current therapies for liver injury include an antiviral therapy and a surgical therapy, with the former being slow-acting and the latter being highly invasive.
Oligospermia has become a common condition in recent years. Low sperm count is the primary indicator of male infertility, which may be caused by: congenital problems with reproductive organs, such as congenital testicular hypoplasia; systemic diseases acquired later in life, such as chronic radiation sickness; or unhealthy living habits and living environments, such as smoking and alcohol consumption. Some infertile men are particularly sensitive to nicotine and alcohols in tobacco and alcohol, and in particular, the spermatogenic cells in the testes are susceptible to these toxic substances, resulting in decreased sperm motility, low survival rates, and reduced sperm count. According to statistics, about 15% of married couples suffer from infertility, with male factors accounting for about 50% of cases. Currently, infertility caused by oligospermia is usually treated with gonadotropin-releasing hormones, gonadotropin or androgen, etc., but the hormonal therapy cannot effect a radical cure and has serious side effects.

Rheumatoid arthritis (RA) is a common chronic, systemic, progressive autoimmune disease, the exact cause of which remains unclear. Its clinical symptoms mainly manifest as joint swelling, pain, and stiffness; and its pathophysiological manifestations include joint synovial cell proliferation and persistent synovial inflammation. If not treated effectively and sufficiently in time, it may lead to joint structural damage and loss of function, eventually leading to joint deformity or even disability. The prolonged course and poor prognosis of rheumatoid arthritis have a significant impact on the lives of patients and their families. Current treatment means are less effective in curing this disease.

Cataract is also known as lens opacity. It is caused by damage to the lens capsule due to aging, genetics, metabolic abnormalities, trauma, radiation, poisoning, and local malnutrition, which increase the permeability of the lens capsule, impair its barrier function, or lead to the metabolic disorders of the lens, resulting in denaturation of lens proteins and opacity formation. The cataract can be classified as congenital or acquired. Currently, the cataract is mostly treated through surgery, which requires intraocular lens implantation or surgical removal depending on individual conditions, with high cost and high risk.

Cushing syndrome (CS), also known as hypercortisolism, is caused by chronic high-dose glucocorticoids, resulting in a typical clinical phenotype and multisystem complications. Glucocorticoids are secreted by the adrenal glands under the control of the hypothalamic-pituitary-adrenal cortex axis, and play a role in regulating various physiological processes, including cell proliferation, cardiovascular tension, immune response, growth, reproduction, behavior, and energy metabolism. CS may lead to many related diseases, severely affecting the patients' quality of life. In the prior art, CS is commonly treated by a surgical therapy combined with a drug therapy. However, the surgical therapy often carries the risk of postoperative Nelson syndrome, while the drug therapy cannot restore patients to normal function, even though it can alleviate some clinical symptoms.

Hashimoto's thyroiditis (HT) is a chronic lymphocytic thyroiditis caused by defects in the autoimmune system. At present, the main therapies for this disease include a thyroid hormone replacement therapy, an immunotherapy, and a surgical therapy, among others. Although these therapies can alleviate the condition of patients to a certain extent, they have no effect on regulating the patients' abnormal immune system, and HT is prone to recurrence.

Chronic obstructive pulmonary disease (COPD) is a chronic destructive disease of the respiratory system, and it is featured with high morbidity and mortality, and primarily affects middle-aged and elderly individuals. Incomplete reversible airflow limitation is a common symptom among COPD patients, typically manifesting as respiratory diseases. Although COPD is a respiratory disease, there are currently no completely effective measures to halt the decline in patients' lung function.

Tinnitus is a common functional disorder, and its incidence increases with the growth of an individual's age, ranging from 10% to 15%, severely affecting the quality of life of those affected. Traditionally, tinnitus is regarded as a disorder of the auditory function. However, when tinnitus exceeds the physiological limit, it becomes symptomatic tinnitus. The occurrence of tinnitus may be caused by various factors, with hearing loss being the most common trigger for tinnitus symptoms. Tinnitus patients suffer long-term distress. Currently, only symptomatic treatment is available, with no radical cure.

Psoriasis is caused by the interaction of various factors such as immunity, genetics and environment. It is mainly mediated by T lymphocytes and involves the participation of multiple immune cells. Psoriasis can affect muscles, joints, nails and even the eyes, and it can coexist with cardiovascular and cerebrovascular diseases, metabolic syndrome, tumors and other autoimmune disorders. The histopathological features of psoriasis generally manifest as hyperkeratosis with parakeratosis, accompanied by significant thickening of the epidermis. Currently, the immunotherapy has replaced antibiotics and become a new approach for psoriasis treatment. However, there are still no drugs with satisfactory efficacy.

Age-related macular degeneration (AMD) refers to age-related changes in the structure of the macular region. Its etiology remains undetermined, and is possibly related to genetics, chronic light damage, nutritional disorders, poisoning, immune diseases, cardiovascular and respiratory systemic diseases, among others. It may also result from the combined effects of multiple factors. At present, low-vision correction methods are predominantly employed for treatment, but with quite limited therapeutic effects. Most of these methods still fail to prevent the loss of central vision in patients.

Retinal pigmentosa (RP) is a progressive hereditary retinal degenerative disease, with the main clinical manifestations as night blindness occurring since childhood and progressive narrowing of the visual field. The global prevalence rate is approximately 1/3000 - 1/7000. Its pathological mechanism is believed to involve progressive apoptosis of photoreceptors caused by primary genetic defects or secondary degeneration of retinal pigment epithelial cells. Currently, there are no effective therapies for RP. According to studies, it is believed that RP is a chronic inflammatory disease, with inflammatory factors and immune regulation processes playing a significant role in the progression of the disease. Over the past few decades, researchers have proposed many strategies for treating RP, such as neurotrophic factors, Chinese medicinal therapies, visual prosthetic devices and gene therapies, which, however, shows unsatisfactory results, and there is still no definitive and effective therapy.

Multiple sclerosis (MS) is an autoimmune demyelinating disease with a complex pathogenesis that affects the central nervous system. This disease is characterized by multiple lesions, a relapsing-remitting course and a high disability rate, severely affecting the normal life of patients. Its pathogenesis remains unclear and may be caused by a combination

of various factors such as immunity, environment and genetics. The currently accepted pathogenesis involves abnormal immune attacks that lead to damage to the blood-brain barrier, allowing various inflammatory factors and immune cells to enter the central nervous system and act on microglia and immune cells in the central nervous system to trigger inflammatory responses that result in myelin loss, neuronal death and axonal damage. Current therapies for multiple sclerosis primarily focus on anti-inflammation, which is, however, only effective during the relapsing and remitting phases of the disease. Therefore, the development of a therapy for multiple sclerosis has become an urgent issue to be addressed.

Hypothyroidism, also called underactive thyroid disease, is a pathological condition where the thyroid hormone functions in the tissues are insufficient or absent. It is more common in women than in men, and its prevalence increases with age. Modern medicine recognizes that hypothyroidism can be caused by various factors, with the majority of cases resulting from autoimmune thyroiditis, radioactive iodine treatment of the thyroid, or thyroid surgery. According to the modern medicine, the thyroid hormone replacement therapy is typically adopted for patients with hypothyroidism. However, long-term use of thyroid hormones can directly stimulate osteoclasts to enhance bone resorption, leading to risks such as osteoporosis, coronary heart disease, and heart failure, among others. Hence, it has become an urgent task to find a safer and more effective therapy for the prevention and treatment of hypothyroidism.

Chronic atrophic gastritis (CAG) is a common clinical disease with incidence and detection rates increasing with age. It refers to a chronic gastric disorder characterized by repeated damage to the gastric mucosal epithelium, resulting in a reduction of intrinsic glands, with or without intestinal metaplasia or pseudo-pyloric metaplasia. Generally, it presents no specific symptoms, and those with symptoms mainly manifest as non-specific dyspeptic symptoms such as epigastric fullness and pain, which may be accompanied by belching, loss of appetite, acid reflux, gastric discomfort, bitter taste, nausea and other symptoms. These symptoms often recur. Currently, there is no effective drug to treat and alleviate all clinical symptoms.

Myocarditis refers to an acute, subacute or chronic inflammatory lesion that is localized or diffuse in the myocardium. It commonly affects children and young adults. Persistent cases may lead to dilated cardiomyopathy and chronic heart failure. Myocarditis can be classified into three types: idiopathic, autoimmune, and infectious. In clinical practice, myocarditis is treated primarily by focusing on intervening in the early acute inflammatory phase. Once it progresses to the chronic phase, the main treatment approach is conservative therapy and reducing the burden on the heart. There are no specific treatment methods available.

Alopecia refers to the loss of scalp hair, which is caused by the autoimmune deficiency in the body. Seborrheic alopecia, alopecia areata and androgenetic alopecia are currently recognized as the three most prevalent types of alopecia. Due to changes in mental stress and dietary habits, seborrheic alopecia has become the predominant type of hair loss today. Minoxidil and finasteride are currently recognized as the drugs for treating seborrheic alopecia. However, during the administration of these two drugs, significant side effects may occur in the human body, such as sexual dysfunction during the use of finasteride, and allergic dermatitis, myocardial infarction and anorexia as adverse reactions to topical minoxidil application. Hence, the search for a drug with good safety and high biological activity against hair loss has become a current priority.

Primary aldosteronism (PA) is a heterogeneous disease caused by excessive aldosterone secretion from the adrenal cortex, leading to increased blood volume, sodium retention, potassium excretion and metabolic alkalosis, with clinical manifestations as hypertension and hypokalemia. Currently, there is no effective therapeutic drug available.

Erectile dysfunction, one of the most common diseases in andrology, refers to the persistent (at least 6 months) inability of the pennis to achieve and maintain sufficient erection for satisfactory sexual intercourse, and it is characterized primarily by the inability of the pennis to achieve or sustain erection and often accompanied by symptoms such as decreased libido and premature ejaculation. Epidemiological data indicate that approximately 52% of men aged 40-70 suffer from varying degrees of erectile dysfunction all over the world. However, the main methods for treating erectile dysfunction involve hormone injections, etc., which have significant side effects.

Periodontitis is a chronic inflammatory disease that damages the integrity of tooth-supporting tissues. Severe periodontitis not only leads to tooth loss but also increases the risk of systemic diseases such as atherosclerosis, rheumatoid arthritis, aspiration pneumonia, and cancer. The current effective means for treating periodontitis involves mechanical removal of local irritants. However, some special tooth structures, such as the root bifurcation area, may restrict the penetration of instruments, preventing the accumulated plaques from being effectively removed. At present, there is an urgent need to develop an effective drug for treating periodontitis.

Cerebral stroke is a severe cerebrovascular disease and is also one of the leading causes of long-term disability and premature death. Most (approximately 80%) of the cerebral stroke cases are ischemic strokes caused by vascular occlusion, which is primarily due to arterial thrombosis. At present, the most effective method for treating ischemic stroke is rapid restoration of blood supply. However, ischemia-reperfusion injury following revascularization may further exacerbate brain damage and neuromotor dysfunction, making neurological function recovery a critical issue that needs urgent resolution.

Epilepsy, one of the most common neurological system diseases in clinical practice, is a chronic brain dysfunction caused by abnormal electrical activity in the brain, with the clinical manifestation as recurrent epileptic seizures. In addition

to the harms caused by the epileptic seizures themselves, cognitive impairment is one of the most common complications in epilepsy patients with recurrent seizures. These patients may experience difficulties in learning and memory, as well as significant reductions in their ability to solve problems and form concepts, etc. Furthermore, although currently available antiepileptic drugs are effective in controlling the epileptic seizures for many epilepsy patients, these drugs may also impair the cognition and behavior of epilepsy patients by upregulating inhibitory neurotransmitters or suppressing neuronal excitability. Hence, it is highly beneficial and urgent for epilepsy patients to develop a drug to improve cognitive impairment in epilepsy patients, or even a drug that both enhances cognition and alleviate epileptic seizures.

Melasma is an acquired chromatodermatosis, typically manifested as symmetrical light brown to dark brown patchy pigmentation on the face, which is classified under the category of " dusty facial complexion " in traditional Chinese medicine. It often occurs to women in the child-bearing period and is a clinically common yet difficult-to-cure cosmetic disease that severely impacts patients' mental health. However, there are currently no effective therapeutic drugs available.

Vitiligo, a common pigmentary disorder in dermatology, is featured with white or milky-white localized spots or patches of varying sizes and shapes, with smooth surfaces, clearly defined borders and absence of itching or pain. It can occur in any part of the body but is most commonly seen on the face, neck, back, trunk, and external genitalia, and in severe cases, the hair may also turn white. Vitiligo significantly affects the physiological and psychological states of patients, and currently, major therapies for vitiligo include the hormone therapy, which has notable side effects.

β-Thalassemia, also known as Mediterranean anemia or globin synthesis disorder, is a hemolytic anemia disease caused by genetic mutations and is one of the most widespread monogenic genetic disorders globally. It is a hemolytic genetic disorder caused by reduced or absent synthesis of β-globin chains, leading to an imbalance between the α-chain/non-α-chain of hemoglobin tetramers. Microcytic hypochromic anemia with elevated HbF and HbA2 levels is its typical clinical feature. Currently, β-thalassemia is one of the most prevalent and severe genetic disorders in provinces south of the Yangtze River in China. However, there are still no effective treatment methods available.

Lumbar disc herniation, the most prevalent disease in spinal surgery, is a common and frequently occurring clinical condition that imposes a heavy burden on the society and families. It is a syndrome results from degenerative changes in the lumbar intervertebral disc, rupture of the annulus fibrosus, and herniation of the nucleus pulposus, which irritates or compresses the nerve roots or cauda equina. It is a common and frequently occurring disease affecting human health and one of the primary causes of clinical pain in waist and lower extremities. With the aging of the population and changes in lifestyle in China, the incidence of intervertebral disc degenerative diseases has been increasing year by year. Current clinical therapies mainly include non-surgical therapies (such as steroidal anti-inflammatory drugs, physical therapy, and pain management) and surgical therapies, none of which can fundamentally prevent the occurrence and progression of lumbar disc herniation. There is an urgent need to develop a therapeutic drug based on the pathogenesis to restore the function of intervertebral discs.

Polycystic ovary syndrome (PCOS) is a heterogeneous disorder affecting multiple systems throughout the body, primarily impacting the reproductive health of women in the child-bearing period, and it is one of the most significant causes of ovulatory infertility in women. The pathological manifestations of PCOS mainly include hormonal imbalances, impaired follicular development and ovulation disorders, abnormally elevated serum androgen levels, insulin resistance, and obesity. This condition not only affects the reproductive function in women but also significantly increases the risk of long-term complications such as type 2 diabetes, cardiovascular diseases, and endometrial cancer in affected patients. Currently, PCOS has a high incidence rate, but existing therapeutic drugs have notable side effects, for example, severe menstrual disorders, weight gain, infertility, and hirsutism. There is an urgent need to develop an effective drug with minimal side effects.

In summary, there are no drugs in the prior art that demonstrate good efficacy for the aforementioned diseases, and there is an urgent need to identify safe and effective therapeutic drugs for these diseases.

## **SUMMARY** OF THE INVENTION

An object of the present invention is to provide uses of naphthalene ring-based small-molecule compounds.

In a first aspect of the present invention, there is provided use of a compound of Formula (I), or a pharmaceutically acceptable salt, stereoisomer, solvate or prodrug thereof, in (i) prevention and/or treatment of and/or (ii) preparation of a medicament for preventing and/or treating the following diseases: reproductive system diseases, autoimmune diseases, skin inflammation, retinal inflammation, ocular degenerative diseases, muscular or skeletal degenerative diseases, neurological diseases, anemia, adrenal diseases, chronic gastritis, myocarditis, periodontitis, hypothyroidism, alopecia, liver injury, tinnitus, and chronic obstructive pulmonary disease,

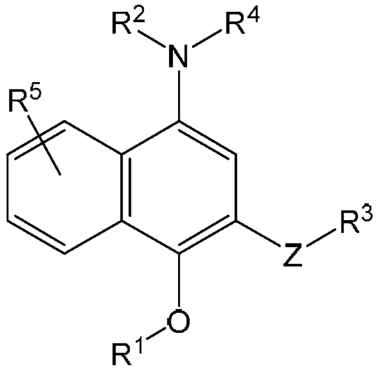

I

wherein Z is

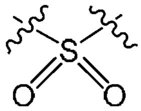 , 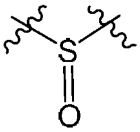 , 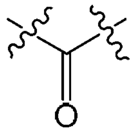 , 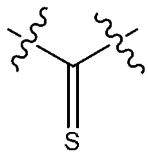

or a sulfur atom;

$R^1$ is hydrogen, $C_{1\sim6}$ alkyl,

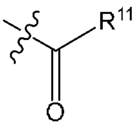 , 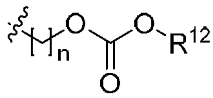 ,

wherein $R^{11}$ and $R^{12}$ are independently $C_{1\sim6}$ alkyl or $C_{1\sim6}$ cycloalkyl, respectively, and n is 1~4;

$R^2$ is hydrogen, $C_{1\sim6}$ alkyl, three- to six-membered cycloalkyl, three- to six-membered epoxyalkyl, phenyl, $C_{1\sim6}$ alkyl with at least one hydrogen substituted with $R^{2-1}$, phenyl with at least one hydrogen substituted with $R^{2-1}$, wherein $R^{2-1}$ is hydroxyl, halogen, amino, or $C_{1\sim6}$ alkoxy,

$R^3$ is

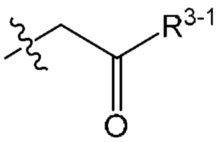 , 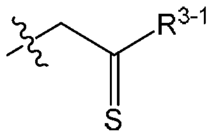 or 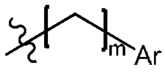 ;

wherein $R^{3-1}$ is hydrogen, hydroxyl, $C_{1\sim6}$ alkyl, $C_{1\sim6}$ alkoxy, $C_{3\sim6}$ cycloalkyl, three- to six-membered epoxyalkyl, amino, $C_{1\sim6}$ amine group, $-CH_2C(O)R^{3-2}$, $-CH_2C(O)OR^{3-2}$, $-CH_2C(O)N$ ($R^{3-2}$ $R^{3-2a}$), and $R^{3-2}$ and $R^{3-2a}$ are independently hydrogen, C1~6 alkyl, or three- to six-membered cycloalkyl respectively,

m is 1 to 6, and

Ar is phenyl, naphthyl, 5- or 6-membered monocyclic heteroaryl, 8- to 10-membered fused bicyclic heteroaryl, phenyl with at least one hydrogen atom substituted with $R^{3-3}$, and naphthylwith at least one hydrogen atom substituted with $R^{3-3}$, 5- or 6-membered monocyclic heteroaryl with at least one hydrogen atom substituted with $R^{3-3}$, 8- to 10-membered fused bicyclic heteroaryl with at least one hydrogen atom substituted with $R^{3-3}$, the $R^{3-3}$ is hydrogen, halogen, $C_{1\sim6}$ alkyl, three-to six-membered cycloalkyl, hydroxy, $C_{1\sim6}$ alkoxy, three- to six-membered epoxyalkyl, $C_{1\sim6}$ haloalkyl, $C_{2\sim6}$ alkenyl, $C_{2\sim6}$ alkynyl, $-N(R^{3-3a}R^{3-3b})$ or phenyl,

$R^{3-3a}$ and $R^{3-3b}$ are independently hydrogen, $C_{1\sim6}$ alkyl, or three- to six-membered cycloalkyl, respectively;

$R^4$ is

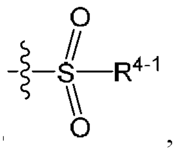 , 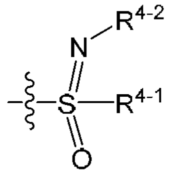 , 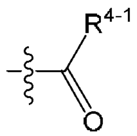 or 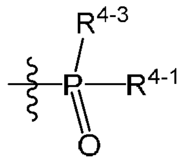 .

wherein $R^{4-1}$ is phenyl, and biphenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, $R^{4-11}$ is hydrogen, halogen, nitro, nitrile, hydroxyl, $C_{1\sim6}$ alkyl, $C_{13\sim6}$ cycloalkyl, $C_{1\sim6}$ alkoxy, -N($R^{4-1a}R^{4-1b}$), phenyl, $C_{1\sim6}$ haloalkyl, $C_{1\sim6}$ haloalkoxy, -C(O)O$R^{4-12}$, -C(O)$R^{4-12}$, -C(O)N($R^{4-1a}R^{4-1b}$), -S(O)2$R^{4-12}$, -S(O)$R^{4-12}$, - OC(O)$R^{4-12}$, -OC(O)O$R^{4-12}$ or

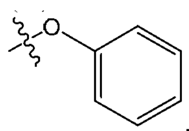 ,

$R^{4-12}$, $R^{4-1a}$ and $R^{4-1b}$ are independently hydrogen, $C_{1\sim6}$ alkyl, $C_{3\sim6}$ cycloalkyl, $C_{2\sim6}$ alkenyl, $C_{2\sim6}$ alkynyl, $C_{1\sim6}$ alkyl substituted with at least one hydrogen by a halogen, $C_{2\sim6}$ alkynyl with at least one hydrogen substituted with a halogen, $C_{3\sim6}$ cycloalkyl with at least one hydrogen substituted with a halogen, C2~6 alkynyl with at least one hydrogen substituted with a halogen, respectively, and $R^{4-1a}$ and $R^{4-1b}$ may be bonded to each other to form a ring, $R^{4-2}$ is $C_{1\sim6}$ alkyl, $C_{3\sim6}$ cycloalkyl, $C_{1\sim6}$ alkyl with at least one hydrogen substituted with a hydroxyl, $C_{3\sim6}$epoxyalkyl, or $R^{4-2}$ is bonded to R2 to form a 4- to 8-membered ring when $R^2$ is $C_{1\sim6}$ alkyl and $R^{4-2}$ is $C_{1\sim6}$ alkyl.

$R^{4-3}$ is $C_{1\sim6}$ alkyl or $C_{1\sim6}$ alkoxy;

the number of $R^5$ is 0~5, and $R^5$, at each occurrence, is independently hydrogen, halogen, nitro, nitrile group, -N+($R^{5-1}$)$_3$, $C_{1\sim6}$ haloalkyl, -C(O)O$R^{5-1}$,-C(O)$R^{5-1}$, -C(O)N($R^{5-1}R^{5-1a}$), -S(O)$_2R^{5-1}$, -S(O)$R^{5-1}$, -S(O)$_2$N($R^{5-1}R^{5-1a}$), -S(O)N($R^{5-1}R^{5-1a}$), -N=C($R^{5-1}$ $R^{5-1a}$), hydroxyl, $C_{1\sim6}$ alkyl, phenyl, phenyl with at least one hydrogen substituted with $R^{5-1}$, $C_{1\sim6}$ alkoxy, -N($R^{5-1}R^{5-1a}$), -N($R^{5-1}$)C(O)$R^{5-1a}$, -N($R^{5-1}$)C(O)O$R^{5-1a}$, -OC(O)$R^{5-1}$, -OC(O)O$R^{5-1}$, -OC(O)N($R^{5-1}R^{5-1a}$) or -S$R^{5-1}$, respectively, wherein $R^{5-1}$, $R^{5-1a}$ and $R^{5-1b}$are independently hydrogen, $C_{1\sim6}$ alkyl, $C_{2\sim6}$ alkenyl, $C_{2\sim6}$ alkynyl, $C_{1\sim6}$ alkywith at least one hydrogen substituted with a halogen, $C_{2\sim6}$ alkenylwith at least one hydrogen substituted with a halogenor $C_{2\sim6}$ alkynyl with at least one hydrogen substituted with a halogen, respectively.

In some preferred embodiments, the Z is

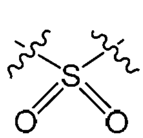 , 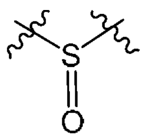 , 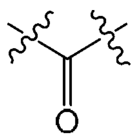 ,

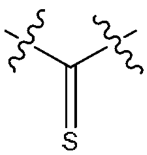

or a sulfur atom;

$R^1$ is hydrogen, $C_{1\sim4}$ alkyl,

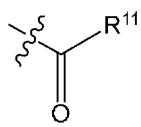 or 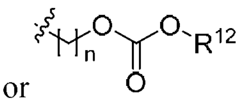 ,

wherein $R^{11}$ and $R^{12}$ are independently $C_{1\sim4}$ alkyl or $C_{1\sim4}$ cycloalkyl, respectively, and n is 1 or 2;

$R^2$ is hydrogen, $C_{1\sim4}$ alkyl, three- to six-membered cycloalkyl, three- to six-membered epoxyalkyl, phenyl, $C_{1\sim4}$ alkyl with at least one hydrogen substituted with $R^{2-1}$, and phenyl with at least one hydrogen substituted with $R^{2-1}$, wherein $R^{2-1}$ is hydroxy, halogen, amino, or $C_{1\sim4}$ alkoxy;

$R^3$ is

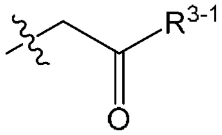 , 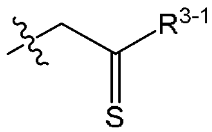 or 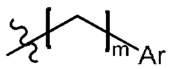 ;

wherein $R^{3-1}$ is hydrogen, hydroxyl, $C_{1\sim4}$ alkyl, $C_{1\sim4}$ alkoxy, $-N(R^{3-2}R^{3-2a})$,

$R^{3-2}$ and $R^{3-2a}$ are independently hydrogen or $C_{1\sim4}$ alkyl, respectively,

m is 1~4.

Ar is phenyl, 5- or 6-membered monocyclic heteroaryl, 5- or 6-membered monocyclic heteroaryl with at least one hydrogen atom substituted with $R^{3-3}$, the $R^{3-3}$ is hydrogen, halogen, $C_{1\sim4}$ alkyl, hydroxyl, $C_{1\sim4}$ alkoxy, $C_{1\sim4}$ haloalkyl, or $-N(R^{3-3a}R^{3-3b})$;

$R^{3-3a}$ and $R^{3-3b}$ are independently hydrogen or $C_{1\sim4}$ alkyl, respectively,

$R^4$ is

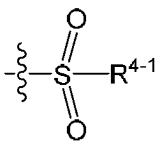 , 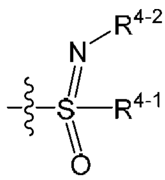 , 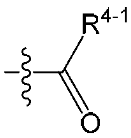 or 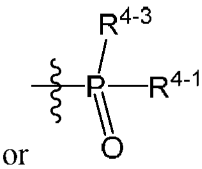 ,

wherein $R^{4-1}$ is phenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, and the $R^{4-11}$ is hydrogen, halogen, nitro, $C_{1\sim4}$ alkyl, $C_{3\sim6}$ cycloalkyl, $C_{1\sim4}$ alkoxy, $-N(R^{4-1a}R^{4-1b})$, phenyl, $C_{1\sim4}$ haloalkyl, $C_{1\sim4}$ haloalkoxy or

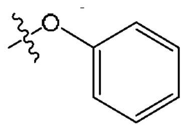 ,

$R^{4-1a}$ and $R^{4-1b}$ are independently hydrogen, $C_{1\sim4}$ alkyl, or $C_{3\sim6}$ cycloalkyl, respectively, and $R^{4-1a}$ and $R^{4-1b}$ may be bonded to each other to form a ring,

$R^{4-2}$ is $C_{1\sim4}$ alkyl, $C_{3\sim5}$ cycloalkyl, $C_{1\sim4}$ alkyl with at least one hydrogen substituted with hydroxyl, $C_{3\sim5}$epoxyalkyl, or $R^{4-2}$ is bonded to $R^2$ to form a 4- to 8-membered ring when $R^2$ is $C_{1\sim4}$ alkyl and $R^{4-2}$ is $C_{1\sim4}$ alkyl,

$R^{4-3}$ is $C_{1\sim4}$ alkyl or $C_{1\sim4}$ alkoxy;

$R^5$ at each occurrence, is independently hydrogen halogen, nitro, nitrile group, $-N+(R^{5-1})_3$, $C_{1\sim4}$ haloalkyl,$C(O)OR^{5-1}$, $-C(O)R^{5-1}$, $-C(O)N(R^{5-1}R^{5-1a})$, $-S(O)_2R^{5-1}$ $-S(O)R^{5-1}$, $-N{=}C(R^{5-1}\ R^{5-1a})$, hydroxyl, $C_{1\sim4}$ alkyl, phenyl, phenyl with at least one hydrogen substituted with $R^{5-1}$, $C_{1\sim4}$ alkoxy, $-N(R^{5-1}R^{5-1a})$, $-N(R^{5-1})C(O)R^{5-1a}$, or $-OC(O)R^{5-1}$, respectively, wherein $R^{5-1}$, $R^{5-1a}$, and $R^{5-1b}$ are independently hydrogen, $C_{1\sim4}$ alkyl, $C_{1\sim4}$ alkyl with at least one hydrogen substituted with a halogen, respectively.

In some preferred embodiments, the Z is

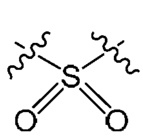 , 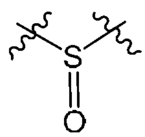 , 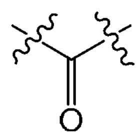 ,

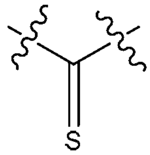

or a sulfur atom;

In some preferred embodiments, $R^1$ is hydrogen,

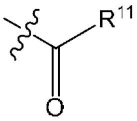 or 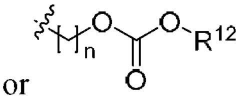,

wherein $R^{11}$ and $R^{12}$ are independently methyl, ethyl, n-propyl or isopropyl, respectively, and n is 1.

In some preferred embodiments, $R^2$ is hydrogen, methyl, ethyl, n-propyl, isopropyl, ethyl with one hydrogen substituted with hydroxyl, n-propyl with one hydrogen substituted with hydroxyl, phenyl,

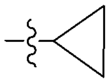, 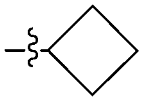, 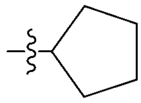,

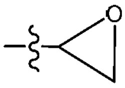, 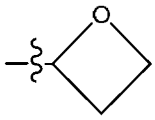, 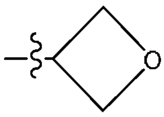, 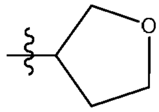 or 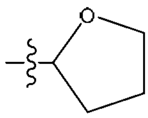.

In some preferred embodiments, $R^3$ is

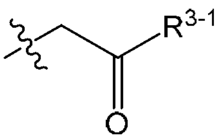, 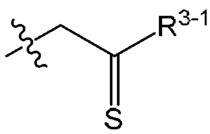

or

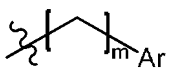;

wherein $R^{3-1}$ is hydrogen, hydroxyl, methyl, ethyl, n-propyl, isopropyl, methoxy, ethoxy, n-propoxy, isopropoxy, n-butoxy, tert-butoxy, sec-butoxy or isobutoxy,

m is 1,

Ar is phenyl, 5- or 6-membered nitrogen-containing monocyclic heteroaryl.

In some preferred embodiments, $R^4$ is

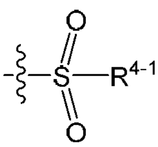, 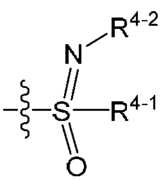, 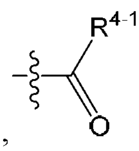

or

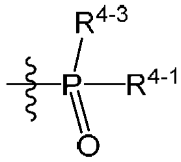,

wherein R4-1 is phenyl, phenyl with at least one hydrogen substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and the $R^{4-11}$ is halogen, nitro, methyl, ethyl, n-propyl, isopropyl,

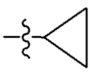, 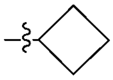, 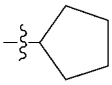, 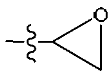, 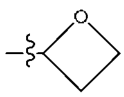, 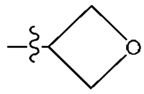,

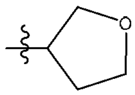, 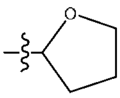, 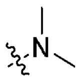, 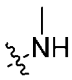, 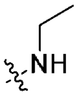, 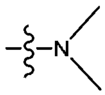, 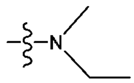, 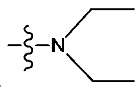, 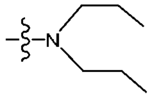,

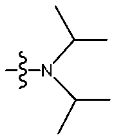, 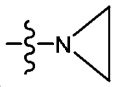, 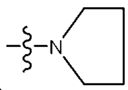, 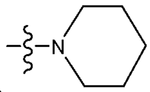,

fluoromethyl, fluoroethyl, fluoro-n-propyl, fluoroisopropyl, chloromethyl, chloroethyl, chloro-n-propyl, chloroisopropyl, bromomethyl, bromoethyl, bromo-n-propyl, bromoisopropyl, iodomethyl, iodoethyl, iodon-propyl, iodoisopropyl, fluoromethoxy, fluoroethoxy, fluoron-propoxy, fluoroisopropoxy, chloromethoxy, chloroethoxy, chloro-n-propoxy, chloroisopropoxy, bromomethoxy, bromoethoxy, bromo-n-propoxy, bromoisopropoxy, Iodooxymethyl, iodoethoxy, iodon-propoxy, iodoisopropoxy, or

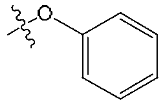,

$R^{4-2}$ is methyl, ethyl, n-propyl, isopropyl, n-butyl, isobutyl, tert-butyl, ethyl with one hydrogen substituted with hydroxyl, n-propyl with one hydrogen substituted with hydroxyl, n-butyl with one hydrogen substituted with hydroxyl.

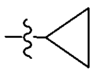, 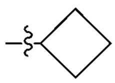,

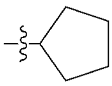, 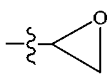, 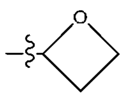, 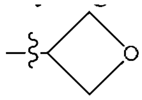, 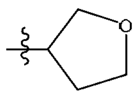, 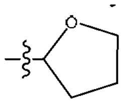

or phenyl, or, $R^{4-2}$ is bonded to $R^2$ to form a 4 to 8-membered ring when $R^2$ is methyl, ethyl or n-propyl and $R^{4-2}$ is methyl, ethyl,

$R^{4-3}$ is methyl, ethyl, n-propyl, isopropyl, methoxy, ethoxy, n-propoxy or isopropoxy.

In some preferred embodiments, $R^5$ , at each occurrence, is independently hydrogen, halogen, nitro, nitrile group, $-N+(R^{5-1})^3$, fluoro-methyl, fluoroethyl, fluoron-propyl, fluoro-isopropyl, chloromethyl, chloroethyl, chloron-propyl, chloro-isopropyl, bromomethyl, bromoethyl, bromo-n-propyl, bromo-isopropyl, $-C(O)OR^{5-1}$, $-C(O)R^{5-1}$, $-C(O)N(R^{5-1}R^{5-1a})$, $-S(O)_2R^{5-1}$, $-S(O)R^{5-1}$, $-N=C(R^{5-1} R^{5-1a})$, hydroxyl, methyl, ethyl, n-propyl, isopropyl, phenyl, phenyl with

at least one hydrogen substituted with $R^{5-1}$, methoxy, ethoxy, n-propoxy, isopropoxy, -N($R^{5-1}R^{5-1a}$), -N($R^{5-1}$)C(O)$R^{5-1a}$, -OC(O)$R^{5-1}$, respectively, wherein $R^{5-1}$, $R^{5-1a}$, and $R^{5-1b}$ are independently hydrogen, methyl, ethyl, n-propyl, isopropyl, fluoro-methyl, fluoroethyl, fluoron-propyl, fluoro-isopropyl, chloro-methyl, chloroethyl, chloro n-propyl, chloro isopropyl, bromo-methyl, bromoethyl, bromo n-propyl, or bromo isopropyl, respectively.

In some preferred embodiments, $R^1$ is hydrogen,

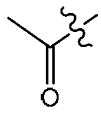 , 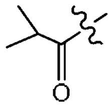

or

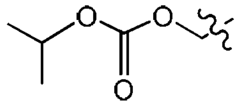 .

In some preferred embodiments, $R^2$ is hydrogen, methyl,

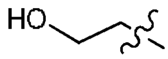 , 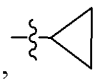 ,

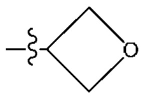

or phenyl.

In some preferred embodiments, $R^3$ is

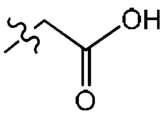 , 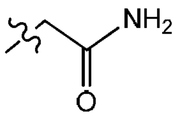 , 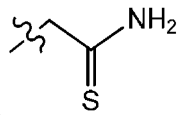 ,

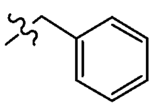 , 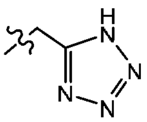 or 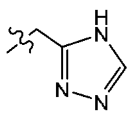 .

In some preferred embodiments, $R^4$ is

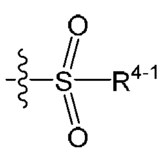 , 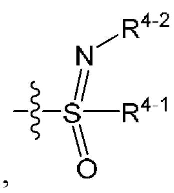 , 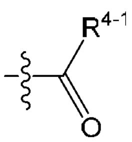

or

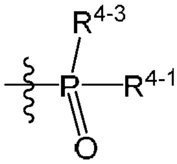

,

wherein $R^{4-1}$ is phenyl, phenyl with at least one hydrogen substituted with $R^{4-11}$, five-membered monocyclic heteroaryl, five-membered monocyclic heteroaryl with at least one hydrogen substituted with $R^{4-11}$, nine-membered fused bicyclic heteroaryl, or naphthyl, wherein $R^{4-11}$ is bromine, fluorine, chlorine, methyl, nitro, phenyl, trifluoromethyl,

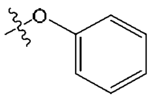

,

methoxy, cyclopropyl, trifluoromethoxy, nitro or

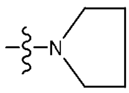

.

$R^{4-2}$ is methyl, ethyl,

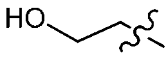 , 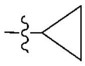 , 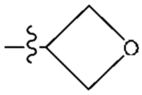

or phenyl, or $R^{4-2}$ is bonded to $R^2$ to form a 4 to 6-membered ringwhen $R^2$ is methyl, ethyl, or n-propyl and $R^{4-2}$ is methyl, ethyl,

$R^{4-3}$ is methoxy, ethoxy, n-propoxy or isopropoxy.

In some preferred embodiments, $R^5$ , at each occurrence, is independently halogen, nitro, nitrile, carboxyl, $-NHC(O)CH_3$, methoxy, or hydroxy, respectively.

In some preferred embodiments, the compound is selected from any one of the following compounds:

| | |
|---|---|
| **I-1** | 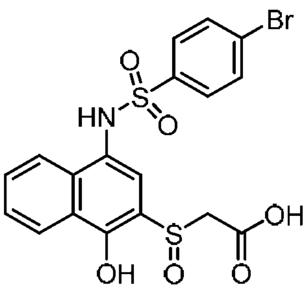 |
| **I-2** | 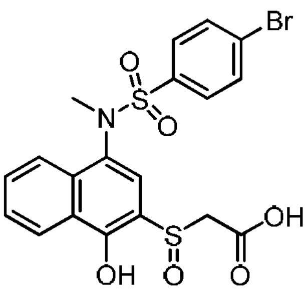 |

(continued)

| | |
|---|---|
| I-3 | 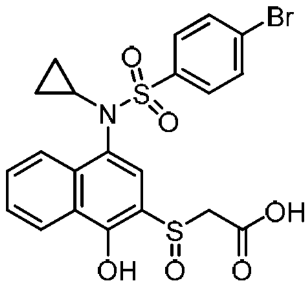 |
| I-4 | 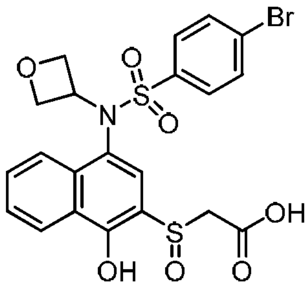 |
| I-5 | 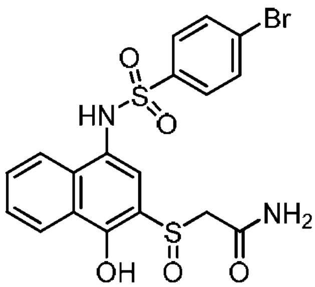 |
| I-6 | 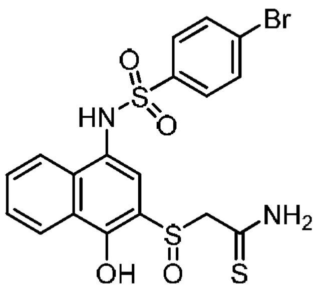 |
| I-7-1 | 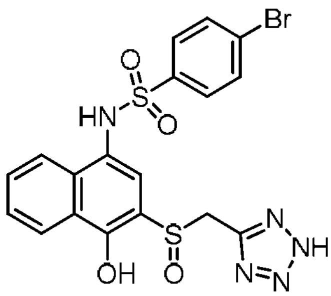 |

(continued)

| | |
|---|---|
| **I-8** | 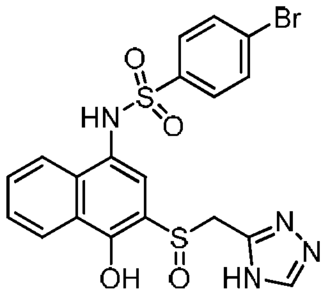 |
| **I-9-1** | 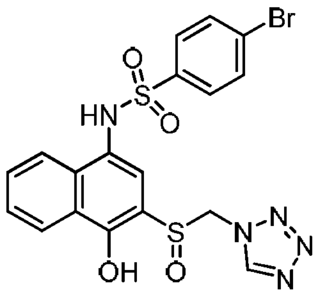 |
| **I-9-2** | 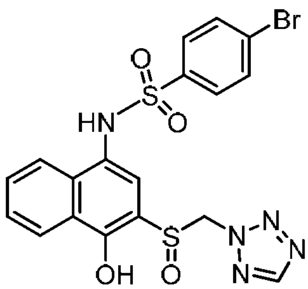 |
| **I-10** | 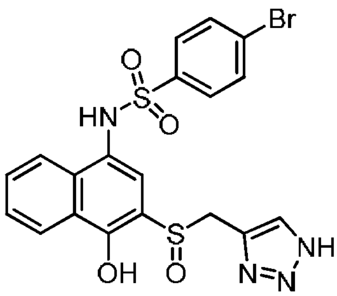 |
| **I-11-1** | 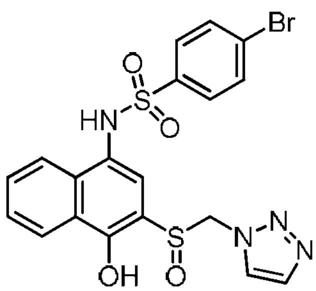 |

(continued)

| | |
|---|---|
| **I-11-2** | 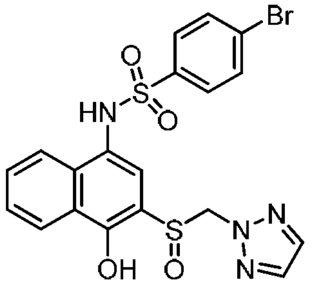 |
| **I-12** | 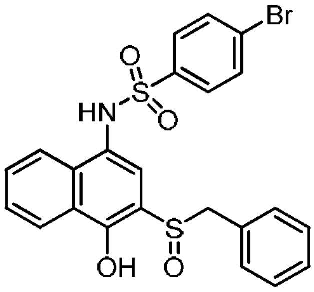 |
| **I-13-1** | 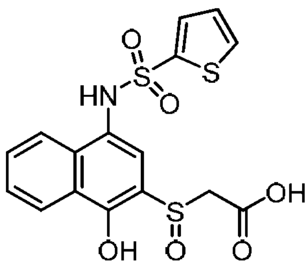 |
| **I-14-1** | 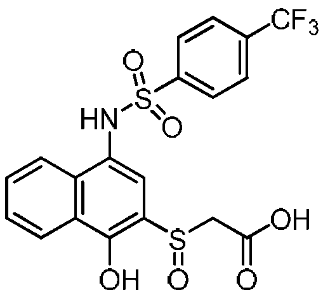 |
| **I-15-1** | 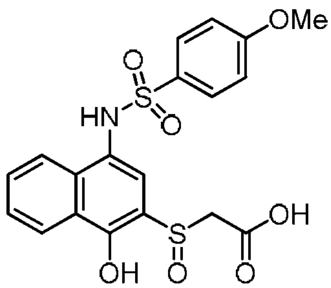 |

(continued)

| | |
|---|---|
| **I-16-1** | 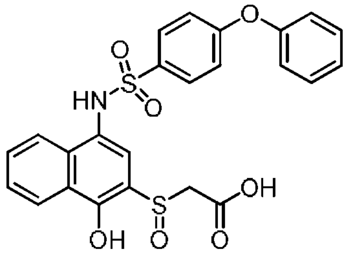 |
| **I-17-1** | 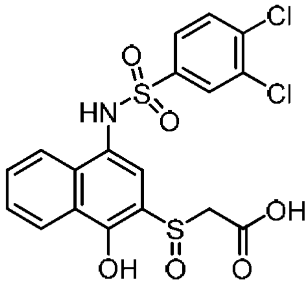 |
| **I-18-1** | 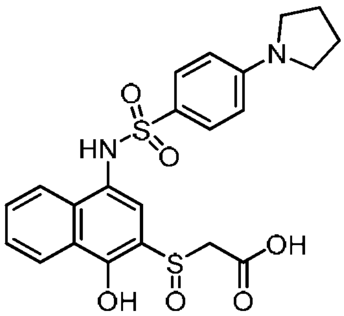 |
| **I-19-1** | 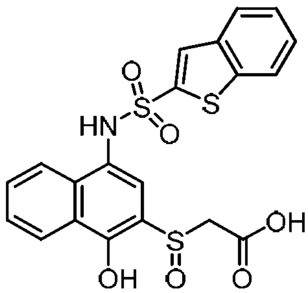 |
| **I-20-1** | 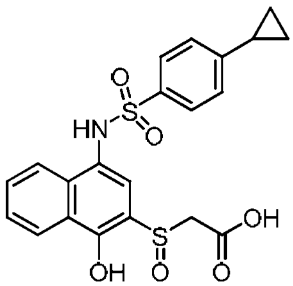 |

(continued)

| | |
|---|---|
| **I-21-1** | 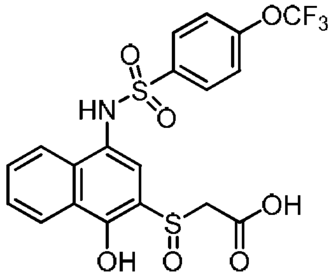 |
| **I-22-1** | 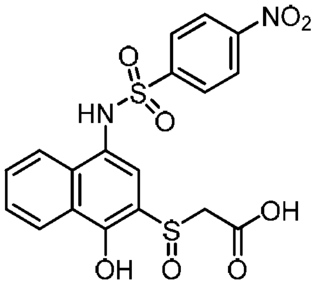 |
| **I-23-1** | 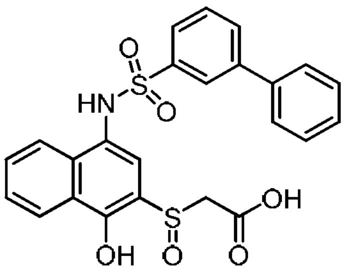 |
| **I-24-1** | 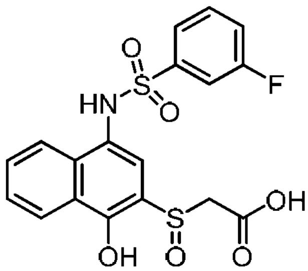 |
| **I-25-1** | 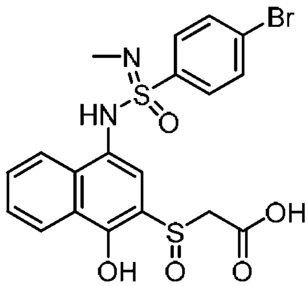 |
| **I-26** | 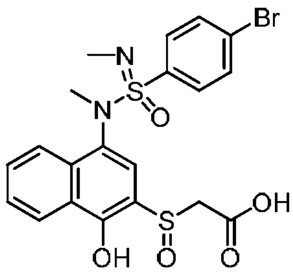 |

(continued)

| | |
|---|---|
| **I-27** | 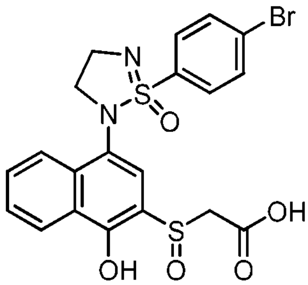 |
| **I-28** | 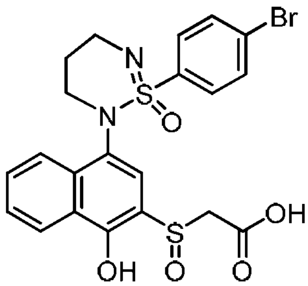 |
| **I-29** | 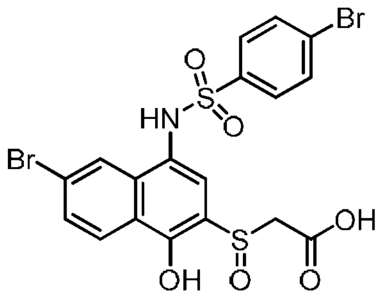 |
| **I-30** | 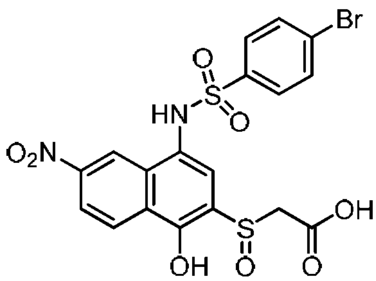 |
| **I-31** | 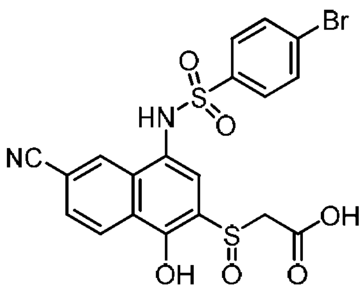 |

(continued)

| | |
|---|---|
| **I-32** | 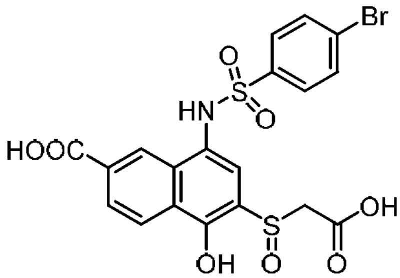 |
| **I-33** | 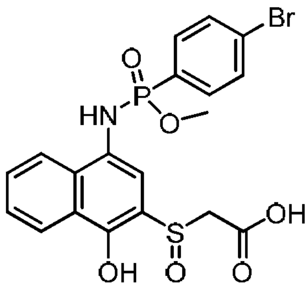 |
| **I-34** | 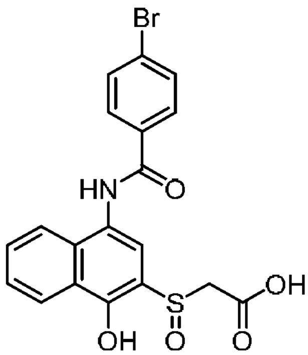 |
| **I-35-1** | 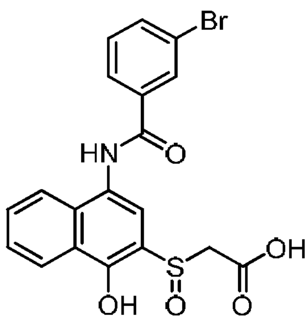 |
| **I-36** | 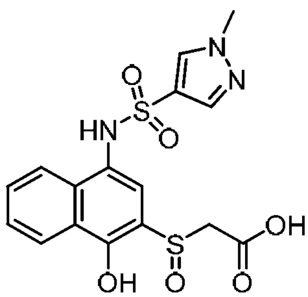 |

(continued)

| | |
|---|---|
| **I-37** | 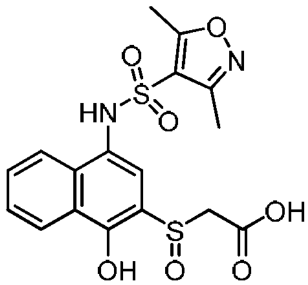 |
| **I-38** | 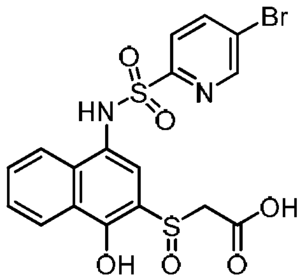 |
| **I-39** | 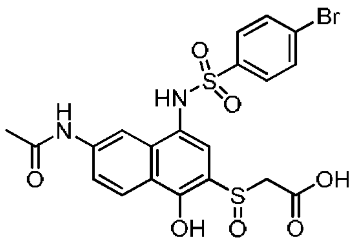 |
| **I-40-1** | 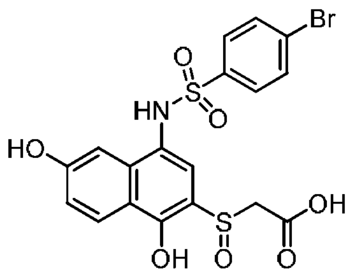 |
| **I-41-1** | 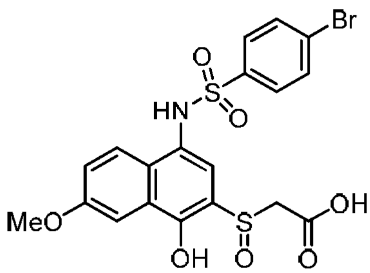 |
| **I-42** | 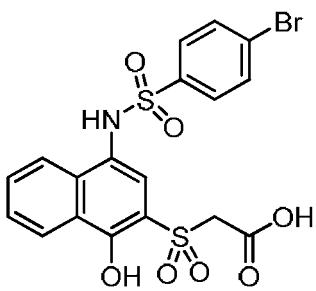 |

(continued)

| | |
|---|---|
| **I-7-2** | 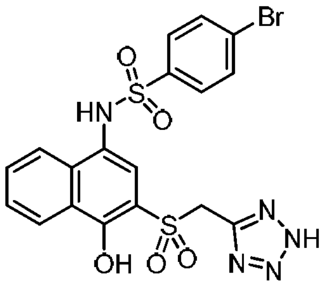 |
| **I-13-2** | 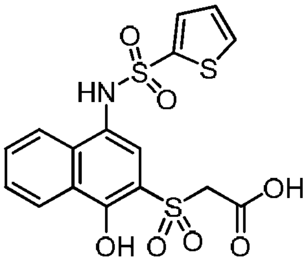 |
| **I-14-2** | 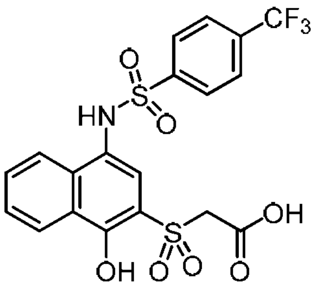 |
| **1-15-2** | 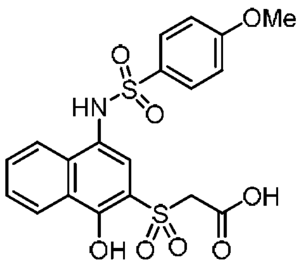 |
| **I-16-2** | 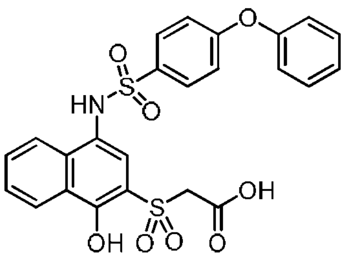 |
| **I-17-2** | 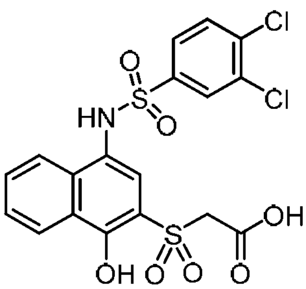 |

(continued)

| | |
|---|---|
| **I-18-2** | 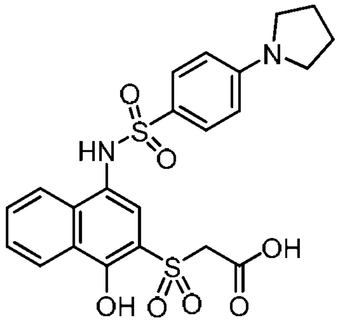 |
| **I-19-2** | 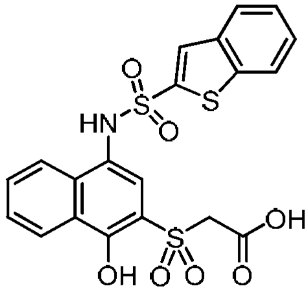 |
| **I-20-2** | 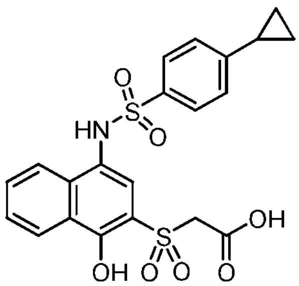 |
| **I-21-2** | 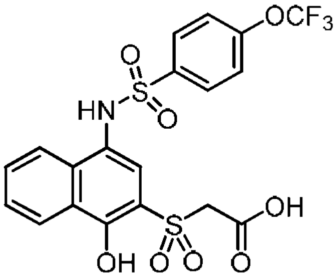 |
| **I-22-2** | 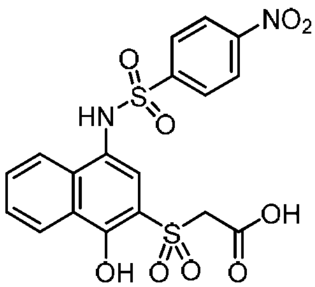 |

(continued)

| | |
|---|---|
| **I-23-2** | 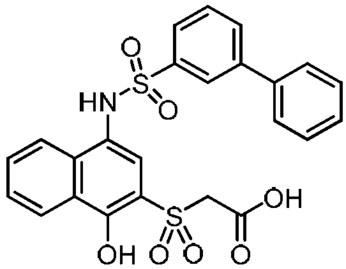 |
| **I-24-2** | 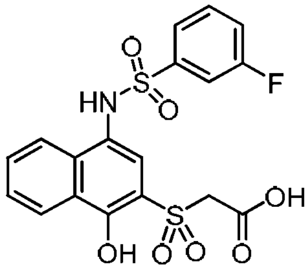 |
| **I-25-2** | 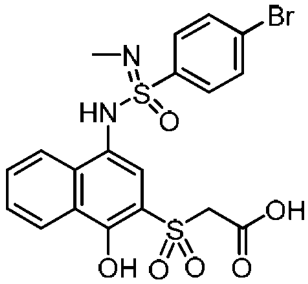 |
| **I-35-2** | 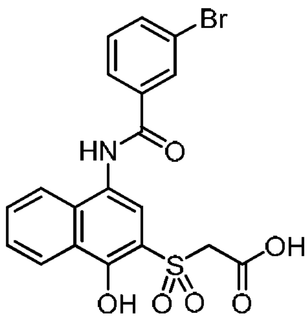 |
| **I-40-2** | 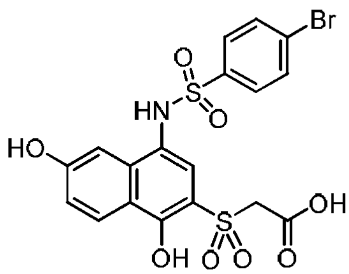 |

(continued)

| | |
|---|---|
| **I-41-2** | 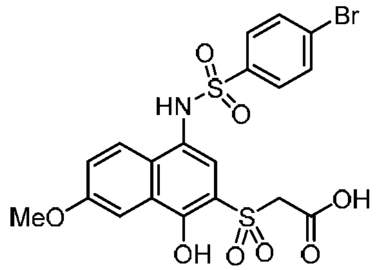 |
| **I-43** | 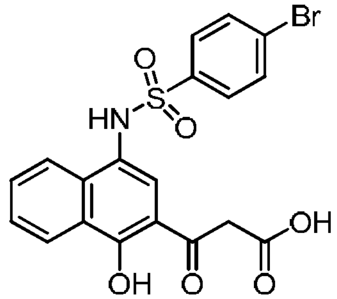 |
| **I-44** | 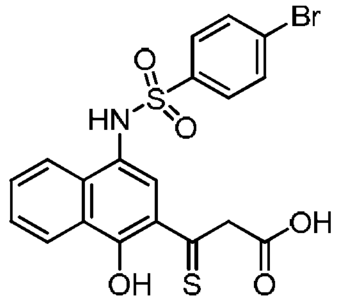 |
| **I-45** | 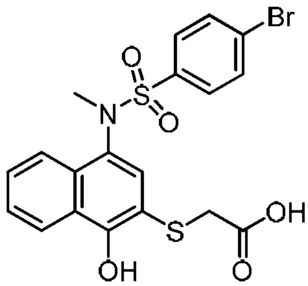 |
| **I-46** | 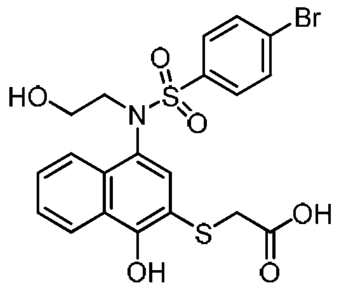 |

(continued)

| | |
|---|---|
| **I-47** | 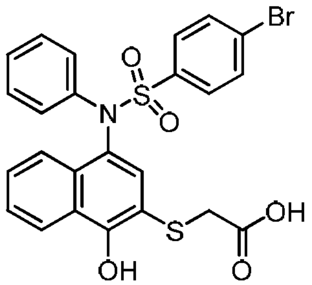 |
| **I-48** | 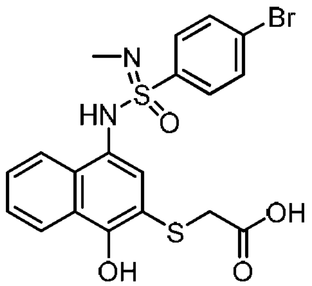 |
| **I-49** | 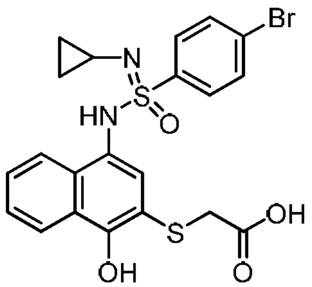 |
| **I-50** | 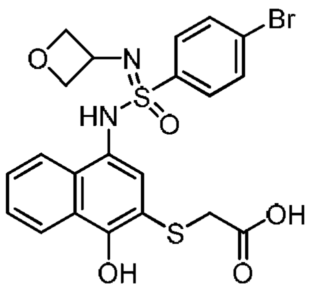 |
| **I-51** | 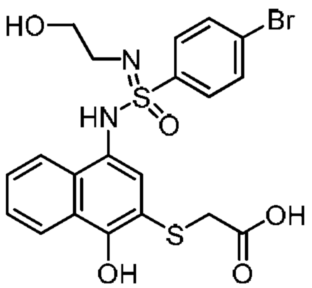 |

(continued)

| | |
|---|---|
| **I-52** | 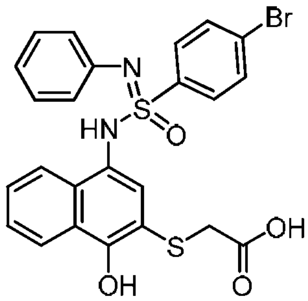 |
| **I-53** | 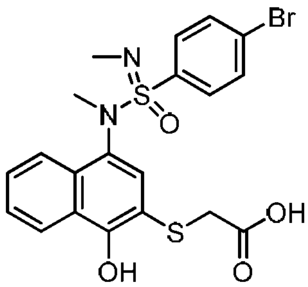 |
| **I-54** | 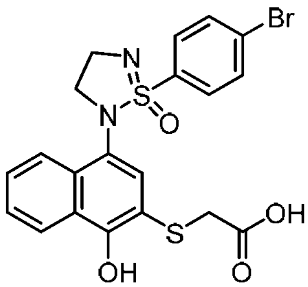 |
| **I-55** | 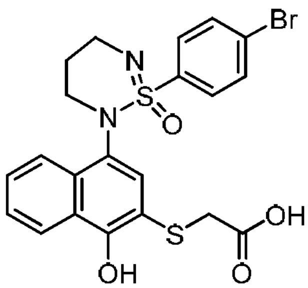 |
| **I-56** | 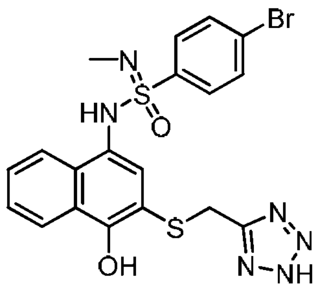 |

(continued)

| | |
|---|---|
| **I-57** | 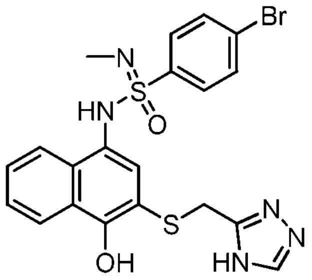 |
| **I-58** | 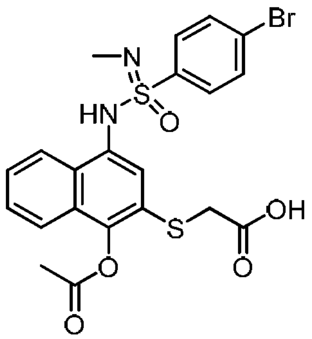 |
| **I-59** | 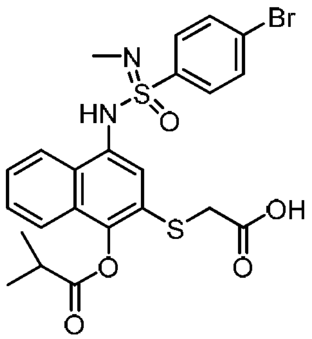 |
| **I-60** | 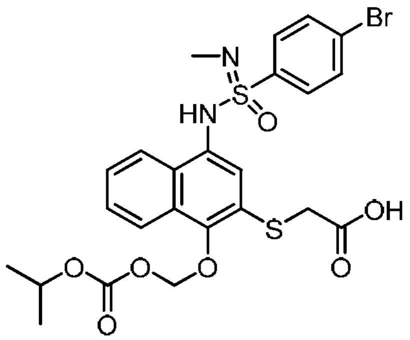 |
| **I-61** | 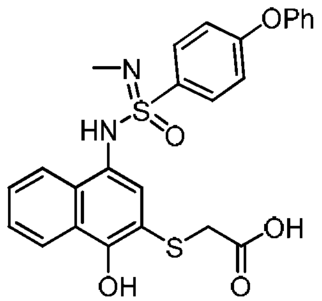 |

(continued)

| | |
|---|---|
| **I-62** | 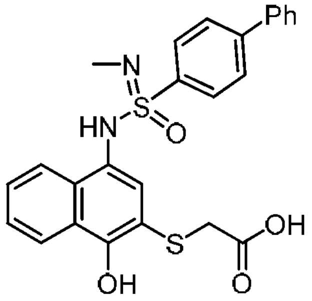 |
| **I-63** | 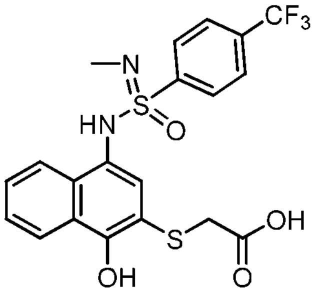 |
| **I-64** | 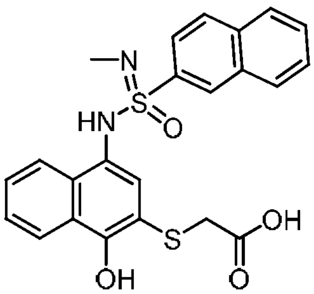 |
| **I-65** | 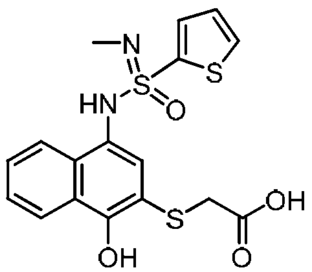 |
| **I-66** | 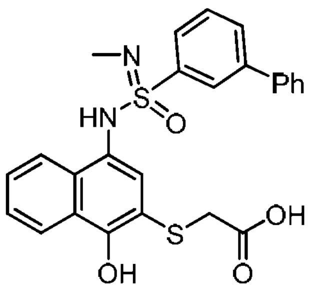 |

(continued)

| | |
|---|---|
| **I-67** | 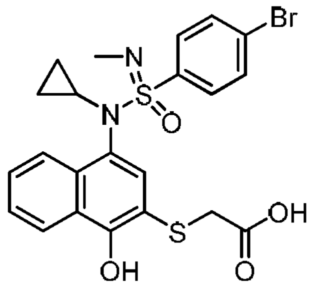 |
| **I-68** | 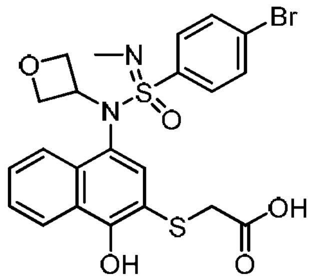 |
| **I-69** | 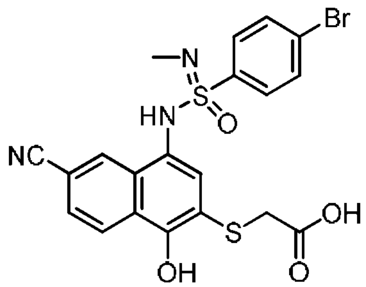 |
| **I-70** | 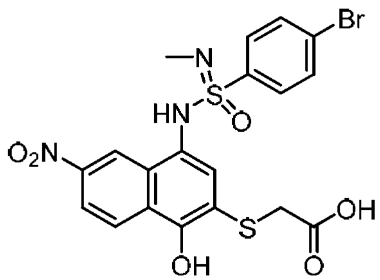 |

In some preferred embodiments, Z is not a sulfur atom.

In some preferred embodiments, in the compound of Formula I, Z is

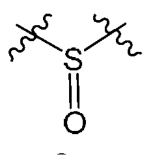

;

$R^1$ is hydrogen; $R^2$ is hydrogen; $R^3$ is hydrogen

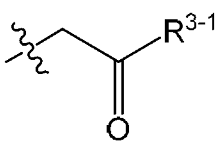

; $R^4$ is

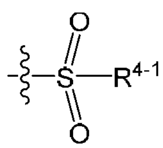

;

wherein $R^{3-1}$ is hydrogen or hydroxyl; and $R^{4-1}$ is phenyl, or phenyl with at least one hydrogen atom substituted by $R^{4-11}$, and $R^{4-11}$ is halogen-substituted methyl, halogen-substituted ethyl, methyl, ethyl or halogen.

In some preferred embodiments, in the compound of Formula I, Z is

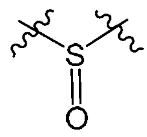
;

$R^1$ is hydrogen; $R^2$ is hydrogen; $R^3$ is hydrogen

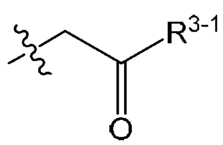
; $R^4$ is
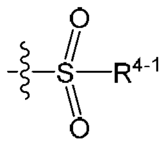
;

wherein $R^{3-1}$ is hydroxyl; and $R^{4-1}$ is phenyl, phenyl with at least one hydrogen atom substituted by trifluoromethyl, or phenyl with at least one hydrogen atom substituted by halogen.

In some preferred embodiments, the compound of Formula I is

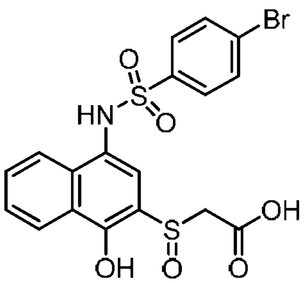
or
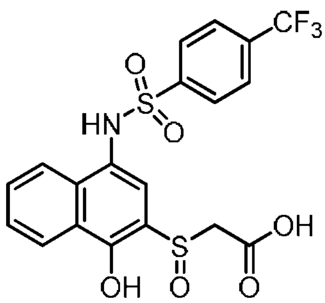
.

In some particularly preferred embodiments, the compound of Formula I is

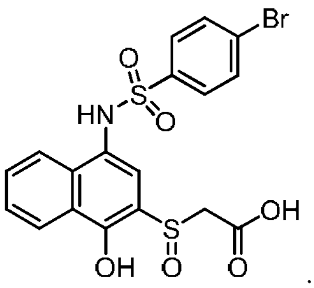
.

In some preferred embodiments, the liver injury is acute alcoholic liver injury, chronic alcoholic liver injury, drug-induced liver injury, non-alcoholic steatohepatitis, or liver injury caused by cholecystitis.

In some preferred embodiments, the reproductive system diseases comprise premature ovarian failure, ovarian insufficiency, polycystic ovary syndrome, oligospermia, and erectile dysfunction.

In some preferred embodiments, the autoimmune diseases comprise systemic lupus erythematosus nephritis, rheumatoid arthritis, multiple sclerosis, and Hashimoto's thyroiditis.

In some preferred embodiments, the skin inflammation comprises psoriasis, melasma, vitiligo, and chronic eczema.

In some preferred embodiments, the retinal inflammation comprises retinitis pigmentosa.

In some preferred embodiments, the eye diseases comprise cataract and age-related macular degeneration.

In some preferred embodiments, the muscular or skeletal degenerative diseases comprise sarcopenia and lumbar disc herniation.

In some preferred embodiments, the neurological diseases comprise epilepsy and cerebral stroke.

In some preferred embodiments, the anemia comprises β-thalassemia.

In some preferred embodiments, the adrenal diseases comprise primary aldosteronism and Cushing's syndrome.

In some preferred embodiments, the chronic gastritis comprises chronic atrophic gastritis.

In some preferred embodiments, the liver injury is acute alcoholic liver injury, chronic alcoholic liver injury, drug-induced liver injury, non-alcoholic steatohepatitis, or liver injury caused by cholecystitis.

In some preferred embodiments, the use of the compound of Formula (I) or the pharmacologically acceptable salt, stereoisomer, solvate or prodrug thereof comprises:

(a1) preparation of a medicament for preventing and/or treating premature ovarian failure; and/or
(a2) prevention and/or treatment of premature ovarian failure; and/or
(bl) preparation of a medicament for preventing and/or treating ovarian insufficiency; and/or
(b2) prevention and/or treatment of ovarian insufficiency; and/or
(c1) preparation of a medicament for preventing and/or treating systemic lupus erythematosus nephritis; and/or
(c2) prevention and/or treatment of systemic lupus erythematosus nephritis; and/or
(d1) preparation of a medicament for preventing and/or treating sarcopenia; and/or
(d2) prevention and/or treatment of sarcopenia; and/or
(e1) preparation of a medicament for preventing and/or treating liver injury; and/or,
(e2) prevention and/or treatment of liver injury; and/or
(f1) preparation of a medicament for preventing and/or treating oligospermia; and/or
(f2) prevention and/or treatment of oligospermia; and/or
(g1) preparation of a medicament for preventing and/or treating rheumatoid arthritis; and/or
(g2) prevention and/or treatment of rheumatoid arthritis; and/or
(h1) preparation of a medicament for preventing and/or treating cataract; and/or
(h2) prevention and/or treatment of cataract; and/or
(i1) preparation of a medicament for preventing and/or treating Cushing's syndrome; and/or
(i2) prevention and/or treatment of Cushing's syndrome; and/or
(j1) preparation of a medicament for preventing and/or treating Hashimoto's thyroiditis; and/or
(j2) prevention and/or treatment of Hashimoto's thyroiditis; and/or
(k1) preparation of a medicament for preventing and/or treating chronic obstructive pulmonary disease; and/or
(k2) prevention and/or treatment of chronic obstructive pulmonary disease; and/or
(11) preparation of a medicament for preventing and/or treating tinnitus; and/or
(l2) prevention and/or treatment of tinnitus; and/or
(m1) preparation of a medicament for preventing and/or treating psoriasis; and/or
(m2) prevention and/or treatment of psoriasis; and/or
(n1) preparation of a medicament for preventing and/or treating chronic eczema; and/or
(n2) prevention and/or treatment of chronic eczema; and/or
(o1) preparation of a medicament for preventing and/or treating age-related macular degeneration; and/or
(o2) prevention and/or treatment of age-related macular degeneration;
(pl) preparation of a medicament for preventing and/or treating retinitis pigmentosa; and/or
(p2) prevention and/or treatment of retinitis pigmentosa;
(q1) preparation of a medicament for preventing and/or treating multiple sclerosis; and/or
(q2) prevention and/or treatment of multiple sclerosis;
(rl) preparation of a medicament for preventing and/or treating hypothyroidism; and/or
(r2) prevention and/or treatment of hypothyroidism;
(s1) preparation of a medicament for preventing and/or treating chronic atrophic gastritis; and/or
(s2) prevention and/or treatment of chronic atrophic gastritis;
(tl) preparation of a medicament for preventing and/or treating myocarditis; and/or
(t2) prevention and/or treatment of myocarditis;
(u1) preparation of a medicament for preventing and/or treating alopecia; and/or
(u2) prevention and/or treatment of alopecia;
(v1) preparation of a medicament for preventing and/or treating primary aldosteronism; and/or
(v2) prevention and/or treatment of primary aldosteronism;
(w1) preparation of a medicament for preventing and/or treating erectile dysfunction; and/or
(w2) prevention and/or treatment of erectile dysfunction;
(x1) preparation of a medicament for preventing and/or treating periodontitis; and/or
(x2) prevention and/or treatment of periodontitis;
(y1) preparation of a medicament for preventing and/or treating cerebral stroke; and/or
(y2) prevention and/or treatment of cerebral stroke;

(z1) preparation of a medicament for preventing and/or treating epilepsy; and/or
(z2) prevention and/or treatment of epilepsy;
(Aa1) preparation of a medicament for preventing and/or treating melasma; and/or
(Aa2) prevention and/or treatment of melasma; and/or
(Ab1) preparation of a medicament for preventing and/or treating vitiligo; and/or
(Ab2) prevention and/or treatment of vitiligo; and/or
(Ac1) preparation of a medicament for preventing and/or treating β-thalassemia; and/or
(Ac2) prevention and/or treatment of β-thalassemia; and/or
(Ad1) preparation of a medicament for preventing and/or treating lumbar disc herniation; and/or
(Ad2) prevention and/or treatment of lumbar disc herniation; and/or
(Ae1) preparation of a medicament for preventing and/or treating polycystic ovary syndrome; and/or,
(Ae2) prevention and/or treatment of polycystic ovary syndrome.

In a second aspect of the present invention, there is provided a method for preventing and/or treating premature ovarian failure, wherein the method comprises the step of: administering to a subject a therapeutically effective amount of a compound of Formula (I) or a pharmaceutically acceptable salt, stereoisomer, solvate or prodrug thereof.
In a third aspect of the present invention, there is provided a method for preventing and/or treating ovarian insufficiency, wherein the method comprises the step of: administering to a subject a therapeutically effective amount of a compound of Formula (I) or a pharmaceutically acceptable salt, stereoisomer, solvate or prodrug thereof.
In a fourth aspect of the present invention, there is provided a method for preventing and/or treating systemic lupus erythematosus nephritis, wherein the method comprises the step of: administering to a subject a therapeutically effective amount of a compound of Formula (I) or a pharmaceutically acceptable salt, stereoisomer, solvate or prodrug thereof.
In a fifth aspect of the present invention, there is provided a method for preventing and/or treating sarcopenia, wherein the method comprises the step of: administering to a subject a therapeutically effective amount of a compound of Formula (I) or a pharmaceutically acceptable salt, stereoisomer, solvate or prodrug thereof.
In a sixth aspect of the present invention, there is provided a method for preventing and/or treating liver injury, wherein the method comprises the step of: administering to a subject a therapeutically effective amount of a compound of Formula (I) or a pharmaceutically acceptable salt, stereoisomer, solvate or prodrug thereof.
In a seventh aspect of the present invention, there is provided a method for preventing and/or treating oligospermia, wherein the method comprises the step of: administering to a subject a therapeutically effective amount of a compound of Formula (I) or a pharmaceutically acceptable salt, stereoisomer, solvate or prodrug thereof.
In an eighth aspect of the present invention, there is provided a method for preventing and/or treating rheumatoid arthritis, wherein the method comprises the step of: administering to a subject a therapeutically effective amount of a compound of Formula (I) or a pharmaceutically acceptable salt, stereoisomer, solvate or prodrug thereof.
In a ninth aspect of the present invention, there is provided a method for preventing and/or treating cataract, wherein the method comprises the step of: administering to a subject a therapeutically effective amount of a compound of Formula (I) or a pharmaceutically acceptable salt, stereoisomer, solvate or prodrug thereof.
In a tenth aspect of the present invention, there is provided a method for preventing and/or treating Cushing's syndrome, wherein the method comprises the step of: administering to a subject a therapeutically effective amount of a compound of Formula (I) or a pharmaceutically acceptable salt, stereoisomer, solvate or prodrug thereof.
In an eleventh aspect of the present invention, there is provided a method for preventing and/or treating Hashimoto's thyroiditis, wherein the method comprises the step of: administering to a subject a therapeutically effective amount of a compound of Formula (I) or a pharmaceutically acceptable salt, stereoisomer, solvate or prodrug thereof.
In a twelfth second aspect of the present invention, there is provided a method for preventing and/or treating chronic obstructive pulmonary disease, wherein the method comprises the step of: administering to a subject a therapeutically effective amount of a compound of Formula (I) or a pharmaceutically acceptable salt, stereoisomer, solvate or prodrug thereof.
In a thirteenth aspect of the present invention, there is provided a method for preventing and/or treating tinnitus, wherein the method comprises the step of: administering to a subject a therapeutically effective amount of a compound of Formula (I) or a pharmaceutically acceptable salt, stereoisomer, solvate or prodrug thereof.
In a fourteenth second aspect of the present invention, there is provided a method for preventing and/or treating psoriasis, wherein the method comprises the step of: administering to a subject a therapeutically effective amount of a compound of Formula (1) or a pharmaceutically acceptable salt, stereoisomer, solvate or prodrug thereof.
In a fifteenth aspect of the present invention, there is provided a method for preventing and/or treating chronic eczema, wherein the method comprises the step of: administering to a subject a therapeutically effective amount of a compound of Formula (I) or a pharmaceutically acceptable salt, stereoisomer, solvate or prodrug thereof.
In a sixteenth aspect of the present invention, there is provided a method for preventing and/or treating age-related macular degeneration, wherein the method comprises the step of: administering to a subject a therapeutically effective amount of a compound of Formula (I) or a pharmaceutically acceptable salt, stereoisomer, solvate or prodrug thereof.

In a seventeenth aspect of the present invention, there is provided a method for preventing and/or treating multiple sclerosis, wherein the method comprises the step of: administering to a subject a therapeutically effective amount of a compound of Formula (I) or a pharmaceutically acceptable salt, stereoisomer, solvate or prodrug thereof.

In an eighteenth aspect of the present invention, there is provided a method for preventing and/or treating hypothyroidism, wherein the method comprises the step of: administering to a subject a therapeutically effective amount of a compound of Formula (I) or a pharmaceutically acceptable salt, stereoisomer, solvate or prodrug thereof.

In a nineteenth aspect of the present invention, there is provided a method for preventing and/or treating chronic atrophic gastritis, wherein the method comprises the step of: administering to a subject a therapeutically effective amount of a compound of Formula (I) or a pharmaceutically acceptable salt, stereoisomer, solvate or prodrug thereof.

In a twentieth aspect of the present invention, there is provided a method for preventing and/or treating myocarditis, wherein the method comprises the step of: administering to a subject a therapeutically effective amount of a compound of Formula (I) or a pharmaceutically acceptable salt, stereoisomer, solvate or prodrug thereof.

In a twenty-first aspect of the present invention, there is provided a method for preventing and/or treating alopecia, wherein the method comprises the step of: administering to a subject a therapeutically effective amount of a compound of Formula (I) or a pharmaceutically acceptable salt, stereoisomer, solvate or prodrug thereof.

In a twenty-second aspect of the present invention, there is provided a method for preventing and/or treating primary aldosteronism, wherein the method comprises the step of: administering to a subject a therapeutically effective amount of a compound of Formula (I) or a pharmaceutically acceptable salt, stereoisomer, solvate or prodrug thereof.

In a twenty-third aspect of the present invention, there is provided a method for preventing and/or treating erectile dysfunction, wherein the method comprises the step of: administering to a subject a therapeutically effective amount of a compound of Formula (I) or a pharmaceutically acceptable salt, stereoisomer, solvate or prodrug thereof.

In a twenty-fourth aspect of the present invention, there is provided a method for preventing and/or treating periodontitis, wherein the method comprises the step of: administering to a subject a therapeutically effective amount of a compound of Formula (I) or a pharmaceutically acceptable salt, stereoisomer, solvate or prodrug thereof.

In a twenty-fifth aspect of the present invention, there is provided a method for preventing and/or treating cerebral stroke, wherein the method comprises the step of: administering to a subject a therapeutically effective amount of a compound of Formula (I) or a pharmaceutically acceptable salt, stereoisomer, solvate or prodrug thereof.

In a twenty-sixth aspect of the present invention, there is provided a method for preventing and/or treating epilepsy, wherein the method comprises the step of: administering to a subject a therapeutically effective amount of a compound of Formula (I) or a pharmaceutically acceptable salt, stereoisomer, solvate or prodrug thereof.

In a twenty-seventh aspect of the present invention, there is provided a method for preventing and/or treating melasma, wherein the method comprises the step of: administering to a subject a therapeutically effective amount of a compound of Formula (I) or a pharmaceutically acceptable salt, stereoisomer, solvate or prodrug thereof.

In a twenty-eighth aspect of the present invention, there is provided a method for preventing and/or treating vitiligo, wherein the method comprises the step of: administering to a subject a therapeutically effective amount of a compound of Formula (I) or a pharmaceutically acceptable salt, stereoisomer, solvate or prodrug thereof.

In a twenty-ninth aspect of the present invention, there is provided a method for preventing and/or treating β-thalassemia, wherein the method comprises the step of: administering to a subject a therapeutically effective amount of a compound of Formula (I) or a pharmaceutically acceptable salt, stereoisomer, solvate or prodrug thereof.

In a thirtieth aspect of the present invention, there is provided a method for preventing and/or treating lumbar disc herniation, wherein the method comprises the step of: administering to a subject a therapeutically effective amount of a compound of Formula (I) or a pharmaceutically acceptable salt, stereoisomer, solvate or prodrug thereof.

In a thirty-first aspect of the present invention, there is provided a method for preventing and/or treating polycystic ovary syndrome, wherein the method comprises the step of: administering to a subject a therapeutically effective amount of a compound of Formula (I) or a pharmaceutically acceptable salt, stereoisomer, solvate or prodrug thereof.

In a thirty-second aspect of the present invention, there is provided a method for preventing and/or treating retinitis pigmentosa, wherein the method comprises the step of: administering to a subject a therapeutically effective amount of a compound of Formula (I) or a pharmaceutically acceptable salt, stereoisomer, solvate or prodrug thereof.

Compared with the prior art, the present invention has at least the following advantages:

(1) the compounds of Formula (I) provided by the present invention, when used for treating premature ovarian failure, can increase ovarian weight and ovarian coefficient, elevate the levels of E2, FSH, and BDNF in the serum of patients, and improve ovarian function, and among the compounds encompassed by Formula (I), Compound I-1 (Tj01-013) demonstrates a therapeutic effect significantly superior to those of other compounds;

(2) the compounds of Formula (I) provided by the present invention, when used for treating ovarian insufficiency, can

increase ovarian weight and ovarian index, elevate the levels of E2 and FSH in the serum of patients, and improve ovarian function, and among the compounds encompassed by Formula (I), Compound I-1 (TJ01-013) demonstrates a therapeutic effect significantly superior to those of other compounds;

(3) the compounds of Formula (I) provided by the present invention, when used for treating systemic lupus erythematosus nephritis, can alleviate proteinuria, reduce the levels of creatinine and blood urea nitrogen, the level of inflammatory factors in kidney tissues and the level of specific antibody associated with systemic lupus erythematosus, and improve the renal function, and among the compounds encompassed by Formula (I), Compound I-1 (TJ01-013) demonstrates a therapeutic effect significantly superior to those of other compounds;

(4) the compounds of Formula (I) provided by the present invention, when used for treating sarcopenia, can increase myotility and gastrocnemius muscle weight, and alleviate the symptoms of sarcopenia, and among the compounds encompassed by Formula (I), Compound I-1 (TJ01-013) demonstrates a therapeutic effect significantly superior to those of other compounds;

(5) the compounds of Formula (I) provided by the present invention, when used for treating liver injury (e.g., acute alcoholic liver injury, chronic alcoholic liver injury, drug-induced liver injury, non-alcoholic steatohepatitis, or liver injury caused by cholecystitis), can downregulate the levels of liver function indicators such as AST, ALT, TG, and TC, and improve liver function, and among the compounds encompassed by Formula (I), Compound I-1 (TJ01-013) demonstrates a therapeutic effect significantly superior to those of other compounds;

(6) the compound of Formula (I) provided by the present invention, when used for treating oligospermia, can increase the level of sex hormones in the body, the testicular index and the number of sperms in semen, and ameliorate oligospermia, and among the compounds encompassed by Formula (I), Compound I-1 (TJ01-013) demonstrates a therapeutic effect significantly superior to those of other compounds;

(7) the compounds of Formula (I) provided by the present invention, when used for treating rheumatoid arthritis, can reduce joint swelling and hind plantar thickness, and ameliorate the symptoms of rheumatoid arthritis, and among the compounds encompassed by Formula (I), Compound I-1 (TJ01-013) demonstrates a therapeutic effect significantly superior to those of other compounds;

(8) the compounds of Formula (I) provided by the present invention, when used for treating cataract, can aleliorate lens opacity and alleviate the symptoms of cataract, and among the compounds encompassed by Formula (I), Compound I-1 (TJ01-013) demonstrates a therapeutic effect significantly superior to those of other compounds;

(9) the compounds of Formula (I) provided by the present invention, when used for treating Cushing's syndrome, can reduce the level of plasma insulin and alleviate the symptoms of Cushing's syndrome, and among the compounds encompassed by Formula (I), Compound I-1 (TJ01-013) demonstrates a therapeutic effect significantly superior to those of other compounds;

(10) the compounds of Formula (I) provided by the present invention, when used for treating Hashimoto's thyroiditis, can reduce the level of the diagnostic marker TPO-Ab for Hashimoto's thyroiditis and ameliorate the symptoms of Hashimoto's thyroiditis, and among the compounds encompassed by Formula (I), Compound I-1 (TJ01-013) demonstrates a therapeutic effect significantly superior to those of other compounds;

(11) the compounds of Formula (I) provided by the present invention, when used for treating chronic obstructive pulmonary disease, can reduce the level of inflammatory factor TNF$\alpha$ in the body, decrease the rise in inflammatory indicators caused by chronic obstructive pulmonary diseases, and alleviate the symptoms of chronic obstructive pulmonary diseases, and among the compounds encompassed by Formula (I), Compound I-1 (TJ01-013) demonstrates a therapeutic effect significantly superior to those of other compounds;

(12) the compounds of Formula (I) provided by the present invention, when used for treating tinnitus, can reduce the amplitude of tinnitus response in patients, and among the compounds encompassed by Formula (I), Compound I-1 (TJ01-013) demonstrates a therapeutic effect significantly superior to those of other compounds and exhibits dose dependency;

(13) the compounds of Formula (I) provided by the present invention, when used for treating psoriasis, can alleviate the symptoms of parakeratosis and epidermal thickening in patients, and among the compounds encompassed by Formula (I), Compound I-1 (TJ01-013) demonstrates a therapeutic effect significantly superior to those of other compounds and exhibits dose dependency;

(14) the compounds of Formula (I) provided by the present invention, when used for treating chronic eczema, can increase the water content of the stratum corneum, reduce water loss, and alleviate the symptoms of eczema, and among the compounds encompassed by Formula (I), Compound I-1 (TJ01-013) demonstrates a therapeutic effect significantly superior to those of other compounds and exhibits dose dependency;

(15) the compounds of Formula (I) provided by the present invention, when used for treating age-related macular degeneration, can improve retinal photosensitivity and restore the normal structure of the retina, and among the compounds encompassed by Formula (I), Compound I-1 (TJ01-013) demonstrates a therapeutic effect significantly superior to those of other compounds;

(16) the compounds of Formula (I) provided by the present invention, when used for treating retinitis pigmentosa to

increase the thickness of the retinal photoreceptor cell layer, can enhance the density of retinal microglia, and ameliorate the symptoms of retinitis pigmentosa, and among the compounds encompassed by Formula (I), Compound I-1 (TJ01-013) demonstrates a therapeutic effect significantly superior to those of other compounds;
(17) the compounds of Formula (I) provided by the present invention, when used for treating multiple sclerosis, can improve the spatial learning and memory abilities of patients, and among the compounds encompassed by Formula (I), Compound I-1 (TJ01-013) demonstrates a therapeutic effect significantly superior to those of other compounds;
(18) the compounds of Formula (I) provided by the present invention, when used for treating hypothyroidism, can increase the levels of T3, T4, FT3, FT4, and FSH, and among the compounds encompassed by Formula (I), Compound I-1 (TJ01-013) demonstrates a therapeutic effect significantly superior to those of other compounds;
(19) the compounds of Formula (I) provided by the present invention, when used for treating chronic atrophic gastritis, can reduce the level of inflammatory factors and increase the level of pepsin, and among the compounds encompassed by Formula (I), Compound I-1 (TJ01-013) demonstrates a therapeutic effect significantly superior to those of other compounds;
(20) the compounds of Formula (I) provided by the present invention, when used for treating myocarditis, can reduce the levels of LVIDd and LVIDs, decrease EF and FS, lower the level of CK-MB, and improve the cardiac function, and among the compounds encompassed by Formula (I), Compound I-1 (TJ01-013) demonstrates a therapeutic effect significantly superior to those of other compounds;
(21) the compounds of Formula (I) provided by the present invention, when used for treating alopecia, can ameliorate the positive infiltration of cells in alopecia areata areas and reduce the level of inflammation, and among the compounds encompassed by Formula (I), Compound I-1 (TJ01-013) demonstrates a therapeutic effect significantly superior to those of other compounds;
(22) the compounds of Formula (I) provided by the present invention, when used for treating primary aldosteronism, can ameliorate the symptoms of sodium retention and potassium excretion and reduce the level of sodium ions while increasing the level of potassium ions, and among the compounds encompassed by Formula (I), Compound I-1 (TJ01-013) demonstrates a therapeutic effect significantly superior to those of other compounds;
(23) the compounds of Formula (I) provided by the present invention, when used for treating erectile dysfunction, can ameliorate the symptoms of penile non-erection or weak erection, enhance the erectile function, and increase the level of sex hormones in the body, and among the compounds encompassed by Formula (I), Compound I-1 (TJ01-013) demonstrates a therapeutic effect significantly superior to those of other compounds;
(24) the compounds of Formula (I) provided by the present invention, when used for treating periodontitis, can improve the condition of alveolar bone and ameliorate the symptoms of periodontal absorption, and Compound I-1 (TJ01-013) demonstrates a therapeutic effect significantly superior to those of other compounds;
(25) the compounds of Formula (I) provided by the present invention, when used for treating cerebral stroke, can reduce the apoptosis rate of hippocampal neurons and improve the cognitive function in patients, and among the compounds encompassed by Formula (I), Compound I-1 (TJ01-013) demonstrates a therapeutic effect significantly superior to those of other compounds;
(26) the compounds of Formula (I) provided by the present invention, when used for treating epilepsy, can alleviate cognitive impairment in patients, ameliorate the frequency of epileptic seizures and disease symptoms, and reduce the level of inflammatory factors, and among the compounds encompassed by Formula (I), Compound I-1 (TJ01-013) demonstrates a therapeutic effect significantly superior to those of other compounds;
(27) the compounds of Formula (I) provided by the present invention, when used for treating melasma, can reduce the area size of melanin-positive cells and the surface density of melanin in the skin tissues of patients, and decrease the number of patchy spots on the face, and among the compounds encompassed by Formula (I), Compound I-1 (TJ01-013) demonstrates a therapeutic effect significantly superior to those of other compounds;
(28) the compounds of Formula (I) provided by the present invention, when used for treating vitiligo, can reduce the number of melanin-containing hair follicles in patients and lower the levels of TYR and MDA in the serum of patients, and among the compounds encompassed by Formula (I), Compound I-1 (TJ01-013) demonstrates a therapeutic effect significantly superior to those of other compounds;
(29) the compounds of Formula (I) provided by the present invention, when used for treating β-thalassemia, can restore the number of red blood cells and hemoglobin in patients and ameliorate the symptoms of anemia, and among the compounds encompassed by Formula (I), Compound I-1 (TJ01-013) demonstrates a therapeutic effect significantly superior to those of other compounds;
(30) the compounds of Formula (I) provided by the present invention, when used for treating lumbar disc herniation, can reduce the activity of PLA2 and the level of inflammatory factors in the nucleus pulposus tissues of patients and improve the lumbar disc function, and among the compounds encompassed by Formula (I), Compound I-1 (TJ01-013) demonstrates a therapeutic effect significantly superior to those of other compounds; and
(31) the compounds of Formula (I) provided by the present invention, when used for treating polycystic ovary syndrome, can reduce the levels of testosterone T, luteinizing hormone LH, and follicle-stimulating hormone FSH in

the serum of patients, increase ovarian weight, and ameliorate the ovulation disorder, and among the compounds encompassed by Formula (I), Compound I-1 (TJ01-013) demonstrates a therapeutic effect significantly superior to those of other compounds.

It should be understood that the above-mentioned technical features of the present invention and the technical features specifically described hereinafter (e.g., in Examples) can be combined with each other within the scope of the present invention to thus form a new or preferred technical solution, Due to space limitation, the details will not be repeated one by one here.

## BRIEF DESCRIPTION OF THE DRAWINGS

One or more embodiments are exemplarily illustrated by means of the accompanying drawings corresponding thereto, and these exemplary illustrations do not limit the embodiments.

FIG. 1 shows graphs for quantitative analysis of body weight, ovarian weight and ovarian index in mice administered with Compound TJ0113 in a chemotherapy-induced premature ovarian failure model according to an embodiment of the present invention;
FIG. 2 shows a graph for quantitative analysis of serum FSH in mice administered with Compound TJ0113 in the chemotherapy-induced premature ovarian failure model according to an embodiment of the present invention;
FIG. 3 shows a graph for quantitative analysis of serum E2 in mice administered with Compound TJ0113 in the chemotherapy-induced premature ovarian failure model according to an embodiment of the present invention;
FIG. 4 shows a graph for quantitative analysis of BDNF in ovarian tissues in mice administered with Compound TJ0113 in the chemotherapy-induced premature ovarian failure model according to an embodiment of the present invention;
FIG. 5 shows graphs for quantitative analysis of body weight, ovarian weight and ovarian index in mice administered with Compound TJ0113 in a naturally aged ovarian insufficiency model according to an embodiment of the present invention;
FIG. 6 shows a graph for quantitative analysis of FSH in mice administered with Compound TJ0113 in the naturally aged ovarian insufficiency model according to an embodiment of the present invention;
FIG. 7 shows a graph for quantitative analysis of E2 in mice administered with Compound TJ0113 in the naturally aged ovarian insufficiency model according to an embodiment of the present invention;
FIG. 8 shows a graph for quantitative analysis of random urine uPCR in mice administered with Compound TJ0113 in a lupus nephritis model according to an embodiment of the present invention;
FIG. 9 shows graphs for quantitative analysis of serum creatinine and blood urea nitrogen in mice administered with Compound TJ0113 in the lupus nephritis model according to an embodiment of the present invention;
FIG. 10 shows a graph for quantitative analysis of serum ANA in mice administered with Compound TJ0113 in the lupus nephritis model according to an embodiment of the present invention;
FIG. 11 shows a graph for quantitative analysis of serum dsDNA in mice administered with Compound TJ0113 in the lupus nephritis model according to an embodiment of the present invention;
FIG. 12 shows graphs for quantitative analysis of TNF$\alpha$ and IL-6 in kidney tissues in mice administered with Compound TJ0113 in the lupus nephritis model according to an embodiment of the present invention;
FIG. 13 shows a graph for quantitative analysis of swimming time of sarcopenia mice administered with Compound TJ0113 according to an embodiment of the present invention;
FIG. 14 shows a graph for quantitative analysis of hindlimb grip strength of sarcopenia mice administered with Compound TJ0113 according to an embodiment of the present invention;
FIG. 15 shows graphs for quantitative analysis of serum AST and ALT after administration of Compound TJ0113 against acute alcoholic liver injury according to an embodiment of the present invention;
FIG. 16 shows graphs for quantitative analysis of TG and TC in liver tissues after administration of Compound TJ0113 against acute alcoholic liver injury according to an embodiment of the present invention;
FIG. 17 shows graphs for quantitative analysis of serum AST and ALT after administration of Compound TJ0113 against chronic alcoholic liver injury according to an embodiment of the present invention;
FIG. 18 shows images of HE-stained liver tissues after administration of Compound TJ0113 against chronic alcoholic liver injury according to an embodiment of the present invention;
FIG. 19 shows images of oil red-stained liver tissues after administration of Compound TJ0113 against chronic alcoholic liver injury according to an embodiment of the present invention;
FIG. 20 shows graphs for quantitative analysis of serum AST and ALT after administration of Compound TJ0113 against drug-induced liver injury model according to an embodiment of the present invention;
FIG. 21 shows a graph for quantitative analysis of serum ALP after administration of Compound TJ0113 against drug-induced liver injury according to an embodiment of the present invention;

FIG. 22 shows graphs for quantitative analysis of serum FSH, LH, and T in an oligospermia model administered with Compound TJ0113 according to an embodiment of the present invention;

FIG. 23 shows graphs for quantitative analysis of wet testis weight and testicular index in the oligospermia model administered with Compound TJ0113 according to an embodiment of the present invention;

FIG. 24 shows a graph for quantitative analysis of sperm density in the oligospermia model administered with Compound TJ0113 according to an embodiment of the present invention;

FIG. 25 shows a graph for quantitative analysis of AI scores in a rheumatoid arthritis model administered with Compound TJ0113 according to an embodiment of the present invention;

FIG. 26 shows a graph for quantitative analysis of swollen foot joint counts in the rheumatoid arthritis model administered with Compound TJ0113 according to an embodiment of the present invention;

FIG. 27 shows a graph for quantitative analysis of hind plantar thickness in the rheumatoid arthritis model administered with Compound TJ0113 according to an embodiment of the present invention;

FIG. 28 shows graphs for quantitative analysis of thymus weight/body weight ratio and spleen index in the rheumatoid arthritis model administered with Compound TJ0113 according to an embodiment of the present invention;

FIG. 29 shows a graph for quantitative analysis of fundus scores in a cataract model administered with Compound TJ0113 according to an embodiment of the present invention;

FIG. 30 shows graphs for quantitative analysis of fundus scores after administration of Compound TJ0113 against Cushing's syndrome according to an embodiment of the present invention;

FIG. 31 shows graphs for quantitative analysis of serum THS and FT3 after administration of Compound TJ0113 against Hashimoto's thyroiditis according to an embodiment of the present invention;

FIG. 32 shows graphs for quantitative analysis of serum TGAb and TPOAb after administration of Compound TJ0113 against Hashimoto's thyroiditis according to an embodiment of the present invention;

FIG. 33 shows graphs for quantitative analysis of serum TNF$\alpha$ and CXCL1 after administration of Compound TJ0113 against chronic obstructive pulmonary disease according to an embodiment of the present invention;

FIG. 34 shows graphs for quantitative analysis of tinnitus indicators after administration of Compound TJ0113 against tinnitus according to an embodiment of the present invention;

FIG. 35 shows a graph for quantitative analysis of skin thickness after administration of Compound TJ0113 against psoriasis according to an embodiment of the present invention;

FIG. 36 shows a graph for quantitative analysis of transepidermal water loss (TWL) after administration of Compound TJ0113 against chronic eczema according to an embodiment of the present invention;

FIG. 37 shows a graph for quantitative analysis of stratum corneum hydration (SCH) after administration of Compound TJ0113 against chronic eczema according to an embodiment of the present invention;

FIG. 38 shows a graph for analysis of steatosis scores after administration of Compound TJ0113 against non-alcoholic steatohepatitis according to an embodiment of the present invention;

FIG. 39 shows a graph for analysis of inflammation level scores after administration of Compound TJ0113 against non-alcoholic steatohepatitis according to an embodiment of the present invention;

FIG. 40 shows a graph for analysis of fibrosis level scores after administration of Compound TJ0113 against non-alcoholic steatohepatitis according to an embodiment of the present invention;

FIG. 41 shows graphs for analysis of liver biochemical indicators ALT, AST, and TBIL levels after administration of Compound TJ0113 against cholecystitis according to an embodiment of the present invention;

FIG. 42 shows a graph for quantitative analysis of the thickness of a photoreceptor cell layer in mice administered with Compound TJ01-013 in a retinitis pigmentosa model according to an embodiment of the present invention;

FIG. 43 shows a graph for quantitative analysis of retinal microglia density in mice administered with Compound TJ01-013 in the retinitis pigmentosa model according to an embodiment of the present invention;

FIG. 44 shows a graph for quantitative analysis of the number of entries into an elevated plus maze by mice administered with Compound TJ01-013 in a multiple sclerosis model according to an embodiment of the present invention;

FIG. 45 shows a graph for quantitative analysis of the distance traveled on an elevated platform by mice administered with Compound TJ01-013 in the multiple sclerosis model according to an embodiment of the present invention;

FIG. 46 shows a graph for quantitative analysis of the time spent in the elevated plus maze by mice administered with Compound TJ01-013 in the multiple sclerosis model according to an embodiment of the present invention;

FIG. 47 shows a graph for quantitative analysis of the number of crossings over a platform by mice administered with Compound TJ01-013 in the multiple sclerosis model according to an embodiment of the present invention;

FIG. 48 shows graphs of quantitative analysis of serum T3 and T4 in mice administered with Compound TJ01-013 in a hypothyroidism model according to an embodiment of the present invention;

FIG. 49 shows graphs of quantitative analysis of serum FT3 and FT4 in mice administered with Compound TJ01-013 in the hypothyroidism model according to an embodiment of the present invention;

FIG. 50 shows a graph for quantitative analysis of serum FSH in mice administered with Compound TJ01-013 in the hypothyroidism model according to an embodiment of the present invention;

FIG. 51 shows a graph for quantitative analysis of serum IgG in mice administered with Compound TJ01-013 in the hypothyroidism model according to an embodiment of the present invention;

FIG. 52 shows graphs for quantitative analysis of serum IL-6, IL-1β and TNF-α in mice administered with Compound TJ01-013 in a chronic atrophic gastritis model according to an embodiment of the present invention;

FIG. 53 shows graphs for quantitative analysis of serum PGI, PGII and GAS in mice administered with Compound TJ01-013 in the chronic atrophic gastritis model according to an embodiment of the present invention;

FIG. 54 shows graphs for quantitative analysis of LVIDd and LVIDs in mice administered with Compound TJ01-013 in a myocarditis model according to an embodiment of the present invention;

FIG. 55 shows graphs for quantitative analysis of EF and FS in mice administered with Compound TJ01-013 in the myocarditis model according to an embodiment of the present invention;

FIG. 56 shows a graph for quantitative analysis of serum CK-MB levels in mice administered with Compound TJ01-013 in the myocarditis model according to an embodiment of the present invention;

FIG. 57 shows graphs for quantitative analysis of the number of CD4+ and CD8+ T cells around hair follicles in mice administered with Compound TJ01-013 in an alopecia model according to an embodiment of the present invention;

FIG. 58 shows graphs for quantitative analysis of inflammatory factors TNF-α and IFN-y in skin tissues of mice administered with Compound TJ01-013 in the alopecia model according to an embodiment of the present invention;

FIG. 59 shows graphs for quantitative analysis of serum Na+ and K+ in mice administered with Compound TJ01-013 in a hyperaldosteronism model according to an embodiment of the present invention;

FIG. 60 shows graphs for quantitative analysis of serum PAC and PRA in mice administered with Compound TJ01-013 in the hyperaldosteronism model according to an embodiment of the present invention;

FIG. 61 shows a graph for quantitative analysis of the number of erections of mice administered with Compound TJ01-013 in an erectile dysfunction model according to an embodiment of the present invention;

FIG. 62 shows graphs for quantitative analysis of the weights of kidney and testis of mice administered with Compound TJ01-013 in the erectile dysfunction model according to an embodiment of the present invention;

FIG. 63 shows a graph for quantitative analysis of the internal diameter of the left spermatic vein of mice administered with Compound TJ01-013 in the erectile dysfunction model according to an embodiment of the present invention;

FIG. 64 shows graphs for quantitative analysis of serum FSH, LH, and T in mice administered with Compound TJ01-013 in the erectile dysfunction model according to an embodiment of the present invention;

FIG. 65 shows a graph for quantitative analysis of CEJ-ABC in mice administered with Compound TJ01-013 in a periodontitis model according to an embodiment of the present invention;

FIG. 66 shows a graph for quantitative analysis of escape latency of a cerebral stroke model administered with Compound TJ01-013 according to an embodiment of the present invention;

FIG. 67 shows a graph for quantitative analysis of the number of passing through a platform by the cerebral stroke model administered with Compound TJ01-013 according to an embodiment of the present invention;

FIG. 68 shows a graph for quantitative analysis of the apoptosis rate in neurons of the cerebral stroke model administered with Compound TJ01-013 according to an embodiment of the present invention;

FIG. 69 shows a graph for quantitative analysis of seizure grade for an epilepsy model administered with Compound TJ01-013 according to an embodiment of the present invention;

FIG. 70 shows a graph for quantitative analysis of the post-stimulation latency in a step-down test for the epilepsy model administered with Compound TJ01-013 according to an embodiment of the present invention;

FIG. 71 shows a graph for quantitative analysis of the post-stimulation latency in a step-through test for the epilepsy model administered with Compound TJ01-013 according to an embodiment of the present invention;

FIG. 72 shows graphs for quantitative analysis of serum inflammatory factors in the epilepsy model administered with Compound TJ01-013 according to an embodiment of the present invention;

FIG. 73 shows graphs for quantitative analysis of the number of melanin-positive cells and melanin number density of a melasma model administered with Compound TJ01-013 according to an embodiment of the present invention;

FIG. 74 shows graphs for quantitative analysis of the target melanin-positive area and melanin area density of the melasma model administered with Compound TJ01-013 according to an embodiment of the present invention;

FIG. 75 shows a graph for quantitative analysis of vitiligo scores of a vitiligo model administered with Compound TJ01-013 according to an embodiment of the present invention;

FIG. 76 shows a graph for quantitative analysis of the proportion of melanin-containing hair follicles in the vitiligo model administered with Compound TJ01-013 according to an embodiment of the present invention;

FIG. 77 shows graphs for quantitative analysis of serum TYR and MDA levels in the vitiligo model administered with Compound TJ01-013 according to an embodiment of the present invention;

FIG. 78 shows a graph for quantitative analysis of hemoglobin Hb in a thalassemia model administered with Compound TJ01-013 according to an embodiment of the present invention;

FIG. 79 shows a graph for quantitative analysis of red blood cell count (RBC) in the thalassemia model administered with Compound TJ01-013 according to an embodiment of the present invention;

FIG. 80 shows a graph for quantitative analysis of the phospholipase A2 activity in nucleus pulposus tissues in a lumbar disc herniation model administered with Compound TJ01-013 according to an embodiment of the present invention;

FIG. 81 shows graphs for quantitative analysis of inflammatory factors TNF-α and IL-6 levels in nucleus pulposus tissues in the lumbar disc herniation model administered with Compound TJ01-013 according to an embodiment of the present invention;

FIG. 82 shows a graph for quantitative analysis of the weight of ovary in a polycystic ovary syndrome model administered with Compound TJ01-013 according to an embodiment of the present invention;

FIG. 83 shows a graph for quantitative analysis of serum E2 levels in the polycystic ovary syndrome model administered with Compound TJ01-013 according to an embodiment of the present invention; and

FIG. 84 shows graphs for quantitative analysis of serum LH, T, and FSH levels in the polycystic ovary syndrome model administered with Compound TJ01-013 according to an embodiment of the present invention.

## DETAILED DESCRIPTION OF THE INVENTION

The inventors of the present invention conducted extensive application exploration on the previously developed compounds of Formula (I) (the compounds of Formula I can be prepared by the method described in Chinese Patent Application No. 202111108417.6, the content of which is incorporated herein by reference in its entirety), and found that the compound exhibited excellent therapeutic efficacy in the treatment of numerous diseases, including reproductive system diseases, autoimmune diseases, skin inflammation, retinal inflammation, ocular degenerative diseases, muscular or skeletal degenerative diseases, neurological diseases, anemia, adrenal diseases, chronic gastritis, myocarditis, periodontitis, hypothyroidism, alopecia, liver injury, tinnitus, and chronic obstructive pulmonary disease, and more specifically, premature ovarian failure, ovarian insufficiency, systemic lupus erythematosus nephritis, sarcopenia, liver injury, oligospermia, rheumatoid arthritis, cataract, Cushing's syndrome, Hashimoto's thyroiditis, chronic obstructive pulmonary disease, tinnitus, psoriasis, chronic eczema, age-related macular degeneration, retinitis pigmentosa, multiple sclerosis, hypothyroidism, chronic atrophic gastritis, myocarditis, alopecia, primary aldosteronism, erectile dysfunction, periodontitis, cerebral stroke, epilepsy, melasma, vitiligo, β-thalassemia, lumbar disc herniation, and polycystic ovary syndrome, among others. The compounds of Formula (I) and the pharmaceutically acceptable salts, stereoisomers, solvates, or prodrugs thereof can dose-dependently alleviate some indicators of the aforementioned diseases, demonstrating significant application value in the prevention and treatment of these diseases.

### Uses of compounds

The present invention relates to the use of a compound of Formula (I) in treatment of, and/or preparation of a medicament for treating at least one disease selected from the following diseases: reproductive system diseases (e.g., premature ovarian failure, ovarian insufficiency, polycystic ovary syndrome, oligospermia, and erectile dysfunction), autoimmune diseases (e.g., systemic lupus erythematosus nephritis, rheumatoid arthritis, multiple sclerosis, Hashimoto's thyroiditis, or alopecia), skin diseases (e.g., psoriasis, melasma, vitiligo, chronic eczema, etc.), ocular diseases (e.g., cataract, age-related macular degeneration, and retinal inflammation (e.g., retinitis pigmentosa)), muscular or skeletal degenerative diseases (e.g., sarcopenia and lumbar disc herniation), neurological diseases (e.g., epilepsy, stroke, etc.), anemia (e.g., β-thalassemia), adrenal diseases (e.g., primary aldosteronism, Cushing's syndrome, etc.), chronic gastritis (e.g., chronic atrophic gastritis), myocarditis, periodontitis, hypothyroidism, alopecia, liver injury (e.g., acute alcoholic liver injury, chronic alcoholic liver injury, drug-induced liver injury, non-alcoholic steatohepatitis, or liver injury caused by cholecystitis, etc.), tinnitus, and chronic obstructive pulmonary disease.

The present invention relates to the use of the compound of Formula (I) in: (a1) preparation of a medicament for preventing and/or treating premature ovarian failure; and/or (a2) prevention and/or treatment of premature ovarian failure; and/or (b1) preparation of a medicament for preventing and/or treating ovarian insufficiency; and/or (b2) prevention and/or treatment of ovarian insufficiency; and/or (c1) preparation of a medicament for preventing and/or treating systemic lupus erythematosus nephritis; and/or (c2) prevention and/or treatment of systemic lupus erythematosus nephritis; and/or (d1) preparation of a medicament for preventing and/or treating sarcopenia; and/or (d2) prevention and/or treatment of sarcopenia; and/or (e1) preparation of a medicament for preventing and/or treating liver injury; and/or (e2) prevention and/or treatment of liver injury; and/or (f1) preparation of a medicament for preventing and/or treating oligospermia; and/or (f2) prevention and/or treatment of oligospermia; and/or (g1) preparation of a medicament for preventing and/or treating rheumatoid arthritis; and/or (g2) prevention and/or treatment of rheumatoid arthritis; and/or (h1) preparation of a medicament for preventing and/or treating cataract; and/or (h2) prevention and/or treatment of cataract; and/or (i1) preparation of a medicament for preventing and/or treating Cushing's syndrome; and/or (i2) prevention and/or treatment of

Cushing's syndrome; and/or (j1) preparation of a medicament for preventing and/or treating Hashimoto's thyroiditis; and/or (j2) prevention and/or treatment of Hashimoto's thyroiditis; and/or (k1) preparation of a medicament for preventing and/or treating chronic obstructive pulmonary disease; and/or (k2) prevention and/or treatment of chronic obstructive pulmonary disease; and/or (11) preparation of a medicament for preventing and/or treating tinnitus; and/or (l2) prevention and/or treatment of tinnitus; and/or (m1) preparation of a medicament for preventing and/or treating psoriasis; and/or (m2) prevention and/or treatment of psoriasis; and/or (n1) preparation of a medicament for preventing and/or treating chronic eczema; and/or (n2) prevention and/or treatment of chronic eczema; and/or (o1) preparation of a medicament for preventing and/or treating age-related macular degeneration; and/or (o2) prevention and/or treatment of age-related macular degeneration; (p1) preparation of a medicament for preventing and/or treating retinitis pigmentosa; and/or (p2) prevention and/or treatment of retinitis pigmentosa; (q1) preparation of a medicament for preventing and/or treating multiple sclerosis; and/or (q2) prevention and/or treatment of multiple sclerosis; (r1) preparation of a medicament for preventing and/or treating hypothyroidism; and/or (r2) prevention and/or treatment of hypothyroidism; (s1) preparation of a medicament for preventing and/or treating chronic atrophic gastritis; and/or (s2) prevention and/or treatment of chronic atrophic gastritis; (t1) preparation of a medicament for preventing and/or treating myocarditis; and/or (t2) prevention and/or treatment of myocarditis; (u1) preparation of a medicament for preventing and/or treating alopecia; and/or (u2) prevention and/or treatment of alopecia; (v1) preparation of a medicament for preventing and/or treating primary aldosteronism; and/or (v2) prevention and/or treatment of primary aldosteronism; (w1) preparation of a medicament for preventing and/or treating erectile dysfunction; and/or (w2) prevention and/or treatment of erectile dysfunction; (x1) preparation of a medicament for preventing and/or treating periodontitis; and/or (x2) prevention and/or treatment of periodontitis; (y1) preparation of a medicament for preventing and/or treating cerebral stroke; and/or (y2) prevention and/or treatment of cerebral stroke; (z1) preparation of a medicament for preventing and/or treating epilepsy; and/or (z2) prevention and/or treatment of epilepsy; (Aa1) preparation of a medicament for preventing and/or treating melasma; and/or (Aa2) prevention and/or treatment of melasma; and/or (Ab1) preparation of a medicament for preventing and/or treating vitiligo; and/or (Ab2) prevention and/or treatment of vitiligo; and/or (Ac1) preparation of a medicament for preventing and/or treating β-thalassemia; and/or (Ac2) prevention and/or treatment of β-thalassemia; and/or (Ad1) preparation of a medicament for preventing and/or treating lumbar disc herniation; and/or (Ad2) prevention and/or treatment of lumbar disc herniation; and/or (Ae1) preparation of a medicament for preventing and/or treating polycystic ovary syndrome; and/or (Ae2) prevention and/or treatment of polycystic ovary syndrome.

The compound of Formula (I) of the present invention is shown as follows:

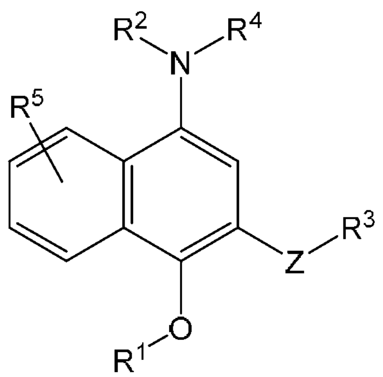

I

wherein Z is

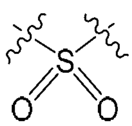 , 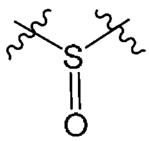 , 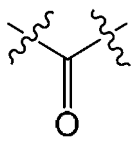 , 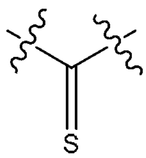

or a sulfur atom;

$R^1$ is hydrogen, $C_{1\sim6}$ alkyl,

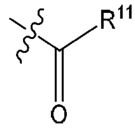 , 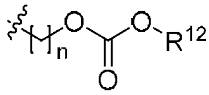 ,

wherein $R^{11}$ and $R^{12}$ are independently C1~6 alkyl or C1~6 cycloalkyl, respectively, and n is 1~4;

$R^2$ is hydrogen, $C_{1\sim6}$ alkyl, three- to six-membered cycloalkyl, three- to six-membered epoxyalkyl, phenyl, $C_{1\sim6}$ alkyl with at least one hydrogen substituted with $R^{2-1}$, phenyl with at least one hydrogen substituted with $R^{2-1}$, wherein $R^{2-1}$ is hydroxyl, halogen,aminogroup, or $C_{1\sim6}$ alkoxyl,

$R^3$ is

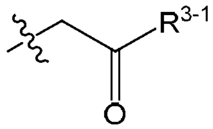 , 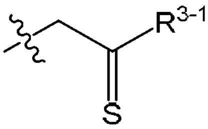 or 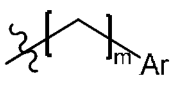 ;

wherein $R^{3-1}$ is hydrogen, hydroxyl, $C_{1\sim6}$ alkyl, $C_{1\sim6}$ alkoxy, $C_{3\sim6}$ cycloalkyl, three- to six-membered epoxyalkyl, aminogroup, $C_{1\sim6}$ amine group, $-CH_2C(O)R^{3-2}$, $-CH_2C(O)OR^{3-2}$, $-CH_2C(O)N$ $(R^{3-2}\ R^{3-2a})$, and $R^{3-2}$ and $R^{3-2a}$ are independently hydrogen, C1~6 alkyl, or three- to six-membered cycloalkyl respectively,

m is from 1 to 6, and

Ar is phenyl, and biphenyl, 5- or 6-membered monocyclic heteroaryl, 8- to 10-membered fused bicyclic heteroaryl, phenyl with at least one hydrogen atom substituted with $R^{3-3}$, and biphenyl with at least one hydrogen atom substituted with $R^{3-3}$, 5- or 6-membered monocyclic heteroaryl with at least one hydrogen atom substituted with $R^{3-3}$, 8- to 10-membered fused bicyclic heteroaryl with at least one hydrogen atom substituted with $R^{3-3}$, the $R^{3-3}$ is hydrogen, halogen, $C_{1\sim6}$ alkyl, three-to six-membered cycloalkyl, hydroxy, $C_{1\sim6}$ alkoxy, three- to six-membered epoxyalkyl, $C_{1\sim6}$ haloalkyl, $C_{2\sim6}$ alkenyl, $C_{2\sim6}$ alkynyl, $-N(R^{3-3a}R^{3-3b})$ or phenyl,

$R^{3-3a}$ and $R^{3-3b}$ are independently hydrogen, $C_{1\sim6}$ alkyl, or three- to six-membered cycloalkyl, respectively;

$R^4$ is

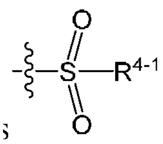 , 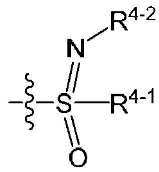 , 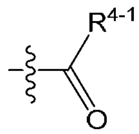 or 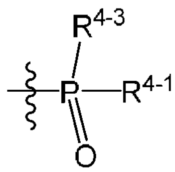 .

wherein $R^{4-1}$ is phenyl, biphenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, $R^{4-11}$ is hydrogen, halogen, nitro, nitrile, hydroxyl, $C_{1\sim6}$ alkyl, $C_{13\sim6}$ cycloalkyl, $C_{1\sim6}$ alkoxy, $-N(R^{4-1a}R^{4-1b})$, phenyl, $C_{1\sim6}$ haloalkyl, $C_{1\sim6}$ haloalkoxy, $-C(O)OR^{4-12}$, $-C(O)R^{4-12}$, $-C(O)N(R^{4-1a}R^{4-1b})$, $-S(O)2R^{4-12}$, $-S(O)R^{4-12}$, $-OC(O)R^{4-12}$, $-OC(O)OR^{4-12}$ or

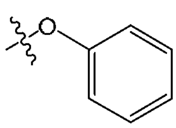 ,

$R^{4-12}$, $R4^{-1a}$ and $R^{4-1b}$ are independently hydrogen, respectively, $C_{1\sim6}$ alkyl, $C_{3\sim6}$ cycloalkyl, $C_{2\sim6}$ alkenyl, C2~6 alkynyl, $C_{1\sim6}$ alkyl substituted with at least one hydrogen by a halogen, $C_{2\sim6}$ alkynyl with at least one hydrogen substituted with a halogen, $C_{3\sim6}$ cycloalkylwith at least one hydrogen substituted by halogen, C2~6 alkynyl with at least one hydrogen substituted with a halogen, and $R^{4-1a}$ and $R^{4-1b}$ are bondable to each other to form a ring,

$R^{4-2}$ is $C_{1\sim6}$ alkyl, $C_{3\sim6}$ cycloalkyl, $C_{1\sim6}$ alkyl with at least one hydrogen substituted with a hydroxyl group, $C_{3\sim6}$epoxyalkyl, or $R^{4-2}$ is bonded to R2 to form a 4- to 8-membered ring when $R^2$ is $C_{1\sim6}$ alkyl and $R^{4-2}$ is $C_{1\sim6}$ alkyl.

$R^{4-3}$ is $C_{1\sim6}$ alkyl or C1~6 alkoxy;

the number of $R^5$ is 0~5, and at each occurrence, $R^5$ is independently hydrogen, halogen, nitro, nitrile, $-N+(R^{5-1})_3$, $C_{1\sim6}$ haloalkyl, $-C(O)OR^{5-1}$,$-C(O)R^{5-1}$, $-C(O)N(R^{5-1}R^{5-1a})$, $-S(O)_2R^{5-1}$, $-S(O)R^{5-1}$, $-S(O)_2N(R^{5-1}R^{5-1a})$, $-S(O)N(R^{5-1}R^{5-1a})$,

-N=C($R^{5-1}$ $R^{5-1a}$), hydroxyl, $C_{1\sim6}$ alkyl, phenyl, phenyl with at least one hydrogen substituted with $R^{5-1}$, $C_{1\sim6}$ alkoxy, -N($R^{5-1}R^{5-1a}$), -N($R^{5-1}$)C(O)$R^{5-1a}$, -N($R^{5-1}$)C(O)O$R^{5-1a}$, -OC(O)$R^{5-1}$, -OC(O)O$R^{5-1}$, -OC(O) N($R^{5-1}R^{5-1a}$) or -S$R^{5-1}$,$R^{5-1}$, $R^{5-1a}$ and $R^{5-1b}$are independently hydrogen, $C_{1\sim6}$ alkyl, $C_{2\sim6}$ alkenyl, C2~6 alkynyl, $C_{1\sim6}$ alkywith at least one hydrogen substituted with a halogen, $C_{2\sim6}$ alkenylwith at least one hydrogen substituted with a halogenor $C_{2\sim6}$ alkynyl with at least one hydrogen substituted with a halogen, respectively.

In some preferred embodiments, the Z is

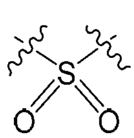, 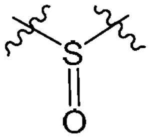, 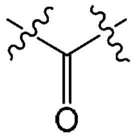,

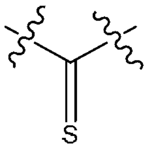

or a sulfur atom;

$R^1$ is hydrogen, $C_{1\sim4}$ alkyl,

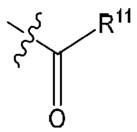 or 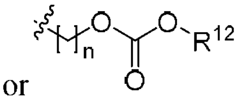

, wherein $R^{11}$ and $R^{12}$ are independently $C_{1\sim4}$ alkyl or $C_{1\sim4}$ cycloalkyl, respectively, and n is 1 or 2;
$R^2$ is hydrogen, $C_{1\sim4}$ alkyl, three- to six-membered cycloalkyl, three- to six-membered epoxyalkyl, phenyl, $C_{1\sim4}$ alkyl with at least one hydrogen substituted with $R^{2-1}$, and phenyl with at least one hydrogen substituted with $R^{2-1}$, wherein $R^{2-1}$ is hydroxy, halogen, amino group, or C $_{1\sim4}$ alkoxy;
$R^3$ is

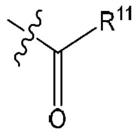, 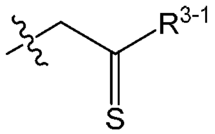 or 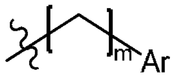;

wherein $R^{3-1}$ is hydrogen, hydroxyl, $C_{1\sim4}$ alkyl, $C_{1\sim4}$ alkoxy, -N($R^{3-2}R^{3-2a}$),
$R^{3-2}$ and $R^{3-2a}$ are independently hydrogen or $C_{1\sim4}$ alkyl, respectively, $R^{3-3a}$ and $R^{3-3b}$ are independently hydrogen or $C_{1\sim4}$ alkyl, respectively,
m is 1~4.
Ar is phenyl, 5- or 6-membered monocyclic heteroaryl, 5- or 6-membered monocyclic heteroaryl with at least one hydrogen atom substituted with $R^{3-3}$, the $R^{3-3}$ is hydrogen, halogen, $C_{1\sim4}$ alkyl, hydroxyl, $C_{1\sim4}$ alkoxy, $C_{1\sim4}$ haloalkyl, or -N($R^{3-3a}R^{3-3b}$);
$R^4$ is

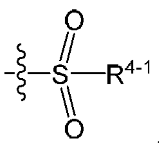, 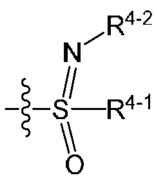, 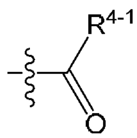 or 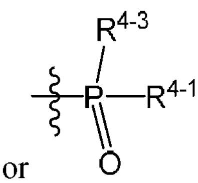,

wherein $R^{4-1}$ is phenyl, phenyl with at least one hydrogen atom substituted with $R^{4-11}$, 5- or 6-membered monocyclic

heteroaryl, 5- or 6-membered monocyclic heteroaryl with at least one hydrogen atom substituted with $R^{4-11}$, and the $R^{4-11}$ is hydrogen, halogen, nitro, $C_{1\sim4}$ alkyl, $C_{3\sim6}$ cycloalkyl, $C_{1\sim4}$ alkoxy, -N($R^{4-1a}R^{4-1b}$), phenyl, $C_{1\sim4}$ haloalkyl, $C_{1\sim4}$ haloalkoxy or

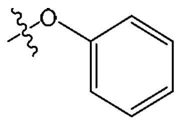,

$R^{4-1a}$ and $R^{4-1b}$ are independently hydrogen, $C_{1\sim4}$ alkyl, or $C_{3\sim6}$ cycloalkyl, respectively, and $R^{4-1a}$ and $R^{4-1b}$are bondable to each other to form a ring,

$R^{4-2}$ is $C_{1\sim4}$ alkyl, $C_{3\sim5}$ cycloalkyl, $C_{1\sim4}$ alkyl with at least one hydrogen substituted with hydroxyl, $C_{3\sim5}$epoxyalkyl, or $R^{4-2}$ is bonded to $R^2$ to form a 4- to 8-membered ring when $R^2$ is $C_{1\sim4}$ alkyl and $R^{4-2}$ is $C_{1\sim4}$ alkyl,

$R^{4-3}$ is $C_{1\sim4}$ alkyl or $C_{1\sim4}$ alkoxy;

at each occurrence, $R^5$ is independently hydrogen halogen, nitro, nitrile, -N+($R^{5-1}$)$_3$, $C_{1\sim4}$ haloalkyl,C(O)O$R^{5-1}$, -C(O)$R^{5-1}$, -C(O)N($R^{5-1}R^{5-1a}$), -S(O)$_2R^{5-1}$, -S(O)$R^{5-1}$, -N=C($R^{5-1}$ $R^{5-1a}$), hydroxyl, $C_{1\sim4}$ alkyl, phenyl, phenyl with at least one hydrogen substituted with $R^{5-1}$, $C_{1\sim4}$ alkoxy, -N($R^{5-1}R^{5-1a}$), -N( $R^{5-1}$)C(O)$R^{5-1a}$, or -OC(O)$R^{5-1}$, respectively, wherein $R^{5-1}$, $R^{5-1a}$, and $R^{5-1b}$ are independently hydrogen, $C_{1\sim4}$ alkyl, $C_{1\sim4}$ alkyl with at least one hydrogen substituted with a halogen, respectively.

In some preferred embodiments, the Z is

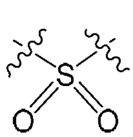, 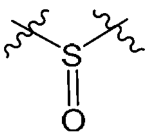, 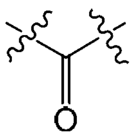,

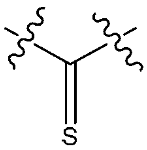

or a sulfur atom;

In some preferred embodiments, $R^1$ is hydrogen,

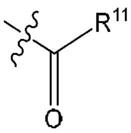 or 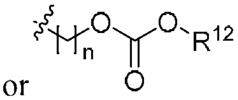,

wherein $R^{11}$ and $R^{12}$ are independently methyl, ethyl, n-propyl or isopropyl, respectively, and n is 1.

In some preferred embodiments, $R^2$ is hydrogen, methyl, ethyl, n-propyl, isopropyl, ethyl with one hydrogen substituted with hydroxyl, n-propyl with one hydrogen substituted with hydroxyl, phenyl,

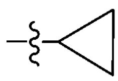, 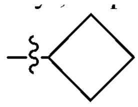, 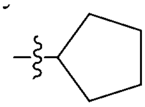,

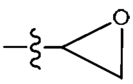, 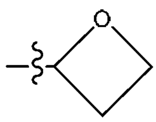, 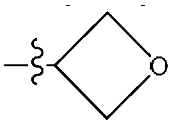, 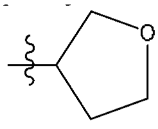 or 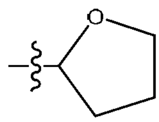.

In some preferred embodiments, $R^3$ is

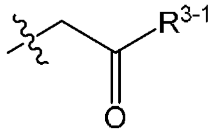 , 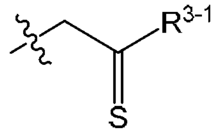

or

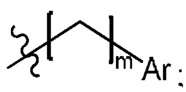

wherein $R^{3-1}$ is hydrogen, hydroxyl, methyl, ethyl, n-propyl, isopropyl, methoxy, ethoxy, n-propoxy, isopropoxy, n-butoxy, tert-butoxy, sec-butoxy or isobutoxy,
m is 1,
Ar is phenyl, 5- or 6-membered nitrogen-containing monocyclic heteroaryl.

In some preferred embodiments, $R^4$ is

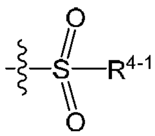 , 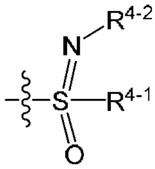 , 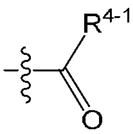

or

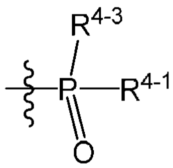 ,

wherein R4-1 is phenyl, phenyl with at least one hydrogen substituted with $R^{4-11}$ 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and the $R^{4-11}$ is halogen, nitro, methyl, ethyl, n-propyl, isopropyl,

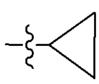 , 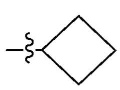 , 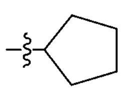 , 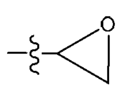 , 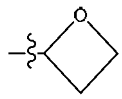 , 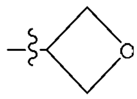 ,

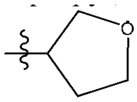 , 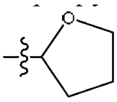 , 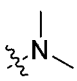 , 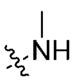 , 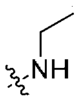 , 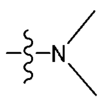 , 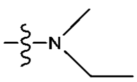 , 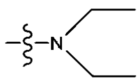 , 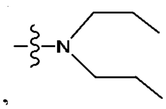 ,

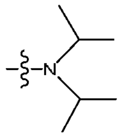 , 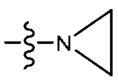 , 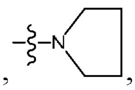 , 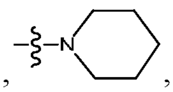 ,

fluoromethyl, fluoroethyl, fluoro-n-propyl, fluoroisopropyl, chloromethyl, chloroethyl, chloro-n-propyl, chloroisopropyl, bromomethyl, bromoethyl, bromo n-propyl, bromo isopropyl, iodomethyl, iodoethyl, iodo n-propyl, iodo isopropyl, fluoromethoxy, fluoroethoxy, fluoroorthopropoxy, fluoroisopropoxy, chloromethoxy, chloroethoxy, chloro n-propoxy,

chloroisopropoxy, bromomethoxy, bromoethoxy, bromo n-propoxy, bromoisopropoxy, iodomethoxy, iodoethoxy, iodo n-propoxy, iodoisopropoxy, or

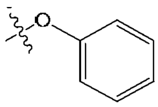,

$R^{4-2}$ is methyl, ethyl, n-propyl, isopropyl, n-butyl, isobutyl, tert-butyl, ethyl with one hydrogen substituted with hydroxyl, n-propyl with one hydrogen substituted with hydroxyl, n-butyl with one hydrogen substituted with hydroxyl,

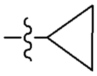, 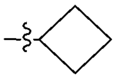,

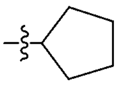, 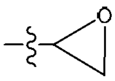, 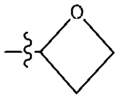, 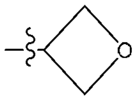, 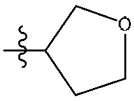, 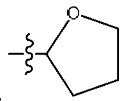

or phenyl, or $R^{4-2}$ is bonded to $R^2$ to form a 4 to 8-membered ring when $R^2$ is methyl, ethyl or n-propyl and $R^{4-2}$ is methyl, ethyl,
$R^{4-3}$ is methyl, ethyl, n-propyl, isopropyl, methoxy, ethoxy, n-propoxy or isopropoxy.

In some preferred embodiments, at each occurrence, $R^5$ is independently hydrogen, halogen, nitro, nitrile, $-N+(R^{5-1})_3$, fluoro-methyl, fluoroethyl, fluoro-n-propyl, fluoro-isopropyl, chloromethyl, chloroethyl, chloro-n-propyl, chloro-isopropyl, bromomethyl, bromoethyl, bromo-n-propyl, bromo-isopropyl, $-C(O)OR^{5-1}$, $-C(O)R^{5-1}$, $-C(O)N(R^{5-1}R^{5-1a})$, $-S(O)_2R^{5-1}$, $-S(O)R^{5-1}$, $-N=C(R^{5-1}\ R^{5-1a})$, hydroxyl, methyl, ethyl, n-propyl, isopropyl, phenyl, phenyl with at least one hydrogen substituted with $R^{5-1}$, methoxy, ethoxy, n-propyloxy, isopropyloxy, $-N(R^{5-1}R^{5-1a})$, $-N(R^{5-1})C(O)R^{5-1a}$, $-OC(O)R^{5-1}$, respectively, wherein $R^{5-1}$, $R^{5-1a}$, and $R^{5-1b}$ are independently hydrogen, methyl, ethyl, n-propyl, isopropyl, fluoro-methyl, fluoroethyl, fluoro-n-propyl, fluoro-isopropyl, chloro-methyl, chloroethyl, chloro n-propyl, chloro isopropyl, bromo-methyl, bromoethyl, bromo n-propyl, or bromo isopropyl, respectively.

In some preferred embodiments, $R^1$ is hydrogen,

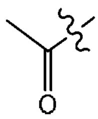, 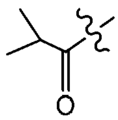

or

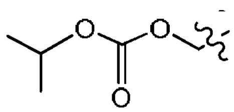

In some preferred embodiments, $R^2$ is hydrogen, methyl,

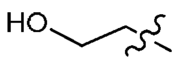, 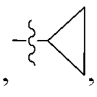,

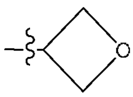

or phenyl.

In some preferred embodiments, $R^3$ is

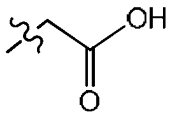 , 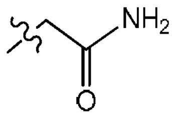 , 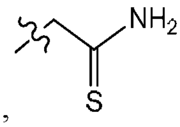 ,

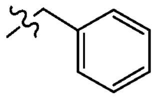 , 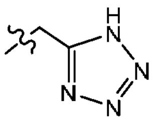 or 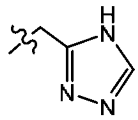 .

In some preferred embodiments, $R^4$ is

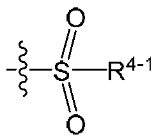 , 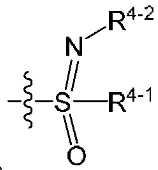 , 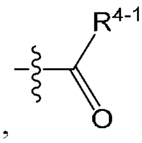

or

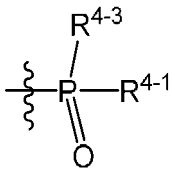 ,

wherein $R^{4-1}$ is phenyl, phenyl with at least one hydrogen substituted with $R^{4-11}$, five-membered monocyclic heteroaryl, five-membered monocyclic heteroaryl with at least one hydrogen substituted with $R^{4-11}$, nine-membered fused bicyclic heteroaryl, or naphthyl, wherein $R^{4-11}$ is bromine, fluorine, chlorine, methyl, nitro, phenyl, trifluoromethyl,

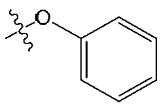 ,

methoxy, cyclopropyl, trifluoromethoxy, nitro or

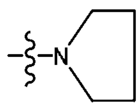 ,

$R^{4-2}$ is methyl, ethyl

, 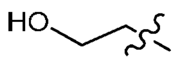 , 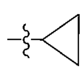 , 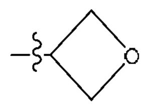

or phenyl, or, $R^{4-2}$ is bonded to $R^2$ to form a 4 to 6-membered ringwhen $R^2$ is methyl, ethyl, or n-propyl and $R^{4-2}$ is methyl, ethyl,

$R^{4-3}$ is methoxy, ethoxy, n-propoxy or isopropoxy.

In some preferred embodiments, at each occurrence, $R^5$ is independently halogen, nitro, nitrile, carboxyl, -NHC(O)CH, methoxy, or hydroxy, respectively.

In some preferred embodiments, the $C_{1\sim6}$ alkyl is $C_{1\sim4}$ alkyl, the $C_{1\sim4}$ alkyl is preferably methyl, ethyl, n-propyl,

isopropyl, n-butyl, isobutyl or tert-butyl, e.g., methyl or ethyl.

In some preferred embodiments, the three to six-membered cycloalkyl is preferably $C_{3\sim5}$ cycloalkyl, said $C_{3\sim5}$cycloalky is preferably

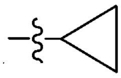, 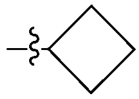

or

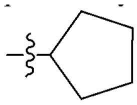,

for example

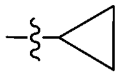.

In some preferred embodiments, said three to six-membered cycloalkyl epoxide is preferably

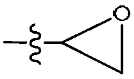, 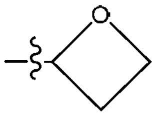, 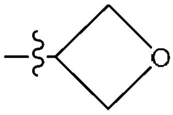, 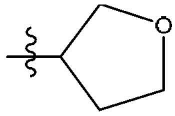

or

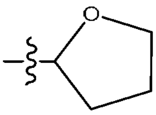,

for example:

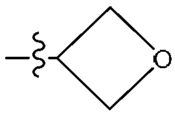.

In some preferred embodiments, the phenyl substituted with $C_{1\sim6}$ alkyl is preferably a phenylsubstituted with $C_{1\sim4}$ alkyl, more preferably a phenyl substituted with any of methyl, ethyl, n-propyl, isopropyl, n-butyl, isobutyl, and tert-butyl.

In some preferred embodiments, the $C_{1\sim6}$ alkoxy is preferably $C_{1\sim4}$ alkoxy, more preferably methoxy, ethoxy, n-propoxy, isopropoxy, n-butoxy, tert-butoxy, sec-butoxy or isobutoxy.

In some preferred embodiments, the -N($R^{3-2}R^{3-2a}$), -N($R^{3-3a}R^{3-3b}$), -N($R^{4-1a}R^{4-1b}$), -N($R^{5-1}R^{5-1a}$) is preferably

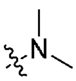, 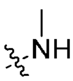, 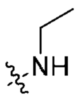, 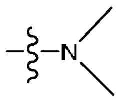, 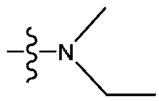,

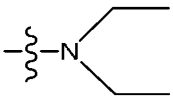 , 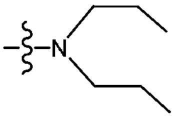 , 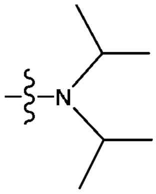 , 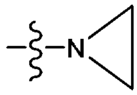 , 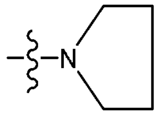

or

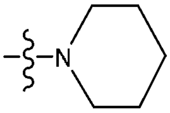.

In some preferred embodiments, the 5 or 6-membered monocyclic heteroarylpreferably is

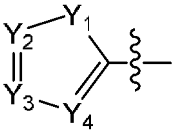 , 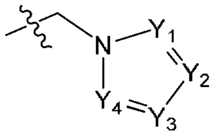 , 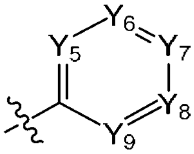 ;

, wherein, $Y_1$, $Y_2$, $Y_3$, $Y_4$, $Y_5$, $Y_6$, $Y_7$, $Y_8$, $Y_9$are independently selected from C, N, O, or S, respectively, and $Y_1$, $Y_2$, $Y_3$, $Y_4$are not all C, $Y_5$, $Y_6$, $Y_7$, $Y_8$, $Y_9$are not all C; the 5- or 6-membered monocyclic heteroaryl is more preferably pyrrolyl, furanyl, thienyl, pyrazolyl, imidazolyl, oxazolyl, isoxazolyl, thiazolyl, pyridyl, pyranyl, thiopyranyl, pyridazinyl, pyrimidinyl, pyrazinyl, piperazinyl, triazolyl, tetrazolyl; the 5- or 6-membered monocyclic heteroaryl is more preferably

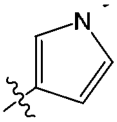 , 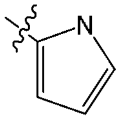 ,

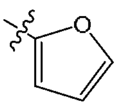 , 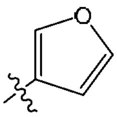 , 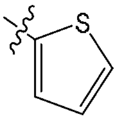 , 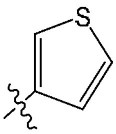 , 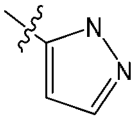 , 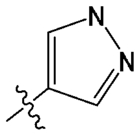 , 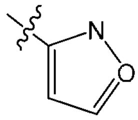 ,

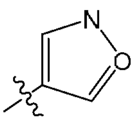 , 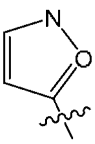 , 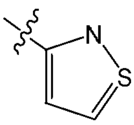 , 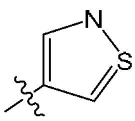 , 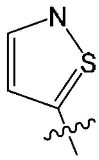 , 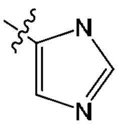 , 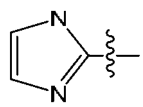 ,

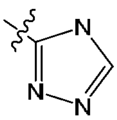 , 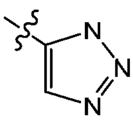 , 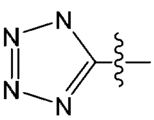 , 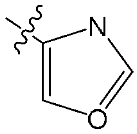 , 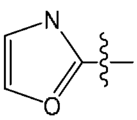 , 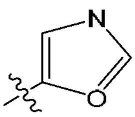 , 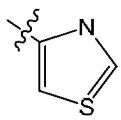 ,

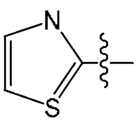 , 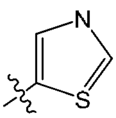 , 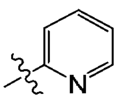 , 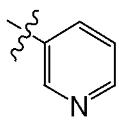 , 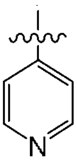 , 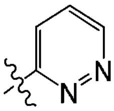 , 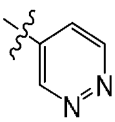 , 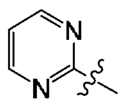 ,

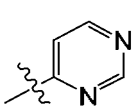, 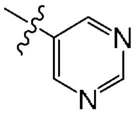, 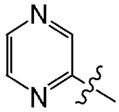, 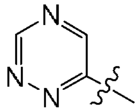, 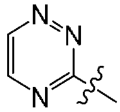, 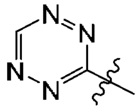, 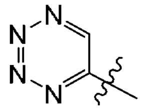,

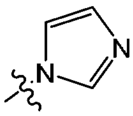, 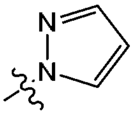, 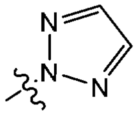, 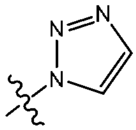, 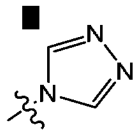, 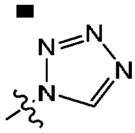, 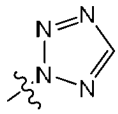,

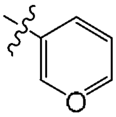, 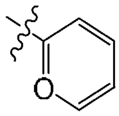, 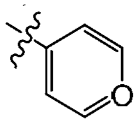, 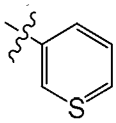, 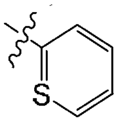, 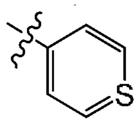;

more preferably, the 5- or 6-membered monocyclic heteroaryl is a 5 or 6-membered nitrogen-containing monocyclic heteroaryl, for example

: 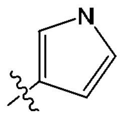, 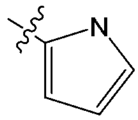,

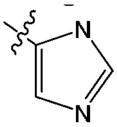, 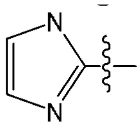, 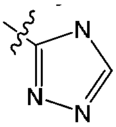, 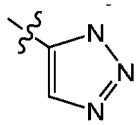, 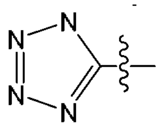, 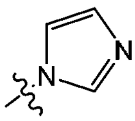, 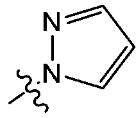,

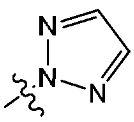, 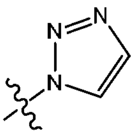, 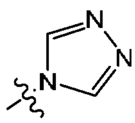, 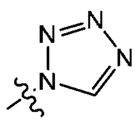, 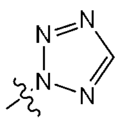 or 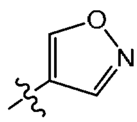;

most preferably, the 5 or 6-membered monocyclic heteroaryl is a 5-membered nitrogen-containing monocyclic heteroaryl, for example:

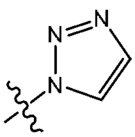, 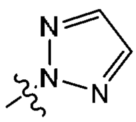

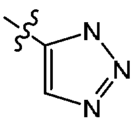, 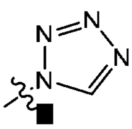, 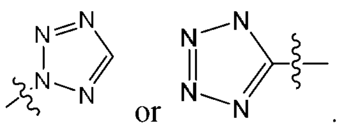 or .

In some preferred embodiments, the 8 to 10-membered fused bicyclic heteroaryl is

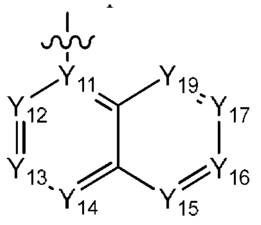
,
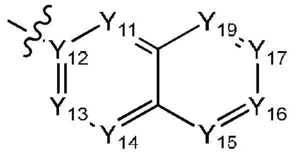
,
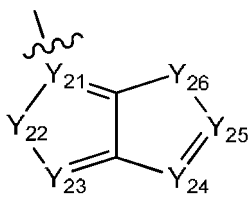
,
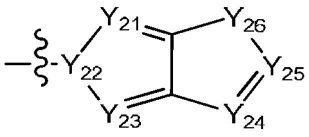
,
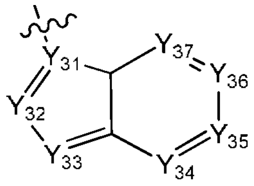
,
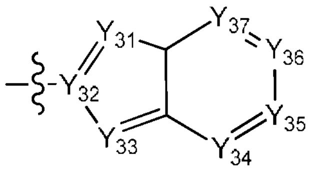
,
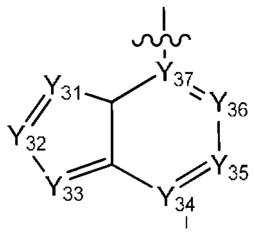
or
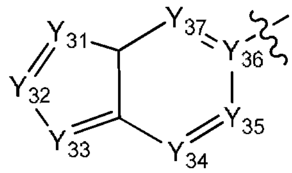
;

wherein $Y_{11}$, $Y_{12}$, $Y_{13}$, $Y_{14}$, $Y_{15}$, $Y_{16}$, $Y_{17}$, $Y_{18}$, and $Y_{19}$ are independently selected from C, N, O, or S, respectively, and $Y_{11}$, $Y_{12}$, $Y_{13}$, $Y_{14}$, $Y_{15}$, $Y_{16}$, $Y_{17}$, $Y_{18}$, and $Y_{19}$ are not allC; $Y_{21}$, $Y_{22}$, $Y_{23}$, $Y_{24}$, $Y_{25}$ and $Y_{26}$ are independently selected from C, N, O or S, respectively, and $Y_{21}$, $Y_{22}$, $Y_{23}$, $Y_{24}$, $Y_{25}$ and $Y_{26}$ are not all C; $Y_{31}$, $Y_{32}$, $Y_{33}$, $Y_{34}$, $Y_{35}$, $Y_{36}$ and $Y_{37}$ are independently selected from C, N, O or S, respectively, and $Y_{31}$, $Y_{32}$, $Y_{33}$, $Y_{34}$, $Y_{35}$, $Y_{36}$ and $Y_{37}$ are not all C; the 8 to 10-membered thickened bicyclic heteroaryl is more preferably indolyl, benzindolyl, benzothienyl, carbazolyl, quinolinyl, pteridinyl, purinyl; and said 8 to 10-membered thickened bicyclic heteroaryl is most preferably

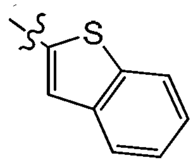
,
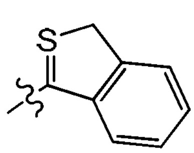
,
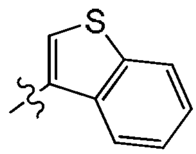
.

In some preferred embodiments, the halogen is preferably fluorine, chlorine, bromine or iodine.

In some preferred embodiments, the $C_{1\sim6}$ haloalkyl is preferably $C_{1\sim3}$ haloalkyl; more preferably fluoromethyl, fluoroethyl, fluoro n-propyl, fluoroisopropyl, chloromethyl, chloroethyl, chloro n-propyl, chloro isopropyl, bromomethyl, bromoethyl, bromo n-propyl, bromo isopropyl, iodomethyl, iodoethyl, iodo n-propyl, iodo isopropyl; most preferably trifluoromethyl.

In some preferred embodiments, the $C_{1\sim6}$ haloalkoxy is preferably $C_{1\sim3}$ haloalkoxy; more preferably fluoromethoxy, fluoroethoxy, fluoro n-propoxy, fluoroisopropoxy, chloromethoxy, chloroethoxy, chloroethoxy, chloro n-propoxy, chloroisopropoxy, bromomethoxy, bromoethoxy, bromo npropoxy, bromoisopropoxy, iodooxymethyl, iodoethoxy, iodo n-propoxy, iodoisopropoxy; most preferably trifluoromethoxy.

In some preferred embodiments, the $C_{2\sim6}$ alkenyl is preferably $C_{2\sim4}$ alkenyl, more preferably $-CH=CH_2$, $-CH=CH-CH_3$, $-CH_2-CH=CH_2$, $-CH=CH-CH_2-CH_3$, $-CH=CH-CH=CH_2$.

In some preferred embodiments, the $C_{2\sim6}$ alkynyl is preferably a $C_{2\sim6}$ alkynyl, more preferably $-C\equiv CH$, $-CH_2-C\equiv CH$, $-CH_2-CH_2-C\equiv CH$, $-CH_2-C\equiv C-CH_3$.

Based on the beneficial effects of improving metabolic stability and reducing toxicity, Z is not a sulfur atom in some preferred embodiments.

In a preferred embodiment of the present invention, in the compound of Formula (I), Z is

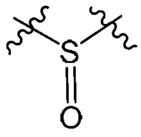

;

$R^1$ is hydrogen; $R^2$ is hydrogen; $R^3$ is hydrogen

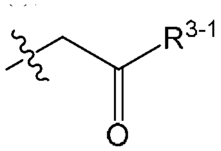

; $R^4$ is

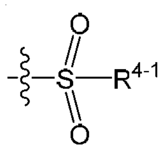

;

wherein $R^{3-1}$ is hydrogen or hydroxyl; and $R^{4-1}$ is phenyl, or phenyl with at least one hydrogen atom substituted by $R^{4-11}$, with $R^{4-11}$ being halogen-substituted methyl, halogen-substituted ethyl, methyl, ethyl or halogen.

In some preferred embodiments, in the compound of Formula I, Z is

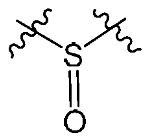

;

$R^1$ is hydrogen; $R^2$ is hydrogen; $R^3$ is hydrogen

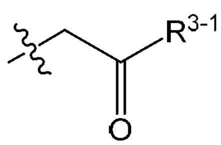

; $R^4$ is

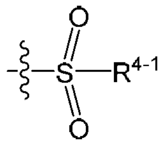

;

wherein $R^{3-1}$ is hydroxyl; and $R^{4-1}$ is phenyl, phenyl with at least one hydrogen atom substituted by trifluoromethyl, or phenyl with at least one hydrogen atom substituted by halogen.

In a particularly preferred embodiment of the present invention, Compound I-1 (also known as TJ01-013 or TJ0113) has a structure shown below. Through extensive experimental researches, the inventors have found that Compound I-1 exhibits superior efficacy compared to other compounds encompassed by Formula (I) in the prevention and/or treatment of at least one disease selected from premature ovarian failure, ovarian insufficiency, systemic lupus erythematosus nephritis, sarcopenia, liver injury, oligospermia, rheumatoid arthritis, cataract, Cushing's syndrome, Hashimoto's thyroiditis, chronic obstructive pulmonary disease, tinnitus, psoriasis, chronic eczema and age-related macular degeneration, and shows a dose-dependent effect on improving the indicators of these diseases.

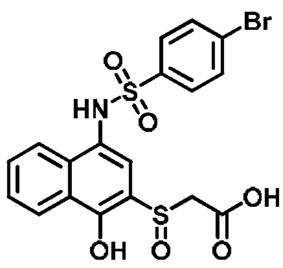

**TJ01-013**

。

Understandably, the derivatives of the compound of Formula (I) in the present invention should also has corresponding uses similar to those of the compound of Formula (I), and can be used for preventing and/or treating at

least one disease selected from premature ovarian failure, ovarian insufficiency, systemic lupus erythematosus nephritis, sarcopenia, liver injury, oligospermia, rheumatoid arthritis, cataract, Cushing's syndrome, Hashimoto's thyroiditis, chronic obstructive pulmonary disease, tinnitus, psoriasis, chronic eczema, and age-related macular degeneration. As used herein, the term "derivatives of the compound" refers to pharmaceutical salts, stereoisomers, solvates or prodrugs of a compound, and these derivatives do not alter the parent ring structure and basic function of the compound but may be modified for factors such as convenience in medicament preparation or improved solubility.

The compound of Formula I as described herein can be prepared by the method disclosed in Chinese Patent Application No. 202111108417.6, the content of which is incorporated herein by reference in its entirety.

As used herein, the terms "pharmaceutical salt" and "pharmacologically acceptable salt" are used interchangeable. In some cases, the pharmaceutically acceptable salt is derived by reacting the compound described herein with an acid or alkali. The term "pharmaceutically acceptable salt" may also refer to a salt formed by reacting a compound having acidic groups with an alkali or an pharmaceutically acceptable addition salt prepared by other methods that have been previously determined. There is no particular limitation on the pharmacologically acceptable salt, provided it can be used in medicaments. Examples of the salt formed by the compound described herein with an alkali include: salts formed with inorganic alkalies such as sodium, potassium, magnesium, calcium, and aluminum; salts formed with organic alkalies such as methylamine, ethylamine, and ethanolamine; or salts formed by reaction with dicyclohexylamine, N-methyl-D-glucamine, or tris(hydroxymethyl)methylamine; salts formed with alkaline amino acids such as lysine and ornithine; and ammonium salts. The salt may be an acid addition salt, with specific examples including: inorganic acids such as hydrochloric acid, hydrobromic acid, hydroiodic acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, methanesulfonic acid and ethanesulfonic acid; and acidic amino acids such as aspartic acid and glutamic acid.

Understandably, the compound of Formula (I) of the present invention or the derivatives thereof may be mixed with pharmaceutically acceptable excipients to obtain pharmaceutical compositions, which should also have corresponding uses to the compound of Formula (I) and can be applied to prevention and/or treatment of at least one disease selected from premature ovarian failure, ovarian insufficiency, systemic lupus erythematosus nephritis, sarcopenia, liver injury, oligospermia, rheumatoid arthritis, cataract, Cushing's syndrome, Hashimoto's thyroiditis, chronic obstructive pulmonary disease, tinnitus, psoriasis, chronic eczema, and age-related macular degeneration.

As used herein, the "excipient" or "pharmaceutically acceptable excipient" means a pharmaceutically acceptable material, composition, or vehicle, such as a liquid or solid filler, diluent, carrier, solvent, or encapsulating material. In an embodiment, each component is "pharmaceutically acceptable" in the sense of being compatible with other ingredients of the pharmaceutical formulation and suitable, suitable for contact with tissues or organs of humans and animals without excessive toxicity, irritation, allergic response, immunogenicity, or other problems or complications, and commensurate with a reasonable benefit/risk ratio. Unless otherwise indicated, the pharmaceutical compositions according to the present invention are prepared in a manner known per se, for example, by conventional mixing, granulation, coating, dissolving, or lyophilization processes. In the preparation of compositions for oral dosage forms, any commonly used pharmaceutical media (e.g., water, glycol, oil, and ethanol), carriers (e.g., starches, sugars, or microcrystalline cellulose), diluents, granulating agents, lubricants, binders, disintegrants and the like may be used. Due to ease of administration, tablets and capsules are the most advantageous unit dosage forms for oral administration, in which case solid pharmaceutical carriers are obviously used.

**Methods for treatment**

The present invention further relates to a method for preventing and/or treating premature ovarian failure, comprising the step of: administering to a subject a therapeutically effective amount of a compound of Formula (I) or a pharmaceutically acceptable salt, stereoisomer, solvate or prodrug thereof.

In the present invention, the "premature ovarian failure" refers to the phenomenon of amenorrhea before the age of 40 caused by ovarian function failure. It is featured with primary or secondary amenorrhea accompanied by elevated blood gonadotropin levels and decreased estrogen levels, along with varying degrees of a series of hypoestrogenic symptoms. Preferably, the premature ovarian failure is chemotherapy-induced premature ovarian failure.

As a method for treating premature ovarian failure, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating premature ovarian failure, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control

of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 30 mg/kg.

As a method for treating premature ovarian failure, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, more preferably at least 12 days, and more preferably at least 14 days).

As a method for treating premature ovarian failure, in a preferred embodiment, a therapeutically effective amount of I-14-1 is administered to the subject.

As a method for treating premature ovarian failure, in a preferred embodiment, a therapeutically effective amount of I-1 (TJ01-013) is administered to the subject . Compared with other compounds of the present invention, Compound I-1 administrated to a test subject can more significantly increase the ovarian weight, the ovarian coefficient, and the level of BDNF in ovarian tissues, to more significantly improve the ovarian function.

The present invention further relates to a method for preventing and/or treating ovarian insufficiency, comprising the step of: administering to a subject a therapeutically effective amount of a compound of Formula (I) or a pharmaceutically acceptable salt, stereoisomer, solvate or prodrug thereof.

In the present invention, the "ovarian insufficiency" refers to a condition in which women under the age of 40 exhibit reduced ovarian function, typically manifested as menstrual abnormalities and anovulation. Preferably, the ovarian insufficiency is caused by natural aging.

As a method for treating ovarian insufficiency, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating ovarian insufficiency, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 30 mg/kg.

As a method for treating ovarian insufficiency, the administration frequency for the compound of Formula (1) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, more preferably at least 20 days, and more preferably at least 30 days).

As a method for treating ovarian insufficiency, in a preferred embodiment, a therapeutically effective amount of I-14-1 is administered to the subject.

As a method for treating ovarian insufficiency, in a preferred embodiment, a therapeutically effective amount of I-1 is administered to the subject.. Compared with other compounds of the present invention, Compound I-1 administrated to a test subject can more significantly increase the ovarian weight, the ovarian coefficient, and the levels of E2 and FSH in the subject’s serum, to more significantly improve the ovarian function.

The present invention further relates to a method for preventing and/or treating systemic lupus erythematosus nephritis, comprising the step of: administering to a subject a therapeutically effective amount of a compound of Formula (I) or a pharmaceutically acceptable salt, stereoisomer, solvate or prodrug thereof.

In the present invention, the terms "systemic lupus erythematosus nephritis" and "lupus nephritis" are used

interchangeably and refer to a disease where systemic lupus erythematosus (SLE) is combined with different pathological types of immune damage in both kidneys, accompanied by obvious clinical manifestations of renal impairment.

As a method for treating systemic lupus erythematosus nephritis, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating systemic lupus erythematosus nephritis, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 10 mg/kg or 30 mg/kg.

As a method for treating systemic lupus erythematosus nephritis, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 14 days, more preferably at least 21 days, more preferably at least 28 days, more preferably at least 35 days, more preferably at least 42 days, more preferably at least 49 days, more preferably at least 56 days, more preferably at least 63 days, and more preferably at least 70 days).

As a method for treating systemic lupus erythematosus nephritis, in a preferred embodiment, a therapeutically effective amount of I-1 (TJ01-013) is administered to the subject. Compared with other compounds of the present invention, Compound I-1 administrated to a test subject can significantly alleviate the occurrence of proteinuria, and significantly reduce the levels of creatinine and blood urea nitrogen, the levels of inflammatory factors in renal tissues and the level of specific antibody against systemic lupus erythematosus, to more significantly improve the renal function.

The present invention further relates to a method for preventing and/or treating sarcopenia, comprising the step of: administering to a subject a therapeutically effective amount of a compound of Formula (I) or a pharmaceutically acceptable salt, stereoisomer, solvate or prodrug thereof.

In the present invention, the "sarcopenia" refers to a syndrome characterized by reduced skeletal muscle mass, decreased muscle strength, and impaired muscle function during the aging process. Sarcopenia is the early clinical manifestation of frailty syndrome.

As a method for treating sarcopenia, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating sarcopenia, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 10 mg/kg or 30 mg/kg.

As a method for treating sarcopenia, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, more preferably at least 20 days, and more

preferably at least 30 days).

As a method for treating sarcopenia, in a preferred embodiment, a therapeutically effective amount of I-14-1 is administered to the subject.

As a method for treating sarcopenia, in a preferred embodiment, a therapeutically effective amount of I-1 (TJ01-013) is administered to the subject. Compared with other compounds of the present invention, Compound I-1 administrated to a test subject can significantly improve myotility and gastrocnemius muscle weight, and obviously alleviate sarcopenia symptoms.

The present invention further relates to a method for preventing and/or treating liver injury, comprising the step of: administering to a subject a therapeutically effective amount of a compound of Formula (I) or a pharmaceutically acceptable salt, stereoisomer, solvate or prodrug thereof.

In the present invention, the term "liver injury" refers to a condition where the liver exhibits varying degrees of functional impairment or damage caused by external stimuli, internal inflammation, or hereditary factors, among others. Preferably, the liver injury is selected from at least one of acute alcoholic liver injury, chronic alcoholic liver injury, drug-induced liver injury, non-alcoholic steatohepatitis, and liver injury caused by cholecystitis.

As a method for treating liver injury, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating liver injury, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 10 mg/kg. When the subject is a human, in a preferred embodiment, the compound of Formula (I) of the present invention is administered to the subject at 1-200 mg/kg, for example, 10 mg/kg.

As a method for treating liver injury, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, and more preferably at least 14 days).

As a method for treating liver injury, in a preferred embodiment, a therapeutically effective amount of I-1 (TJ01-013) is administered to the subject. Compared with other compounds of the present invention, Compound I-1 administrated to a test subject can significantly down-regulate the levels of liver function indicators such as AST, ALT, TG, and TC, to significantly improve the liver function.

The present invention further relates to a method for preventing and/or treating oligospermia, comprising the step of: administering to a subject a therapeutically effective amount of a compound of Formula (I) or a pharmaceutically acceptable salt, stereoisomer, solvate or prodrug thereof.

In the present invention, the term "oligospermia" refers to a condition in which the number of sperms in semen is lower than that of a normal healthy fertile male.

As a method for treating oligospermia, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating oligospermia, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is

administered to the subject at 1-200 mg/kg, for example, 30 mg/kg. When the subject is a human, in a preferred embodiment, the compound of Formula (I) of the present invention is administered to the subject at 1-200 mg/kg, for example, 10 mg/kg or 30 mg/kg.

As a method for treating oligospermia, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, more preferably at least 20 days, and more preferably at least 30 days).

As a method for treating oligospermia, in a preferred embodiment, a therapeutically effective amount of I-14-1 is administered to the subject.

As a method for treating oligospermia, in a preferred embodiment, a therapeutically effective amount of I-1 (TJ01-013) is administered to the subject. Compared with other compounds of the present invention, Compound I-1 administrated to a test subject can significantly increase the level of sex hormones in the body, the testicular index and the number of sperms in semen, and markedly ameliorate the symptoms of oligospermia.

The present invention further relates to a method for preventing and/or treating rheumatoid arthritis, comprising the step of: administering to a subject a therapeutically effective amount of a compound of Formula (I) or a pharmaceutically acceptable salt, stereoisomer, solvate or prodrug thereof.

In the present invention, the term "rheumatoid arthritis (RA)" is a chronic systemic autoimmune disease. It mainly affects joints throughout the body and manifests as multiple and symmetric diffuse proliferative synovitis, causing damage to the joint cartilage and the joint capsule, eventually leading to joint ankylosis and deformity.

As a method for treating rheumatoid arthritis, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating rheumatoid arthritis, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 30 mg/kg.

As a method for treating rheumatoid arthritis, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, and more preferably at least 14 days).

As a method for treating rheumatoid arthritis, in a preferred embodiment, a therapeutically effective amount of I-1 (TJ01-013) is administered to the subject. Compared with other compounds of the present invention and dexamethasone, administering Compound I-1 to a test subject can significantly reduce joint swelling, markedly decrease hind plantar thickness, and notably ameliorate symptoms of rheumatoid arthritis.

The present invention further relates to a method for preventing and/or treating cataract, comprising the step of: administering to a subject a therapeutically effective amount of a compound of Formula (I) or a pharmaceutically acceptable salt, stereoisomer, solvate or prodrug thereof.

In the present invention, the term "cataract" is a disease caused by damage to the lens capsule due to many factors such as aging, genetics, metabolic abnormalities, trauma, radiation, poisoning, and local malnutrition, which increase the permeability of the lens capsule, impair its barrier function, or lead to the metabolic disorders of the lens, resulting in denaturation of lens proteins and opacity formation.

As a method for treating cataract, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating cataract, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 0.1-200 mg/kg, for example, 1 mg/kg. When the subject is a human, in a preferred embodiment, the compound of Formula (I) of the present invention is administered to the subject at 0.1-200 mg/kg, for example, 1 mg/kg.

As a method for treating cataract, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, and more preferably at least 10 days).

As a method for treating cataract, in a preferred embodiment, a therapeutically effective amount of I-14-1 is administered to the subject.

As a method for treating cataract, in a preferred embodiment, a therapeutically effective amount of I-1 (TJ01-013) is administered to the subject. Compared with other compounds of the present invention, Compound I-1 administrated to a test subject can significantly ameliorate lens opacity and markedly relieve the symptoms of cataract.

The present invention further relates to a method for preventing and/or treating Cushing's syndrome, comprising the step of: administering to a subject a therapeutically effective amount of a compound of Formula (I) or a pharmaceutically acceptable salt, stereoisomer, solvate or prodrug thereof.

In the present invention, the term "Cushing syndrome" refers to a series of diseases caused by excessive secretion of glucocorticoids (mostly cortisol) from the adrenal cortex due to various reasons. In the elderly, it is often caused by long-term use of glucocorticoid drugs. Adrenal cortical carcinoma and ectopic ACTH syndrome-induced hypercortisolism are more common in the elderly than in younger individuals.

As a method for treating Cushing's syndrome, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating Cushing's syndrome, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 15 mg/kg. When the subject is a human, in a preferred embodiment, the compound of Formula (I) of the present invention is administered to the subject at 1-200 mg/kg, for example, 15 mg/kg.

As a method for treating Cushing's syndrome, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, and more preferably at least 14 days).

As a method for treating Cushing's syndrome, in a preferred embodiment, a therapeutically effective amount of I-14-1 is administered to the subject.

As a method for treating Cushing's syndrome, in a preferred embodiment, a therapeutically effective amount of I-1 (TJ01-013) is administered to the subject. Compared with other compounds of the present invention, Compound I-1 administrated to a test subject can significantly reduce the levels of plasma insulins and markedly alleviate the symptoms of Cushing's syndrome.

The present invention further relates to a method for preventing and/or treating Hashimoto's thyroiditis, comprising the step of: administering to a subject a therapeutically effective amount of a compound of Formula (I) or

a pharmaceutically acceptable salt, stereoisomer, solvate or prodrug thereof.

In the present invention, the term "Hashimoto's thyroiditis" refers to immunologic dysfunction in the body, in which case autoantibodies against thyroid follicular epithelial cell antigen components (such as thyroglobulin, mitochondria, peroxidase, etc.) are produced, leading to damage and dysfunction of thyroid tissue cells.

As a method for treating Hashimoto's thyroiditis, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating Hashimoto's thyroiditis, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 15 mg/kg. When the subject is a human, in a preferred embodiment, the compound of Formula (I) of the present invention is administered to the subject at 1-200 mg/kg, for example, 15mg/kg or 30 mg/kg.

As a method for treating Hashimoto's thyroiditis, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, and more preferably at least 14 days).

As a method for treating Hashimoto's thyroiditis, in a preferred embodiment, a therapeutically effective amount of I-1 (TJ01-013) is administered to the subject. Compared with other compounds of the present invention, Compound I-1 administrated to a test subject can significantly reduce the level of a diagnostic marker TPO-Ab for Hashimoto's thyroiditis and markedly alleviate the symptoms of Hashimoto's thyroiditis.

The present invention further relates to a method for preventing and/or treating chronic obstructive pulmonary disease, comprising the step of: administering to a subject a therapeutically effective amount of a compound of Formula (I) or a pharmaceutically acceptable salt, stereoisomer, solvate or prodrug thereof.

In the present invention, the term "chronic obstructive pulmonary disease (COPD)" refers to a collective term for chronic airway obstructive diseases. It mainly refers to two diseases with irreversible airway obstruction, namely, chronic bronchitis and emphysema. It exhibits polygenic inheritance. Its onset is slow, with a long course, and the main symptoms include chronic cough, expectoration, shortness of breath, dyspnea, wheezing, and chest tightness.

As a method for treating chronic obstructive pulmonary disease, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating chronic obstructive pulmonary disease, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 7.5 mg/kg, 15 mg/kg or 30 mg/kg.

As a method for treating chronic obstructive pulmonary disease, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 14 days, and more

preferably at least 21 days).

As a method for treating chronic obstructive pulmonary disease, in a preferred embodiment, a therapeutically effective amount of I-1 (TJ01-013) is administered to the subject. Compared with other compounds of the present invention, Compound I-1 administrated to a test subject significantly reduces the level of inflammatory factor TNF$\alpha$ in the body, and the increase in inflammatory indicators caused by chronic obstructive pulmonary disease, and obviously alleviate the symptoms of chronic obstructive pulmonary disease.

The present invention further relates to a method for preventing and/or treating tinnitus, comprising the step of: administering to a subject a therapeutically effective amount of a compound of Formula (I) or a pharmaceutically acceptable salt, stereoisomer, solvate or prodrug thereof.

In the present invention, the term "tinnitus" refers to the symptom of subjectively hearing continuous sounds in the absence of external acoustic stimulation. It is caused by the pathological stimulation of the auditory receptors and their conduction pathways.

As a method for treating tinnitus, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating tinnitus, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 7.5 mg/kg, 15 mg/kg or 30 mg/kg.

As a method for treating tinnitus, the administration frequency for the compound of Formula (I) of the present invention is preferably once daily, twice daily, three times daily, once every two days, once every three days, or once weekly. In a preferred embodiment, the administration is performed once daily for at least 2 consecutive days (preferably at least 3 days, and more preferably at least 4 days).

As a method for treating tinnitus, in a preferred embodiment, a therapeutically effective amount of I-1 (TJ01-013) is administered to the subject. Compared with other compounds of the present invention, Compound I-1 administrated to a test subject can significantly reduce the amplitude of tinnitus response in patients.

The present invention further relates to a method for preventing and/or treating psoriasis, comprising the step of: administering to a subject a therapeutically effective amount of a compound of Formula (I) or a pharmaceutically acceptable salt, stereoisomer, solvate or prodrug thereof.

In the present invention, the term "psoriasis" refers to a disease caused by polygenic defects. It occurs under stimulation by various inducing factors such as trauma, infection, or drugs. The typical skin lesions manifest as well-defined red patches covered with silvery-white scales. When these scales are scraped off, a shiny film can be seen, and upon scraping the film, pinpoint bleeding can be observed. It commonly occurs on the extensor surfaces of the limbs and the lumbar and sacral regions. In severe cases, it may affect the entire body and joints. Preferably, the psoriasis is induced by the imiquimod emulsion.

As a method for treating psoriasis, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating psoriasis, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 7.5 mg/kg, 15 mg/kg or 30 mg/kg.

As a method for treating psoriasis, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, and more preferably at least 10 days).

As a method for treating psoriasis, in a preferred embodiment, a therapeutically effective amount of I-1 (TJ01-013) is administered to the subject. Compared with other compounds of the present invention, Compound I-1 administrated to a test subject can significantly alleviate the symptoms of parakeratosis and epidermal thickening in patients.

The present invention further relates to a method for preventing and/or treating chronic eczema, comprising the step of: administering to a subject a therapeutically effective amount of a compound of Formula (I) or a pharmaceutically acceptable salt, stereoisomer, solvate or prodrug thereof.

In the present invention, the terms "chronic eczema" and "eczema" are used interchangeably and refer to the inflammation of the superficial dermis and epidermis caused by various internal and external factors, with a tendency for exudation. Its etiology is complex and is generally considered to be related to allergic reactions. Clinically, it manifests as symmetrically distributed polymorphic lesions, intense itching, erythema, moistness, and clustered papulovesicles, and it tends to recur frequently and is a type of chronic disease.

As a method for treating chronic eczema, the administration mode for the compound of Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating chronic eczema, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 7.5 mg/kg, 15 mg/kg or 30 mg/kg.

As a method for treating chronic eczema, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, more preferably at least 12 days, and more preferably at least 14 days).

As a method for treating chronic eczema, in a preferred embodiment, a therapeutically effective amount of I-1 (TJ01-013) is administered to the subject. Compared with other compounds of the present invention, Compound I-1 administrated to a test subject can significantly increase the stratum corneum hydration, significantly reduce water loss, and markedly alleviate the symtoms of eczema.

The present invention further relates to a method for preventing and/or treating age-related macular degeneration, comprising the step of: administering to a subject a therapeutically effective amount of a compound of Formula (I) or a pharmaceutically acceptable salt, stereoisomer, solvate or prodrug thereof.

In the present invention, the term "age-related macular degeneration (ARMD)" refers to senescent changes in the structure of the macular region. The main manifestations include a decline in the phagocytic and digestive capacity of retinal pigment epithelial cells for the rod outer segments disc membranes of photoreceptor cells, leading to the retention of incompletely digested disc membrane remnants in the basal cytoplasm of the cells, after which the remnants are subsequently excreted extracellularly and deposited in Bruch's membranes to form drusen (more pronounced in the macular region), which results in macular degeneration. In the early stages, it often manifests as decreased vision, while in the late stages, it manifests as central vision loss (a dark spot in the central visual field) and blurred vision, with severe cases leading to blindness.

As a method for treating age-related macular degeneration, the administration mode for the compound of

Formula (I) of the present invention is not limited, and preferably includes: enteral administration, for example, oral or rectal administration; or parenteral administration, for example, intramuscular, intravenous, intranasal or transdermal administration. In a preferred embodiment, the compound of Formula (I) or the pharmacologically acceptable salt, stereoisomer, solvate or prodrug thereof is administered to the subject by oral gavage.

As a method for treating age-related macular degeneration, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 7.5 mg/kg, 15 mg/kg or 30 mg/kg.

As a method for treating age-related macular degeneration, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, more preferably at least 12 days, and more preferably at least 14 days).

As a method for treating age-related macular degeneration, in a preferred embodiment, a therapeutically effective amount of I-1 (TJ01-013) is administered to the subject. Compared with other compounds of the present invention, Compound I-1 administrated to a test subject can significantly improve retinal photoperceptivity and restore the normal retinal structure.

The present invention further relates to a method for preventing and/or treating retinitis pigmentosa, comprising the step of: administering to a subject a therapeutically effective amount of a compound of Formula (I) or a pharmaceutically acceptable salt, stereoisomer, solvate or prodrug thereof.

In the present invention, the term "retinal pigmentosa (RP)" refers to a progressive hereditary retinal degenerative disease, with the main clinical manifestations as night blindness occurring since childhood and progressive narrowing of the visual field. The global prevalence rate is approximately 1/3000 - 1/7000. Its pathological mechanism is believed to involve progressive apoptosis of photoreceptors caused by primary genetic defects or secondary degeneration of retinal pigment epithelial cells. Currently, there are no effective therapies for RP. According to studies, it is believed that RP is a chronic inflammatory disease, with inflammatory factors and immune regulation processes playing a significant role in the progression of the disease. Over the past few decades, researchers have proposed many strategies for treating RP, such as neurotrophic factors, Chinese medicinal therapies, visual prosthetic devices and gene therapies, which, however, shows unsatisfactory results, and there is still no definitive and effective therapy.

As a method for treating retinitis pigmentosa, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating retinitis pigmentosa, the administration frequency for the compound of Formula (I) of the present invention is preferably once daily, twice daily, three times daily, once every two days, once every three days, or once weekly. In a preferred embodiment, the administration is performed once daily for at least 1 week (preferably at least 3weeks, and more preferably at least 8 weeks).

As a method for treating retinitis pigmentosa, in a preferred embodiment, a therapeutically effective amount of I-1 (TJ01-013) is administered to the subject. Compared with other compounds of the present invention, Compound I-1 administrated to a test subject can significantly reduce the density of posterior retinal microglia and significantly increase the thickness of the photosensitive cell layer.

The present invention further relates to a method for preventing and/or treating multiple sclerosis, comprising the

step of: administering to a subject a therapeutically effective amount of a compound of Formula (I) or a pharmaceutically acceptable salt, stereoisomer, solvate or prodrug thereof.

As a method for treating multiple sclerosis, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating multiple sclerosis, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 21 days, more preferably at least 35 days, more preferably at least 49 days, and more preferably at least 84 days).

As a method for treating multiple sclerosis, in a preferred embodiment, a therapeutically effective amount of I-1 (TJ01-013) is administered to the subject. Compared with other compounds of the present invention, Compound I-1 administrated to a test subject reduces the number of entries in open arms and the open arm traveling distance and time for the test subjects in the elevated plus maze test, and can increase the number of crossing the platform as well as the spatial cleaning and memory abilities for the experimental subjects in the water maze test.

The present invention further relates to a method for preventing and/or treating hypothyroidism, comprising the step of: administering to a subject a therapeutically effective amount of a compound of Formula (I) or a pharmaceutically acceptable salt, stereoisomer, solvate or prodrug thereof.

As a method for treating hypothyroidism, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating hypothyroidism, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 21 days, and more preferably at least 28 days).

As a method for treating hypothyroidism, in a preferred embodiment, a therapeutically effective amount of I-1 (TJ01-013) is administered to the subject. Compared with other compounds of the present invention, Compound I-1 administrated to a test subject can significantly increase the levels of T3 and T4, and the levels of FT3 and FT4 in serum, and reduce the level of FSH.

The present invention further relates to a method for preventing and/or treating chronic atrophic gastritis, comprising the step of: administering to a subject a therapeutically effective amount of a compound of Formula (I) or a pharmaceutically acceptable salt, stereoisomer, solvate or prodrug thereof.

As a method for treating chronic atrophic gastritis, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating chronic atrophic gastritis, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 21 days, more preferably at least 35 days, more preferably at least 42 days, more preferably at least 49 days, and more preferably at least 56 days).

As a method for treating chronic atrophic gastritis, in a preferred embodiment, a therapeutically effective amount of I-1 (TJ01-013) is administered to the subject. Compared with other compounds of the present invention, Compound I-1 administrated to a test subject can significantly reduce the levels of IL-1$\beta$, IL-6, and TNF-$\alpha$ in serum while increasing the levels of PGI, PGII, and GAS in serum.

The present invention further relates to a method for preventing and/or treating myocarditis, comprising the step of: administering to a subject a therapeutically effective amount of a compound of Formula (I) or a pharmaceutically acceptable salt, stereoisomer, solvate or prodrug thereof.

As a method for treating myocarditis, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating myocarditis, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 21 days, and more preferably at least 28 days).

As a method for treating myocarditis, in a preferred embodiment, a therapeutically effective amount of I-1 (TJ01-013) is administered to the subject. Compared with other compounds of the present invention, Compound I-1 administrated to a test subject can reduce the levels of LVIDd and LVIDs, increase the levels of EF and FS, and decrease the level of CK-MB.

The present invention further relates to a method for preventing and/or treating alopecia, comprising the step of: administering to a subject a therapeutically effective amount of a compound of Formula (I) or a pharmaceutically acceptable salt, stereoisomer, solvate or prodrug thereof.

As used in the present invention, the term "alopecia" refers to the loss of scalp hair caused by the autoimmune deficiency in the body, including seborrheic alopecia, alopecia areata, androgenetic alopecia, etc.

As a method for treating alopecia, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating alopecia, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 21 days, and more preferably at least 28 days).

As a method for treating alopecia, in a preferred embodiment, a therapeutically effective amount of I-1 (TJ01-013)

is administered to the subject. Compared with other compounds of the present invention, Compound I-1 administrated to a test subject can significantly reduce the levels of IFN-$\gamma$ and TNF-$\alpha$ in skin tissues, and decrease the total number and proportion of pathological hair follicles.

The present invention further relates to a method for preventing and/or treating primary aldosteronism, comprising the step of: administering to a subject a therapeutically effective amount of a compound of Formula (I) or a pharmaceutically acceptable salt, stereoisomer, solvate or prodrug thereof.

As used in the present invention, the term "primary aldosteronism (PA)" is a heterogeneous disease caused by excessive aldosterone secretion from the adrenal cortex, leading to increased blood volume, sodium retention, potassium excretion and metabolic alkalosis, with clinical manifestations as hypertension and hypokalemia.

As a method for treating primary aldosteronism, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating primary aldosteronism, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 21 days, and more preferably at least 28 days).

As a method for treating primary aldosteronism, in a preferred embodiment, a therapeutically effective amount of I-1 (TJ01-013) is administered to the subject. Compared with other compounds of the present invention, Compound I-1 administrated to a test subject can ameliorate the symptoms of sodium retention and potassium excretion and reduce the level of PAC.

The present invention further relates to a method for preventing and/or treating erectile dysfunction, comprising the step of: administering to a subject a therapeutically effective amount of a compound of Formula (I) or a pharmaceutically acceptable salt, stereoisomer, solvate or prodrug thereof.

As a method for treating erectile dysfunction, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating erectile dysfunction, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 21 days, more preferably at least 35 days, more preferably at least 49 days, more preferably at least 63 days, and more preferably at least 84 days).

As a method for treating erectile dysfunction, in a preferred embodiment, a therapeutically effective amount of I-1 (TJ01-013) is administered to the subject. Compared with other compounds of the present invention, Compound I-1 administrated to a test subject can significantly reduce the levels of luteinizing hormone (LH) and follicle-stimulating hormone (FSH), increase the levels of sex hormone testosterone (T), and improve the frequency and duration of erections in patients.

The present invention further relates to a method for preventing and/or treating periodontitis, comprising the step of: administering to a subject a therapeutically effective amount of a compound of Formula (I) or a pharmaceutically acceptable salt, stereoisomer, solvate or prodrug thereof.

As a method for treating periodontitis, the administration dose for the compound of Formula (I) of the present

invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating periodontitis, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 21 days, more preferably at least 35 days, more preferably at least 49 days, and more preferably at least 56 days).

As a method for treating periodontitis, in a preferred embodiment, a therapeutically effective amount of I-1 (TJ01-013) is administered to the subject. Compared with other compounds of the present invention, Compound I-1 administrated to a test subject can significantly improve the periodontal conditions and the alveolar bone growth, and alleviate the symptoms of periodontitis.

The present invention further relates to a method for preventing and/or treating cerebral stroke, comprising the step of: administering to a subject a therapeutically effective amount of a compound of Formula (I) or a pharmaceutically acceptable salt, stereoisomer, solvate or prodrug thereof.

As a method for treating cerebral stroke, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating cerebral stroke, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 21 days, more preferably at least 35 days, more preferably at least 49 days, and more preferably at least 56 days).

As a method for treating cerebral stroke, in a preferred embodiment, a therapeutically effective amount of I-1 (TJ01-013) is administered to the subject. Compared with other compounds of the present invention, Compound I-1 administrated to a test subject can shorten the escape latency of the experimental subjects in the water maze test, increase their number of passing through the platform, improve memory and cognitive abilities, and decrease the apoptosis rate of hippocampal neurons in the brain.

The present invention further relates to a method for preventing and/or treating epilepsy, comprising the step of: administering to a subject a therapeutically effective amount of a compound of Formula (I) or a pharmaceutically acceptable salt, stereoisomer, solvate or prodrug thereof.

As a method for treating epilepsy, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200

mg/kg, for example, 1 mg/kg, 2.5mg/kg or 5 mg/kg.

As a method for treating epilepsy, the administration frequency for the compound of Formula (I) of the present invention is preferably once daily, twice daily, three times daily, once every two days, once every three days, or once weekly. In a preferred embodiment, the administration is performed once daily for at least 3 consecutive days (preferably at least 5 days, more preferably at least 7 days, more preferably at least 10 days, more preferably at least 12 days, and more preferably at least 21 days).

As a method for treating epilepsy, in a preferred embodiment, a therapeutically effective amount of I-1 (TJ01-013) is administered to the subject. Compared with other compounds of the present invention, Compound I-1 administrated to a test subject can prolong the post-stimulation latency of the experimental subjects in a step-down test, increase their latency in the step-through test, improve the cognitive function, and reduce the level of inflammatory factors in serum.

The present invention further relates to a method for preventing and/or treating melasma, comprising the step of: administering to a subject a therapeutically effective amount of a compound of Formula (I) or a pharmaceutically acceptable salt, stereoisomer, solvate or prodrug thereof.

As a method for treating melasma, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating melasma, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 28 days, more preferably at least 42 days, and more preferably at least 56 days).

As a method for treating melasma, in a preferred embodiment, a therapeutically effective amount of I-1 (TJ01-013) is administered to the subject. Compared with other compounds of the present invention, Compound I-1 administrated to a test subject can significantly reduce the area and distribution of pathological skins, as well as the number and density of spots.

The present invention further relates to a method for preventing and/or treating vitiligo, comprising the step of: administering to a subject a therapeutically effective amount of a compound of Formula (I) or a pharmaceutically acceptable salt, stereoisomer, solvate or prodrug thereof.

As a method for treating vitiligo, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating vitiligo, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 28 days, more preferably at least 42 days, and more preferably at least 56 days).

As a method for treating vitiligo, in a preferred embodiment, a therapeutically effective amount of I-1 (TJ01-013) is administered to the subject. Compared with other compounds of the present invention, Compound I-1 administrated to a test subject can significantly increase the activity of tyrosinase and reduce the content of MDA.

The present invention further relates to a method for preventing and/or treating β-thalassemia, comprising the step of: administering to a subject a therapeutically effective amount of a compound of Formula (I) or a pharmaceutically acceptable salt, stereoisomer, solvate or prodrug thereof.

As a method for treating β-thalassemia, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating β-thalassemia, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 28 days, more preferably at least 42 days, and more preferably at least 56 days).

As a method for treating β-thalassemia, in a preferred embodiment, a therapeutically effective amount of I-1 (TJ01-013) is administered to the subject. Compared with other compounds of the present invention, Compound I-1 administrated to a test subject can significantly increase the count levels of hemoglobin (Hb) and red blood cells, as well as the levels of Hb and RBC, and alleviate the symptoms of anemia.

The present invention further relates to a method for preventing and/or treating lumbar disc herniation, wherein the method comprises the step of: administering to a subject a therapeutically effective amount of a compound of Formula (I) or a pharmaceutically acceptable salt, stereoisomer, solvate or prodrug thereof.

As a method for treating lumbar disc herniation, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200 mg/kg, for example, 1 mg/kg, 2.5mg/kg or 5 mg/kg.

As a method for treating lumbar disc herniation, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 21 days, and more preferably at least 28 days).

As a method for treating lumbar disc herniation, in a preferred embodiment, a therapeutically effective amount of I-1 (TJ01-013) is administered to the subject. Compared with other compounds of the present invention, Compound I-1 administrated to a test subject can significantly reduce the levels of TNF-α and IL-6 in the nucleus pulposus tissues and markedly decrease the activity of PLA2 in the nucleus pulposus tissues.

The present invention further relates to a method for preventing and/or treating polycystic ovary syndrome, wherein the method comprises the step of: administering to a subject a therapeutically effective amount of a compound of Formula (I) or a pharmaceutically acceptable salt, stereoisomer, solvate or prodrug thereof.

As a method for treating polycystic ovary syndrome, the administration dose for the compound of Formula (I) of the present invention is a pharmaceutically effective dose, i.e., a therapeutically effective amount. The term "therapeutically effective amount" refers to an amount of the compound sufficient to provide a therapeutic effect in the treatment or control of a disease or disorder, or sufficient to delay or minimize one or more symptoms associated with the disease or disorder. Generally, the effective dose varies for different subjects. In the present application, the terms "subject" and "test subject" are used interchangeably and refer to an animals, for example, mammals, including but not limited to primates (for example, humans), cattle, sheep, goats, horses, dogs, cats, rabbits, rats, mice or the like. In a specific embodiment, the subject is a human.

When the subject is a mouse, in a preferred embodiment, the compound of Formula (I) of the present invention is administered to the subject at 1-200 mg/kg, for example, 7.5 mg/kg, 15 mg/kg or 30 mg/kg. When the subject is a human, in a preferred embodiment, the compound of Formula (I) of the present invention is administered to the subject at 1-200

mg/kg, for example, 1 mg/kg, 2.5mg/kg or 5 mg/kg.

As a method for treating polycystic ovary syndrome, the administration frequency for the compound of Formula (I) of the present invention is preferably once daily, twice daily, three times daily, once every two days, once every three days, or once weekly. In a preferred embodiment, the administration is performed once daily for at least 7 consecutive days (preferably at least 14 days, more preferably at least 21 days, and more preferably at least 28 days).

As a method for treating polycystic ovary syndrome, in a preferred embodiment, a therapeutically effective amount of I-1 (TJ01-013) is administered to the subject. Compared with other compounds of the present invention, Compound I-1 administrated to a test subject can significantly reduce the levels of T, LH, and FSH, and significantly improve the ovarian function.

Terms

As used herein, the term "alkyl" refers to a straight or branched saturated aliphatic hydrocarbon group. The term "$C_{1-6}$ alkyl" refers to a straight or branched alkyl group with 1 to 6 carbon atoms, for example, but not limited to: methyl, ethyl, n-propyl, isopropyl, n-butyl, isobutyl, tert-butyl, sec-butyl, n-pentyl, 1,1-dimethylpropyl, 1,2-dimethylpropyl, 2, 2-dimethylpropyl, 1-ethylpropyl, 2-methylbutyl, 3-methylbutyl, n-hexyl, 1-ethyl-2-methylpropyl, 1,1,2-trimethylpropyl, 1, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof, etc. The term "$C_{1-4}$ alkyl" refers to a straight or branched alkyl group having 1 to 4 carbon atoms. When the $C_{1-4}$ appears at the terminal of a molecule, non-limiting examples of the $C_{1-4}$ alkyl include: methyl, ethyl, n-propyl, isopropyl, n-butyl, isobutyl, sec-butyl, and tert-butyl; or, when two moieties of a molecule are linked via the alkyl group, the non-limiting examples include: $-CH_2-$, $-CH_2-CH_2-$, $-CH(CH_3)-$, $-CH_2-CH_2-CH_2-$, $-CH(C_2H_5)-$, and $-C(CH_3)_2-$. Each hydrogen atom on the carbon of $C_{1-4}$ alkyl can be substituted by a substituent further listed herein.

As used herein, the term "alkenyl" refers to a straight or branched hydrocarbon chain with at least one carbon-carbon double bond. Each hydrogen on the carbon of alkenyl can be substituted by a substituent further listed herein. The term "$C_{2\sim6}$ alkenyl" refers to a straight or branched hydrocarbon chain having 1 to 6 carbon atoms, with at least one carbon-carbon double bond. When $C_{2\sim6}$ alkenyl appears at the terminal of a molecule, non-limiting examples include: $-CH=CH_2$, $-CH=CH-CH_3$, $-CH_2-CH=CH_2$, $-CH=CH-CH_2-CH_3$, and $-CH=CH-CH=CH_2$; or, when two moieties of a molecule are linked via this alkenyl, non-limiting examples include: $-CH=CH-$. Each hydrogen on the carbon of $C_{2\sim6}$ alkenyl can be substituted by a substituent further listed herein.

As used herein, the term "alkynyl" refers to a straight or branched hydrocarbon chain with at least one carbon-carbon triple bond. Each hydrogen on the carbon of alkynyl can be substituted by a substituent further listed herein. The term "$C_{2\sim6}$ alkynyl" refers to a straight or branched hydrocarbon chain having 1 to 6 carbon atoms, with at least one carbon-carbon triple bond. When $C_{2\sim6}$ alkynyl appears at the terminal of a molecule, non-limiting examples include: $-C\equiv CH$, $-CH_2-C\equiv CH$, $-CH_2-CH_2-C\equiv CH$, and $-CH_2-C\equiv C-CH_3$, or, when two moieties of a molecule are linked via this alkynyl, non-limiting examples include $-C\equiv C-$. Each hydrogen on the carbon of $C_{2\sim6}$ alkynyl can be substituted by a substituent further listed herein.

As used herein, the term "alkoxy" refers to a group having the structure "-O-alkyl," with the alkyl defined as above. The term "$C_{1-6}$ alkoxy" refers to an alkoxy group having 1 to 6 carbon atoms, and its non-limiting examples include methoxy, ethoxy, n-propoxy, isopropoxy, n-butoxy, tert-butoxy, isobutoxy, n-pentoxy, etc.

As used herein, the term "amido" refers to a group formed by substituting at least one hydrogen atom in an amino group with an alkyl group. For example: in the amido group

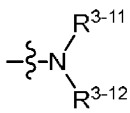,

either of $R^{3-11}$ and $R^{3-12}$ is an alkyl group, and the other is hydrogen; or both $R^{3-11}$ and $R^{3-12}$ are alkyl groups; when both $R^{3-11}$ and $R^{3-12}$ are alkyl groups, $R^{3-11}$ and $R^{3-12}$ may form a ring by bonding.

As used herein, the term "haloalkyl" refers to an alkyl group with one or more (e.g., 1, 2, 3, 4, or 5) hydrogen atoms substituted by halogen, where the alkyl is defined as above.

As used herein, "(O)" refers to

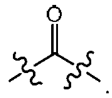

. In an embodiment, $-CH_2C(O) R^{3-2}$ refers to

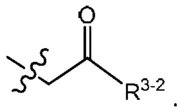

As used herein, the term "haloalkoxy" refers to an alkoxy group with one or more hydrogen atoms substituted by halogen, where the alkoxy is defined as above.

As used herein, the terms "aryl", "aryl ring" and "aromatic ring" are used interchangeably and refer to an all-carbon monocyclic group, an all-carbon non-fused polycyclic group (with rings linked by a covalent bond, in a non-fused way) or an all-carbon fused polycyclic group (i.e., rings sharing adjacent pairs of carbon atoms), where at least one ring in the group is aromatic, that is, it has a conjugated $\pi$ electron system with a cyclic structure.

As used herein, the term "heteroaryl" refers to an aryl group in which at least one ring carbon atom constituting the aryl is substituted by a heteroatom, which is a non-carbon atom, such as S, N or O.

As used herein, the term "monocyclic heteroaryl" refers to a heteroaryl group having only one aromatic ring, where the heteroaryl is defined as above. The term "5- or 6-membered monocyclic heteroaryl" refers to a monocyclic heteroaryl group having 5 or 6 ring atoms, where 1, 2 or 3 ring atoms are heteroatoms selected from nitrogen, oxygen or S(=O)m' (with m' being an integer ranging from 0 to 2). Its non-limiting examples include: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, etc.

As used herein, the term "fused ring heteroaryl" refers to a heteroaryl group having at least two aromatic rings, in which two ring atoms are shared adjacently, where the heteroaryl is defined as above. The term "fused bicyclic heteroaryl" refers to a fused ring heteroaryl group having two aromatic rings, where the fused ring heteroaryl is defined as above. The term "8- to 10-membered fused bicyclic heteroaryl" refers to a fused bicyclic heteroaryl group having 8 to 10 ring atoms, of which 1, 2, 3, 4 or 5 ring atoms are heteroatoms selected from nitrogen, oxygen or S(=O)m' (with m' being an integer ranging from 0 to 2). Its non-limiting examples include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, etc.

For clearer description of the objects, technical solutions and advantages of the embodiments of the present invention, the present invention is further described hereinafter in conjunction with specific embodiments. It should be understood that these examples are intended only to illustrate the present invention, rather than limiting the scope of the present invention. In the examples below, experimental methods without clear indication of specific conditions are generally carried out following the conventional conditions or the conditions recommended by the manufacturers. Unless otherwise specified, percentages and parts refer to weight percentages and parts by weight. The experimental materials and reagents as used in the following examples are commercially available, unless otherwise specially indicated.

Unless otherwise specified, all the technical and scientific terms used herein have the same meaning as those generally understood by those of ordinary skill in the technical field to which the present application belongs. It should be noted that the terms used herein are intended only to describe the specific embodiments, rather than limiting the exemplary embodiments of the present application.

**Example 1: Alleviating Premature Ovarian Insufficiency and Delaying Progression of Ovarian Insufficiency**

1) Chemotherapy-induced POF mouse models

Infertility following premature ovarian failure is one of the most severe side effects of chemotherapies in young female cancer patients. However, there is no effective therapy for gonadotoxic damage in the ovaries. Cyclophosphamide (Cy) is a commonly used chemotherapeutic drug that may induce severe ovarian damage and is a recognized risk factor for POF. Cyclophosphamide can induce primordial follicle depletion.

C57BL/6J mice (weighing approximately 20-25 g) aged 8 weeks were first adaptively fed for 7 days, and then

randomly divided into three groups based on body weight: a control group (Control, n = 8), a model group (Cy, treated with a solvent 0.5% CMC-Na, n = 8), a TJ01-013 treatment group (Cy + TJ01-013, orally gavaged with 30 mg/kg TJ01-013, n = 8), and an I-14-1 treatment group (Cy + I-14-1, orally gavaged with 30 mg/kg I-14-1, n = 8). The model group and the TJ01-013 treatment group received a single intraperitoneal injection of cyclophosphamide (75 mg/kg, 200-300μl), and the control group received a single intraperitoneal injection of an equal volume of normal saline. After 12 days of administration, samples were collected, and the body weight, ovarian weight, and serum hormone levels of E2 and FSH were measured.

2) Determination of ovarian index

The body weight and bilateral ovarian wet weight of mice in each group were measured using an electronic balance and an analytical balance, respectively. The ovarian index of each mice in each group was calculated based on the body weight (g) and the bilateral ovarian wet weight (mg). The formula was shown below:

$$\text{ovarian index} = \text{bilateral ovarian wet weight (mg)/mouse body weight (g)}.$$

As shown in FIG. 1, there was no significant change in body weight of mice after 14 days of chemotherapy and treatment, but the chemotherapy significantly reduced the ovarian weight, which was significantly improved after treatment with TJ01-013 and I-14-1. Similarly, the chemotherapy significantly decreased the ovarian index (the ovarian-to-body weight ratio), which was also significantly improved after treatment with TJ01-013 and I-14-1, with TJ01-013 showing better efficacy than I-14-1.

3) Detection of serum hormone levels by ELISA.

At the experimental endpoint, retro-orbital blood collection was performed. The blood was let stand at room temperature for 45 minutes, then centrifuged at 12,000g for 10 minutes. The supernatant (serum) was collected, and the levels of E2, FSH, and BDNF in serum were detected by ELISA.

From the results shown in FIG. 2, the serum FSH concentration significantly increased after chemotherapy-based model establishment as compared to the normal model group, and the serum FSH level significantly decreased after treatment with TJ01-013 and I-14-1. As shown in FIG. 3, the serum E2 level significantly decreased after chemotherapy-based model establishment, and the serum E2 level significantly increased after treatment with I-14-1 and TJ01-013. The results indicate that both TJ01-013 and I-14-1 treatments significantly improve the ovarian function in mice.

4) Detection of BDNF in ovarian tissues by ELISA.

Brain-derived neurotrophic factor (BDNF), a widely studied neurotrophic factor, is now also referred to as an ovarian endocrine factor. Substantial evidence indicates that BDNF plays a role in ovarian follicular development. Upon reaching the experimental endpoint, the mice were euthanized, and the ovaries were extracted and ground to measure the BDNF levels in ovarian tissues via ELISA.

From the results shown in FIG. 4, the BDNF levels in ovarian tissues significantly decreased after chemotherapy-based model establishment, and significantly increased after treatment with TJ01-013 and I-14-1, indicating that both TJ01-013 and I-14-1 treatments improved the ovarian function in mice.

TJ0113 demonstrated superior effects on improving the FSH concentration, serum E2 levels, and BDNF levels as compared to I-14-1, particularly showing a more significant advantage in reducing the serum FSH concentration.

**Example 2: Treatment of Naturally Aged Ovarian Insufficiency**

1) Establishment of naturally aged POF (NA-POF) model

The naturally aged model (NA-POF) simulated the conditions of progressive ovarian degeneration. Female C57BL/6J mice aged 9-10 months were purchased and raised until 12 months old. The mice were randomly divided into three groups: a control group (Control, orally gavaged with 0.5% CMC-Na, n = 8), a TJ01-013 treatment group (TJ01-013, orally gavaged with 30 mg/kg TJ01-013, n = 8), and an I-14-1 treatment group (I-14-1, orally gavaged with 30 mg/kg I-14-1, n = 8). Administration was performed daily for a total of 30 days. At the experimental endpoint, the body weight, ovarian weight, ovarian index, and serum hormone levels of E2 and FSH in the mice were measured.

2) Determination of ovarian index

The body weight and bilateral ovarian wet weight of mice in each group were measured using an electronic balance and an analytical balance, respectively. The ovarian index of each mice in each group was calculated based on the body weight (g) and the bilateral ovarian wet weight (mg). The formula was shown below:

$$\text{ovarian index} = \text{bilateral ovarian wet weight (mg)/mouse body weight (g).}$$

From the results shown in FIG. 5, there was no significant change in the body weight of naturally aged mice after treatment with TJ01-013 and I-14-1. However, aging led to a significant decrease in ovarian weight, which was significantly improved after treatment with TJ01-013 and I-14-1. Similarly, aging led to a significant decline in ovarian index, which was significantly improved after treatment with TJ01-013. Although the treatment with 1-14-1 did not show a significant increase, an upward trend was observed.

3) Detection of serum hormone levels by ELISA.

At the experimental endpoint, retro-orbital blood collection was performed. The blood was let stand at room temperature for 45 minutes, then centrifuged at 12,000g for 10 minutes. The supernatant (serum) was collected, and the levels of E2 and FSH in serum were detected by ELISA.

As shown in FIG. 6, the FSH levels in serum significantly decreased after post-aging treatment with TJ01-013. As shown in FIG. 7, the E2 levels in serum significantly increased after post-aging treatment with TJ01-013 and I-14-1. The results indicate that both TJ01-013 and I-14-1 treatments can to an extent ameliorate the age-related decline in the ovarian function in mice, with TJ01-013 demonstrating superior efficacy.

**Example 3: Treatment of Lupus Nephritis**

1) Model establishment and dosing

MRL/lpr mice are the most classical animal model for lupus and immune-related diseases. The MRL/lpr animal model ultimately develops a systemic lupus erythematosus-like disease, characterized by generalized lymphadenectasis, skin lesions, alopecia, aggressive arthritis, and immune complex deposition glomerulonephritis. The onset typically occurs at 14-16 weeks of age. In consideration of the gender-related pathogenesis of SLE (with a female-to-male incidence ratio of approximately 9:1), female MRL/lpr mice were used in this study.

Female MRL/lpr mice aged 10 weeks were housed in an SPF environment with a 12-hour light/dark cycle at the temperature maintained at 24-26°C. The mice were randomly divided into three groups based on body weight: a model group (Control, n = 8, orally gavaged with 0.5% CMC-Na), a TJ01-013 10 mg/kg treatment group (10 mg/kg, n = 8, orally gavaged with 10 mg/kg TJ01-013), and a TJ01-013 30 mg/kg treatment group (30 mg/kg, n = 8, orally gavaged with 30 mg/kg TJ01-013). After one week of acclimatization, daily drug administration commenced at Week 11. Starting from week 11, the random urine protein and creatinine concentrations were monitored every two weeks for the mice in each group, and the urine protein-to-creatinine ratio (uPCR) of the mice was calculated. The administration lasted for 10 weeks, after which retro-orbital blood collection was conducted to collect the serum. The serum creatinine, blood urea nitrogen, serum ANA, and anti-dsDNA concentrations were detected.

2) Detection of urinary protein and urinary creatinine concentrations

The levels of urinary protein and urinary creatinine were measured using Coomassie brilliant blue staining. From the results shown in FIG. 8, the normal group and the TJ01-013 treatment group exhibited substantially consistent trends in uPCR during continuous monitoring after 10 weeks of administration, and both were significantly lower than the MRL/lpr model group, indicating that TJ01-013 treatment alleviated the occurrence of proteinuria.

3) Detection of serum creatinine and blood urea nitrogen

Serum creatinine and blood urea nitrogen were detected using a biochemical analyzer. From the results shown in FIG. 9, the levels of serum creatinine and blood urea nitrogen in MRL/lpr model mice were significantly higher than those in normal mice, and after treatment with TJ01-013, the levels of creatinine and blood urea nitrogen were significantly lower than those in the model group, indicating that TJ01-013 treatment significantly improved the renal function.

4) Detection of serum ANA and anti-dsDNA concentrations

The levels of serum ANA and anti-dsDNA were detected by ELISA. From the results shown in FIGs. 10-11, the ANA and dsDNA levels in the serum of MRL/lpr model mice were significantly higher than those in normal mice, and after treatment with TJ01-013, the ANA and dsDNA levels were significantly lower than those in the model group, indicating that TJ01-013 treatment significantly reduced the levels of specific antibodies for systemic lupus erythematosus.

5) Detection of inflammatory factor levels in kidney tissues

The kidney tissues were placed in a homogenizer and thoroughly minced with clean scissors; 400 μl of a lysis buffer was added to the homogenizer, and after homogenization, the kidney tissues were placed on ice and repeatedly ground on the ice multiple times until the kidney tissues were as thoroughly ground as possible; and after 30 minutes of lysis, the lysate was transferred to a 1.5 ml centrifuge tube using a pipette, and centrifuged at 12,000 rpm for 10 minutes at 4°C, and the supernatant was stored at -20°C. The inflammatory factors TNFα and IL-6 in kidney tissue proteins were detected using ELISA.

From the results shown in FIG. 12, the levels of TNFα and IL-6 in the kidney tissues of the MRL/lpr model mice were significantly higher than those in normal mice, and after treatment with TJ01-013, the levels of TNFα and IL-6 were significantly reduced compared to the model group, indicating that TJ01-013 treatment significantly reduced the inflammatory level in the kidney tissues.

**Example 4: Treatment of Sarcopenia**

1) Model establishment and grouping

Male mice were weighed and then randomly divided into a blank group (normal, n = 8), a model control group (Control, orally gavaged with 0.5% CMC-Na, n = 8), a TJ01-013 treatment group (TJ01-013, orally gavaged at 30 mg/kg, n = 8), and an I-14-1 treatment group (I-14-1, orally gavaged with 30 mg/kg I-14-1, n = 8). Both the model control group and the treatment groups were subjected to continuous intramuscular injection of hydrocortisone into the left and right hind limbs for 7 days, followed by continuous subcutaneous injection of D-galactose in the neck for 40 days to promote the formation of sarcopenia in mice. In the experiment, daily administration was conducted after model establishment, for a total of 30 days. After the experiment, corresponding grip strength and swimming tests were conducted to evaluate the muscle strength.

2) Muscle strength evaluation test:

Swimming test: Warm water at 37°C was added to a basin with a diameter of 50 cm; mice were allowed to pre-swim in the basin for 20 min, with the water level maintained such that the mice could not touch the bottom; and after the physical strength of the mice was consumed to some extent, the hind limb movement time of the mice within 5 min was then measured.

Grip strength test: A grip strength gauge was vertically fixed, with its hook end tied with a rough rope; mice were allowed to cling to the rope, and their tails were pulled downward to create resistance when the mice attempted to climb upward; grip strength values were recorded; and the test was repeated three times to obtain an average value.

The above tests were repeated at the same time three days before the end of the experiment, and the test results were collected and averaged.

As shown in FIG. 13, the swimming time of sarcopenia mice was significantly shorter than that of the normal group, and after treatment with TJ01-013, the swimming time of the sarcopenia mice significantly increased, while treatment with I-14-1 showed a trend of increased swimming time. As shown in FIG. 14, the grip strength of the sarcopenia mice was significantly lower than that of the normal group. After treatment with TJ01-013 and I-14-1, the grip strength of the sarcopenia mice significantly increased. These results indicate that TJ01-013 and I-14-1 treatments can alleviate the progression of sarcopenia to treat sarcopenia.

**Example 5: Treatment of Acute Alcoholic Liver Injury**

1) Model establishment

C57BL/6J mice aged 8 weeks were adaptively fed for 7 days, and then randomly divided into three groups: a blank control group (Normal, n = 8), a model control group (ETOH, n = 8), a positive control group (ETOH + NAC, n = 8), and a TJ01-013 administration group (ETOH + TJ01-013, n = 8). Administration was performed via gavage at a dose of 5ml/kg

based on the body weight of the mice. The blank control group and the model control group received a vehicle, 0.5% CMC-Na, by gavage, the positive control group was treated with 400 mg/kg N-acetylcysteine (NAC), the TJ01-013 treatment group received 10 mg/kg TJ01-013, each for three consecutive days. Four hours after the last administration, all groups, except for the blank group, were intragastrically administered two doses of 50% alcohol (10 ml/kg) at an interval of 12 hours, while the blank group received an equivalent amount of normal saline. Two hours after the last administration of alcohol, retro-orbital blood collection was conducted; the blood was allowed to stand at room temperature for 1 hour, and then centrifuged at 4°C; the supernatant was taken to measure the corresponding serum indices; and the mice were sacrificed by cervical dislocation to excise the livers rapidly.

2) Determination of the levels of liver function indices: aspartate aminotransferase (AST), alanine aminotransferase (ALT), triglyceride (TG), and total cholesterol (TC)

The prepared mouse serum was tested for AST and ALT according to the operating procedures of the biochemical analyzer. The livers were ground to extract the supernatant of liver tissues and then measure the levels of TG and TC in the liver tissues, followed by significance analysis.

AST, ALT, TG, and TC are commonly used clinical indicators for detecting liver diseases. When hepatocytes are damaged, the permeability of the liver cell membrane increases, leading to the release of various enzymes into the blood. As shown in FIG. 15, compared with the normal group, the model control group exhibited significantly increased levels of ALT and AST, and the TJ01-013 treatment significantly inhibited the levels of ALT. Although the inhibitory effect on AST showed no significant difference as compared to the model group, there was a inhibition trend, which was better than that of the positive control NAC. As shown in FIG. 16, compared with the normal group, the model control group also exhibited significantly increased levels of TG and TC in the liver tissues, and the TJ01-013 treatment significantly inhibited the levels of TG and TC. These results indicated that TJ01-013 had a certain reversal effect on acute alcoholic liver injury and was significantly superior to that of the positive control drug.

**Example 6: Treatment of Chronic Alcoholic Liver Injury**

1) Model establishment

Male C57BL/6J mice aged 8 weeks were randomly divided into 4 groups based on body weight: a normal control group (PF, without subsequent alcohol-based model establishment, n = 8), a model control group (AF, n = 8), a positive drug treatment group (AF + Metadoxine, n = 8), and a TJ01-013 treatment group (AF + TJ01-013, n = 8). The experiment consisted of the following 5 phases: a 3-day environmental adaptation period (provided with pellet feed), a 3-day liquid feed adaptation period (provided with conventional or low-fat liquid feed without ethanol), an ethanol-containing liquid feed transition period (sequentially provided with conventional liquid feed containing 1%-4% ethanol for 2 days each), and a model establishment period (provided with liquid feed containing 5% ethanol continuously for 4 weeks). Administration began at the start of the formal model establishment period, with gavage administered at a dose of 5 ml/kg based on the body weight of the mice. The normal control group and the model control group received a vehicle, 0.5% CMC-Na, by gavage, the positive control group was treated with 65 mg/kg metadoxine, and the TJ01-013 treatment group was treated with 10 mg/kg TJ01-013, each for four weeks. After the administration was completed, the mice had their eyeballs removed for blood collection, and then sacrificed by cervical dislocation, and the livers were quickly extracted and fixed in 4% paraformaldehyde for later use in tissue staining.

2) Determination of the levels of liver function indices: aspartate aminotransferase (AST), alanine aminotransferase (ALT), triglyceride (TG), and total cholesterol (TC).

The prepared mouse serum was tested for AST, ALT, TG, and TC according to the operating procedures of the biochemical analyzer. The livers were ground to extract the supernatant of liver tissues and then measure the levels of TG and TC in the liver tissues, followed by significance analysis.

As shown in FIG. 17, compared with the normal control group, the model control group exhibited significantly increased levels of ALT and AST, both the positive control drug and TJ01-013 treatments significantly reduced the levels of ALT and AST, and the TJ01-013 treatment exhibited superior inhibitory effects on ALT and AST as compared to the positive control. These results indicated that TJ01-013 had a certain effect on alleviating the affected biochemical indicators caused by chronic alcoholic liver injury.

3) HE staining analysis of liver tissues

After blood collection from mouse eyeballs, cardiac perfusion was conducted with PBS; each mosue was

anesthetized and fixed on a foam board with needles; the skin and ribs of the chest and abdomen were cut to expose the heart; the injection needle was inserted into the left ventricle of the mouse, and the pericardium was cut open at the same time; perfusion was conducted with PBS at a rate of 7 rpm, until the liver turned white, then perfusion was stopped; and the liver was then removed and immersed in 4% paraformaldehyde for fixation for 72 hours. Subsequently, tissue embedding, sectioning, and HE staining were conducted, with the specific operations detailed below.

Tissue embedding

The steps for tissue embedding were as follows: ethanol dehydration: dehydrating the tissues successively in ethanol solutions of varying concentrations (75%, 85%, 95%, 100%, 100%), each for 40 minutes; transparentizing: immersing the tissues sequentially in three xylene baths, each for 30 minutes; wax impregnation: immersing the tissues sequentially in three paraffin baths, with the first bath for 1 hour, the second for 1.5 hours, and the third for 2 hours; embedding: pouring liquid paraffin into a mold box, then placing the wax-impregnated tissue block flat at the bottom, with the cut surface facing downward, removing an embedding frame after the paraffin was solidified, trimming the wax block after the paraffin was cooled and hardened completely, and retaining an appropriate amount of paraffin around the tissues for sectioning.

Slice preparation

The pre-cooled wax block was fixed on a paraffin microtome, the cut surface of the wax block was kept parallel to the blade edge which was typically at a tilt angle of 15 degrees, and a rotary thruster was rotated to adjust the section thickness to 3 μm to produce uniformly thick sections. With a brush held in a left hand, the handle of the microtome was rotated by a right hand. After the section was discharged, it was gently lifted up with the brush, picked up by forceps and placed face-up in a slide box, in which the water temperature was around 40°C. The section was flattened and then removed. To mount the section, one end of a slide was held with the left hand and vertically submerged in water to attach the section, while the forceps were held in the right hand to assist in positioning the section onto two-thirds of the slide. After mounting, the slide was air-dried slightly and placed on a slide warmer at 60°C for 1 hour, followed by baking in an oven for 2 hours.

Dewaxing of paraffin sections to hydration

The paraffin sections were sequentially placed in xylene I for 15 min, xylene II for 15 min, absolute ethanol I for 10 min, absolute ethanol II for 10 min, 95% alcohol for 10 min, and 85% alcohol for 10 min, for gradient dewaxing.

HE staining

The paraffin sections were stained with hematoxylin for 5-10 min, rinsed with tap water, differentiated with 1% hydrochloric acid alcohol for several seconds, rinsed with tap water, then allowed to return to blue with a saturated lithium carbonate aqueous solution for 1 min, rinsed with running water for several seconds, immersed in an eosin staining solution for several seconds, and rinsed with running water.

Dehydration, mounting, and image acquisition

The paraffin sections were sequentially placed in 75% ethanol for 2 min, 85% ethanol for 2 min, absolute ethanol for 2 min, absolute ethanol for 2 min, and xylene for 2 min for transparentizing. The section was then removed from xylene and mounted to the slide with neutral balsam. Microscopic examination and image acquisition analysis.

As shown in FIG. 18, the HE staining analysis results of liver tissues indicated that the chronic alcoholic fatty damage led to blurred hepatocyte structure and severe histological abnormalities, and the TJ01-013 treatment alleviated the severe liver tissue abnormalities caused by chronic alcohol consumption.

3) Oil red staining of liver tissues

The kidney tissues were fixed with 4% paraformaldehyde for 48 hours, dehydrated using sucrose, sliced with a freezing microtome, allowed to return to room temperature, and rinsed with distilled water to remove the embedding agent; and the tissues were immersed in 60% isopropanol for 2 min, then stained with an Oil Red O working solution for 2-5 min, adjusted in color under a microscope with 60% isopropanol, immediately rinsed with water, counterstained with hematoxylin to return to blue under running water, mounted to a slide with glycerin gelatin, and finally examined under a microscope.

As shown in FIG. 19, the microscopic examination results of oil red-stained liver tissued showed that the fat level in the chronic alcoholic steatohepatitis model group was significantly increased, and the TJ01-013 treatment alleviated the fat accumulation caused by chronic alcohol consumption.

**Example 7: Treatment of Drug-induced Liver Injury**

1) Model establishment

APAP-induced liver injury was a commonly used drug-induced liver injury mode. Male C57BL/6J mice aged 8 weeks were randomly divided into five groups (n = 8) based on body weight: a normal control group (normal, n = 8), a model control group (APAP, n = 8), a positive drug treatment group (APAP + silymarin, 50 mg/kg, n = 8), a TJ01-013 low-concentration treatment group (APAP + 13L, 10 mg/kg, n = 8), and a TJ01-013 high-concentration treatment group (APAP + 13H, 30 mg/kg, n = 8). Administration was performed via oral gavage at a dose of 5ml/kg based on the body weight of the mice. The blank group and the model group received a vehicle, 0.5% CMC-Na, by gavage, the positive drug treatment group received 50 mg/kg silymarin, the TJ01-013 low-concentration treatment group received 10 mg/kg TJ01-013, and the TJ01-013 high-concentration treatment group received 30 mg/kg TJ01-013, each for three consecutive days. Four hours after the last administration, all groups, except for the normal control group, were administered 500 mg/kg APAP (10 ml/kg) by oral gavage, while the normal control group received an equivalent volume of normal saline. Six hours after APAP model establishment, retro-orbital blood collection were condcuted; the blood was allowed to stand at room temperature for 1 hour, and then centrifuged at 4°C; and the supernatant was taken to measure the corresponding serum indices..

2) Determination of levels of liver function indices: aspartate aminotransferase (AST), alanine aminotransferase (ALT), alkaline phosphatase (ALP), and cholesterol (CHOL)

The levels of aspartate aminotransferase (AST), alanine aminotransferase (ALT), alkaline phosphatase (ALP), and cholesterol (CHOL) were determined according to the steps of the biochemical analyzer.

As shown in FIG. 20, compared with the normal group, the model control group exhibited significantly increased levels of ALTand AST, and both the positive control drug and the TJ01-013 treatment significantly reduced the levels of ALT and AST. As shown in FIG. 21, compared with the normal group, the model control group exhibited significantly increased levels of ALP in serum, and both the positive control drug treatment and the TJ01-013 treatment significantly inhibited the levels of ALP. These results indicate that TJ01-013 shows a certain alleviating effect on APAP-induced drug-induced liver injury.

**Example 8: Treatment of Oligospermia**

1) Model establishment

Male C57BL/6J mice were numbered by body weight and then randomly divided into three groups according to the random number table: a normal control group (normal, n = 8), a model control group (Control, n = 8), a TJ01-013 treatment group (TJ01-013, n = 8), and an I-14-1 treatment group (I-14-1, n = 8). The normal control group was intraperitoneally injected with 2 ml/d of normal saline, and the model control group and drug treatment groups were intraperitoneally injected with 0.1% cyclophosphamide at 2 mg/d for 5 consecutive days to establish the oligospermia models. From the sixth day, the normal control group and the model control group received 0.5% CMC-Na by gavage, the TJ01-013 treatment group received 30 mg/kg TJ01-013 by gavage, and the I-14-1treatment group received 30 mg/kg I-14-1 by gavage, with a gavage volume of 5 ml/kg, for 30 consecutive days. After the administration was completed, the mice were weighed and subjected to retro-orbital blood collection. After euthanasia, the bilateral testes and epididymides of the mice were isolated, adherent fat and other connective tissues were removed and then dried with filter paper, and the wet weight of the testes was measured using an analytical balance.

2) Determination of serum sex hormoness

After orbital blood collection, the blood was kept at room temperature for 30 min and centrifuged at 12,000 rpm for 10 min to collect a supernatant, i.e., the serum. The levels of sex hormones testosterone (T), luteinizing hormone (LH), and follicle-stimulating hormone (FSH) in mouse serum were measured using a biochemical analyzer.

From the results shown in FIG. 22, the model group exhibited significantly higher levels of FSH, LH, and T in serum than those in the normal control group; the levels of FSH and T in serum significantly decreased after the treatment with TJ01-013 and I-14-1; the TJ01-013 treatment also significantly reduced the level of LH in serum; and the 1-14-1 treatment showed a decreasing trend in the level of LH. These results indicate that TJ01-013 and I-14-1 can improve the

serum levels of sex hormones in mice, with TJ01-013 showing superior efficacy to I-14-1.

3) Determination of testicular index

The body weight and bilateral testis wet weight of mice in each group were measured using an electronic balance and an analytical balance, respectively. The ovarian index of each mice in each group was calculated based on the mouse body weight (g) and the bilateral testis wet weight (mg): The formula was shown below:

testicular index = bilateral testis wet weight (mg)/mosue body weight (g) $\times$ 100%.

As shown in FIG. 23, the mice in the model group exhibited reduced testis wet weight as compared to the normal control group. After treatment with TJ01-013 and I-14-1, the testis wet weight increased. Although there were no significant differences among the groups, certain trends were observed. Compared with the normal control group, the testicular index of the model group significantly decreased. After treatment with TJ01-013 and I-14-1, the testicular index increased, showing a significant difference. This indicated that the TJ01-013 treatment and the 1-14-1 treatment improved the quality of the testis with oligospermia, with TJ01-013 being significantly more effective than I-14-1.

4) Detection of sperm density

Collection of mouse semen: The isolated unilateral mouse epididymis was placed in 1 ml of pre-prepared normal saline at 37°C, minced with ophthalmic scissors, and incubated in a 37°C water bath for 15 minutes. After the sperm completely swim out of the epididymal tissues, the mixture was vortexed using a turbine mixer to prepare a sperm suspension, which was then stored in a 37°C water bath for later use. The number sperms was calculated using a blood cell counting plate.
As shown in FIG. 24, the sperm density in the model group was significantly lower than that in the normal control group, indicating a significant decline in the reproductive function of the model mice. Compared to the model control group, the TJ01-013 and I-14-1 treatment groups showed significantly increased sperm density, with a statistical significance. The results indicated that both TJ01-013 and I-14-1 were effective in treating oligospermia and increasing the sperm count in mice, with TJ01-013 showing significantly better efficacy than I-14-1.

**Example 9: Treatment of Rheumatoid Arthritis**

1) Model establishment

C57BL/6J mice aged 8 weeks were adaptively fed for 1 week, and then randomly divided into 4 groups: a normal control group (Normal, n = 8), a positive control group (Control, n = 8), a positive control group (Dex, a dexamethasone group, 0.2 mg/kg, n = 8), and a TJ01-013 treatment group (TJ01-013, 30 mg/kg, n = 8). All groups, except for the normal control group, were subjected to model establishment. Bovine type II collagen was mixed with a complete Freund's adjuvant at 1:1 and emulsified on ice at 4°C using a syringe to prepare the antigen. Then, 0.1 ml of the antigen was intradermally injected at the base of the mouse tail. Supplementary immunization was performed on Day 21 after the initial model establishment. The normal control group was injected with an equivalent dose of normal saline at the same time. From the 28th day after the initial immunization, gavage administration was conducted for a total of 14 days.

2) Arthritis score

The arthritis index (AI) score of each mouse was evaluated every 4 days starting from the initial immunization. The degree of arthritis in each paw was graded into 5 levels from none to severe on a scale of 0-4, with 4 being the most severe. The sum of the scores of all four paws of each mouse was recorded as the AI score, with a maximum score of 16. AI scoring criteria: 0 point: no redness or swelling; 1 point: redness and swelling in the small toe joint; 2 points: redness and swelling in the small toe and metatarsal joints; 3 points: redness and swelling in the entire paw below the ankle joint; 4 points: redness and swelling or deformity in the entire paw including the ankle joint. From the results shown in FIG. 25, after the initial immunization, the AI scores of the mice in the model group were significantly higher than those of the normal group, and the Al scores of the Dex intervention treatment group were significantly lower than those of the model control group, with an increasingly significant difference. The TJ01-013 treatment also significantly reduced the AI scores of the model mice.

3) Immune function assessment

Starting from the initial immunization, the number of swollen joints in the mouse paws was evaluated every 4 days. Since each paw consisted of five phalangeal joints and one ankle or wrist joint, the maximum number of swollen joints per mouse was 24 for all four paws. Meanwhile, the bilateral rear plantar thicknesses were measured using a vernier caliper, and the hind plantar thickness was calculated as the average of the bilateral plantar thicknesses. After sacrificing the mice at the end of the experiment, the body weight, thymus, and spleen of each mouse were weighed to calculate the immune organ index. The calculation formula was as follows: immune organ index = immune organ weight (mg)/body weight (g).

As shown in FIGs. 26 and 27, after the initial immunization, the number of swollen joints and hind plantar thickness in the model group were significantly higher than those in the normal control group. Both dexamethasone (Dex) and TJ01-013 treatments reduced the number of swollen joints and the hind plantar thickness. From the results shown in FIG. 28, the thymus index and the spleen index of the mice in the model control group were significantly higher than those in the normal control group, and both the Dex treatment and the TJ01-013 treatment reduced the thymus index and the spleen index in the model mice. These results indicate that TJ01-013 can significantly alleviate the symptoms of rheumatoid arthritis.

**Example 10: Treatment of Cataract**

10-day-old SD rats that have not opened their eyes yet were selected and randomly divided into 4 groups: a normal control group (normal, n = 10), a model control group (Vehicle, n = 10), an I-14-1 treatment group (I-14-1,1 mg/kg, n = 10), and a TJ01-013 high-dose treatment group (TJ01-013, 10 mg/kg, n = 10). All groups, except for the normal control group, were administered a 3mmol/L solution of sodium selenite powder in sterile normal saline via a single intraperitoneal injection at a dosing volume of 10ml/kg to establish the model. The normal control group received an equivalent dose of normal saline via intraperitoneal injection. The administration period started on the day of model establishment. The treatment groups received daily intraperitoneal injections of the corresponding doses of I-14-1 and TJ01-013 for 10 consecutive days, while the model group and the normal control group received daily intraperitoneal injections of the vehicle (5% DMSO + 20% PEG400 + 65% sterile normal saline). At the endpoint, all animals should have their eyes open, with the fundus observed using an ophthalmoscope, and the lens opacity was examined and scored using a slit-lamp microscope.

From the results shown in FIG. 29, both TJ01-013 and I-14-1 alleviated the selenite-induced lens opacity in rats, with TJ01-013 exhibiting a stronger alleviating effect.

**Example 11 Treatment of Cushing's Syndrome**

Male C57BL/6J mice aged 7 weeks were acclimatized for one week and then randomly divided into four groups based on body weight: a normal control group (normal, n = 10), a model control group (Control, n = 10), an I-14-1 15 mg/kg treatment group (I-14-1, n = 10), and a TJ01-013 15 mg/kg treatment group (TJ01-013, n = 10). All groups, except for the normal control group, were fed with drinking water containing 100 μg/mL corticosterone for two weeks to establish a Cushing's syndrome mouse model. The mice in the normal control group were fed normally. Administration began during model establishment. The normal control group and the model control group were administered with the vehicle (0.5% CMC-Na) by gavage, while the treatment groups received corresponding concentrations of I-14-1 and TJ01-013, with the dosing volume of 10 ml/kg for a total of 14 days in each case. After the administration was completed, the mice were subjected to retro-orbital blood collection; the blood was placed in anticoagulant tubes and centrifuged at 12,000 rpm for 10 minutes; and the supernatant was collected for detection of the blood glucose and insulin levels.

From the results shown in FIG. 30, compared with the normal control group, the model control group exhibited a significant increase in blood glucose levels, and the TJ01-013 treatment reduced the blood glucose levels, showing a significant difference compared to the model group. Compared with the normal control group, the model control group exhibited a significant increase in the fasting insulin level in plasma, and both the TJ01-013 treatment and the I-14-1 treatment reduced the fasting insulin level in plasm, showing a significant difference compared to the model group. These results indicate that both TJ01-013 and I-14-1 can alleviate Cushing's syndrome, with TJ01-013 showing superior efficacy.

**Example 12 Treatment of Hashimoto's thyroiditis**

Male SD rats aged 5 weeks were adaptively fed for 1 week, and then randomly divided into five groups based on body weight: a normal control group (normal, n = 10), a model control group (Control, n = 10), a TJ01-013 7.5 mg/kg treatment group (7.5 mg/kg, n = 10), a TJ01-013 15 mg/kg treatment group (15 mg/kg, n = 10), and a TJ01-013 30 mg/kg treatment group (30 mg/kg, n = 10). All groups, except for the normal control group, were fed with 0.64 g/L high-iodine

water. After 14 days of high-iodine water feeding, 0.2 ml of water-in-oil Tg formed by full emulsification of the complete Freund's adjuvant (CFA) was injected subcutaneously at multiple sites on both feet for a total of 2 injections with a 2-day interval therebetween, serving as the initial immunization. After two weeks, 0.2 ml of water-in-oil Tg formed by full emulsification of the complete Freund's adjuvant (CFA) was injected subcutaneously at multiple sites on the back for a total of 4 injections with a 7-day interval between every two injections, serving as the supplementary immunization. Administration began at the start of the primary immunization. The normal control group and the model control group were treated with a vehicle (0.5% CMC-Na) via oral gavage, and the TJ01-013 treatment groups were treated with corresponding concentrations of TJ01-013 at a dosing volume of 10 ml/kg for 6 weeks. After the administration was completed, blood was collected from the retro-orbital veins of the rats. The blood was let stand at room temperature for 30 minutes, then centrifuged at 6,000rpm for 10 minutes to obtain an upper serum. The levels of TGAb, TPO-Ab, TSH, and FT3 in the serum were detected using the enzyme-linked immunosorbent assay (ELISA).

TSH promotes thyroid growth and is an important indicator for assessing the thyroid function. It primarily stimulates the metabolic rate of epithelial cells in the target organ and promotes the synthesis of nucleic acids and proteins within the cells, leading to glandular hypertrophy. Since the level of FT3 in serum was not affected by thyroxine-binding globulin, it serves as a fundamental indicator for detecting the thyroid dysfunction. As shown in FIG. 31, the serum TSH level in the model group was significantly increased as compared to the normal control group, and the TJ01-013 treatment at each dose reduced the serum TSH level. The FT3 level in the model group was significantly decreased as compared to the normal control group, and the TJ01-013 treatment increased the FT3 level.

Thyroglobulin (TGAb) produces a complement after entering the blood and then binds to the produced complement, damaging follicular epithelial cells. The levels of TGAb and TPOAb in serum have high diagnostic value for Hashimoto's thyroiditis. As shown in FIG. 32, the serum TGAb level in the model group was significantly increased compared with the normal control group, and the high-dose TJ01-013 30 mg/kg treatment reduced the serum TGAb level. The TPO-Ab level in the model group was significantly increased as compared to the normal control group, and the TJ01-013 treatment at 15 mg/kg and 30 mg/kg could reduce the TPO-Ab level.

**Example 13: Treatment of Chronic Obstructive Pulmonary Disease**

Male SD rats aged 5 weeks were adaptively fed for 1 week, and then randomly divided into five groups based on body weight: a normal control group (normal, n = 10), a model control group (Control, n = 10), a TJ01-013 7.5 mg/kg treatment group (7.5 mg/kg, n = 10), a TJ01-013 15 mg/kg treatment group (15 mg/kg, n = 10), and a TJ01-013 30 mg/kg treatment group (30 mg/kg, n = 10). The model was replicated by intratracheal injection of lipopolysaccharide combined with passive smoke exposure. The rats in all groups, except for the normal control group, were anesthetized by intraperitoneal injection of pentobarbital sodium on the first day of model establishment. Each rat was fixed on the operating table, the neck hair was removed and disinfected, and a midline incision of about 0.5 cm was made in the neck. The subcutaneous tissues and fascia were separated to expose the trachea. The rat's head was elevated at 45 degrees, and 0.2 mL of LPS with a concentration of 1 mg/mL was slowly injected into the rat's airway using a 1 mL syringe. After injection, the rat was lifted by the tail and slowly rotated and shaken to ensure uniform distribution of the drug in the airway and both lungs. Finally, the surgical wound was sutured. From the second day after the operation, passive smoke exposure was conducted on the rats. A cigarette was inserted into a smoking chamber, and the rats were exposed to smoke once a day for 1 hour. Approximately 10 cigarettes were consumed. During the smoke exposure process, the smoke chamber was tapped once approximately every 5 minutes to avoid uneven smoke diffusion concentration in the airways and lung tissues. The normal control group underwent a sham surgery without smoke exposure. From the second day after the model establishment, the administration was cconducted. The normal control group and the model control group were intragastrically administered with a vehicle (0.5% CMC-Na), and the TJ01-013 treatment groups received the corresponding concentrations of TJ01-013. The dosing volume was 10 ml/kg, and the administration was carried out for 3 weeks. After the administration, blood was collected from the retroocular vein of the rats at room temperature and left to stand at room temperature for 30 minutes. Then, the blood was centrifuged at 6,000rpm for 10 minutes to obtain the upper layer serum, which was tested for the levels of TNF$\alpha$ and CXCL1 using the enzyme-linked immunosorbent assay (ELISA) method.

From the results shown in FIG. 33, the levels of TNF$\alpha$ and CXCL1 in the serum of the model group were significantly higher than those in the normal control group; the treatments with 15 mg/kg and 30 mg/kg TJ01-013 reduced the levels of CXCL1 in serum; and the treatment with 30 mg/kg TJ01-013 also decreased the level of TNF$\alpha$ in serum, with a significant difference. These results indicate that TJ01-013 treatment can reduce the elevation of inflammatory indicators caused by chronic obstructive pulmonary disease and alleviate symptoms.

**Example 14: Treatment of Tinnitus**

Male C57BL/6 mice aged 7 weeks with normal hearing were screened for absence of tinnitus based on their acoustic startle (AS) responses and gap pre-pulse inhibition of the acoustic startle (GPIAS) responses. The mice were

randomly divided into five groups: a normal control group (normal, n = 10), a model control group (Control, n = 10), a TJ01-013 7.5 mg/kg treatment group (7.5 mg/kg, n = 10), a TJ01-013 15 mg/kg treatment group (15 mg/kg, n = 10), and a TJ01-013 30 mg/kg treatment group (30 mg/kg, n = 10). All groups, except for the normal control group, were intraperitoneally injected with 450 mg/kg sodium salicylate to establish the model, and the normal control group was intraperitoneally injected with an equivalent volume of saline. Administration began on the day of model establishment, the normal control group and the model control group were intragastrically administered with a vehicle (0.5% CMC-Na), and the TJ01-013 treatment groups received TJ01-013 at corresponding concentrations, with a dosing volume of 10 ml/kg. After 4 consecutive days of administration, the acoustic startle response was measured again 3 hours after the last dose. The behavioral test involves the AS response combined with the GPLAS response, which is a behavioral experiment used to determine the presence of tinnitus. All mice were placed in transparent boxes for 15 minutes of acclimation for 3 consecutive days prior to the start of the experiment. The background noise provided in the experiment was 8 kHz and 16 kHz narrowband noise with a bandwidth of 1 kHz, for the purpose of determining the frequency range at which tinnitus occured. During the GPIAS experiment, each mouse was placed in a transparent box located in a dark chamber, and the transparent box was placed on a sensitive piezoelectric sensor, by which the voltage generated was proportional to the magnitude of the response elicited by the sound produced by a digital signal processor. The output of the platform was amplified, sampled, stored in a computer, and subsequently analyzed offline. GPIAS consisted of 100 trials with pre-gap (gap test) and 100 trials without pre-gap (no gap test). The pre-gap and no-gap trials were conducted using a random pairing method. The degree of Tinnitus was calculated as the percentage ratio of the response amplitude elicited by the gap trials to that elicited by no-gap trials (Ratio = gap/no gap × 100%).

From the results shown in FIG. 34, the ratio of AS response amplitude in the Tinnitus model group induced by sodium salicylate injection was significantly higher than that in the vehicle group under noise exposure at 8 kHz and 16 kHz, indicating successful model establishment; and the ratio of AS response amplitude in the TJ01-013 protection group was significantly lower than that in the model group, showing dose-dependency, which indicates that TJ01-013 significantly ameliorated the symptoms of tinnitus in mice.

**Example 15: Treatment of Psoriasis**

After 1 week of acclimation, 8-week-old m

Male C57BL/6J mice aged 8 weeks were adaptively fed for 1 week, and then randomly divided into five groups based on body weight: a normal control group (normal, n = 10), a model control group (Control, n = 10), a TJ01-013 7.5 mg/kg treatment group (7.5 mg/kg, n = 10), a TJ01-013 15 mg/kg treatment group (15 mg/kg, n = 10), and a TJ01-013 30 mg/kg treatment group (30 mg/kg, n = 10). Two days before the experiment, the back hair was shaved to obtain an area of approximately 2.5 cm × 4 cm, and 2% sodium sulfide was used for depilation. The control group was topically applied with 60 mg of vaseline on the shaved back area daily, while the model group and the TJ01-013 treatment groups were topically applied with 60 mg of imiquimod cream on the shaved back area daily. Administration started from the first day of model establishment and lasted for 10 days. The normal control group was intraperitoneally injected with an equivalent volume of normal saline, and the administration began on the day of model establishment. The normal control group and the model control group were intragastrically administered with a vehicle (0.5% CMC-Na), while the TJ01-013 treatment groups received the corresponding concentration of TJ01-013 at a dosing volume of 10 ml/kg. After the completion of administration, the skin tissues from the back of the mice were collected and prepared into tissue sections; and four measurement points were selected on each section to measure the thickness, and the average value was calculated. The thickness of the skin was used to represent the severity of the psoriasis-like skin condition.

As shown in FIG. 35, the model group exhibited significantly increased skin thickness as compared to the normal control group, and the TJ01-013 treatment significantly reduced the skin thickness as compared to the model control group, indicating that TJ01-013 significantly alleviated psoriasis-like symptoms in mice and presented dose-dependent improvement.

**Example 16: Significant Therapeutic Effect on Chronic Eczema**

Balb/c mice aged 6-8 weeks were adaptively fed for 1 week, and then randomly divided into five groups based on body weight: a normal control group (normal, n = 10), a model control group (Control, n = 10), a TJ01-013 7.5 mg/kg treatment group (7.5 mg/kg, n = 10), a TJ01-013 15 mg/kg treatment group (15 mg/kg, n = 10), and a TJ01-013 30 mg/kg treatment group (30 mg/kg, n = 10). Two days before the experiment, the back hair was shaved to obtain an area of approximately 2.5 cm × 4 cm, and 2% sodium sulfide was used for depilation. All groups, except for the normal control group, were sensitized by applying 60 μL of 6% DNCB (2,4-dinitrochlorobenzene) to the shaved area for the first time. Two weeks later, the back was shaved again to an area of approximately 3 cm × 4 cm, and 100 μL of 0.2% DNCB was applied to induce the reaction once a week for four consecutive weeks. The behavioral statuses of the mice in each group were

observed. The appearance of obvious eczema-like symptoms, such as scabbing, roughness, and dark red patches, indicated successful model establishment. After successful model establishment, drug administration began. The normal control group and the model control group were intragastrically administered with a vehicle (0.5% CMC-Na), and the TJ01-013 treatment group was administered with the corresponding concentration of TJ01-013 at a dosing volume of 10 ml/kg. The administration cycle was 14 days. After the completion of administration, a GPSkin Barrier detector was used to measure skin indicators such as transepidermal water loss (TEWL) and stratum corneum hydration (SCH) in each group of mice at Weeks 0, 1 and 2 of intervention.

From the results shown in FIG. 36, the transepidermal water loss (TEWL) index in mice indicated that the model group treated with DNCB and the TJ0113 protection group showed a significant increase before drug intervention, indicating successful model establishment. With prolonged administration time and increased dosage, the TJ0113 protection group exhibited reduced skin water loss, suggesting a gradually enhanced protective effect. As shown in FIG. 37, the stratum corneum hydration (SCH) index in mice demonstrated that the model group treated with DNCB and the TJ0113 protection group showed a significant decrease before drug intervention, indicating successful model establishment. With prolonged administration time and increased dosage, the TJ0113 protection group exhibited increased stratum corneum hydration, suggesting a gradually enhanced protective effect. The above results indicate that TJ0113 has a significant therapeutic effect on eczema.

**Example 17: Treatment of Non-Alcoholic Steatohepatitis (NASH)**

Male SD rats weighing 200-300 g were randomly divided into the following four groups: a model control group (Control, n = 10), a TJ01-013 3 mg/kg treatment group (3 mg/kg, n = 10), a TJ01-013 10 mg/kg treatment group (10 mg/kg, n = 10), and a TJ01-013 30 mg/kg treatment group (30 mg/kg, n = 10). The model group and the treatment groups were fed a choline-deficient/high-fat diet (CD/HFD). After continuous feeding for 4 weeks, intraperitoneal injection of sodium nitrite was performed once a week for three consecutive weeks to induce steatohepatitis and liver fibrosis symptoms. The administration cycle started from the first intraperitoneal injection of sodium nitrite, with continuous oral gavage for 8 weeks, once daily. The model group was synchronously administered with a vehicle (0.5% CMC-Na) via oral gavage. At the endpoint, liver tissues were collected for histopathological scoring (Non-Alcoholic Steatohepatitis NAS score).

Tissue embedding, slicing, and Masson staining were performed as follows:

(a) Tissue embedding

ethanol dehydration: dehydrating the tissues successively in ethanol solutions of varying concentrations (75%, 85%, 95%, 100%, 100%), each for 40 minutes; transparentizing: immersing the tissues sequentially in three xylene baths, each for 30 minutes; wax impregnation: immersing the tissues sequentially in three paraffin baths, with the first bath for 1 hour, the second for 1.5 hours, and the third for 2 hours; and embedding: pouring liquid paraffin into a mold box, then placing the wax-impregnated tissue block flat at the bottom, with the cut surface facing downward, removing an embedding frame after the paraffin was solidified, trimming the wax block after the paraffin was cooled and hardened completely, and retaining an appropriate amount of paraffin around the tissues for sectioning.

(b) Slice preparation

The pre-cooled wax block was fixed on a paraffin microtome, the cut surface of the wax block was kept parallel to the blade edge which was typically at a tilt angle of 15 degrees, and a rotary thruster was rotated to adjust the section thickness to 3 μm to produce uniformly thick sections. With a brush held in a left hand, the handle of the microtome was rotated by a right hand. After the section was discharged, it was gently lifted up with the brush, picked up by forceps and placed face-up in a slide box, in which the water temperature was around 40°C. The section was flattened and then removed. To mount the section, one end of a slide was held with the left hand and vertically submerged in water to attach the section, while the forceps were held in the right hand to assist in positioning the section onto two-thirds of the slide. After mounting, the slide was air-dried slightly and placed on a slide warmer at 60°C for 1 hour, followed by baking in an oven for 2 hours.

(c) Dewaxing of paraffin sections

The paraffin sections were sequentially placed in xylene I for 15 min, xylene II for 15 min, absolute ethanol I for 10 min, absolute ethanol II for 10 min, 95% alcohol for 10 min, and 85% alcohol for 10 min, for gradient dewaxing.

(d) Masson staining

Paraffin sections were stained with hematoxylin for 5-10 minutes, rinsed with tap water, then stained with a Masson's ponceau acid fuchsin solution for 5-10 minutes, briefly immersed in 2% acetic acid aqueous solution, differentiated with 1% phosphomolybdic acid for 3-5 minutes, directly stained with aniline blue for 5 minutes without water washing, and briefly immersed in 0.2% acetic acid aqueous solution.

(e) Dehydration, mounting, and image acquisition

The paraffin sections were sequentially placed in 75% ethanol for 2 min, 85% ethanol for 2 min, absolute ethanol for 2 min, absolute ethanol for 2 min, and xylene for 2 min for transparentizing. The section was then removed from xylene and mounted to the slide with neutral balsam. Microscopic examination and image acquisition analysis.

Tissue embedding, slicing, and Masson staining were performed as follows:

The tissues were embedded in an embedding medium for frozen slicing, with the section thickness of approximately 6-10 μm. After brief washing with distilled water, these sections were rinsed in 60% isopropanol for 20-30 seconds, immersed in an oil red solution for 5-10 minutes for staining, rinsed with 60% isopropanol to remove the excess staining solution, and washed with distilled water; nuclei were lightly stained with Mayer's hematoxylin; the sections were differentiated with 1% hydrochloric acid alcohol; and the sections were rinsed with running water for 10 minutes, mounted with a mounting medium, and examined under a microscope.

From the results shown in FIG. 38, the TJ01-013 treatment alleviated steatosis in liver tissues; and as shown in FIGs. 39 and 40, the TJ01-013 treatment reduced the inflammation and fibrosis levels in liver tissues. The above experimental results indicate that the TJ01-013 treatment alleviate the NASH symptoms to some extent.

**Example 18 Treatment of Cholecystitis and Liver Injury**

Male SD rats weighing 200-300 g were randomly divided into five groups: a normal control group (Sham, n = 10), a model control group (Vehicle, n = 10), a TJ01-013 3 mg/kg treatment group (3 mg/kg, n = 10), a TJ01-013 10 mg/kg treatment group (10 mg/kg, n = 10), and a TJ01-013 30 mg/kg treatment group (30 mg/kg, n = 10). The model establishment was performed using bacterial infection combined with common bile duct ligation as follows. After the rats in the model group and the treatment groups were anesthetized with ether, the abdominal cavity was opened to expose the gallbladder, bile was aspirated using a 1 mL syringe, the common bile duct was ligated, and bacteria (0.1 mL, Escherichia coli 1*104 CFU/mL) were injected above the ligation site. The puncture site was ligated, and the abdomen was closed to complete the model establishment. Purulent inflammatory reactions were formed within one week. The sham operation group was injected with sterile normal saline into the common bile duct without ligation. The administration period started on the day of model establishment. The treatment groups received different doses of TJ0113 daily via oral gavage for 10 consecutive days, and the model group received a vehicle (0.5% CMC-Na) daily via oral gavage. At the endpoint, blood was collected via dissection, and tested for the levels of aspartate aminotransferase (AST), alanine aminotransferase (ALT), and total bilirubin (TBIL).

From the results shown in FIG. 41, compared to the sham operation group, the model group exhibited a significant increase in the liver-related biochemical indicators ALT, AST, and TBIL, and after the TJ01-013 treatment, the levels of ALT, AST, and TBIL decreased, with the 10 mg/kg treatment group showing a significant difference, which indicates that the TJ01-013 treatment alleviated the liver damage caused by cholecystitis.

**Example 19: Treatment of Age-related Macular Degeneration**

C57BL/6J mice aged 4 months were randomly divided into four groups: a normal control group (normal, n = 10), a model control group (Control, n = 10), an I-14-1 10 mg/kg treatment group (3 mg/kg, n = 10), and a TJ01-013 10 mg/kg treatment group (10 mg/kg, n = 10). A mouse model of age-related macular degeneration was established by combining chronic light exposure and hydroquinone feeding. The specific model establishment method was as follows. Mice in the model group and the treatment group were placed in an LED light exposure device with a wavelength of 400-750 nm; the light intensity was measured using a lux meter to ensure that the light intensity measured at the animal's head level was up to 2500 lx, with 12 hours of daily light exposure; meanwhile, the mice were fed with a diet based on a pure synthetic formulation containing hydroquinone at 8 g/(kg·bw). The mice in the normal group were fed with the same formulation diet without hydroquinone, and were maintained under normal circadian rhythm conditions. The animals were raised for a total of 3.5 months, with the administration period starting from 1.5 months after model establishment and lasting for 2 months. The treatment groups received daily oral gavage of 10 mg/kg TJ01-013 and I-14-1, and the model group received daily oral gavage oof a vehicle (0.5% CMC-Na). At the endpoint, changes in the retinal function and structure were observed for the mice in each group using electroretinogram (ERG), optical microscopy, and electron microscopy; retinal cell apoptosis

was observed using the TUNEL method; and the expression and distribution of vascular endothelial growth factor (VEGF) and CD31 in the retina and choroid were detected by immunofluorescence.

Table 1. Comparison of electroretinogram (ERG) test results among groups

| Group | Dark-adapted 0.01 ERG (μV) | Dark-adapted 3.0 ERG (μV) | Dark-adapted 3.0 oscillatory potentials (μV) | Light-adapted 3.0 ERG (μV) | Flashing light response (μV) |
|---|---|---|---|---|---|
| Normal | 224.60±24.78 | 361.50±57.18 | 566.04±138.2 1 | 144.01±21.37 | 168.07±35.71 |
| Control | 116.00±17.03 | 139.10±28.25 | 291.20±59.55 | 46.09±8.91 | 49.78±15.09 |
| I-14-1 | 190.32±57.44 | 206.70±52.02 | 379.00±83.12 | 96.70±28.50 | 106.22±38.09 |
| TJ01-01 3 | 183.03±68.41 | 269.37±69.21 | 463.60±91.32 | 112.04±37.91 | 105.76±50.63 |

The optical microscopic observation of the retinal structure showed that in the normal group, the layers of the retina were arranged in an orderly manner, with a uniform cell form and a continuous and neat retinal pigment epithelial layer. Compared with the normal group, the model group exhibited looser arrangement of retinal layers, reduced photoreceptor cells (compared to the normal group), atrophic changes in the retinal pigment epithelial layer, disrupted Bruch's membrane structure, ingrowth of vascular-like tissues, partial thickening and thinning, and a significant decrease in photoreceptor cell count as compared to the normal group. The TJ01-013 and I-14-1 treatment groups showed varying degrees of improvement in the above conditions.

The electron microscopic observation of the retina revealed that for in the normal group, the photoreceptor cell discs of the mice were clearly structured and neatly arranged, with abundant mitochondria in pigment epithelial cells, numerous and long apical microvilli, and a regular Bruch's membrane structure with uniform thickness. In the model group, the photoreceptor cell discs of the mice were loosely deformed, showing changes such as separation, fragmentation, and pyknosis. The pigment granules in the retinal pigment epithelial cells were reduced, the apical microvilli were sparser and shorter compared to the normal group, deposits were observed beneath the retinal pigment epithelial cells (RPE), and the structure of the Bruch's membrane was irregular with uneven thickness, partial interruptions, and endothelial cell ingrowth. The TJ01-013 and 1-14-1 treatment groups showed varying degrees of improvement in the above conditions.

The results indicate that both 1-14-1 and TJ01-013 can alleviate the age-related macular degeneration in the model mice as induced by chronic light exposure and hydroquinone feeding, including improving retinal photoreceptor function and restoring normal retinal structure, with TJ01-013 being significantly more effective than 1-14-1.

**Example 20: Treatment of Retinitis Pigmentosa and Delaying its Progression**

1. Model establishment

Pde6a D670G/D670G mice aged 16 weeks were adaptively fed for 7 days. The pathogenic mechanism of this mouse model involved a reduction or failure in the hydrolysis rate of cyclic guanosine monophosphate (cGMP), leading to excessive accumulation of cGMPs in cells and thus a permanently open state of the CNG channels, resulting in the death of rod cells. The mice randomly divided into three groups based on body weight, namely, a model group (Control, orally gavaged with 0.5% CMC-Na, n = 8), a TJ01-013 treatment group (TJ01-013, orally gavaged with 30 mg/kg TJ01-013, n = 8), and an 1-14-1 treatment group (I-14-1, orally gavaged with 30 mg/kg 1-14-1, n = 8). An additional normal control group was set up using normal C57BL/6J mice (WT, treated with 0.5% CMC-Na via oral gavage, n = 8) at the same age. In this experiment, the administration was conducted for 8 weeks, after which the detection was conducted by confocal laser scanning ophthalmoscopy imaging and retinal immunofluorescence staining.

2. Confocal laser scanning ophthalmoscopy imaging

After anesthesia, the mice were positioned using a custom-made bottle cap to ensure the anterior part of their heads remained relaxed during imaging. Throughout the imaging process, a hydrating gel was used to maintain the mouse cornea in a constantly moist state. With the Spectralis infrared mode, large retinal vessels were located and focused, and the handle was pushed forward to achieve uniform illumination for the fundus image in the field of view. The fundus retinal images centered on the optic nerves were acquired. Following retinal scanning, the acquired images were statistically analyzed using ImageJ software.

As shown in FIG. 42, the thickness of the photoreceptor cell layer was measured and comparatively analyzed. Compared with the control group, the model group exhibited a significant decrease in the thickness of the photoreceptor

cell layer, with a statistically significant difference. After treatment with TJ01-013 and 1-14-1, the thickness of the photoreceptor cell layer significantly increased, both showing statistically significant differences, and TJ01-013 showed a therapeutic effect significantly superior to that of 1-14-1.

3. Retinal immunofluorescence staining

At the experimental endpoint, the mice were euthanized to harvest their eyeballs, which were then placed in 4% paraformaldehyde. After soaking for 5 minutes, the mouse eyeballs were dissected under a microscope, the anterior part of each eyeball was removed, and the retina was extracted and spread in a 96-well cell culture plate and soaked in PBST three times, each for 15 minutes, followed by addition of 4% paraformaldehyde solution for 20-min fixation and soaking in PBST three times, each for 10 minutes. The GFAP antibody was diluted in a certain amount of PBST solution according to the instructions, and the mixture was added dropwise into the 96-well cell culture plate and incubated overnight at 4°C. After washing with PBST, Hoechst was added, and the retina was incubated at room temperature for 15 minutes. After washing with PBST, the retina was transferred to a pre-treated glass slide. With forceps, the retinal ganglion cell side was placed facing the glass slide, and the photoreceptor cell layer was positioned away from the glass slide, with the entire retina spread out. A drop of anti-fluorescence mounting medium was added to the retinal section on the glass slide, covered with a coverslip, and care was taken to avoid air bubbles. Observation was performed using a confocal microscope.

As shown in FIG. 43, the retinal microglia density in the retinitis pigmentosa (RP) mouse model was significantly higher compared to the control group, with a statistically significant difference. After treatment with TJ01-013 and 1-14-1, the retinal microglia density significantly decreased, and both TJ01-013 and 1-14-1 showed statistically significant differences, with TJ01-013 showing a therapeutic effect significantly superior to that of 1-14-1.

**Experimental Example 21: Treatment of Multiple Sclerosis**

Multiple sclerosis (MS) is an autoimmune demyelinating disease with a complex pathogenesis that affects the central nervous system. This disease is characterized by multiple lesions, a relapsing-remitting course and a high disability rate, severely affecting the normal life of patients. Its pathogenesis remains unclear and may be caused by a combination of various factors such as immunity, environment and genetics. The currently accepted pathogenesis involves abnormal immune attacks that lead to damage to the blood-brain barrier, allowing various inflammatory factors and immune cells to enter the central nervous system and act on microglia and immune cells in the central nervous system to trigger inflammatory responses that result in myelin loss, neuronal death and axonal damage. Current therapies for multiple sclerosis primarily focus on anti-inflammation, which is, however, only effective during the relapsing and remitting phases of the disease. Therefore, the development of a therapy for multiple sclerosis has become an urgent issue to be addressed.

A CUP model can be used to simulate multiple sclerosis, in which case demyelination symptoms are induced in mice through dietary supplementation with CUP. CUP is a copper chelator that is added to the animals' diet through dietary supplementation, ultimately causing oligodendrocyte apoptosis and leading to demyelination.

1. Model establishment

C57BL/6 female mice aged 7-8 weeks were adaptively fed for 1 week, and then randomly divided into four groups based on body weight: a normal control group (Control, treated with 0.5% CMC-Na by oral gavage, n = 8), a model group (model, treated with 0.5% CMC-Na by oral gavage, n = 8), a TJ01-013 treatment group (TJ01-013, treated with 30 mg/kg TJ01-013 by oral gavage, n = 8), and an I-14-1 treatment group (I-14-1, treated with 30 mg/kg I-14-1 by oral gavage, n = 8). The normal control group was fed with basal feed throughout for 12 weeks, while the model group, the TJ01-013 treatment group, and the 1-14-1 treatment group were fed with 0.2% CUP diet throughout. The administration was started in all groups 6 weeks after the model establishment, for a total of 12 weeks, with a dosing volume of 5 ml/kg.

2. Behavioral test

(1) Elevated plus maze test

An elevated plus maze consists of two open arms (35 cm×6 cm) and two closed arms (35 cm×6 cm×14 cm), as well as a central area (6 cm×6 cm) elevated 70cm above the ground. Mice were placed in the central area facing the open arms. Prior to testing, the mice were allowed to explore a 50cm×50cm open field for 5 minutes. During testing, the time spent in open arms, distance traveled in open arms, and number of entries into open arms of the mice were recorded. The maze was wiped with 75% alcohol after each mouse test. From the results shown in FIG. 44, the number of entries in open arms for the mice in the model group was significantly increased as compared to the control group.

After treatment with TJ01-013 and 1-14-1, the number of entries of the mice into open arms was significantly reduced, showing statistically significant differences, and TJ01-013 showed a therapeutic effect significantly superior to that of 1-14-1. As shown in FIG. 45, compared with the control group, the distance for the mice to travel in the open arms was significantly increased in the model group, and after treatment with TJ01-013 and 1-14-1, the distance for the mice to travel in the open arms was significantly reduced, with TJ01-013 showing a therapeutic effect significantly superior to that of 1-14-1 and demonstrating statistically significant difference. As shown in FIG. 46, compared with the control group, the time for the mice to enter the open arms was significantly increased in the model group, and after treatment with TJ01-013 and 1-14-1, the time for the mice to enter the open arms was significantly reduced, showing statistically significant differences, and TJ01-013 showed a therapeutic effect significantly superior to that of I-14-1..

(2) Water maze test

The mice were placed in a basin having a diameter of 70 cm and a height of 30 cm, with water temperature maintained at approximately 22-24°C. The basin was divided into four quadrants, and a platform with a diameter of 5 cm was placed in the quadrant of interest, submerged 1 cm below the water surface. Mice were acclimated for 1 day and trained for 4 days before the formal test. During training, the mice were placed in the water facing a basin wall and given 60 seconds to locate a platform. If unsuccessful, they were guided on the the platform for a 30-second learning period. The time taken by mice to find the platform during training was recorded. On the test day, the platform was removed, and the number of platform crossings by the mice after entering from the quadrant opposite to the target quadrant was recorded to assess the spatial learning and memory abilities of the mice. As shown in FIG. 47, the number of platform crossings by the model group was significantly reduced compared with the normal group. After treatment with TJ01-013 and 1-14-1, the number of platform crossings by the mice increased, and TJ01-013 showed a therapeutic effect significantly superior to that of 1-14-1.

**Experimental Example 22: Treatment of Hypothyroidism**

Hypothyroidism, also called underactive thyroid disease, is a pathological condition where the thyroid hormone functions in the tissues are insufficient or absent. It is more common in women than in men, and its prevalence increases with age. Modern medicine recognizes that hypothyroidism can be caused by various factors, with the majority of cases resulting from autoimmune thyroiditis, radioactive iodine treatment of the thyroid, or thyroid surgery. According to the modern medicine, the thyroid hormone replacement therapy is typically adopted for patients with hypothyroidism. However, long-term use of thyroid hormones can directly stimulate osteoclasts to enhance bone resorption, leading to risks such as osteoporosis, coronary heart disease, and heart failure, among others. Hence, it has become an urgent task to find a safer and more effective therapy for the prevention and treatment of hypothyroidism.

(1) Model establishment

SD rats aged 7-8 weeks were adaptively fed for 1 week, and then randomly divided into four groups based on body weight: a normal control group (Control, treated with 0.5% CMC-Na by oral gavage, n = 8), a model group (model, treated with 0.5% CMC-Na by oral gavage, n = 8), a TJ01-013 treatment group (TJ01-013, treated with 30 mg/kg TJ01-013 by oral gavage, n = 8), and an 1-14-1 treatment group (I-14-1, treated with 30 mg/kg 1-14-1 by oral gavage, n = 8). The model group, the TJ01-013 treatment group, and the I-14-1 treatment group were intragastrically administered 0.1% propylthiouracil for 15 consecutive days, while the normal control group was intragastrically administered 0.1% normal saline at a dose of 10ml/kg. After model establishment, blood was collected from the eyeballs and centrifuged at 3,000rpm to isolate serum, which was tested to assess the thyroid function and determine the success of the model establishment. After four weeks of continuous treatment, administration was terminated, blood was collected, and serum was separated to measure the levels of triiodothyronine (T3), tetraiodothyronine (T4), free triiodothyronine (FT3), free tetraiodothyronine (FT4), thyroid-stimulating hormone (TSH), and IgG.

(2) Determination of serum hormones

The enzyme-linked immunosorbent assay (ELISA) was used, which is based on the principle of immobilizing antigens or antibodies and labeling the antigens or antibodies with enzymes. During the assay, the test specimen reacted with the antigens or antibodies on the surface of a solid-phase carrier; washing was conducted to separate antigen-antibody complexes formed on the solid-phase carrier from other substances; and enzyme-labeled antigens or antibodies were then added, which also bound to the solid-phase carrier through reaction. After addition of the substrate for the enzymatic reaction, the substrate is catalyzed by the enzyme into a colored product, the amount of which is directly correlated with the quantity of the substance being tested in the specimen. Quantitative analysis was performed using a

microplate reader.

From the results shown in FIG. 48, the model group exhibited significantly decreased levels of T3 and T4 as compared to the normal control group; and after treatment with TJ01-013 and 1-14-1, the levels of T3 and T4 increased, with TJ01-013 showing a therapeutic effect significantly superior to that of 1-14-1, exhibiting a statistically significant difference. As shown in FIG. 49, the model group exhibited significantly decreased levels of FT3 and FT4 in serum as compared to the normal control group; and after treatment with TJ01-013 and 1-14-1, the levels of FT3 and FT4 increased, with TJ01-013 showing a therapeutic effect significantly superior to that of I-14-1. As shown in FIG. 50, the model group exhibited significantly increased levels of FSH in serum as compared to the normal control group; and after treatment with TJ01-013 and 1-14-1, the level of FSH decreased, with significant differences, with TJ01-013 showing a therapeutic effect significantly superior to that of 1-14-1. As shown in FIG. 51, the model group exhibited significantly increased levels of IgG in serum as compared to the normal control group; and after treatment with TJ01-013 and 1-14-1, the level of IgG decreased, with statistically significant differences, and TJ01-013 showed a therapeutic effect significantly superior to that of I-14-1.

**Experimental Example 23: Treatment of Atrophic Gastritis**

Chronic atrophic gastritis (CAG) is a common clinical disease with incidence and detection rates increasing with age. It refers to a chronic gastric disorder characterized by repeated damage to the gastric mucosal epithelium, resulting in a reduction of intrinsic glands, with or without intestinal metaplasia or pseudo-pyloric metaplasia. Generally, it presents no specific symptoms, and those with symptoms mainly manifest as non-specific dyspeptic symptoms such as epigastric fullness and pain, which may be accompanied by belching, loss of appetite, acid reflux, gastric discomfort, bitter taste, nausea and other symptoms. These symptoms often recur. Currently, there is no effective drug to treat and alleviate all clinical symptoms.

(1) Model establishment

SD rats aged 7-8 weeks were adaptively fed for 1 week, and then randomly divided into four groups based on body weight: a normal control group (Control, treated with 0.5% CMC-Na by oral gavage, n = 8), a model group (model, treated with 0.5% CMC-Na by oral gavage, n = 8), a TJ01-013 treatment group (TJ01-013, treated with 30 mg/kg TJ01-013 by oral gavage, n = 8), and an I-14-1 treatment group (I-14-1, treated with 30 mg/kg 1-14-1 by oral gavage, n = 8). Hp strains were used to establish the model, where frozen Helicobacter pylori (Hp) was taken from a -80°C freezer, slowly thawed at room temperature, pipetted and mixed for homogenization, cultured on a Columbia blood agar medium until reaching the logarithmic growth phase, and then prepared into an Hp suspension using normal saline, and the Hp suspension was adjusted to $1\times10^{12}$cfu/ml by turbidimetry.

After 1 week of adaptive feeding, the rats were randomly divided into 2 groups: a normal control group (Control, treated with 0.5% CMC-Na by oral gavage, n = 8) and a model group (24 rats). The control group was given normal diet and water, while the model group was subjected to a multi-factor composite model establishment method. The rats were allowed to freely drink 120 mg/L MNNG solution daily and fed with feed containing 0.03 g/kg ranitidine. After 2 days of feeding, the rats were fasted for 1 day. On the first day of subsequent feeding, the rats were given 15% NaCl by gavage, and on the second day, they were given 10 ml/kg MNNG solution by gavage. On the fasting day, the rats were given 40% ethanol by gavage. After continuous model establishment until the 6th week, the model group was fasted for 12 h and then given formulated antibiotics (amoxicillin + metronidazole) by gavage for 3 consecutive days to eliminate gastric bacteria. On the 4th day, they were pretreated with indomethacin. Every other day, each rat was given 2 ml of 50 g/L sodium bicarbonate solution by gavage, followed by 1 ml of Helicobacter pylori suspension by gavage 15 minutes later, once daily for a total of 6 times. After successful model establishment, the rats were divided into three groups: a model group (model, treated with 0.5% CMC-Na by oral gavage, n = 8), a TJ01-013 treatment group (TJ01-013, treated with 30 mg/kg TJ01-013 by oral gavage, n = 8), and an 1-14-1 treatment group (1-14-1, treated with 30 mg/kg 1-14-1 by oral gavage, n = 8). Administration was initiated and continued for 8 weeks. After the pharmaceutical intervention, blood was collected from the heart, and serum was extracted. The levels of interleukin-1$\beta$ (IL-1$\beta$), interleukin-6 (IL-6), tumor necrosis factor-$\alpha$ (TNF-$\alpha$), pepsinogen I (PGI), pepsinogen II (PGII), and gastrin (GAS) in the serum of rats in each group were detected by enzyme-linked immunosorbent assay (ELISA).

As shown in FIG. 52, the model group exhibited significantly increased levels of Il-1$\beta$, IL-6 and TNF-$\alpha$ in serum as compared to the normal control group; and both the TJ01-013 treatment and the I-14-1 treatment reduced the levels of Il-1$\beta$, IL-6 and TNF-$\alpha$ in serum, showing a significant difference, and TJ01-013 showed a therapeutic effect significantly superior to that of I-14-1.

As shown in FIG. 53, the model group exhibited significantly decreased levels of PGI, PGII and GAS in serum as compared to the normal control group; and both the TJ01-013 treatment and the 1-14-1 teratment increased the level of PGI, PGII and GAS in serum, showing statistically significant differences, and TJ01-013 showed a therapeutic effect

significantly superior to that of I-14-1.

**Experimental Example 24: Treatment of Myocarditis**

Myocarditis refers to an acute, subacute or chronic inflammatory lesion that is localized or diffuse in the myocardium. It commonly affects children and young adults. Persistent cases may lead to dilated cardiomyopathy and chronic heart failure. Myocarditis can be classified into three types: idiopathic, autoimmune, and infectious. In clinical practice, myocarditis is treated primarily by focusing on intervening in the early acute inflammatory phase. Once it progresses to the chronic phase, there are no specific treatment methods available, and the main treatment approach is a conservative therapy and involves reducing the burden on the heart.

(1) Model establishment

Male SPF-grade Lewis rats aged 8 weeks were adaptively fed for 1 week to establish an autoimmune myocarditis model. The rats were divided into two groups: a normal control group (normal rats, conventional feeding) and a model group. In the model group, porcine myocardial myoglobin (for autoimmune induction) and the Freund's adjuvant were fully mixed at 1:1 to obtain an immunogenic mixture, and then a total of 0.2 ml of the immunogenic mixture was injected subcutaneously at multiple sites of the rats, including the bilateral groins and axillas, where the immunogenic mixture was a homogeneous emulsion prepared by dissolving purified porcine myocardial myoglobin in a PBS solution at a concentration controlled to be 10g/L, and under sterile conditions, the mixture was mixed with a complete Freund's adjuvant (CFA) at 1:1 by volume and emulsified thoroughly. In the normal control group, the rats were immunized with a PBS and CFA emulsion without myoglobin. From the 14th day after immunization, grouping and administration were conducted. There were three groups, namely, a model group (model, treated with 0.5% CMC-Na by oral gavage, n = 8), a TJ01-013 treatment group (TJ01-013, treated with 30 mg/kg TJ01-013 by oral gavage, n = 8), and an I-14-1 treatment group (I-14-1, treated with 30 mg/kg 1-14-1 by oral gavage, n = 8). An additional normal control group (Control, treated with 0.5% CMC-Na via oral gavage, n = 8) was set up. Administration began immediately after grouping and lasted for 4 weeks. After administration, the rats were weighed and anesthetized, and blood was collected to extract serum for detection of corresponding physiological indices. Following euthanasia, the rat's hearts were excised and weighed to calculate the cardiac index.

(2) Echocardiogram for cardiac function assessment

After the administration was completed, the rats were weighed and anesthetized via intraperitoneal injection of 10% chloral hydrate. After the hair on the chest of each rat was removed, echocardiography was performed in a supine position slightly to the left, with measurements repeated three times and averaged. The detected indices included left ventricular internal diameter at end-diastole (LVIDd), left ventricular internal diameter at end-systole (LVIDs), fractional shortening (FS), and ejection fraction (EF). From the results shown in FIG. 54, the model group exhibited significantly increased LVIDd and LVIDs after immunization model establishment as compared to the normal control group; and both the TJ01-013 treatment and the I-14-1 treatment reduced the levels of LVIDd and LVIDs, with TJ01-013 showing a therapeutic effect significantly superior to that of 1-14-1. As shown in FIG. 56, the model group exhibited significantly decreased EF and FS after immunization model establishment as compared to the normal control group; and both the TJ01-013 treatment and the 1-14-1 treatment reduced the levels of EF and FS, with TJ01-013 showing a therapeutic effect significantly superior to that of I-14-1. As shown in FIG. 56, the model group exhibited significantly increased level of CK-MB after immunization model establishment as compared to the normal control group; and both the TJ01-013 treatment and the 1-14-1 treatment reduced the level of CK-MB, with TJ01-013 showing a therapeutic effect significantly superior to that of I-14-1.

**Experimental Example 25: Treatment of Alopecia**

Alopecia refers to the loss of scalp hair, which is caused by the autoimmune deficiency in the body. Seborrheic alopecia, alopecia areata and androgenetic alopecia are currently recognized as the three most prevalent types of alopecia. Due to changes in mental stress and dietary habits, seborrheic alopecia has become the predominant type of hair loss today. Minoxidil and finasteride are currently recognized as the drugs for treating seborrheic alopecia. However, during the administration of these two drugs, significant side effects may occur in the human body, such as sexual dysfunction during the use of finasteride, and allergic dermatitis, myocardial infarction and anorexia as adverse reactions to topical minoxidil application. Hence, the search for a drug with good safety and high biological activity against hair loss has become a current priority.

(1) Alopecia areata model establishment

Healthy male C3H/HeJ mice aged 6-8 weeks were selected. After one week of adaptive feeding, imiquimod was used for model establishment. The model establishment involved topical application of imiquimod at different sites and different time intervals. Each time, the mice were carefully removed, and approximately 0.05 g of imiquimod ointment was applied evenly to the neck skin of each mouse using a clean medical cotton swab, covering an area of about 1.5×1.5 cm2. The application was performed three times a week. Before each administration, the local area was cleaned with saline to remove residual drug from the previous application. During the operation, actions were kept as gentle as possible to ensure even application, avoiding skin scratches or mechanical hair loss caused by repeated friction. During the experiment, the formation of an alopecia areata area being larger than 1×1 cm2 at an administration site indicated successful model establishment, after which grouping was conducted. There were three groups, including: a model group (model, orally gavaged with 0.5% CMC-Na, n = 8), a TJ01-013 treatment group (TJ01-013, orally gavaged with 30 mg/kg TJ01-013, n = 8), and an I-14-1 treatment group (I-14-1, orally gavaged with 30 mg/kg 1-14-1, n = 8). An additional normal control group (Control, treated with 0.5% CMC-Na via oral gavage, n = 8) was set up, with Vaseline applied to the neck skin. After grouping, administration began and continued for 4 weeks. After the administration was completed, the mice were euthanized, and the skin tissue from the mouse's neck area where obvious patchy alopecia areata had formed was removed using ophthalmic surgical scissors, and then stored in 4% paraformaldehyde and in a -80°C refrigerator. The skin tissue of each experimental mouse as fixed in 4% paraformaldehyde was retrieved, washed, dehydrated, and transparentized, then embedded in paraffin, followed by microtome slicing and staining.

The staining results were judged as follows: the presence of brown-yellow granules in the cell membrane or cytoplasm was considered positive. Under a high power microscope (400×), the field of view was sequentially moved, and five fields were randomly selected. The total number of positive infiltrating cells and the number of hair follicles in these five fields was counted. The mean value of the total number of positive cells divided by the number of hair follicles was taken as the number of positive cell infiltrations per hair follicle in the section. That is: number of positive infiltrating cells per hair follicle = total number of positive infiltrating cells/number of hair follicles. The statistical results of anti-CD4 and anti-CD8 immunohistochemical staining were shown in FIG. 57. During the stable phase after the formation of alopecia areata induced by imiquimod in model mice, the number of infiltrating CD4+ T and CD8+ T cells in the skin significantly increased, particularly around the hair follicles, hair bulbs, and blood vessels in the dermal matrix segment, with a higher number of infiltrating CD4+ T cells. Both the TJ0113 treatment and the IL-14-1 treatment significantly reduced the numbers of CD4+ T and CD8+ T cells, with TJ01-013 showing a superior therapeutic effect to that of I-14-1.

(2) Measurement of levels of IFN-γ and TNF-α in skin tissuesby ELISA

Frozen skin tissue samples were homogenized and then centrifuged at 12,000g for 10 minutes to collect a supernatant, and the levels of IFN-γ and TNF-α in mouse skin tissues were quantified using a mouse IFN-γ quantitative detection kit. From the detection results shown in FIG. 58, the model group exhibited significantly increased levels of IFN-γ and TNF-α in skin tissues as compared to the normal control group. Both the TJ0113 treatment and the I-14-1 treatment reduced the levels of IFN-γ and TNF-α after administration, with the TJ0113 treatment showing a significant difference, indicating its superior therapeutic effect over I-14-1.

## Experimental Example 26: Treatment of Hyperaldosteronism

(1) Model establishment

Healthy male SD rats aged 6-8 weeks were adaptively fed for one week before model establishment. After anesthesia with 20% urethane via intraperitoneal injection, the dorsal scapular region of each rat was depilated and disinfected. A micro-osmotic pump containing an aldosterone solution was subcutaneously implanted for continuous administration over 4 weeks. Grouping were conducted after successful model establishment. There were three groups, including: a model group (model, orally gavaged with 0.5% CMC-Na, n = 8), TJ01-013 treatment group (TJ01-013, orally gavaged with 30 mg/kg TJ01-013, n = 8), and an 1-14-1 treatment group (I-14-1, orally gavaged with 30 mg/kg I-14-1, n = 8). An additional normal control group (Control, treated with 0.5% CMC-Na via oral gavage, n = 8) was set up, followed by administrationfor 4 consecutive weeks, after which the rats were anesthetized with 20% urethane, the abdominal wall skin was incised to expose and isolate the inferior vena cava and collect venous blood for monitoring the serum concentrations of K+ and Na+, PAC, and PRA.

From the results shown in FIG. 59, compared with the normal control group, the model group effected significant sodium retention and potassium excretion, as well as significantly increased levels of sodium ions and significantly decreased levels of potassium ions, in the serum. After treatment with TJ0113 and I-14-1, the symptoms of sodium retention and potassium excretion were ameliorated, the level of sodium ions was reduced, and the level of potassium ions

was simultaneously increased. Moreover, TJ01-013 showed a therapeutic effect significantly superior to that of 1-14-1. As shown in FIG. 60, the model group exhibited significantly increased levels of PAC as compared to the normal control group, and after treatment with TJ0113 and 1-14-1, the level of PAC was reduced; and the model group exhibited significantly decreased levels of PRA rate as compared to the normal control group, and after treatment with TJ0113 and 1-14-1, the level of PRA rate was increased; and TJ01-013 showed a therapeutic effect significantly superior to that of 1-14-1.

**Experimental Example 27: Treatment of Erectile Dysfunction**

Erectile dysfunction, one of the most common diseases in andrology, refers to the persistent (at least 6 months) inability of the pennis to achieve and maintain sufficient erection for satisfactory sexual intercourse, and it is characterized primarily by the inability of the pennis to achieve or sustain erection and often accompanied by symptoms such as decreased libido and premature ejaculation. Epidemiological data indicate that approximately 52% of men aged 40-70 suffer from varying degrees of erectile dysfunction all over the world.

(1) Model establishment

Normal male SD rats aged 6 weeks were selected. After one week of adaptive feeding, the rats were fasted for 24 h, and then randomly divided into three groups according to a random number table, with their body weights recorded. The rats were anesthetized by intraperitoneal injection of pentobarbital sodium at a dose of 40 mg/kg. The rats were fixed on an operating table, and a midline longitudinal abdominal incision was made to expose the abdominal cavity; the left renal vein, left adrenal vein, and inferior vena cava were separately dissected; a syringe needle with the diameter of 0.8 mm was placed between the left adrenal vein and the inferior vena cava; at the intersection of the left renal vein and the inferior vena cava, the needle was placed between the left renal vein and the inferior vena cava using a 4-0 silk thread, followed by ligation and need removal; the abdominal cavity was rinsed with 0.9% sodium chloride injection and treated with penicillin sodium for anti-infection; and then the incision was sutured in sequence. Penicillin sodium (200,000 units/time) was injected to prevent infection, which was postoperatively administered for 3 consecutive days. Grouping and administration were commenced after successful model establishment. There were 3 groups, including: a model group (model, orally gavaged with 0.5% CMC-Na, n = 8), TJ01-013 treatment group (TJ01-013, orally gavaged with 30 mg/kg TJ01-013, n = 8), and an I-14-1 treatment group (I-14-1, orally gavaged with 30 mg/kg I-14-1, n = 8). An additional normal control group (Control, treated with 0.5% CMC-Na via oral gavage, n = 8) was set up, followed by administration for 12 weeks. After administration, the penile erectile function was tested, in which case the rats were euthanatized, venous blood was collected, centrifuged at 12,000g for 10min to collect a supernatant for testing of the serum levels of sex hormones testosterone (T), luteinizing hormone (LH), and follicle-stimulating hormone (FSH). The rats in each group were dissected to measure the weight of the bilateral kidneys and testes and the inner diameter of the left spermatic vein.

(2) Observation method for penile erectile function

Apomorphine (APO) was injected one day before the experiment and at the end of the 12th week, respectively, to test the penile erectile function of rats in each group. The rats from each group were placed into observation cages and allowed to adapt for 15 minutes with dimmed lighting and a quiet environment. APO was injected into the loose skin on the back of the neck of each rat, after which the rats were immediately placed back to the cages. The number of penile erections and the number of licking the glans and penis within 30 minutes were observed and recorded. Counting criteria: Congestion of the glans and appearance of penile erection at the distal end were denoted as one erection; and prolonged licking of the glans or penis was also denoted as one erection. As shown in FIG. 61, the model group exhibited significantly fewer penile erections as compared to the normal group, with a statistically significant difference. After treatment with TJ0113 and 1-14-1, the number of penile erections was significantly increased; and TJ01-013 showed a therapeutic effect significantly superior to that of I-14-1.

After euthanasia, the rats in each group were dissected to measure the weight of the bilateral kidneys and testes and the inner diameter of the left spermatic vein. As shown in FIG. 62, compared with the normal group, the model group showed no change in the kidney weight of rats but significantly reduced testicular weight, with a statistically significant difference. After treatment with TJ0113 and 1-14-1, there was no significant effect on the kidney weight, but the testicular weight was significantly increased; and TJ01-013 showed a therapeutic effect significantly superior to that of I-14-1.

As shown in FIG. 63, the model group exhibited significantly reduced inner diameter of the left spermatic vein of rats as compared to the normal group, with a statistically significant difference. After treatment with TJ0113 and 1-14-1, the inner diameter of the left spermatic vein was significantly increased; and TJ01-013 showed a therapeutic effect significantly superior to that of I-14-1.

The detection results of serum hormone levels were shown in FIG. 64. Compared with the normal group, the model group exhibited significantly increased levels of luteinizing hormone (LH) and follicle-stimulating hormone (FSH) in

the rat serum, while the levels of sex hormone testosterone (T) were significantly decreased, showing statistically significant differences. After treatment with TJ0113 and 1-14-1, the levels of luteinizing hormone (LH) and follicle-stimulating hormone (FSH) in serum were reduced, the level of sex hormone testosterone (T) was increased, and the TJ01-013 treatment showed a significant difference, indicating that J01-013 had a therapeutic effect significantly superior to that of 1-14-1.

**Experimental Example 28: Treatment of Periodontitis**

Periodontitis is a chronic inflammatory disease that damages the integrity of tooth-supporting tissues. Severe periodontitis not only leads to tooth loss but also increases the risk of systemic diseases such as atherosclerosis, rheumatoid arthritis, aspiration pneumonia, and cancer. The current effective means for treating periodontitis involves mechanical removal of local irritants. However, some special tooth structures, such as the root bifurcation area, may restrict the penetration of instruments, preventing the accumulated plaques from being effectively removed.

(1) Model establishment

SPF male C57BL/6 mice aged 8-11 weekds were adaptively feed them for one week for periodontitis model establishment. The mice were anesthetized by intraperitoneal injection of pentobarbital sodium, and then placed in pre-treated 50 ml centrifuge tubes; a 5-0 silk thread with the length of 2.5 mm was knotted at both ends; the mouse oral cavity was opened with a mouth opener; and the silk thread was placed between the first and second maxillary molars on the right side of each mouse using toothed forceps. Grouping and administration were commenced seven days after model establishment. There were 3 groups in total, including: a model group (model, orally gavaged with 0.5% CMC-Na, n = 8), TJ01-013 treatment group (TJ01-013, orally gavaged with 30 mg/kg TJ01-013, n = 8), and an I-14-1 treatment group (I-14-1, orally gavaged with 30 mg/kg 1-14-1, n = 8). An additional normal control group (Control, treated with 0.5% CMC-Na via oral gavage, n = 8) was set up, followed by administration for 8 weeks, and the mice were euthanized at the end of administration.

(2) Detection of changes in alveolar bone height in each group by Micro-CT scanning

The specific method was as follows: euthanizing the mice, quickly isolating the right maxillary tissue, fixing it with 4% paraformaldehyde for 24h, performing three-dimensional reconstruction after Micro-CT scanning of the tissue, measuring the distance from the cementoenamel junction (CEJ) to the alveolar bone crest (ABC) at the distal root of the first molar and the mesial root of the second molar on the buccal side of the right maxilla, and observing changes in alveolar bone height in each group.

As shown in FIG. 65, after Micro-CT scanning and three-dimensional reconstruction of mouse tissue, the CEJ-ABC value at the distal root of the first molar and the mesial root of the second molar on the buccal side of the right maxilla was measured. The results showed that compared with the normal control group, the model group exhibited a significantly increased CEJ-ABC value, indicating successful establishment of the periodontitis mouse model. After treatment with both TJ0113 and 1-14-1, the CEJ-ABC value was significantly increased, indicating the therapeutic effects of TJ0113 and 1-14-1, and TJ01-013 showed a therapeutic effect significantly superior to that of I-14-1.

**Experimental Example 29: Treatment of Cerebral Stroke**

Cerebral stroke is a disease that severely endangers human health, with a globally increasing incidence year by year. It is also the disease with the highest disability rate in China, imposing a serious burden on the society and the economy. Post-cerebral stroke rehabilitation is currently a critical and challenging issue that urgently needs to be addressed in clinical practice.

Cerebral stroke is a severe cerebrovascular disease and is also one of the leading causes of long-term disability and premature death. Most (approximately 80%) of the cerebral stroke cases are ischemic strokes caused by vascular occlusion, which is primarily due to arterial thrombosis. At present, the most effective method for treating ischemic stroke is rapid restoration of blood supply. However, ischemia-reperfusion injury following revascularization may further exacerbate brain damage and neuromotor dysfunction, making neurological function recovery a critical issue that needs urgent resolution.

(1) Model establishment

A middle cerebral artery occlusion (MCAO) model is a focal cerebral ischemia model and also the most widely used cerebral ischemia model. This model has the pathogenesis similar to that of cerebral ischemic stroke, with the

features of simple operation and accurate control in reperfusion. The middle cerebral artery (MCA) supplies the largest blood flow area, and its occlusion primarily leads to cortical and striatal lesions. The extent of infarction depends on the location and duration of occlusion, as well as the collateral circulation blood volume of the MCA.

Male SD rats aged 7-8 weeks were selected and adaptively fed for 1 week to establish a rat middle cerebral artery occlusion model. After inducing anesthesia in rats using a small animal anesthesia machine, maintenance anesthesia was immediately initiated; the rats were fixed in a supine position on the operating table, and the midline of the neck and the right surgical area were shaved; after disinfection with alcohol and iodophor, a midline incision was made in the neck; and under a microscope, the carotid artery was carefully isolated along the tissue to avoid damaging blood vessels and nerves and to minimize bleeding. Small arterial branches were cut off using an electrocoagulation pen, and the external carotid artery was ligated, with a loose knot tied for future use. The proximal ends of the internal carotid artery and common carotid artery were clipped using arterial clips. The external carotid artery was cut off at a 45° angle using surgical scissors, and a suture thread was carefully inserted, and a marker point reaching the bifurcation of the internal and external carotid arteries indicated that the suture thread head had reached the middle cerebral artery. The skin was sutured, and symptoms were observed after the rats woke up to evaluate the neurological function. Two hours after surgery, the suture was cut and removed, and the residual end of the external carotid artery was tightly ligated. The skin was re-sutured, and attention should be paid to the postoperative care of the rats. Grouping and administration were commenced after model establishment. There were 3 groups in total, including: a model group (model, orally gavaged with 0.5% CMC-Na, n = 8), TJ01-013 treatment group (TJ01-013, orally gavaged with 30 mg/kg TJ01-013, n = 8), and an 1-14-1 treatment group (I-14-1, orally gavaged with 30 mg/kg I-14-1, n = 8). An additional normal control group (Control, treated with 0.5% CMC-Na via oral gavage, n = 8) was set up, followed by corresponding surgical procedures without carotid artery ligation or suture insertion, and administration for 8 consecutive weeks, after which the cognitive function of the rats was assessed using the water maze.

The cerebral ischemic stroke leads to insufficient perfusion of brain tissue, making it prone to ischemia and hypoxia during surgery. The massive accumulation of calcium ions in the brain tissue can damage the brain, resulting in postoperative cognitive decline.

(2) Water maze test

On the evening before the experiment, the laboratory and water maze were cleaned, the original water was drained from the water maze, animal excrement should be removed thoroughly, and the laboratory should be well-ventilated and free of unusual odors. The water maze was filled with clean water, with the water level maintained approximately 2 cm above the platform. All four groups of rats were trained for 5 days to adapt to the water maze. The above training consisted of a 4-days place navigation test and a 1-day spatial probe test. The water maze apparatus was a circular pool divided equally into quadrants I, II, III, and IV by four equidistant points on the pool wall. A circular hidden platform was placed at the center of quadrant IV. The pool was surrounded by fixed reference objects, and both the pool and platform were black. Black ink was poured into the water and thoroughly mixed to obscure the circular platform from the rats. The water temperature was maintained at 22-24°C, and the indoor lighting was stable. For each group of rats, the water maze test was conducted after administration to assess the cognitive function of the experimental animals. Each time, the rat was placed into the pool facing the wall from one of the three quadrants other than the quadrant containing the platform. If the rat failed to locate the hidden platform beneath the water surface in the target quadrant within 2 minutes, it was guided to the platform in the target quadrant and allowed to stay for 15 seconds. The rats in the four groups underwent this training for 4 days, with 4 sessions per day (2 in the morning and 2 in the afternoon). After each day's training, the experimenter thoroughly cleaned the water maze apparatus to prevent residual rat odors from affecting the results of the experiment on the following day. The time taken from placing each rat into the water facing the wall until it reached the hidden platform in the target quadrant was recorded and defined as the escape latency, with the average of four results taken. A 60-second time limit was defined for each navigation experiment. If the rat failed to locate the hidden platform in the target quadrant within the defined time, the experimenter guided the rat to the hidden platform, aiming to make the rat stay on the platform for 15 seconds to reinforce memory. The escape latency for that test herein was denoted as 60 seconds. Experimental preparations included towels and a hair dryer. After each swimming session, the rat was dried with the towel and then with the hair dryer to prevent colds. The experiment was conducted in a quiet environment to minimize disturbances. As shown in FIG. 66, the model group exhibited a significant increase in escape latency as compared to the normal control group, and after treatment with both TJ0113 and 1-14-1, the escape latency was shortened, indicating that TJ0113 and 1-14-1 had therapeutic effects, with TJ01-013 showing a significantly superior therapeutic effect to that of 1-14-1.

After the above experiments were completed, the platform was removed for the spatial exploration test. Each rat was placed into the water pool facing the pool wall at the midpoint of the quadrant opposite to the circular hidden platform, the rat was allowed to swim in the pool for 60 seconds, and the number of times that the rat crossed the location of the hidden circular platform in the center of the target quadrant within 60 seconds was denoted as the number of platform

crossings. As shown in FIG. 67, the model group exhibited a significant decrease in the number of platform crossings as compared to the normal control group; after treatment with both TJ0113 and 1-14-1, increased the number of platform crossings was reduced, with TJ01-013 showing a significant difference in shortening the escape latency, indicating that TJ0113 and I-14-1 had therapeutic effects, and TJ01-013 showed a therapeutic effect significantly superior to that of 1-14-1.

The apoptosis rate of hippocampal neurons in rats was measured by flow cytometry.

After the administration was completed and behavioral experiments were performed, the rats in all groups were intraperitoneally injected with chloral hydrate and then sacrificed; and the rats were quickly decapitated to remove the brain, and the hippocampal tissues were rapidly isolated on ice. Approximately 1g of hippocampal tissue was weighed and placed on a 100-mesh copper net; the surface connective tissue membrane was cut off; a buffer solution was added; the hippocampal tissue was minced, and gently rubbed with forceps and filtered to prepare a single-cell suspension; the cell suspension was then filtered a second time through a 300-mesh nylon net to remove large clumps; the collected cell suspension was centrifuged in a centrifuge for 5 minutes at 3000 r/min with a centrifugation radius of 10 cm and a centrifugal force of 13,000g, after discarding the supernatant, 500 μl of 1X buffer solution was added to prepare a single-cell suspension with a concentration of 1×105~5×105/L; then, 5 μl of Annexin V was first added, followed by the addition of 10 μl of propidium iodide (PI); and the mixture was thoroughly mixed and then incubated in the dark for 5 minutes. The operation was performed by referring to the steps and instructions in the manual in combining with practical experience. The fluorescence intensity of apoptotic hippocampal neurons was detected by flow cytometry, and the apoptosis rate of hippocampal neurons was calculated. As shown in FIG. 68, the model group exhibited a significant increase in the apoptosis rate of hippocampal neurons as compared to the normal control group; and after treatment with both TJ0113 and 1-14-1, the apoptosis rate of hippocampal neurons was significantly reduced, indicating that TJ0113 and I-14-1 had therapeutic effects, with TJ01-013 showing a therapeutic effect significantly superior to that of I-14-1.

### Experimental Example 30: Treatment of Epilepsy

Epilepsy, one of the most common neurological system diseases in clinical practice, is a chronic brain dysfunction caused by abnormal electrical activity in the brain, with the clinical manifestation as recurrent epileptic seizures. In addition to the harms caused by the epileptic seizures themselves, cognitive impairment is one of the most common complications in epilepsy patients with recurrent seizures. These patients may experience difficulties in learning and memory, as well as significant reductions in their ability to solve problems and form concepts, etc. Furthermore, although currently available antiepileptic drugs are effective in controlling the epileptic seizures for many epilepsy patients, these drugs may also impair the cognition and behavior of epilepsy patients by upregulating inhibitory neurotransmitters or suppressing neuronal excitability. Hence, it is highly beneficial and urgent for epilepsy patients to develop a drug to improve cognitive impairment in epilepsy patients, or even a drug that both enhances cognition and alleviate epileptic seizures.

Clean-grade male ICR mice aged 8-10 weeks were selected for the preparation of chronic epilepsy mouse models. The mice were randomly divided into four groups: a normal control group (Control, treated with 0.5% CMC-Na by oral gavage, n = 8), a model group (Model, treated with 0.5% CMC-Na by oral gavage, n = 8), a TJ01-013 treatment group (TJ01-013, treated with 30 mg/kg TJ01-013 by oral gavage, n = 8), and an I-14-1 treatment group (I-14-1, treated with 30 mg/kg 1-14-1 by oral gavage, n = 8). Starting from the first day of the experiment, the mice in all groups, except for the normal control group, were intraperitoneally injected with PTZ at 40 mg/kg between 10-11 AM daily, while the normal control group was injected with an equivalent volume of normal saline, each for a total of 21 days. Administration began immediately after model establishment was initiated, with one dose administered daily for a total of 21 days. Corresponding tests were conducted after the completion of administration.

(1) Animal behavioral observation

The grading standard for epileptic seizures was based on the Racine scale: Grade 0, no convulsive response, and normal behavioral state; Grade I, facial muscle spasms, manifested as rhythmic chewing, blinking, whisker movement, etc.; Grade II, neck muscle spasms, manifested as head nodding with or without tail erection; Grade III, forelimb clonus or rhythmic twitching; Grade IV, hindlimb stiffness or stiff standing; and Grade V, generalized clonus, stiff standing and falling, with loss of balance. After the experiment was completed, animal behaviors were observed, with the results shown in FIG. 69. The normal control group exhibited no epileptic seizures, and the model group exhibited Grade V epileptic seizures. Both the TJ0113 treatment and the I-14-1 treatment reduced the seizure grade after administration, with TJ0113 showing a significant difference in reducing epileptic seizures, indicating that the therapeutic effect of TJ01-013 is significantly superior to that of I-14-1.

(2) Step-down test

The step-down test is an experimental method designed based on the instinct of laboratory mice to immediately step down from an elevated platform to explore the surrounding environment, where the experimental mice are placed on a high platform, and an electrical stimulus is administered at the moment they step down. Subsequently, the mice will avoid stepping down from the platform to evade the electrical stimulus, whereas mice with weaker learning and memory abilities will retain their instinct and step down. After each round of the experiment, 75% alcohol is sprayed to wiped away odor interference between the mice. This experiment is conducted at the end of administration. The experimental apparatus consists of a cubic box with a bottom (10 cm × 10 cm) electric grid and a height of 30 cm, containing a platform with the height of 3.2 cm and the diameter of 4.2. The experimental procedures and processes of this experiment are as follows. (1) The mice were placed at the bottom of the experimental apparatus without electrification, allowing them to freely explore and adapt to the environment for 10 s. (2) The mice were placed on the elevated platform within the apparatus, the time taken for the mice to step down was recorded as initial latency, and an electrical stimulus (50 Hz, 20 V, 5 s) was administered. (3) After 24 h, the mice were placed on the platform again, and the time taken for the mice to step down was recorded and recorded as step-down latency. The time for the mice to stay on the elevated platform should not exceed 60 s, or else, still recorded as 60 s. From the results shown in FIG. 70, the post-stimulation latency was significantly decreased in the model group as compared to the normal control group, indicating cognitive dysfunction in the mice of the model group. Both the TJ0113 treatment and the I-14-1 treatment increased the latency to some extent after administration, with TJ0113 showing a significant difference, indicating that the therapeutic effect of TJ01-013 is significantly superior to that of I-14-1.

(3) Step-through test

The step-through test is an experimental method designed based on the tendency of mice to prefer dark environments over bright ones, where the experimental mice are placed in the bright compartment of an apparatus with connected bright and dark compartments, and an electric stimulus is administered when the mice enter the dark compartment. Consequently, the mice will avoid the electric stimulus by staying in the bright compartment, whereas mice with weaker learning and memory abilities will maintain their natural tendency and enter the dark compartment. After each round of the experiment, 75% alcohol is sprayed to wipe away odor interference between mice. This experiment is conducted at the end of administration. The experimental procedures and processes of this experiment are as follows. (1) Each mouse was placed in a bright compartment with its back facing a dark compartment, the time taken for the mouse to enter the dark compartment was denoted as initial latency; and the small door was closed, and electrical stimulation (50 Hz, 20 V, 5 s) was administered to the mouse. (2) After 24 hours, the mice were placed in the bright compartment again, and the time taken for the mice to enter the dark compartment was recorded as step-through latency. From the results shown in FIG. 71, the post-stimulation latency was significantly decreased in the model group as compared to the normal control group, indicating cognitive dysfunction in the mice of the model group. Both the TJ0113 treatment and the 1-14-1 treatment increased the latency to some extent after administration, with TJ0113 showing a significant difference, indicating that the therapeutic effect of TJ01-013 is significantly superior to that of I-14-1.

(4) After the experiment was completed, blood was collected from the mice to measure the levels of TNF-α and Il-1β in serum.

Twenty-four hours after the water maze test, the whiskers of the mice were trimmed, and the eyeballs were removed for blood collection. Approximately 0.5 mL of blood was collected into a centrifuge tubes, left to stand for 2 hours, and then centrifuged (3000 rpm/min, 10 min) to collect a supernatant, which was stored frozen for later use. The levels of TNF-α and IL-1β in serum were detected by ELISA.

Principle of ELISA: Samples, standards, and HRP-labeled detection antibodies were sequentially added to the pre-coated microplate wells containing mouse TNF-α and IL-1β capture antibodies, followed by incubation and thorough washing. Color development was performed using a substrate TMB, which turned blue under the catalysis of peroxidase and then converted into final yellow under the action of acid. The absorbance (OD value) was measured at 450 nm wavelength using a microplate reader, and the sample concentration was calculated. From the results shown in FIG. 72, the model group exhibited significantly increased levels of TNF-α and IL-1β in serum as compared to the normal control group, indicating elevated inflammatory levels in the serum of the model group. After treatment with both TJ0113 and 1-14-1, the levels of TNF-α and IL-1β in serum were reduced to some extent, and TJ01-013 showed a therapeutic effect significantly superior to that of I-14-1.

**Experimental Example 31: Treatment of Melasma**

Melasma is an acquired chromatodermatosis, typically manifested as symmetrical light brown to dark brown

patchy pigmentation on the face, which is classified under the category of " dusty facial complexion " in traditional Chinese medicine. It often occurs to women in the child-bearing period and is a clinically common yet difficult-to-cure cosmetic disease that severely impacts patients' mental health. With changes in living environments and improvements in living standards, melasma has increasingly become one of the most frequently discussed and researched diseases in both the medical and beauty fields nowadays.

The establishment of melasma animal models plays an important role in studying the etiology, pathological mechanisms, and treatment approaches of melasma. As the etiology and mechanisms of melasma are not very clear, there is no well-established method for establishing animal models. Currently, there are three commonly used methods for establishing melasma animal models at home and abroad: ultraviolet irradiation model establishment, progesterone injection model establishment , and model establishment based on ultraviolet irradiation combined with progesterone, with mice being the primary choice. The specific implementation method for ultraviolet irradiation model establishment involves depilating the modeling area of an experimental mouse and exposing it to medium-wave ultraviolet irradiation for 30-60 minutes daily, for 4-8 weeks. The specific implementation method for progesterone model establishment involves injecting a certain dose of progesterone into the hind leg root of an experimental mouse daily, for 4-8 weeks. However, some research results indicate that due to the regulation of hormones by the body's feedback mechanisms, the method for model establishment by inducing melasma with progesterone should not replicate animal models by blindly controlling the dosage. The method for model establishment based on ultraviolet irradiation combined with progesterone can simulate the multifactorial clinical characteristics of melasma, but may also increase influencing factors on the experiment.

In the melasma model establishment method, progesterone was injected to attack female mice to replicate melasma animal models. The model establishment was performed by adaptively feeding female C57BL/6J mice aged 8 weeks for one week, and then carrying out intramuscular injection at the thigh root at a dose of 20 mg/kg body weight (0.4% progesterone oil solution, 5 ml/kg), once daily, alternating on both sides, for a total of 28 days. Grouping and administration were commenced after model establishment. There were 3 groups in total, including: a model group (Model, orally gavaged with 0.5% CMC-Na, n = 8), TJ01-013 treatment group (TJ01-013, orally gavaged with 30 mg/kg TJ01-013, n = 8), and an I-14-1 treatment group (I-14-1, orally gavaged with 30 mg/kg I-14-1, n = 8). An additional normal control group (Control, treated with 0.5% CMC-Na via oral gavage, n = 8) was set up, with daily intramuscular injection of normal saline at 5ml/kg. Administration were conducted once daily for 8 weeks. At the end of the administration, the mice were euthanized, and the pathological morphological observation was conducted on the melanin-positive target skin MC using immunohistochemical labeling.

Pathological morphological observation of melanin-positive target skin using immunohistochemical labeling

After 28 days of model establishment, the mice were euthanized by cervical dislocation, and the central back skin was depilated using an 8% Na2S aqueous solution. A piece of hairless skin tissue (approximately 2cmx3cm) was excised, and a portion (approximately 2cm×1cm) was taken tehrefrom, fixed in 10% formalin, and routinely embedded in paraffin for slicing, followed by dewaxing to hydration. Melanin granules in the epidermis were visualized using the Strept Avidin-Biotin Complex immunohistochemical method, with HMB45 (melanoma) as the primary antibody, DAB for color development, and hematoxylin for counterstaining. In place of the primary antibody, PBS was used as a negative control, while a human skin tissue was used as the positive control. The distribution and secretory function of melanocytes in the epidermis were observed. Immunohistochemical steps: Routine paraffin slices were dewaxed to hydration, and incubated with 3% H2O2 deionized water for 5 min; the tissue slices were pretreated based on the primary antibody; the primary antibody was dropwise added, and the slice was incubated at 37°C for 1 h or at 4°C overnight, followed by PBS washing three times, for 2 min each; a ready-to-use SABC kit was dropwise added and incubated at 37°C for 30 min, followed by PBS washing three times, for 2 min each; DAB color development was performed for 5-10 min; the slice was washed with distilled water for 10-15 min; routine counterstaining was performed with hematoxylin for 5 seconds; routine dehydration, transparentizing and slide mounting were performed; and staining results were as follows: the positive appeared brown and was mainly distributed in the cytoplasm and cell membrane, with nuclei counterstained blue by the hematoxylin. With the skin of each mouse in each group, one pathological section was prepared; the BI-2000 computer image analysis system was used for quantitative analysis of target images; for each section, five non-overlapping fields were randomly observed under a 40× microscope objective, and color images were acquired and stored; the number of melanin-positive targets (melanin-positive cells distributed in the epidermis and adnexal epithelial cell cytoplasm, showing brown reaction), average target area, melanin area density (the ratio of target area to statistical field area), and number density (the ratio of target number to statistical field area) were calculated for the five fields of each section and each group of animals after model establishment. As shown in FIG. 73, the model group exhibited a significant increase in the number of melanin-positive cell targets and the melanin number density as compared to the normal control group, indicating successful model establishment. After treatment with both TJ0113 and I-14-1, the number of melanin-positive cell targets and the melanin number density were reduced to some extent, with TJ01-013 showing a therapeutic effect significantly superior to that of 1-14-1. As shown in FIG. 74, the model group exhibited a significantly increase in the target area of melanin-positive cells and the melanin

area density as compared to the normal control group, indicating successful model establishment. After treatment with both TJ0113 and 1-14-1, the target area of melanin-positive cells and the melanin area density were reduced to some extent, with TJ01-013 showing a therapeutic effect significantly superior to that of I-14-1.

**Experimental Example 32: Treatment of Vitiligo**

Vitiligo, a common pigmentary disorder in dermatology, is featured with white or milky-white localized spots or patches of varying sizes and shapes, with smooth surfaces, clearly defined borders and absence of itching or pain. It can occur in any part of the body but is most commonly seen on the face, neck, back, trunk, and external genitalia, and in severe cases, the hair may also turn white. Epidemiological surveys indicate that vitiligo typically begins in childhood or adolescence, with approximately half of vitiligo patients developing the condition before the age of 20, and its incidence gradually decreases with age. Clinical studies show that the prevalence of vitiligo is generally higher in individuals with darker skin tones, with the yellow race falling between the white race and the black race. In China, the prevalence of vitiligo in the Han population is 0.09%-2.7%, with an incidence rate of approximately 0.2%-1% in rural areas and about 0.3% in urban residents. There is no significant difference between males and females.

Model establishment:

Black or black-spotted guinea pigs weighed 300-350 g were adaptively fed for 1 week, and then, sodium sulfide was used to remove a 4×4 cm2 area of black skin on the back of each guinea pig. The depilated area was then treated with 5% H2O2, 1 mL each time, twice daily, until the hair of guinea pigs turned white and their skin became pale. After approximately 50 consecutive days, a vitiligo animal model was established. Grouping and administration were commenced after model establishment. There were 3 groups in total, including: a model group (Model, orally gavaged with 0.5% CMC-Na, n = 8), TJ01-013 treatment group (TJ01-013, orally gavaged with 30 mg/kg TJ01-013, n = 8), and an I-14-1 treatment group (I-14-1, orally gavaged with 30 mg/kg 1-14-1, n = 8). An additional normal control group (Control, treated with 0.5% CMC-Na via oral gavage, n = 8) was set up, with distilled water applied to the depilated area at 1 mL per application, twice daily. All groups received intragastric administration at a volume of 10ml/kg daily. Administration was conducted once daily for 8 weeks. After completion of administration, the guinea pigs were visually observed; and blood was collected from the guinea pigs via venous puncture to measure the levels of MDA and MAO in serum.

1. Visual observation: Upon completion of the experiment, hair depigmentation was assessed daily, and the depigmented areas were divided into treated and untreated regions, with each region scored out of 5 points, totaling 10 points per mouse. Scoring criteria for hair depigmentation in mice: 0 point for no depigmentation; 1 point for depigmentation area between 0% and 10% (0%~10%); 2 points for depigmentation area between 11% and 25% (11% ~25%); 3 points for depigmentation area between 26% and 50% (26%~50%); 4 points for depigmentation area between 51% and 75% (51%~75%); and 5 points for depigmentation area between 76% and 100% (76%~100%). Comparison of skin and hair whitening between the blank control group and the model group indicated the successful establishment of a vitiligo model in guinea pigs. The TJ01-013 treatment group exhibited brown-black skin with visible melanin pigmentation, significant repigmentation, and occasional small white spots; and the 1-14-1 treatment group showed brown-black skin with visible melanin pigmentation, noticeable repigmentation, and frequent small white spots. From the scoring results shown in FIG. 75, both the TJ01-013 treatment and the I-14-1 treatment improved the symptoms of vitiligo, with TJ01-013 exhibiting a superior therapeutic effect to that of I-14-1.

In the vitiligo model, the distribution of melanin in hair follicles is a primary factor, and the count of pigmented hair follicles per unit area directly reflects the distribution level of melanin in the skin. A piece of 1cm×1cm skin tissue was taken for HE staining, followed by histological observation of the skin tissue and hair follicle counting. Under a light microscope, 50 hair follicles were observed, and the proportion of hair follicles containing melanin was calculated. From the results shown in FIG. 76, the proportion of hair follicles containing melanin was significantly decreased in the model group as compared to the normal control group. Both TJ0113 and 1-14-1 increased the proportion of hair follicles containing melanin to a certain extent after administration, with TJ01-013 exhibiting a significantly superior therapeutic effect to that of I-14-1.

Serum index test: One hour after the last administration, the guinea pigs in each group were anesthetized with 20% urethane (0.5 ml per 100 g of body weight), and 3 ml of blood was collected from the abdominal aorta. The blood was added to heparin sodium anticoagulant tubes and centrifuged at 2,500 rpm/min for 10 minutes to separate the supernatant, which was then stored in a -4°C refrigerator. The contents of TYR and MDA in the serum were detected by ELISA. From the results shown in FIG. 77, the activity of tyrosinase TYR in the serum was significantly decreased in the model group as compared to the normal control group. Both TJ0113 and 1-14-1 increased the activity of tyrosinase to a certain extent after administration, with the TJ01-013 treatment showing a significant difference. Compared with the normal control group, the model group exhibited significantly increased levels of malondialdehyde MDA in the serum, and both

TJ0113 and I-14-1 reduced the levels of MDA to a certain extent after administration, with TJ01-013 showing a significant difference in therapeutic effect. The results indicated that the therapeutic effect of TJ01-013 was significantly superior to that of I-14-1.

**Experimental Example 33: β-Thalassemia**

β-Thalassemia, also known as Mediterranean anemia or globin synthesis disorder, is a hemolytic anemia disease caused by genetic mutations and is one of the most widespread monogenic genetic disorders globally. It is a hemolytic genetic disorder caused by reduced or absent synthesis of β-globin chains, leading to an imbalance between the α-chain/non-α-chain of hemoglobin tetramers. Microcytic hypochromic anemia with elevated HbF and HbA2 levels is its typical clinical feature. Currently, β-thalassemia is one of the most prevalent and severe genetic disorders in provinces south of the Yangtze River in China.

Model establishment: NCG-X mice are derived by introducing a W41 point mutation into the c-kit gene using the Cas9 technology based on an NCG severe immunodeficiency background model. This line is featured with T/B/NK cell immunodeficiency and hematopoietic stem cell function inhibition, making it an excellent model for human hematopoietic stem cell transplantation and thalassemia research.

NCG-X male mice aged 6-8 weeks were adaptively fed for 1 week, and then randomly divided into three groups: a model group (Model, treated with 0.5% CMC-Na by oral gavage, n = 8), a TJ01-013 treatment group (TJ01-013, treated with 30 mg/kg TJ01-013 by oral gavage, n = 8), and an I-14-1 treatment group (I-14-1, treated with 30 mg/kg 1-14-1 by oral gavage, n = 8). An addition normal control group (Control, treated with 0.5% CMC-Na via oral gavage, n = 8) was set up, where normal NCG mice were selected without any treatment. After grouping, administration was conducted for a total of 4 weeks. Upon completion of the administration, blood was collected from the eyeballs to detect the levels of peripheral blood RBC and Hb in the mice.

From the results shown in FIGs. 78 and 79, the model group exhibited significantly reduced levels of hemoglobin (Hb) and red blood cell count (RBC), and both the TJ01-013 treatment and the I-14-1 treatment increased the levels of Hb and RBC to some extent after administration, with the TJ01-013 treatment showing a significant difference. The results indicated that the therapeutic effect of TJ01-013 was significantly superior to that of 1-14-1.

**Experimental Example 34L Treatment of Lumbar Disc Herniation**

Lumbar disc herniation, the most prevalent disease in spinal surgery, is a common and frequently occurring clinical condition that imposes a heavy burden on the society and families. It is a syndrome results from degenerative changes in the lumbar intervertebral disc, rupture of the annulus fibrosus, and herniation of the nucleus pulposus, which irritates or compresses the nerve roots or cauda equina. It is a common and frequently occurring disease affecting human health and one of the primary causes of clinical pain in waist and lower extremities. With the aging of the population and changes in lifestyle in China, the incidence of intervertebral disc degenerative diseases has been increasing year by year. Current clinical therapies mainly include non-surgical therapies (such as steroidal anti-inflammatory drugs, physical therapy, and pain management) and surgical therapies, none of which can fundamentally prevent the occurrence and progression of lumbar disc herniation.

A normal intervertebral disc is located between two adjacent vertebral bodies and serves as a structure that maintains spinal height, bears loads, and disperses stress. The intervertebral disc consists of an outer layered annulus fibrosus, an inner gel-like nucleus pulposus, and upper and lower cartilage endplates, where the nucleus pulposus cells are involved in the synthesis and secretion of extracellular matrix and cytokines, which are crucial for maintaining the function of the intervertebral disc.

Model establishment: SD rats aged 7-8 weeks were adaptively fed for 1 week, and then subjected to the model establishment of intervertebral disc herniation. The rats were anesthetized by intraperitoneal injection of 2% sodium pentobarbital, with their limbs fixed. The back hair of the rats were removed with tissue scissors, followed by routine disinfection and laying of sterile sheets. The tails of the rats were tightened with rubber band, and under sterile conditions, two intervertebral discs were harvested from the 3rd and 4th caudal vertebrae of each rat. The central annulus fibrosus of the harvested intervertebral discs was punctured with a 10 ml needle to expose the nucleus pulposus so as to create a ruptured form, and then placed in physiological saline for later use. After routine disinfection of the back with iodophor, an incision of about 2cm long was made by centering on the L5 spinous process, the intervertebral disc was implanted into the muscle layer, and then suturing was conducted layer by layer; 800,000 units of penicillin sodium was intramuscularly injected; erythromycin ointment was applied to the wound, to establish a model successfully. After the rats recovered from anesthesia, they were placed in breeding cages, and intramuscularly injected with 800,000 units of penicillin sodium for 5 days after the operation, so as to prevent infection. After successful model establishment, the rats were divided into three groups, including: a model group (Model, orally gavaged with 0.5% CMC-Na, n = 8), , TJ01-013 treatment group (TJ01-013, orally gavaged with 30 mg/kg TJ01-013, n = 8), and an 1-14-1 treatment group (I-14-1, orally gavaged with

30 mg/kg 1-14-1, n = 8). An additional normal control group (Control, treated with 0.5% CMC-Na via oral gavage, n = 8) was set up, where SD rats only underwent surgical procedures without nucleus pulposus extraction and implantation. After grouping, administration was conducted for a total of 4 weeks. After the administration was completed, the rats were euthanized to extract the transplanted nucleus pulposus specimens from each group. The nucleus pulposus to be tested was weighed and homogenized with PBS on ice at a ratio of 1:9 to collect a supernatant, and the levels of phospholipase A2 (PLA2), IL-6, and TNF-$\alpha$ in the tissue homogenate were detected by ELISA. From the experimental results shown in FIG. 80, after 4 weeks of oral gavage administration in successfully modeled rats, the model group exhibited significantly increased activity of PLA2 in the nucleus pulposus tissue as compared to the normal control group. Both the TJ0113 treatment and the I-14-1 treatment reduced the activity of PLA2 in the nucleus pulposus tissues to some extent after administration, with the TJ01-013 treatment showing a significant difference. The results indicated that the therapeutic effect of TJ01-013 was significantly superior to that of 1-14-1. As shown in FIG. 81, after 4 weeks of oral gavage administration in successfully modeled rats, the model group exhibited significantly increased levels of inflammatory factors TNF-$\alpha$ and IL-6 in the nucleus pulposus tissue as compared to the normal control group. Both the TJ0113 treatment and the I-14-1 treatment reduced the levels of TNF-$\alpha$ and IL-6 in the nucleus pulposus tissues to some extent after administration, with the TJ01-013 treatment showing a significant difference. The results indicated that the therapeutic effect of TJ01-013 was significantly superior to that of 1-14-1.

**Experimental Example 35: Treatment of Polycystic Ovary Syndrome**

Polycystic ovary syndrome (PCOS) is a heterogeneous disorder affecting multiple systems throughout the body, primarily impacting the reproductive health of women in the child-bearing period, and it is one of the most significant causes of ovulatory infertility in women. The pathological manifestations of PCOS mainly include hormonal imbalances, impaired follicular development and ovulation disorders, abnormally elevated serum androgen levels, insulin resistance, and obesity. This condition not only affects the reproductive function in women but also significantly increases the risk of long-term complications such as type 2 diabetes, cardiovascular diseases, and endometrial cancer in affected patients.

Model establishment:

Female SD rats aged 8-9 weeks were numbered with picric acid and adaptively fed for 1 week. Letrozole (10 mg) was dissolved in 50 ml of physiological saline to prepare a 0.2 mg/ml letrozole gavage solution. The PCOS group was intragastrically administered 1 ml of letrozole gavage solution daily. The dosage for gavage was increased with the increase of the body weight, to achieve a final letrozole treatment concentration of 1 mg/kg/d, and the administration was conducted for 23 consecutive days. After model establishment, the rats were randomly divided into three groups, including: a model group (Model, orally gavaged with 0.5% CMC-Na, n = 8), , TJ01-013 treatment group (TJ01-013, orally gavaged with 30 mg/kg TJ01-013, n = 8), and an I-14-1 treatment group (I-14-1, orally gavaged with 30 mg/kg 1-14-1, n = 8). An additional normal control group (Control, treated with 0.5% CMC-Na via oral gavage, n = 8) was set up, followed by intragastric administration of 1 ml of normal saline daily for 23 consecutive days. The administration began after grouping, once daily for a total of 4 weeks.

12-Hour fasting and weighing were performed the day before the end of administration. After anesthesia with an appropriate amount of tribromoethanol based on body weight, the abdominal skin of each rat was thoroughly disinfected; the abdominal skin and peritoneum were incised layer by layer with scissors, and blood was collected from the abdominal aorta; and the blood was left at room temperature for 20 minutes and centrifuged at 3,000 rpm for 15 minutes to collect a supernatant, which was properly aliquoted and stored in a -80°C freezer. The viscera was dissected to locate the free bilateral ovaries, excess fat and fascia were removed from the ovarian surfaces, both ovaries were weighed, and the ovarian weight and ovarian index were determined. As shown in FIG. 82, the model group exhibited significantly upregulated ovarian weight as compared to the normal control group, and after treatment with both TJ0113 and 1-14-1, the ovarian weight was reduced to some extent, with TJ01-013 showing a therapeutic effect significantly superior to that of I-14-1.

Determination of serum hormone levels:

The concentrations of testosterone (T), estradiol (E2), luteinizing hormone (LH), and follicle-stimulating hormone (FSH) in rat serum were measured following the instructions of the enzyme-linked immunosorbent assay (ELISA) kit. From the results shown in FIG. 83, the model control group exhibited a significant decrease in the level of estradiol (E2) as compared to the normal control group, and both the TJ0113 treatment and the 1-14-1 treatment increased the level of E2 to some extent, with the TJ01-013 treatment showing a significant difference. As shown in FIG. 84, compared with the normal control group, the model control group exhibited a significant decrease in the levels of serum testosterone (T), luteinizing hormone (LH), and follicle-stimulating hormone (FSH), and both the TJ0113 treatment and the 1-14-1 treatment reduced

the levels of T, LH, and FSH to some extent, with TJ01-013 showing a significant difference, indicating that TJ01-013 has a therapeutic effect significantly superior to that of I-14-1.

Those skilled in the art will appreciate that the above embodiments provide specific examples for implementing the present invention. In practical applications, various modifications can be made in form and detail without departing from the spirit and scope of the invention.

## Claims

**1.** Use of a compound of Formula (I), or a pharmaceutically acceptable salt, stereoisomer, solvate or prodrug thereof, in treatment of and/or preparation of a medicament for treating at least one disease selected from the group consisting of: reproductive system diseases, autoimmune diseases, skin diseases, eye diseases, muscular or skeletal degenerative diseases, neurological diseases, anemia, adrenal diseases, chronic gastritis, myocarditis, periodontitis, hypothyroidism, alopecia, liver injury, tinnitus, and chronic obstructive pulmonary disease,

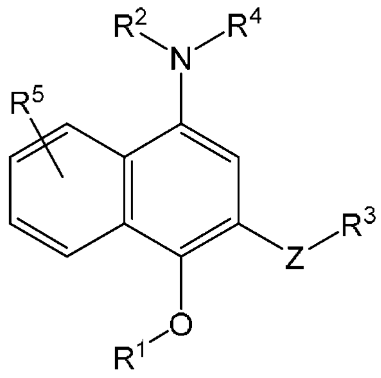

I

wherein Z is atom;

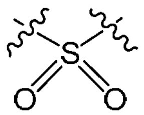, 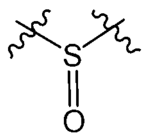, 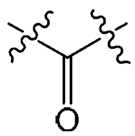, 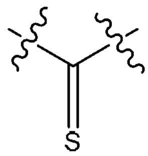

or a sulfur

$R^1$ is hydrogen, $C_{1\sim6}$ alkyl,

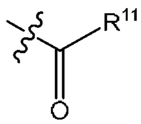, 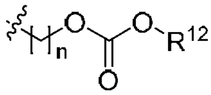,

wherein $R^{11}$ and $R^{12}$ are independently $C_{1\sim6}$ alkyl or $C_{1\sim6}$ cycloalkyl, respectively, and n is 1~4;

$R^2$ is hydrogen, $C_{1\sim6}$ alkyl, three- to six-membered cycloalkyl, three- to six-membered cyclooxyalkyl, phenyl, $C_{1\sim6}$ alkyl with at least one hydrogen substituted with $R^{2-1}$, phenyl with at least one hydrogen substituted with $R^{2-1}$, wherein $R^{2-1}$ is hydroxyl, halogen, amino, or $C_{1\sim6}$ alkoxy,

$R^3$ is

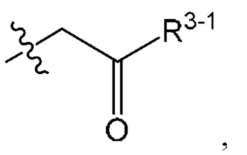, 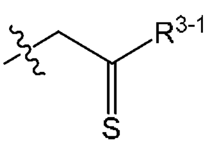 or 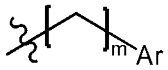;

wherein $R^{3-1}$ is hydrogen, hydroxyl, $C_{1\sim6}$ alkyl, $C_{1\sim6}$ alkoxy, $C_{3\sim6}$ cycloalkyl, three- to six-membered cyclooxyalkyl, amino, $C_{1\sim6}$ amine group, $-CH_2C(O)R^{3-2}$, $-CH_2C(O)OR^{3-2}$, $-CH_2C(O)N$ ($R^{3-2}$ $R^{3-2a}$), and $R^{3-2}$ and $R^{3-2a}$ are independently hydrogen, $C_{1\sim6}$ alkyl, or three- to six-membered cycloalkyl respectively,
m is 1 to 6, and
Ar is phenyl, naphthyl, 5- or 6-membered monocyclic heteroaryl, 8- to 10-membered fused bicyclic heteroaryl, phenyl with at least one hydrogen atom substituted with $R^{3-3}$, naphthylwith at least one hydrogen atom substituted with $R^{3-3}$, 5- or 6-membered monocyclic heteroaryl with at least one hydrogen atom substituted with $R^{3-3}$, 8- to 10-membered fused bicyclic heteroaryl with at least one hydrogen atom substituted with $R^{3-3}$, and the $R^{3-3}$ is hydrogen, halogen, $C_{1\sim6}$ alkyl, three- to six-membered cycloalkyl, hydroxy, $C_{1\sim6}$ alkoxy, three- to six-membered epoxyalkyl, $C_{1\sim6}$ haloalkyl, $C_{2\sim6}$ alkenyl, $C_{2\sim6}$ alkynyl, $-N(R^{3-3a}R^{3-3b})$ or phenyl,
$R^{3-3a}$ and $R^{3-3b}$ are independently hydrogen, $C_{1\sim6}$ alkyl, or three- to six-membered cycloalkyl, respectively;
$R^4$ is

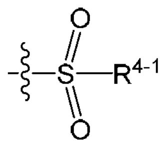 , 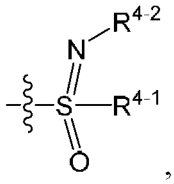 , 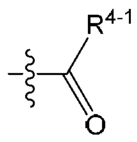 or 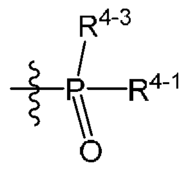 ,

wherein $R^{4-1}$ is phenyl, naphthyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and $R^{4-11}$ is hydrogen, halogen, nitro, nitrile, hydroxyl, $C_{1\sim6}$ alkyl, $C_{13\sim6}$ cycloalkyl, $C_{1\sim6}$ alkoxy, $-N(R^{4-1a}R^{4-1b})$, phenyl, $C_{1\sim6}$ haloalkyl, $C_{1\sim6}$ haloalkoxy, $-C(O)OR^{4-12}$, $-C(O)R^{4-12}$, $-C(O)N(R^{4-1a}R^{4-1b})$, $-S(O)_2R^{4-12}$, $-S(O)R^{4-12}$, $-OC(O)R^{4-12}$, $-OC(O)OR^{4-12}$ or

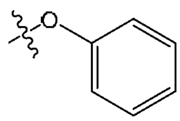 ,

$R^{4-12}$, R4$^{-1a}$ and $R^{4-1b}$ are independently hydrogen, $C_{1\sim6}$ alkyl, $C_{3\sim6}$ cycloalkyl, $C_{2\sim6}$ alkenyl, $C_{2\sim6}$ alkynyl, $C_{1\sim6}$ alkyl substituted with at least one hydrogen by a halogen, $C_{2\sim6}$ alkynyl with at least one hydrogen substituted with a halogen, $C_{3\sim6}$ cycloalkyl with at least one hydrogen substituted with a halogen, $C_{2\sim6}$ alkynyl with at least one hydrogen substituted with a halogen, respectively, and $R^{4-1a}$ and $R^{4-1b}$may be bonded to each other to form a ring,
$R^{4-2}$ is $C_{1\sim6}$ alkyl, $C_{3\sim6}$ cycloalkyl, $C_{1\sim6}$ alkyl with at least one hydrogen substituted with a hydroxyl group, $C_{3\sim6}$epoxyalkyl, or $R^{4-2}$ is bonded to $R^2$ to form a 4- to 8-membered ring when $R^2$ is $C_{1\sim6}$ alkyl and $R^{4-2}$ is $C_{1\sim6}$ alkyl,
$R^{4-3}$ is $C_{1\sim6}$ alkyl or $C_{1\sim6}$ alkoxy;
the number of $R^5$ is 0~5, and $R^5$, at each occurrence, is independently hydrogen, halogen, nitro, nitrile, $-N+(R^{5-1})_3$, $C_{1\sim6}$ haloalkyl, $-C(O)OR^{5-1}$,$-C(O)R^{5-1}$, $-C(O)N(R^{5-1}R^{5-1a})$, $-S(O)2R^{5-1}$, $-S(O)R^{5-1}$, $-S(O)2N(R^{5-1}R^{5-1a})$, $-S(O)N(R^{5-1}R^{5-1a})$, $-N=C(R^{5-1}$ $R^{5-1a})$, hydroxyl, $C_{1\sim6}$ alkyl, phenyl, phenyl with at least one hydrogen substituted with $R^{5-1}$, $C_{1\sim6}$ alkoxy, $-N(R^{5-1}R^{5-1a})$, $-N(R^{5-1})C(O)R^{5-1a}$, $-N(R^{5-1})C(O)OR^{5-1a}$, $-OC(O)R^{5-1}$, $-OC(O)OR^{5-1}$, $-OC(O)N(R^{5-1}R^{5-1a})$ or $-SR^{5-1}$, respectively, wherein $R^{5-1}$, $R^{5-1a}$ and $R^{5-1b}$are independently hydrogen, $C_{1\sim6}$ alkyl, $C_{2\sim6}$ alkenyl, $C_{2\sim6}$ alkynyl, $C_{1\sim6}$ alkywith at least one hydrogen substituted with a halogen, $C_{2\sim6}$ alkenylwith at least one hydrogen substituted with a halogenor $C_{2\sim6}$ alkynyl with at least one hydrogen substituted with a halogen, respectively.

**2.** The use according to claim 1, whereinthe Z is

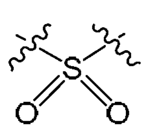 , 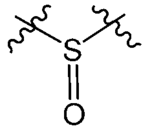 ,

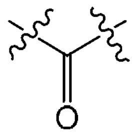, 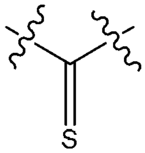

or a sulfur atom;

$R^1$ is hydrogen, $C_{1\sim4}$ alkyl,

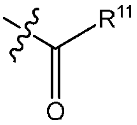 or 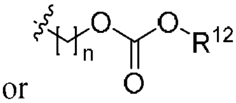,

wherein $R^{11}$ and $R^{12}$ are independently $C_{1\sim4}$ alkyl or $C_{1\sim4}$ cycloalkyl, respectively, and n is 1 or 2;
$R^2$ is hydrogen, $C_{1\sim4}$ alkyl, three- to six-membered cycloalkyl, three- to six-membered cyclooxyalkyl, phenyl, $C_{1\sim4}$ alkyl with at least one hydrogen substituted with $R^{2-1}$, and phenyl with at least one hydrogen substituted with $R^{2-1}$, wherein $R^{2-1}$ is hydroxy, halogen, amino, or $C_{1\sim4}$ alkoxy;
$R^3$ is

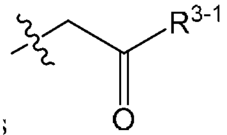, 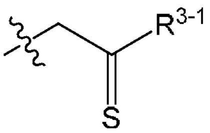 or 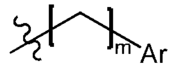;

wherein $R^{3-1}$ is hydrogen, hydroxyl, $C_{1\sim4}$ alkyl, $C_{1\sim4}$ alkoxy, $-N(R^{3-2}R^{3-2a})$,
$R^{3-2}$ and $R^{3-2a}$ are independently hydrogen or $C_{1\sim4}$ alkyl, respectively,
m is 1~4.
Ar is phenyl, 5- or 6-membered monocyclic heteroaryl, 5- or 6-membered monocyclic heteroaryl with at least one hydrogen atom substituted with $R^{3-3}$, the $R^{3-3}$ is hydrogen, halogen, $C_{1\sim4}$ alkyl, hydroxyl, $C_{1\sim4}$ alkoxy, $C_{1\sim4}$ haloalkyl, or $-N(R^{3-3a}R^{3-3b})$;
$R^{3-3a}$ and $R^{3-3b}$ are independently hydrogen or $C_{1\sim4}$ alkyl, respectively,
$R^4$ is

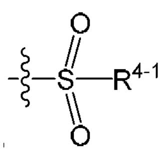 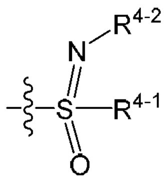 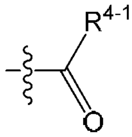 or 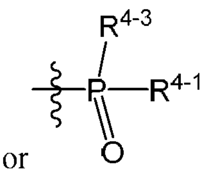

wherein $R^{4-1}$ is phenyl, phenyl with at least one hydrogen atom substituted with $R^{4-11}$ 5- or 6-membered monocyclic heteroaryl, 5- or 6-membered monocyclic heteroaryl with at least one hydrogen atom substituted with $R^{4-11}$, and the $R^{4-11}$ is hydrogen, halogen, nitro, $C_{1\sim4}$ alkyl, $C_{3\sim6}$ cycloalkyl, $C_{1\sim4}$ alkoxy, $-N(R^{4-1a}R^{4-1b})$ phenyl, $C_{1\sim4}$ haloalkyl, $C_{1\sim4}$ haloalkoxy or

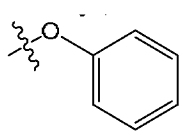,

$R^{4-1a}$ and $R^{4-1b}$ are independently hydrogen, $C_{1\sim4}$ alkyl, or $C_{3\sim6}$ cycloalkyl, respectively, and $R^{4-1a}$ and $R^{4-1b}$may be bonded to each other to form a ring,
$R^{4-2}$ is $C_{1\sim4}$ alkyl, $C_{3\sim5}$ cycloalkyl, $C_{1\sim4}$ alkyl with at least one hydrogen substituted with hydroxyl, $C_{3\sim5}$cyclooxyalkyl, or $R^{4-2}$ is bonded to $R^2$ to form a 4- to 8-membered ring when $R^2$ is $C_{1\sim4}$ alkyl and $R^{4-2}$ is $C_{1\sim4}$ alkyl,

$R^{4-3}$ is $C_{1\sim4}$ alkyl or $C_{1\sim4}$ alkoxy;
$R^5$,at each occurrence, is independently hydrogen, halogen, nitro, nitrile, -N+($R^{5-1}$)$_3$, $C_{1\sim4}$ haloalkyl,C(O)O$R^{5-1}$, -C(O)$R^{5-1}$, -C(O)N($R^{5-1}R^{5-1a}$), -S(O)$_2R^{5-1}$, -S(O)$R^{5-1}$, -N=C($R^{5-1}$ $R^{5-1a}$), hydroxyl, $C_{1\sim4}$ alkyl, phenyl, phenyl with at least one hydrogen substituted with $R^{5-1}$, $C_{1\sim4}$ alkoxy, -N($R^{5-1}R^{5-1a}$), -N($R^{5-1}$)C(O)$R^{5-1a}$, or -OC(O)$R^{5-1}$, respectively, wherein $R^{5-1}$, $R^{5-1a}$, and $R^{5-1b}$ are independently hydrogen, $C_{1\sim4}$ alkyl, $C_{1\sim4}$ alkyl with at least one hydrogen substituted with a halogen, respectively.

**3.** The use according to claim 1, whereinthe Z is

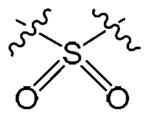 , 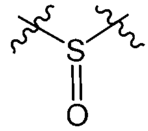 ,

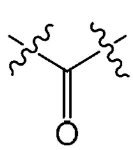 , 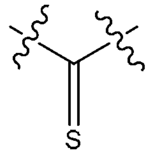

or a sulfur atom;

and/or, $R^1$ is hydrogen,

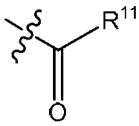 or 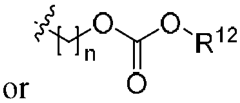 ,

wherein $R^{11}$ and $R^{12}$ are independently methyl, ethyl, n-propyl or isopropyl, respectively, and n is 1;
and/or, $R^2$ is hydrogen, methyl, ethyl, n-propyl, isopropyl, ethyl with one hydrogen substituted with hydroxyl, n-propyl with one hydrogen substituted with hydroxyl, phenyl,

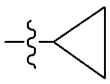 , 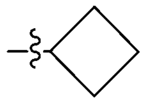 , 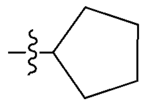 , 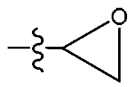 , 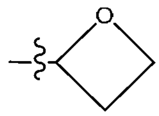 ,

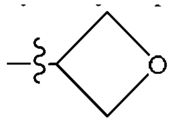 , 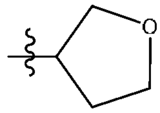 or 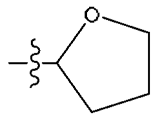 ;

and/or $R^3$ is

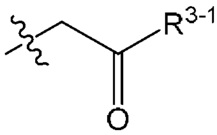 , 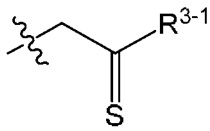 or 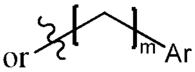 ;

wherein $R^{3-1}$ is hydrogen, hydroxyl, methyl, ethyl, n-propyl, isopropyl, methoxy, ethoxy, n-propoxy, isopropoxy, n-butoxy, tert-butoxy, sec-butoxy or isobutoxy,
m is 1,
Ar is phenyl, 5- or 6-membered nitrogen-containing monocyclic heteroaryl;
and/or $R^4$ is

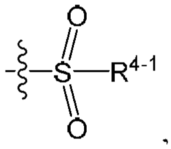, 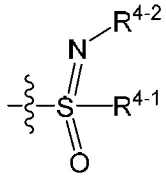, 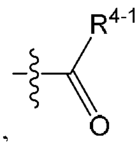 or 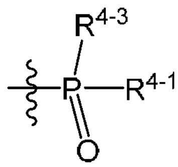,

wherein $R^{4-1}$ is phenyl, phenyl with at least one hydrogen substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and the $R^{4-11}$ is halogen, nitro, methyl, ethyl, n-propyl, isopropyl,

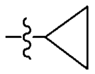, 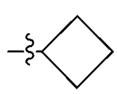, 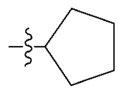, 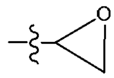, 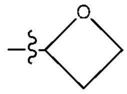, 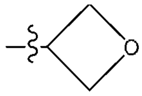,

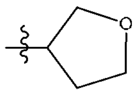, 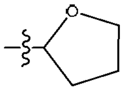, 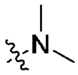, 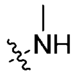, 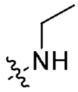, 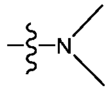, 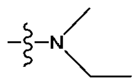, 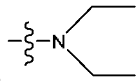, 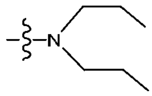,

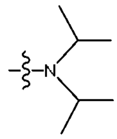, 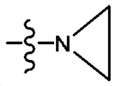, 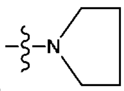, 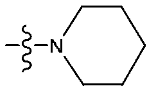,

fluoromethyl, fluoroethyl, fluoro-n-propyl, fluoroisopropyl, chloromethyl, chloroethyl, chloro-n-propyl, chloroisopropyl, bromomethyl, bromoethyl, bromo-n-propyl, bromoisopropyl, iodomethyl, iodoethyl, iodo-n-propyl, iodoisopropyl, fluoroethoxy, fluoroethoxy, fluoroorthopropoxy, fluoroisopropoxy, chloromethoxy, chloroethoxy, chloro-n-propoxy, chloroisopropoxy, bromomethoxy, bromoethoxy, bromo-n-propoxy, bromoisopropoxy, iodomethoxy, iodoethoxy, iodo-n-propoxy, iodoisopropoxy, or

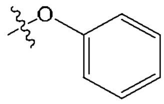,

$R^{4-2}$ is methyl, ethyl, n-propyl, isopropyl, n-butyl, isobutyl, tert-butyl, ethyl with one hydrogen substituted with hydroxyl, n-propyl with one hydrogen substituted with hydroxyl, n-butyl with one hydrogen substituted with hydroxyl,

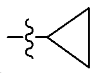, 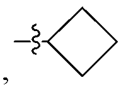,

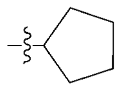, 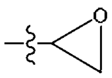, 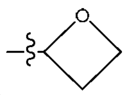, 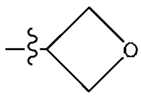, 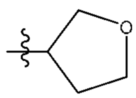, 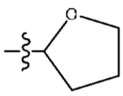

or phenyl, or, $R^{4-2}$ is bonded to $R^2$ to form a 4 to 8-membered ring when $R^2$ is methyl, ethyl or n-propyl and $R^{4-2}$ is methyl, ethyl,

$R^{4-3}$ is methyl, ethyl, n-propyl, isopropyl, methoxy, ethoxy, n-propoxy or isopropoxy;

and/or, $R^5$, at each occurrence, is independently hydrogen, halogen, nitro, nitrile, $-N+(R^{5-1})^3$, fluoromethyl, fluoroethyl, fluoro-n-propyl, fluoroisopropyl, chloromethyl, chloroethyl, chloro-n-propyl, chloroisopropyl, bromomethyl, bromoethyl, bromo-n-propyl, bromoisopropyl, $-C(O)OR^{5-1}$, $-C(O)R^{5-1}$, $-C(O)N(R^{5-1}R^{5-1a})$, $-S(O)_2R^{5-1}$, $-S(O)R^{5-1}$, $-N{=}C(R^{5-1}\ R^{5-1a})$, hydroxyl, methyl, ethyl, n-propyl, isopropyl, phenyl, phenyl with at least one hydrogen substituted with $R^{5-1}$, methoxy, ethoxy, n-propyloxy, isopropyloxy, $-N(R^{5-1}R^{5-1a})$, $-N(R^{5-1})C(O)R^{5-1a}$,

-OC(O)$R^{5-1}$, respectively, wherein $R^{5-1}$, $R^{5-1a}$, and $R^{5-1b}$ are independently hydrogen, methyl, ethyl, n-propyl, isopropyl, fluoromethyl, fluoroethyl, fluoro-n-propyl, fluoroisopropyl, chloromethyl, chloroethyl, chloro-n-propyl, chloroisopropyl, bromomethyl, bromoethyl, bromo-n-propyl, or bromoisopropyl, respectively.

**4.** The use according to claim 1, whereinthe Z is

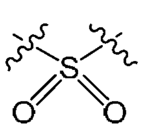, 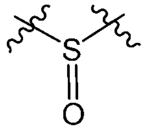,

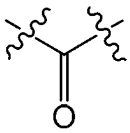, 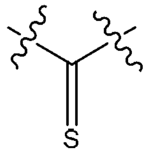

or a sulfur atom;

and/or, $R^1$ is hydrogen,

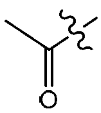, 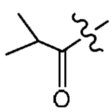 or 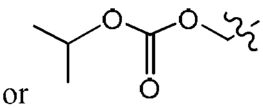;

and/or, $R^2$ is hydrogen, methyl

, 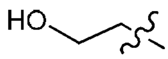, 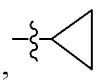, 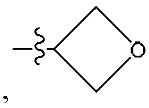

or phenyl;

and/or, $R^3$ is

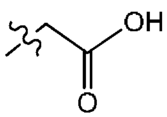, 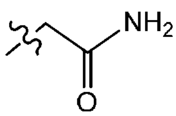, 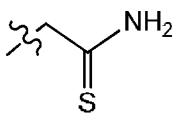, 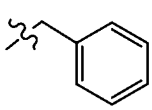, 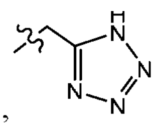

or

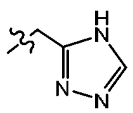;

and/or $R^4$ is

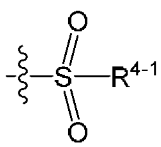, 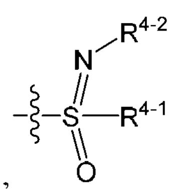, 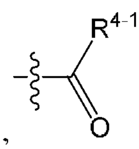 or 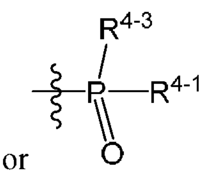,

wherein $R^{4-1}$ is phenyl, phenyl with at least one hydrogen substituted with $R^{4-11}$, five-membered monocyclic heteroaryl, five-membered monocyclic heteroaryl with at least one hydrogen substituted with $R^{4-11}$, nine-membered fused bicyclic heteroaryl, or naphthyl, wherein $R^{4-11}$ is bromine, fluorine, chlorine, methyl, nitro, phenyl, trifluoromethyl

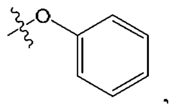,

methoxy, cyclopropyL trifluoromethoxy, nitro or

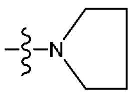;

$R^{4-2}$ is methyl, ethyl

, 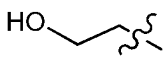, 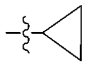, 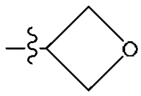

or phenyl, or, $R^{4-2}$ is bonded to $R^2$ to form a 4 to 6-membered ringwhen R is methyl, ethyl, or n-propyl and $R^{4-2}$ is methyl, ethyl,

$R^{4-3}$ is methoxy, ethoxy, n-propoxy or isopropoxy;

and/or,$R^5$, at each occurrence, is independently halogen, nitro, nitrile, carboxyl, -NHC(O)$CH_3$, methoxy, or hydroxy, respectively;

**5.** The use according to claim 1, whereinthe compound is selected from any one of the following compounds:

| | |
|---|---|
| **I-1** | 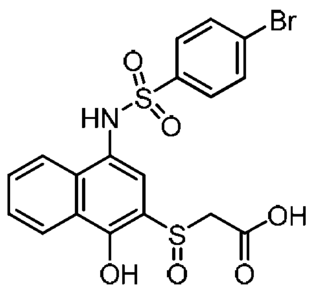 |
| **I-2** | 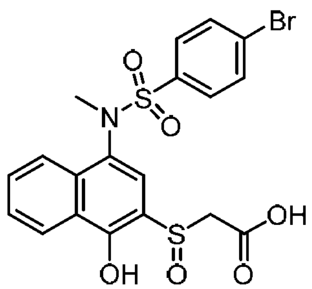 |

(continued)

| | |
|---|---|
| I-3 | 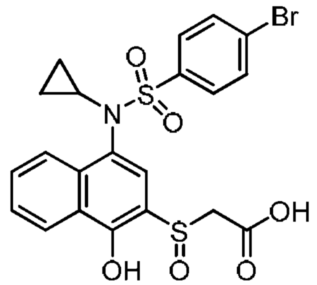 |
| I-4 | 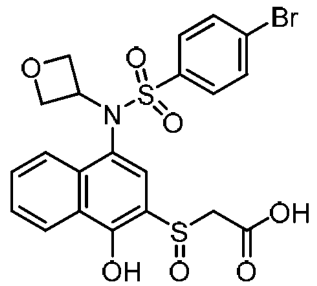 |
| I-5 | 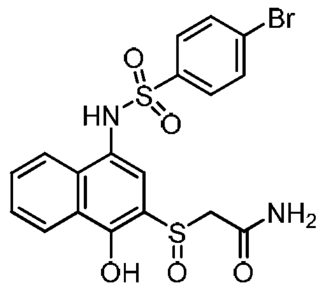 |
| I-6 | 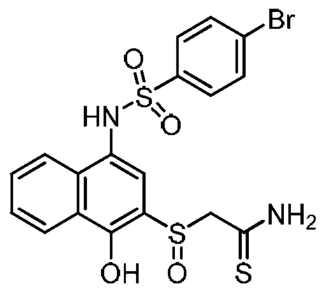 |
| I-7-1 | 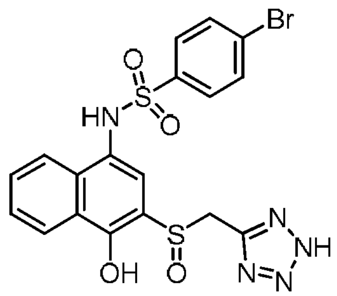 |

(continued)

| | |
|---|---|
| **I-8** | 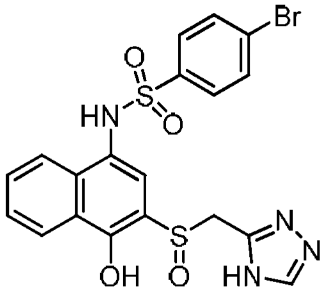 |
| **I-9-1** | 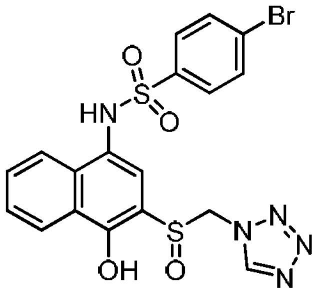 |
| **I-9-2** | 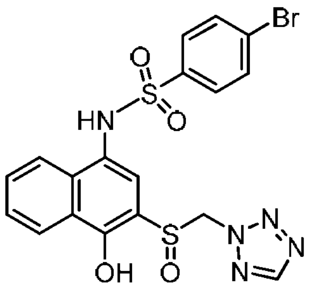 |
| **I-10** | 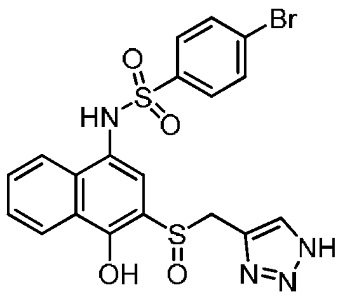 |
| **I-11-1** | 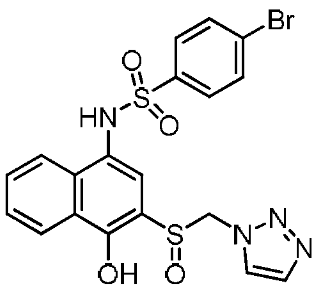 |

(continued)

| | |
|---|---|
| **I-11-2** | 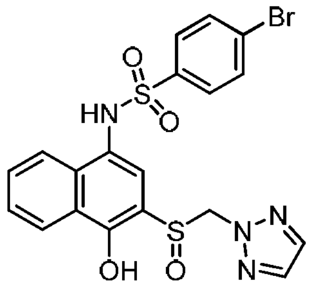 |
| **I-12** | 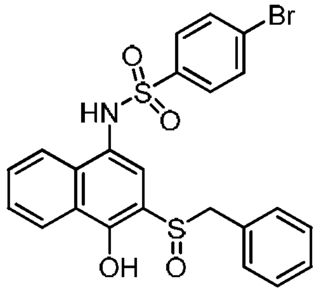 |
| **I-13-1** | 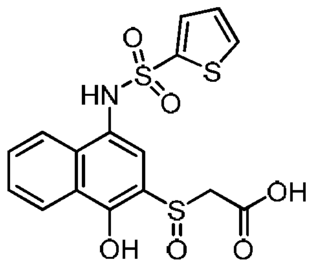 |
| **I-14-1** | 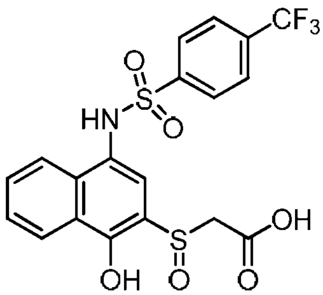 |
| **I-15-1** | 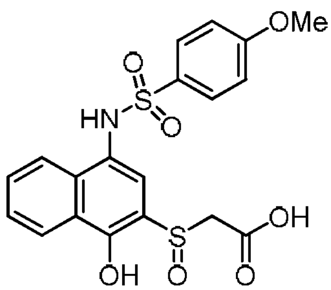 |

(continued)

| | |
|---|---|
| **I-16-1** | 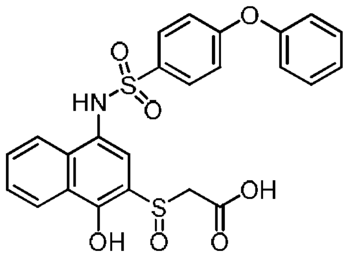 |
| **I-17-1** | 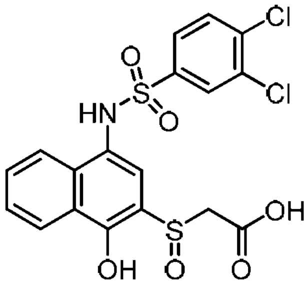 |
| **I-18-1** | 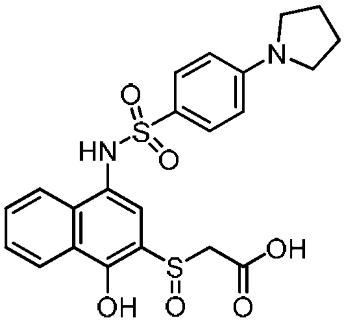 |
| **I-19-1** | 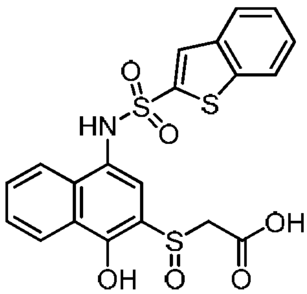 |
| **I-20-1** | 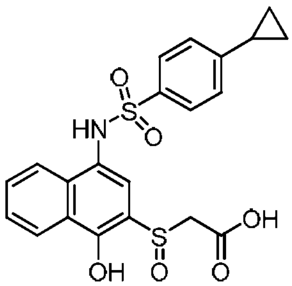 |

(continued)

| | |
|---|---|
| **I-21-1** | 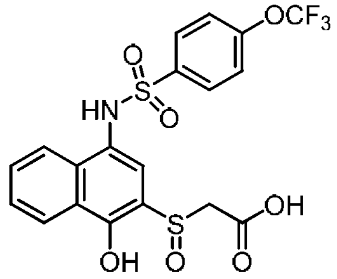 |
| **I-22-1** | 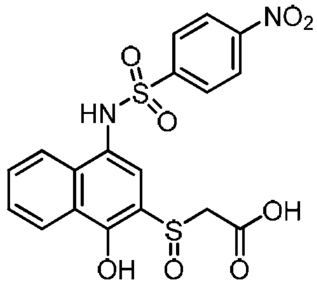 |
| **I-23-1** | 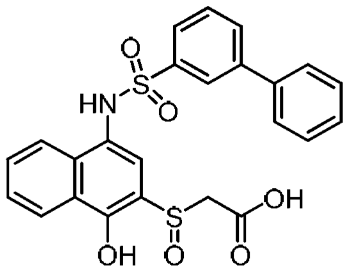 |
| **I-24-1** | 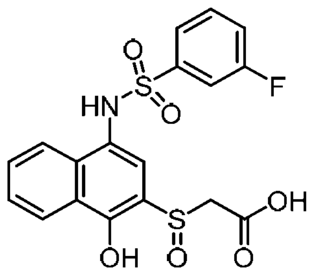 |
| **I-25-1** | 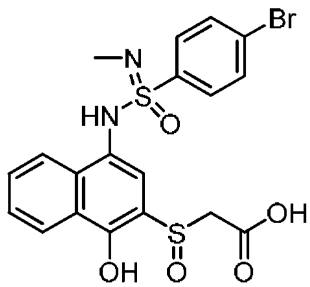 |
| **I-26** | 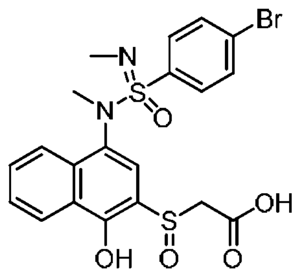 |

(continued)

| | |
|---|---|
| **I-27** | 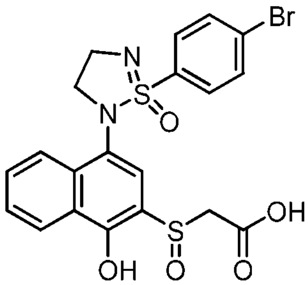 |
| **I-28** | 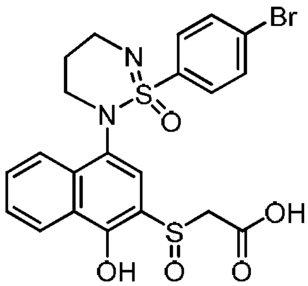 |
| **I-29** | 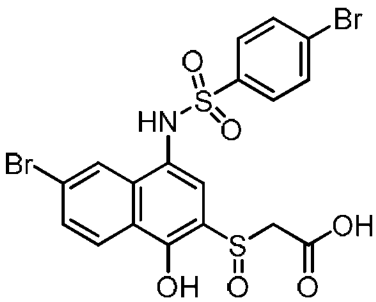 |
| **I-30** | 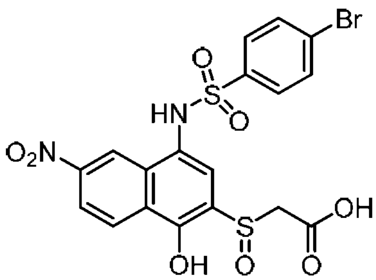 |
| **I-31** | 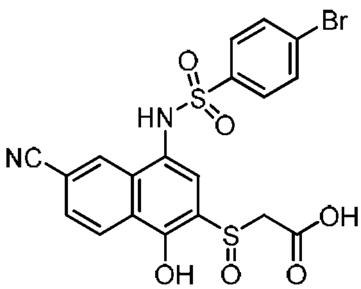 |

(continued)

| | |
|---|---|
| **I-32** | 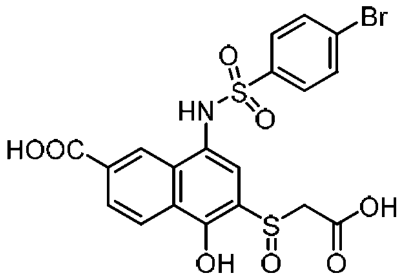 |
| **I-33** | 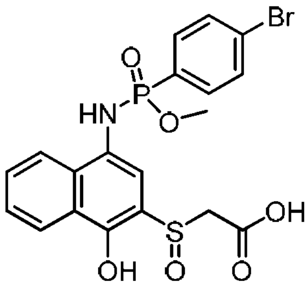 |
| **I-34** | 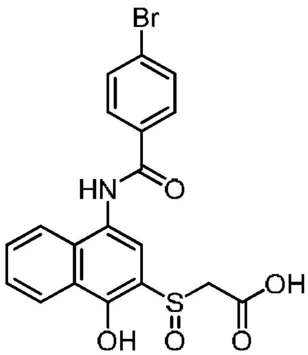 |
| **I-35-1** | 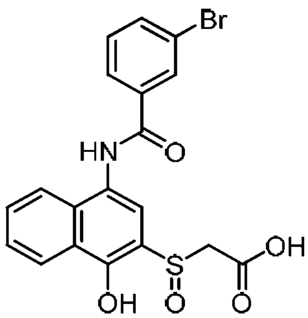 |
| **I-36** | 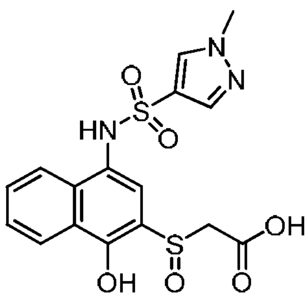 |

(continued)

| | |
|---|---|
| **I-37** | 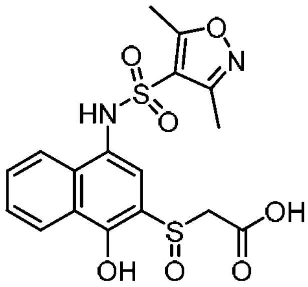 |
| **I-38** | 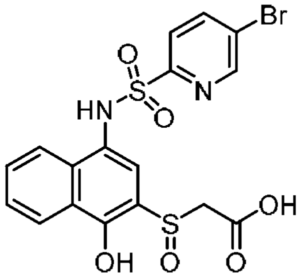 |
| **I-39** | 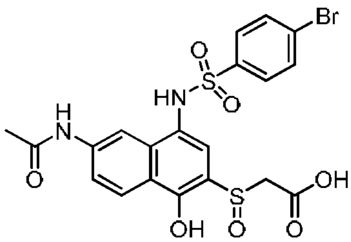 |
| **I-40-1** | 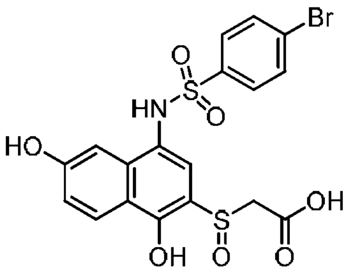 |
| **I-41-1** | 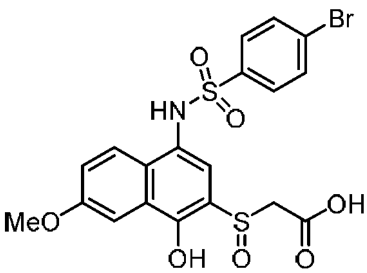 |
| **I-42** | 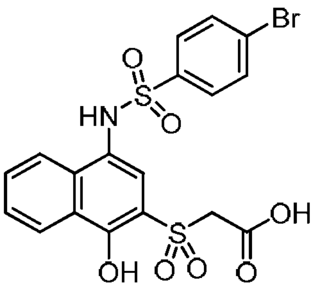 |

(continued)

| | |
|---|---|
| **I-7-2** | 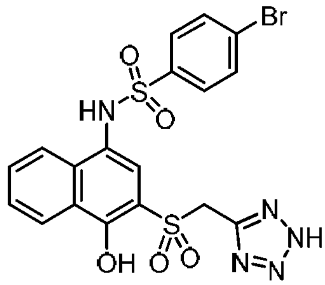 |
| **I-13-2** | 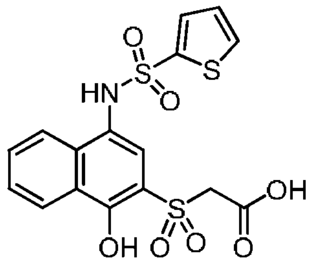 |
| **I-14-2** | 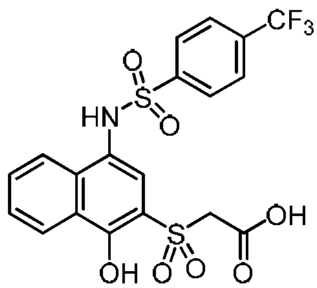 |
| **I-15-2** | 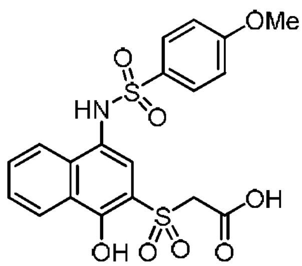 |
| **I-16-2** | 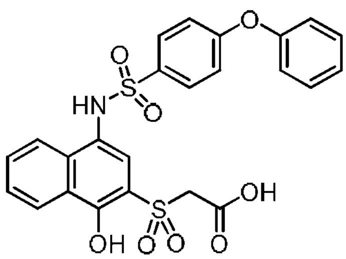 |
| **I-17-2** | 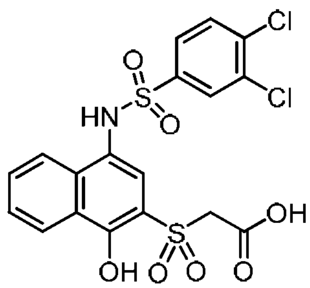 |

(continued)

| | |
|---|---|
| I-18-2 | 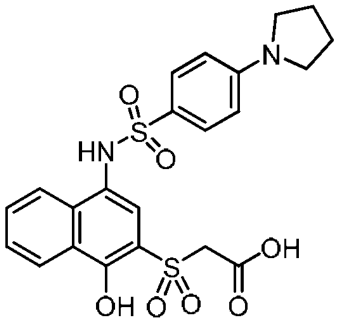 |
| I-19-2 | 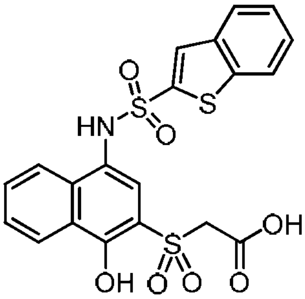 |
| I-20-2 | 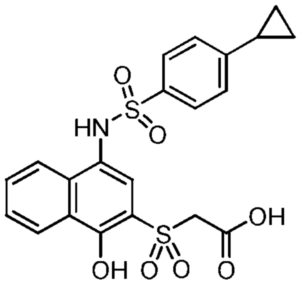 |
| I-21-2 | 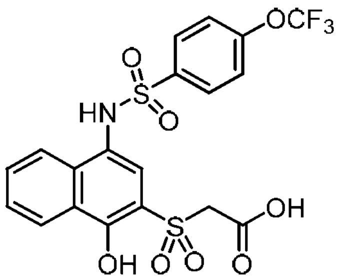 |
| I-22-2 | 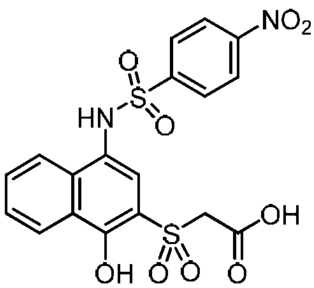 |

(continued)

| | |
|---|---|
| I-23-2 | 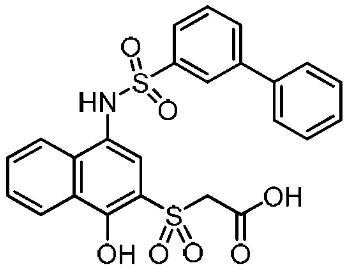 |
| I-24-2 | 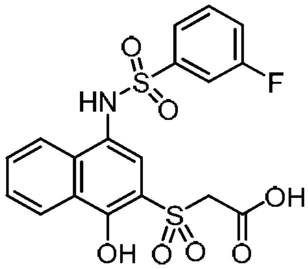 |
| I-25-2 | 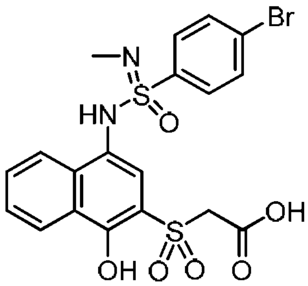 |
| I-35-2 | 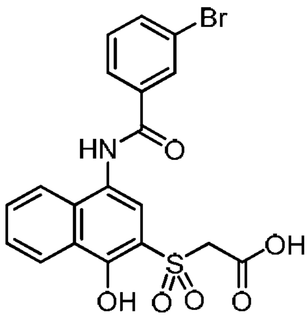 |
| I-40-2 | 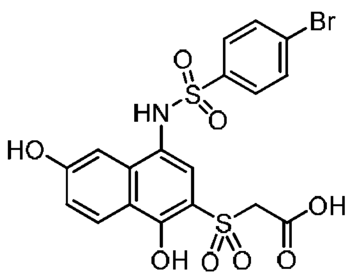 |

(continued)

| | |
|---|---|
| **I-41-2** | 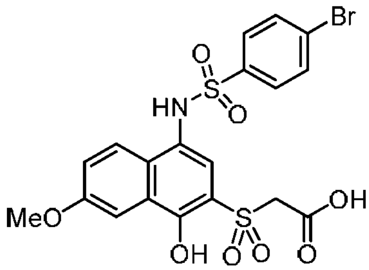 |
| **I-43** | 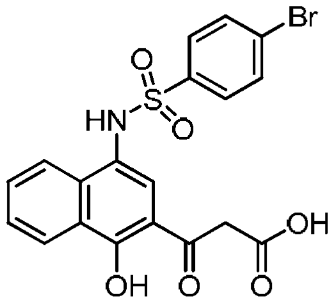 |
| **I-44** | 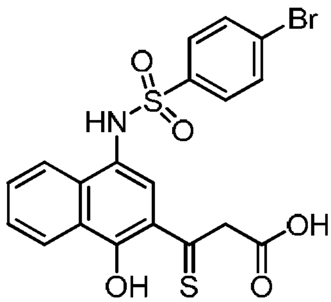 |
| **I-45** | 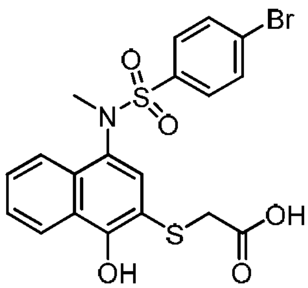 |
| **I-46** | 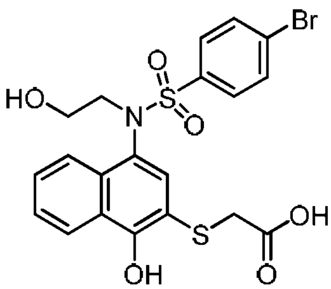 |

(continued)

| | |
|---|---|
| **I-47** | 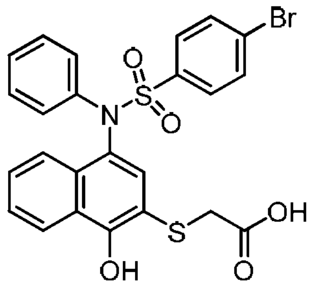 |
| **I-48** | 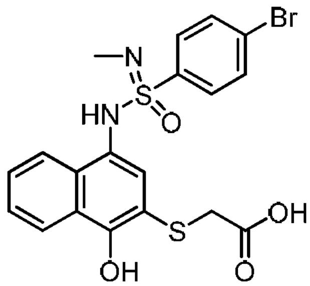 |
| **I-49** | 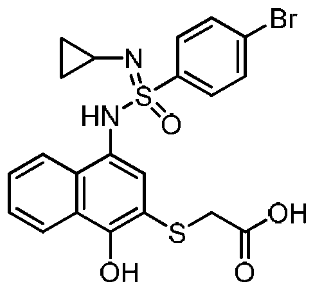 |
| **I-50** | 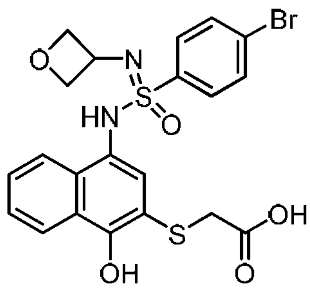 |
| **I-51** | 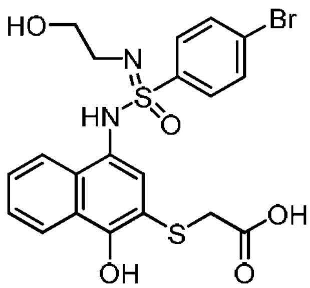 |

(continued)

| | |
|---|---|
| **I-52** | 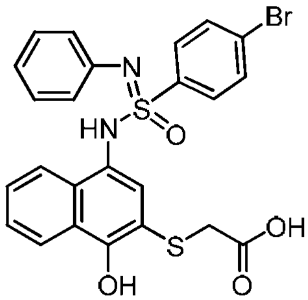 |
| **I-53** | 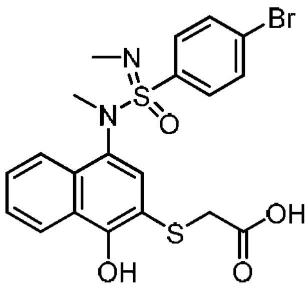 |
| **I-54** | 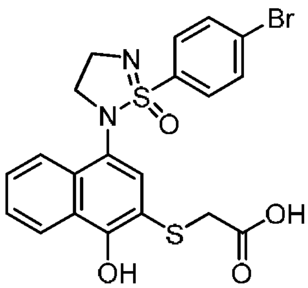 |
| **I-55** | 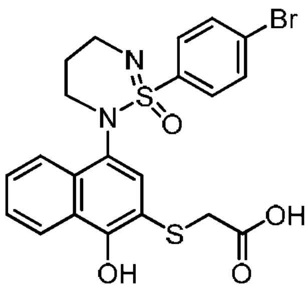 |
| **I-56** | 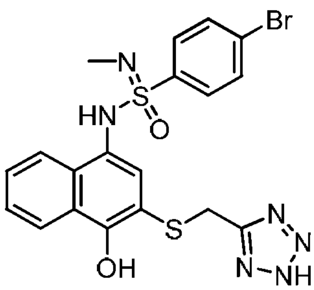 |

(continued)

| | |
|---|---|
| **I-57** | 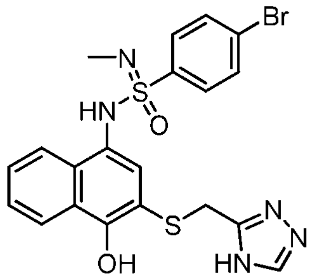 |
| **I-58** | 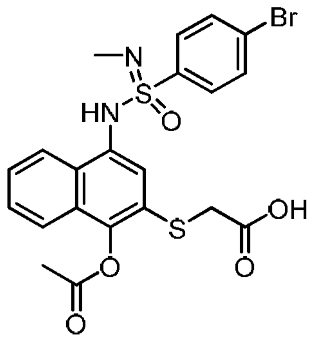 |
| **I-59** | 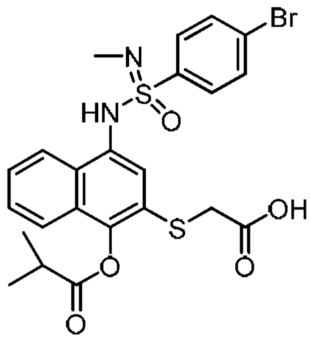 |
| **I-60** | 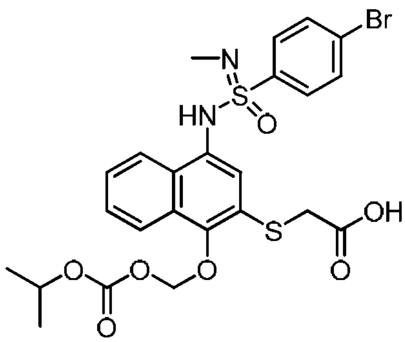 |
| **I-61** | 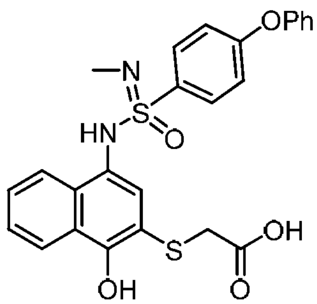 |

(continued)

| | |
|---|---|
| **I-62** | 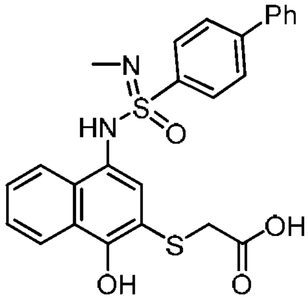 |
| **I-63** | 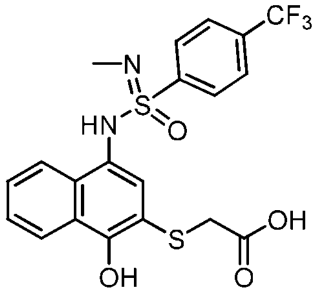 |
| **I-64** | 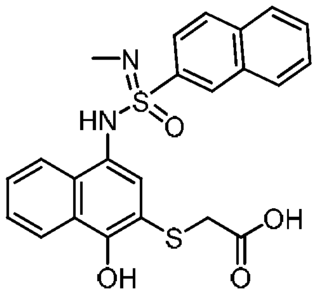 |
| **I-65** | 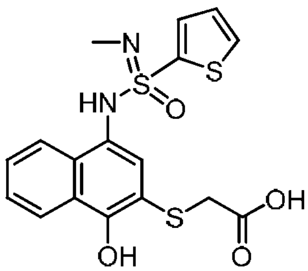 |
| **I-66** | 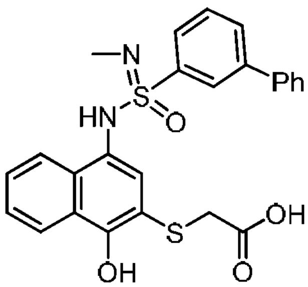 |

(continued)

| | |
|---|---|
| **I-67** | 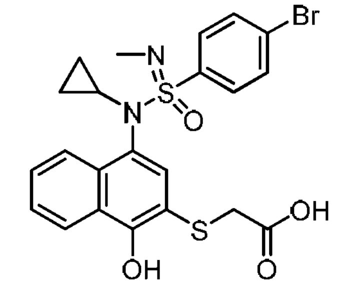 |
| **I-68** | 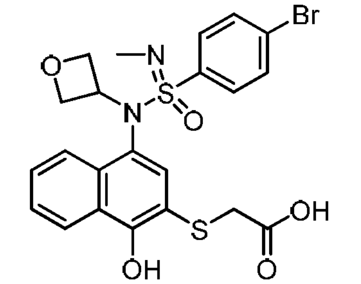 |
| **I-69** | 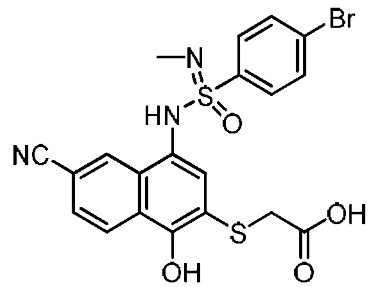 |
| **I-70** | 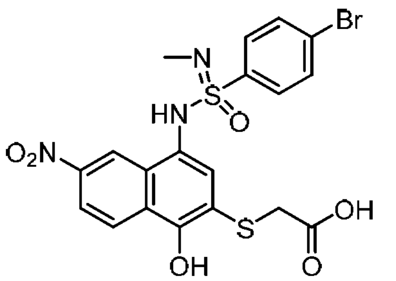 |

**6.** The use according to claim 1, wherein Z is

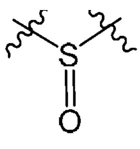

;

$R^1$ is hydrogen; $R^2$ is hydrogen; $R^3$ is hydrogen,

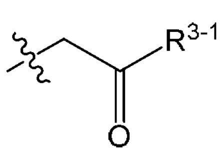

; $R^4$ is

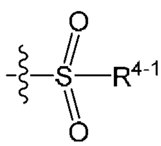

;

wherein $R^{3-1}$ is hydrogen or hydroxyl; and $R^{4-1}$ is phenyl, or phenyl with at least one hydrogen atom substituted by $R^{4-11}$, and $R^{4-11}$is halogen-substituted methyl, halogen-substituted ethyl, methyl, ethyl, or halogen.

**7.** The use according to claim 6, wherein $R^{3-1}$ is hydroxyl; and/or, $R^{4-1}$ is phenyl, phenyl with at least one hydrogen atom substituted by trifluoromethyl, or phenyl with at least one hydrogen atom substituted by halogen.

**8.** The use according to claim 7, wherein the compound of Formula I is

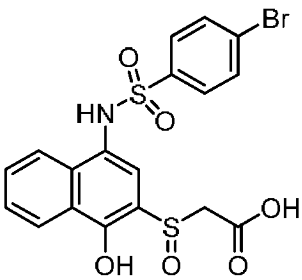

or

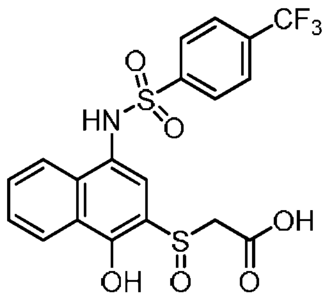

.

**9.** The use according to claim 1, wherein the reproductive system diseases comprise premature ovarian failure, ovarian insufficiency, polycystic ovary syndrome, oligospermia, and erectile dysfunction;

the autoimmune diseases comprise systemic lupus erythematosus nephritis, rheumatoid arthritis, multiple sclerosis, and Hashimoto's thyroiditis;
the skin diseases comprise psoriasis, melasma, vitiligo, and chronic eczema;
the eye diseases comprise cataract, age-related macular degeneration, and retinitis pigmentosa;
the muscular or skeletal degenerative diseases comprise sarcopenia and lumbar disc herniation;
the neurological diseases comprise epilepsy and cerebral stroke;
the anemia comprises β-thalassemia;
the adrenal diseases comprise primary aldosteronism and Cushing's syndrome;
the chronic gastritis comprises chronic atrophic gastritis; and/or
the liver injury is acute alcoholic liver injury, chronic alcoholic liver injury, drug-induced liver injury, non-alcoholic steatohepatitis, or liver injury caused by cholecystitis.

**10.** The use according to claim 1, comprising:

(a1) preparation of a medicament for preventing and/or treating premature ovarian failure; and/or
(a2) prevention and/or treatment of premature ovarian failure; and/or
(b1) preparation of a medicament for preventing and/or treating ovarian insufficiency; and/or
(b2) prevention and/or treatment of ovarian insufficiency; and/or
(c1) preparation of a medicament for preventing and/or treating systemic lupus erythematosus nephritis; and/or
(c2) prevention and/or treatment of systemic lupus erythematosus nephritis; and/or
(d1) preparation of a medicament for preventing and/or treating sarcopenia; and/or
(d2) prevention and/or treatment of sarcopenia; and/or
(e1) preparation of a medicament for preventing and/or treating liver injury; and/or,
(e2) prevention and/or treatment of liver injury; and/or
(f1) preparation of a medicament for preventing and/or treating oligospermia; and/or
(f2) prevention and/or treatment of oligospermia; and/or
(g1) preparation of a medicament for preventing and/or treating rheumatoid arthritis; and/or
(g2) prevention and/or treatment of rheumatoid arthritis; and/or
(h1) preparation of a medicament for preventing and/or treating cataract; and/or
(h2) prevention and/or treatment of cataract; and/or
(i1) preparation of a medicament for preventing and/or treating Cushing's syndrome; and/or
(i2) prevention and/or treatment of Cushing's syndrome; and/or
(j1) preparation of a medicament for preventing and/or treating Hashimoto's thyroiditis; and/or
(j2) prevention and/or treatment of Hashimoto's thyroiditis; and/or
(k1) preparation of a medicament for preventing and/or treating chronic obstructive pulmonary disease; and/or
(k2) prevention and/or treatment of chronic obstructive pulmonary disease; and/or

(11) preparation of a medicament for preventing and/or treating tinnitus; and/or
(l2) prevention and/or treatment of tinnitus; and/or
(m1) preparation of a medicament for preventing and/or treating psoriasis; and/or
(m2) prevention and/or treatment of psoriasis; and/or
(n1) preparation of a medicament for preventing and/or treating chronic eczema; and/or
(n2) prevention and/or treatment of chronic eczema; and/or
(o1) preparation of a medicament for preventing and/or treating age-related macular degeneration; and/or
(o2) prevention and/or treatment of age-related macular degeneration;
(p1) preparation of a medicament for preventing and/or treating retinitis pigmentosa; and/or
(p2) prevention and/or treatment of retinitis pigmentosa;
(q1) preparation of a medicament for preventing and/or treating multiple sclerosis; and/or
(q2) prevention and/or treatment of multiple sclerosis;
(r1) preparation of a medicament for preventing and/or treating hypothyroidism; and/or
(r2) prevention and/or treatment of hypothyroidism;
(s1) preparation of a medicament for preventing and/or treating chronic atrophic gastritis; and/or
(s2) prevention and/or treatment of chronic atrophic gastritis;
(t1) preparation of a medicament for preventing and/or treating myocarditis; and/or
(t2) prevention and/or treatment of myocarditis;
(u1) preparation of a medicament for preventing and/or treating alopecia; and/or
(u2) prevention and/or treatment of alopecia;
(v1) preparation of a medicament for preventing and/or treating primary aldosteronism; and/or
(v2) prevention and/or treatment of primary aldosteronism;
(w1) preparation of a medicament for preventing and/or treating erectile dysfunction; and/or
(w2) prevention and/or treatment of erectile dysfunction;
(x1) preparation of a medicament for preventing and/or treating periodontitis; and/or
(x2) prevention and/or treatment of periodontitis;
(y1) preparation of a medicament for preventing and/or treating cerebral stroke; and/or
(y2) prevention and/or treatment of cerebral stroke;
(z1) preparation of a medicament for preventing and/or treating epilepsy; and/or
(z2) prevention and/or treatment of epilepsy;
(Aa1) preparation of a medicament for preventing and/or treating melasma; and/or
(Aa2) prevention and/or treatment of melasma; and/or
(Ab1) preparation of a medicament for preventing and/or treating vitiligo; and/or
(Ab2) prevention and/or treatment of vitiligo; and/or
(Ac1) preparation of a medicament for preventing and/or treating β-thalassemia; and/or
(Ac2) prevention and/or treatment of β-thalassemia; and/or
(Ad1) preparation of a medicament for preventing and/or treating lumbar disc herniation; and/or
(Ad2) prevention and/or treatment of lumbar disc herniation; and/or
(Ae1) preparation of a medicament for preventing and/or treating polycystic ovary syndrome; and/or,
(Ae2) prevention and/or treatment of polycystic ovary syndrome.

**11.** A method for preventing and/or treating (a) premature ovarian failure; and/or (b) ovarian insufficiency; and/or (c) systemic lupus erythematosus nephritis; and/or (d) sarcopenia; and/or (e) liver injury; and/or (f) oligospermia; and/or (g) rheumatoid arthritis; and/or (h) cataract; and/or (i) Cushing's syndrome; and/or (j) Hashimoto's thyroiditis; and/or (k) chronic obstructive pulmonary disease; and/or (l) tinnitus; and/or (m) psoriasis; and/or (n) chronic eczema; and/or (o) age-related macular degeneration; and/or (p) retinitis pigmentosa; and/or (q) multiple sclerosis; and/or (r) hypothyroidism; and/or (s) chronic atrophic gastritis; and/or (t) myocarditis; and/or (u) alopecia; and/or (v) primary aldosteronism; and/or (w) erectile dysfunction; and/or (x) periodontitis; and/or (y) cerebral stroke; and/or (z) epilepsy; and/or (Aa) melasma; and/or (Ab) vitiligo; and/or (Ac) β-thalassemia; and/or (Ad) lumbar disc herniation; and/or (Ae) polycystic ovary syndrome, wherein the method comprises the step of: administering to a subject a therapeutically effective amount of a compound of Formula (I) or a pharmaceutically acceptable salt, stereoisomer, solvate or prodrug thereof.

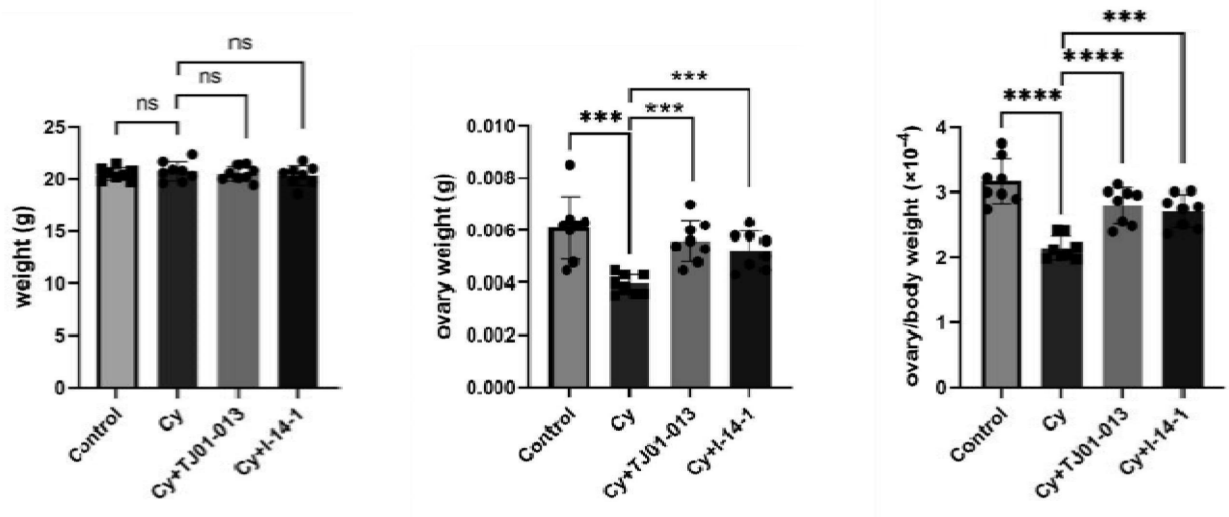
ns
ns
ns
weight (g)
25
20
15
10
5
0
Control
Cy
Cy+TJ01-013
Cy+I-14-1
***
***
***
ovary weight (g)
0.010
0.008
0.006
0.004
0.002
0.000
****
****
***
ovary/body weight (×10$^{-4}$)
4
3
2
1
0

FIG. 1

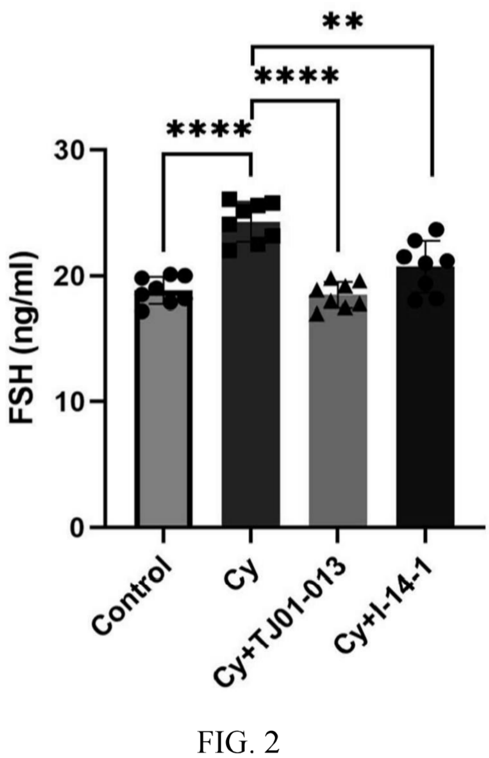
**
****
****
FSH (ng/ml)
30
20
10
0
Control
Cy
Cy+TJ01-013
Cy+I-14-1

FIG. 2

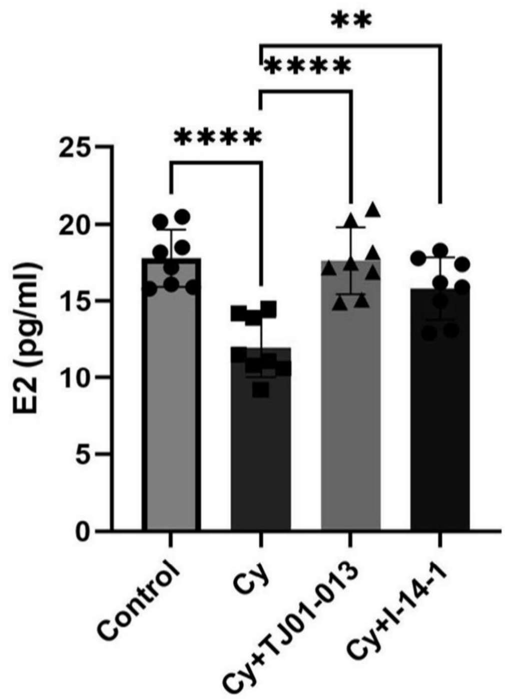
**
****
****
25
20
15
10
5
0
E2 (pg/ml)
Control
Cy
Cy+TJ01-013
Cy+I-14-1

FIG. 3

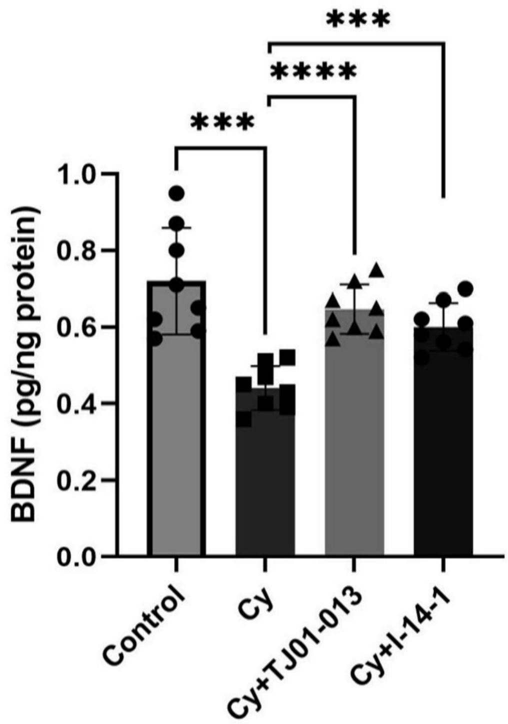
***
****
***
1.0
0.8
0.6
0.4
0.2
0.0
BDNF (pg/ng protein)
Control
Cy
Cy+TJ01-013
Cy+I-14-1

FIG. 4

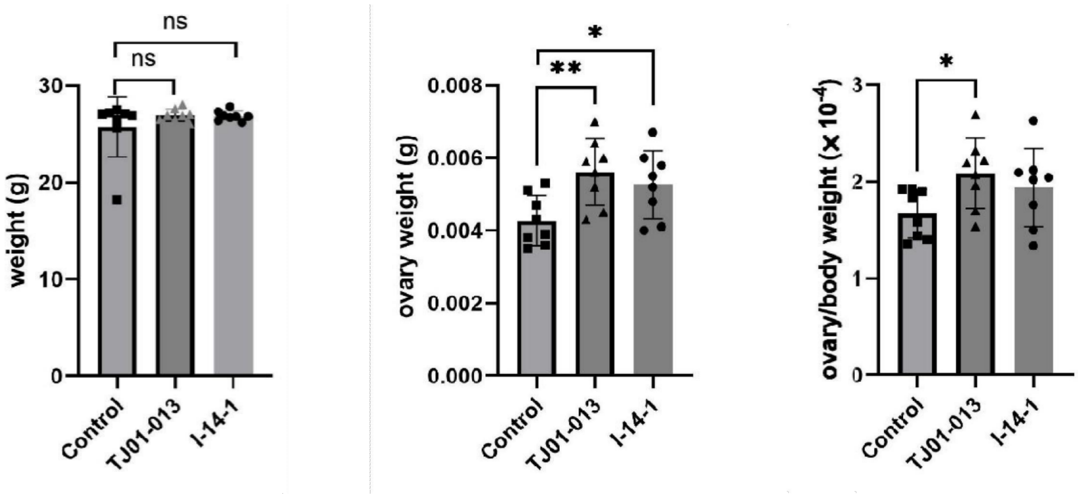


FIG. 5

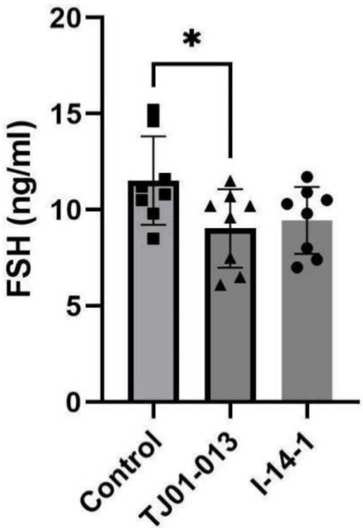


FIG. 6

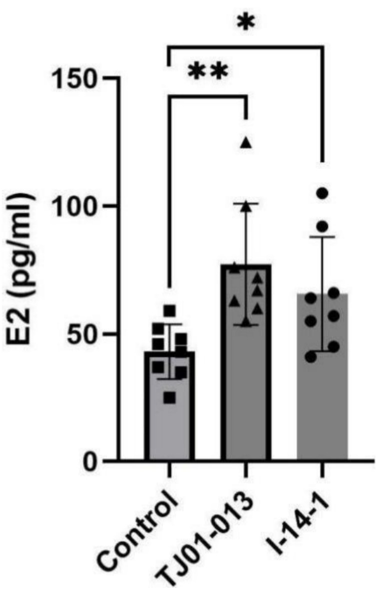


FIG. 7

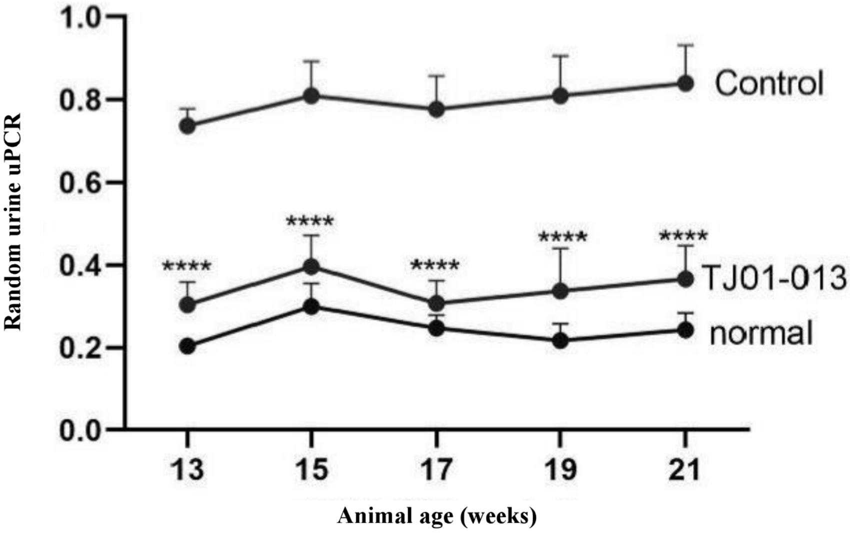
1.0
0.8
0.6
0.4
0.2
0.0
Random urine uPCR
Control
TJ01-013
normal
****
****
****
****
****
13
15
17
19
21
Animal age (weeks)

FIG. 8

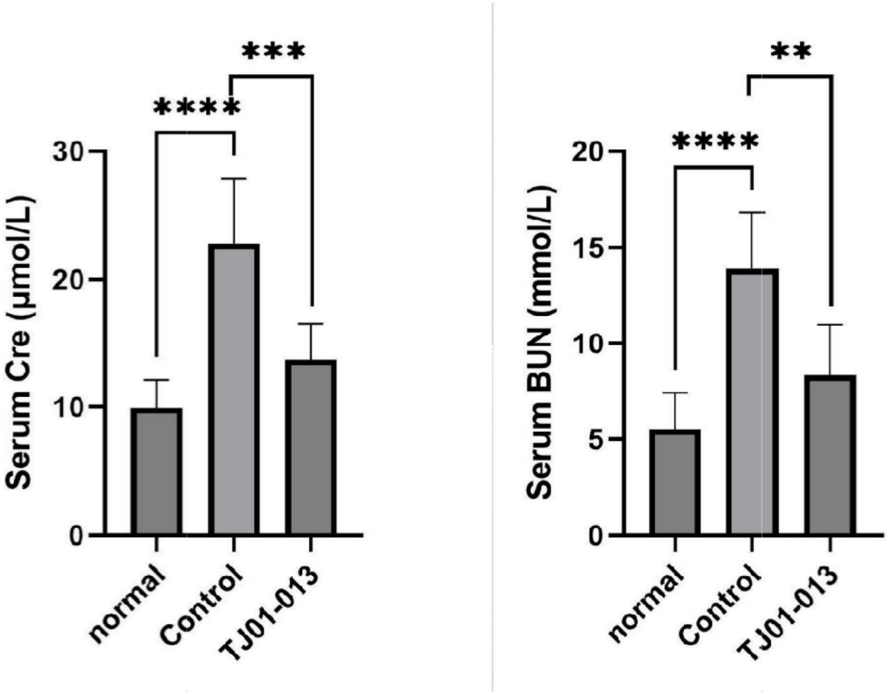
Serum Cre (μmol/L)
30
20
10
0
****
***
normal
Control
TJ01-013
Serum BUN (mmol/L)
20
15
10
5
0
****
**
normal
Control
TJ01-013

FIG. 9

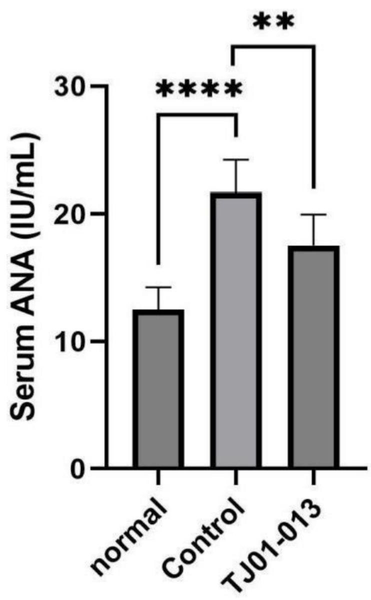
**
****
30
20
10
0
Serum ANA (IU/mL)
normal
Control
TJ01-013

FIG. 10

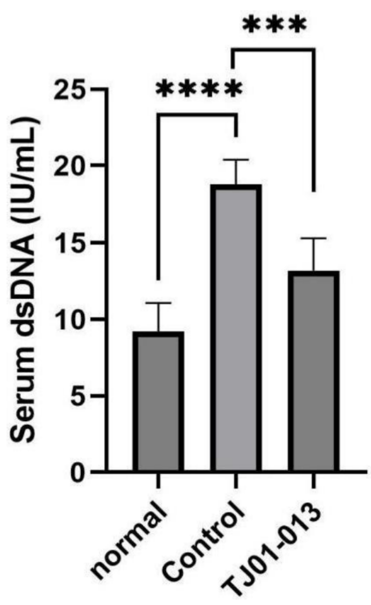
***
****
25
20
15
10
5
0
Serum dsDNA (IU/mL)
normal
Control
TJ01-013

FIG. 11

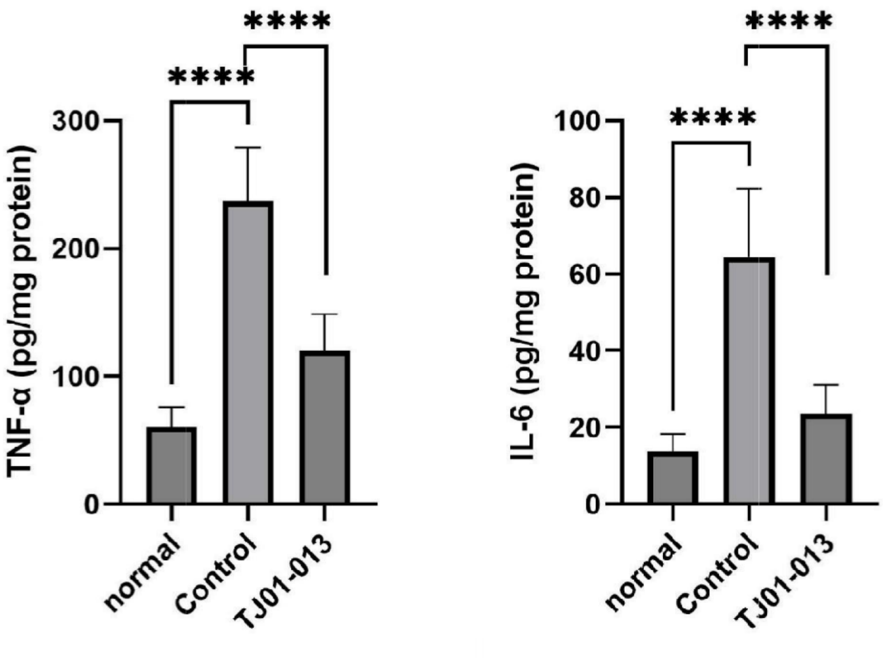
TNF-α (pg/mg protein)
300
200
100
0
****
****
normal
Control
TJ01-013
IL-6 (pg/mg protein)
100
80
60
40
20
0
****
****
normal
Control
TJ01-013

FIG. 12

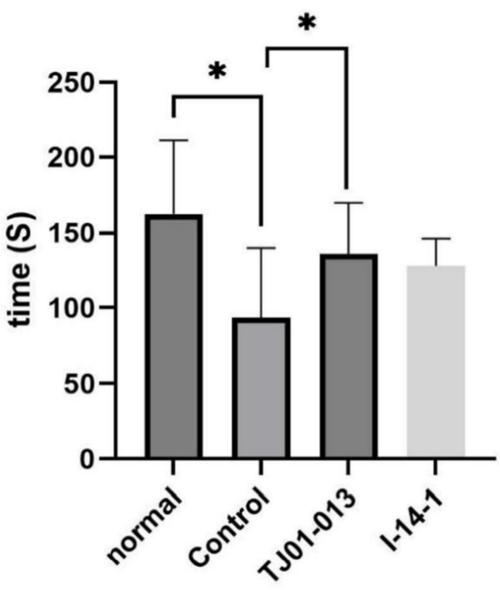
time (S)
250
200
150
100
50
0
*
*
normal
Control
TJ01-013
I-14-1

FIG. 13

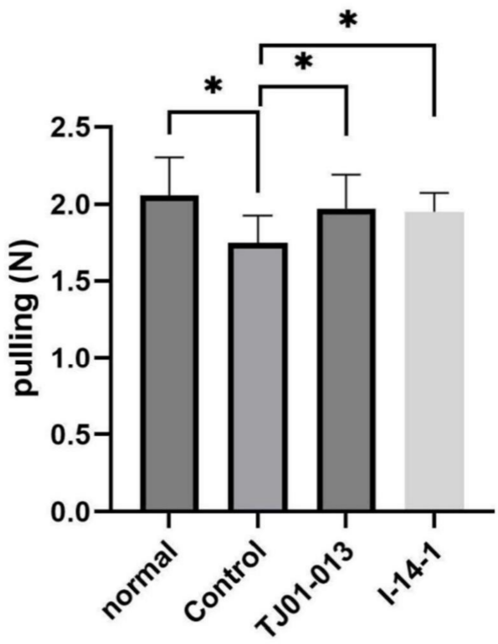

pulling (N)
2.5
2.0
1.5
1.0
0.5
0.0
*
*
*
normal
Control
TJ01-013
I-14-1


FIG. 14

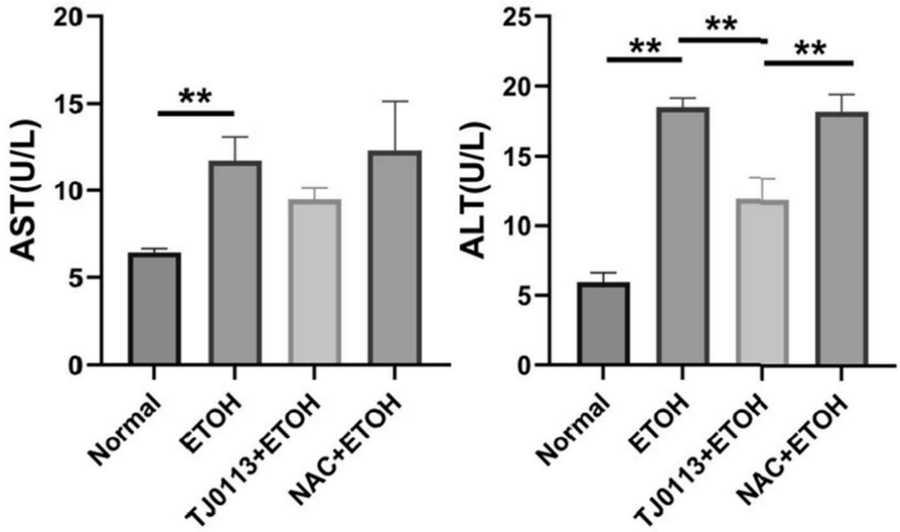

AST(U/L)
20
15
10
5
0
**
Normal
ETOH
TJ0113+ETOH
NAC+ETOH
ALT(U/L)
25
20
15
10
5
0
**
**
**
Normal
ETOH
TJ0113+ETOH
NAC+ETOH


FIG. 15

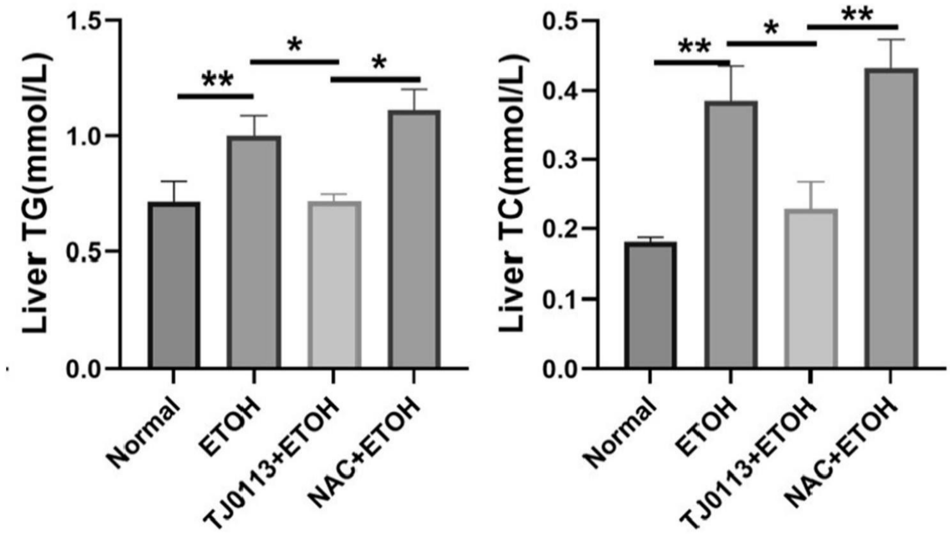


FIG. 16

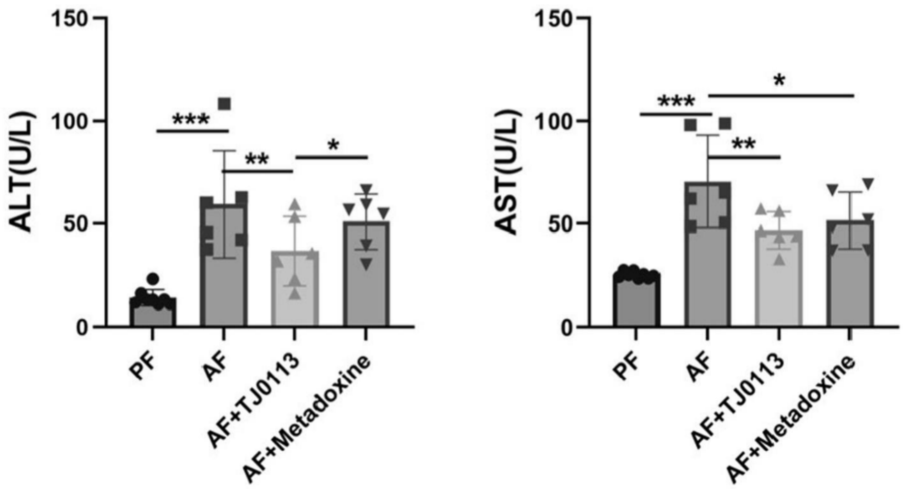


FIG. 17

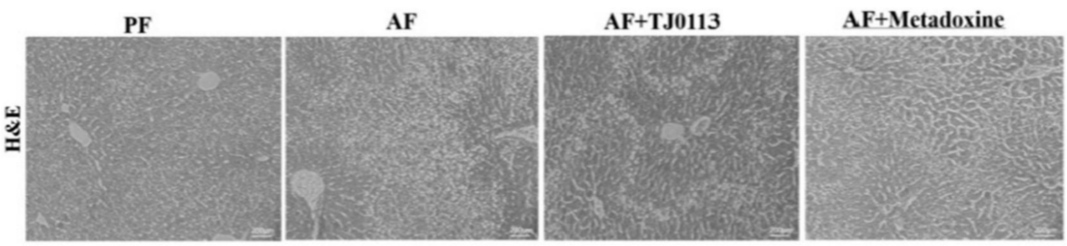


FIG. 18

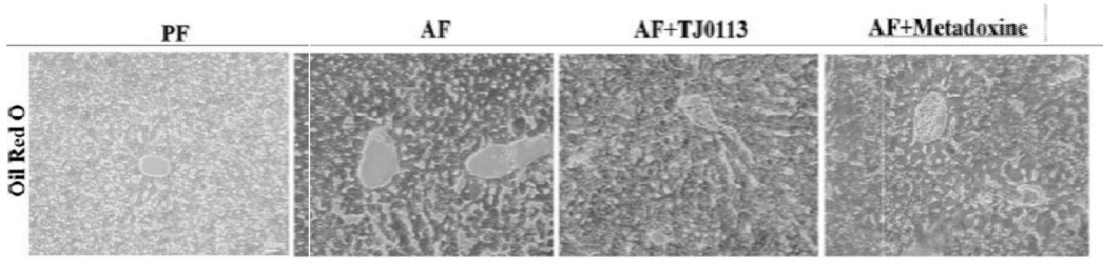


FIG. 19

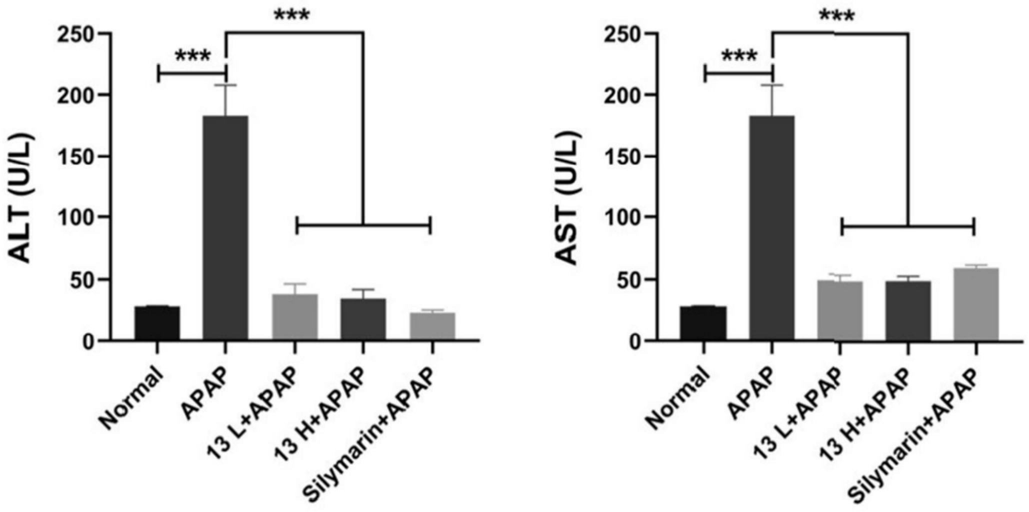


FIG. 20

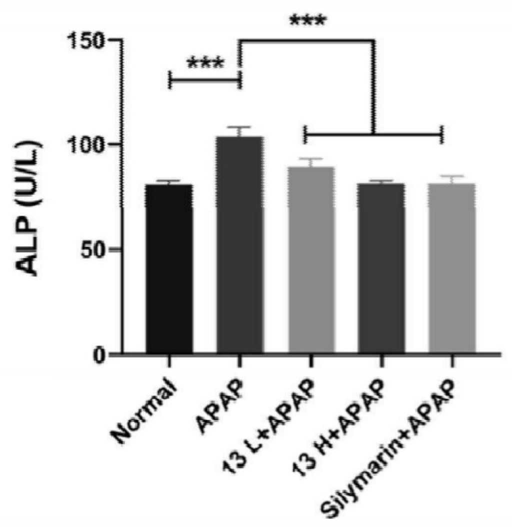


FIG. 21

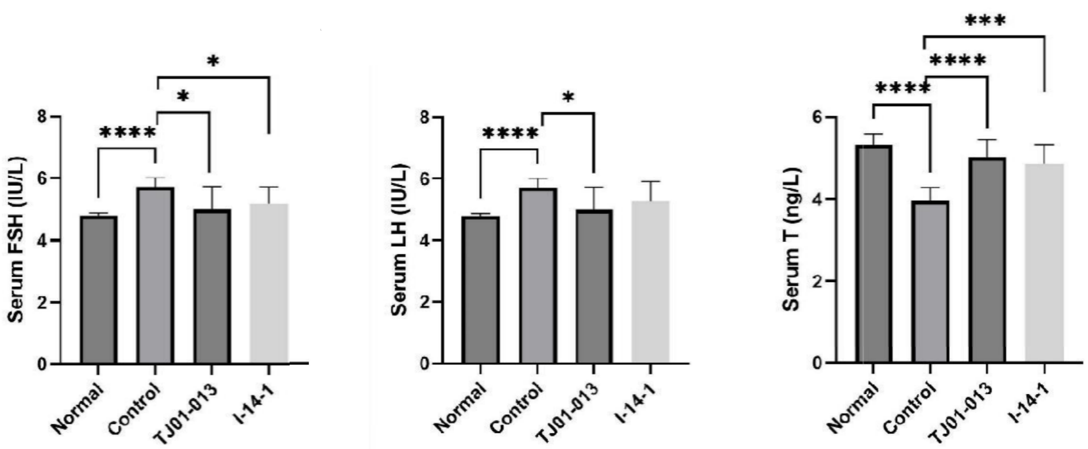


FIG. 22

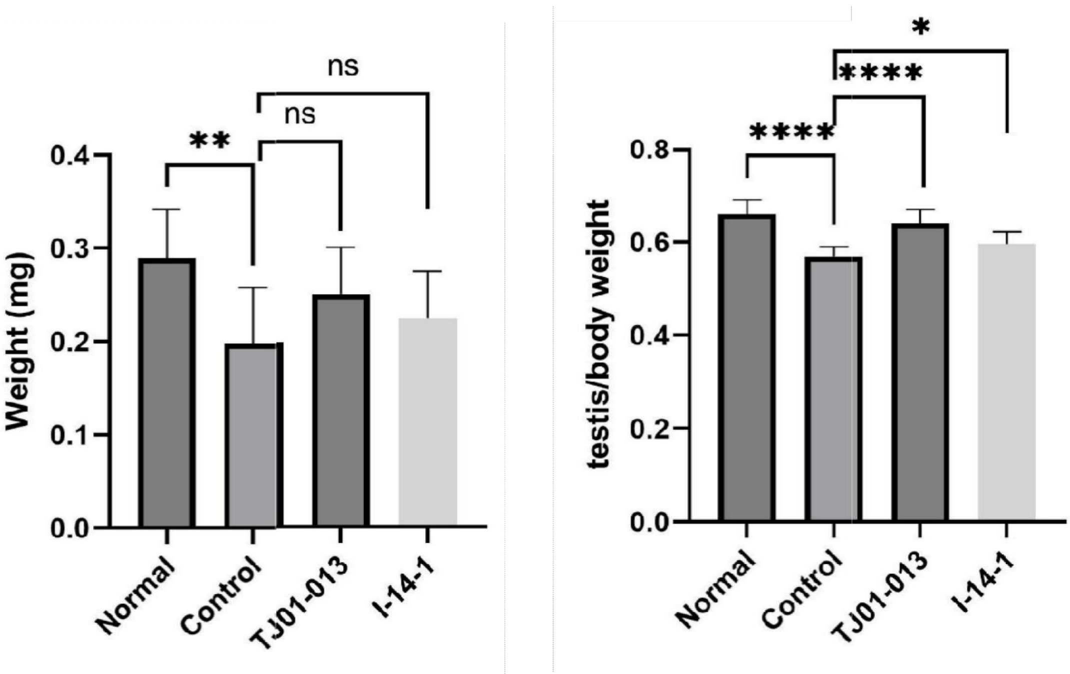

ns
ns
**
Weight (mg)
0.4
0.3
0.2
0.1
0.0
Normal
Control
TJ01-013
I-14-1
*
****
****
testis/body weight
0.8
0.6
0.4
0.2
0.0
Normal
Control
TJ01-013
I-14-1


FIG. 23

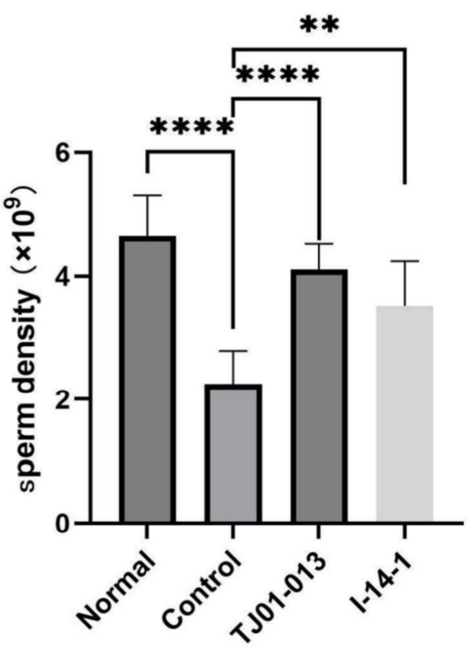

**
****
****
sperm density（×10$^9$）
6
4
2
0
Normal
Control
TJ01-013
I-14-1


FIG. 24

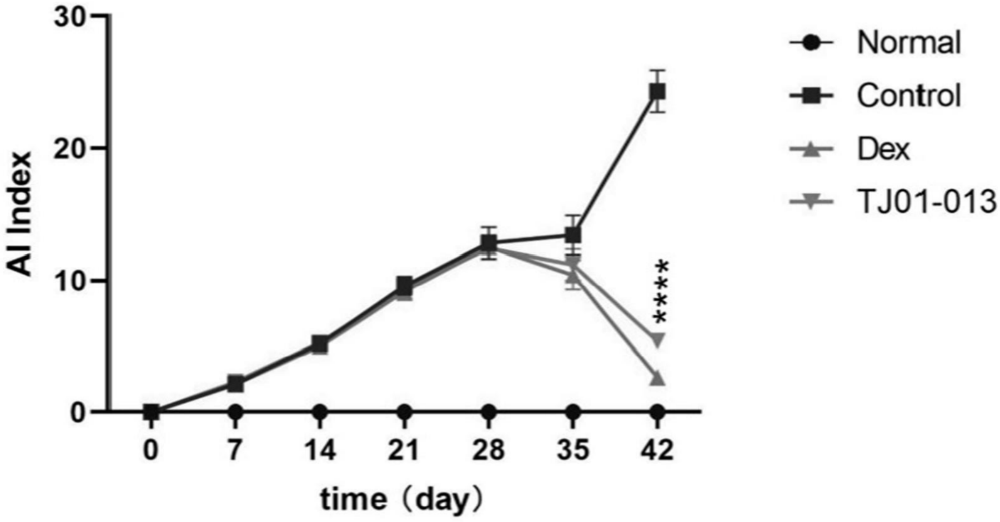


FIG. 25

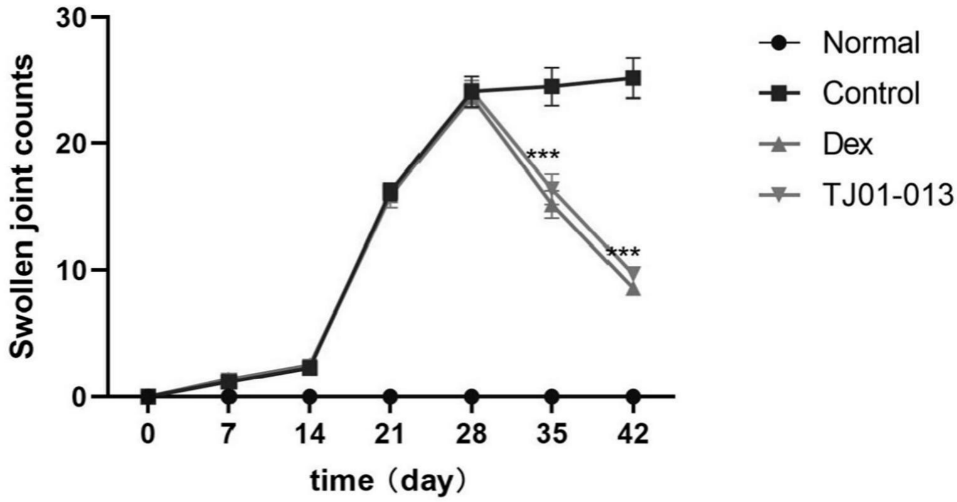


FIG. 26

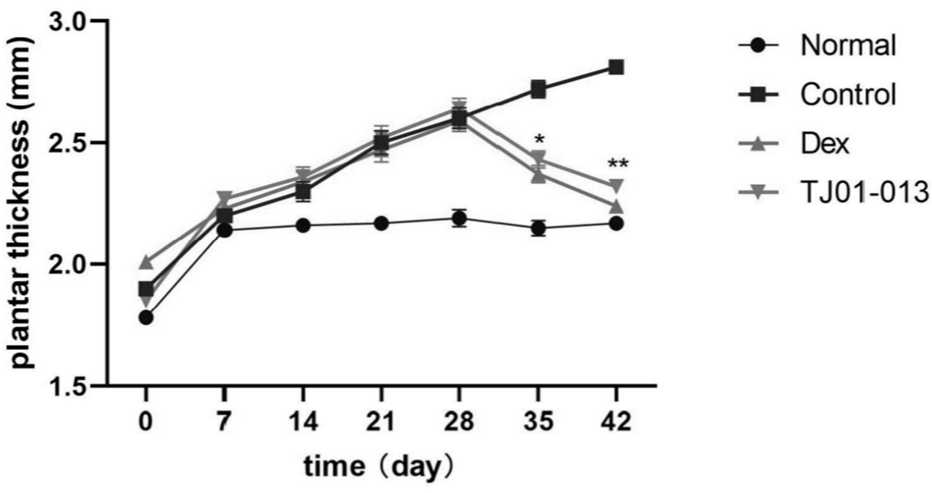


FIG. 27

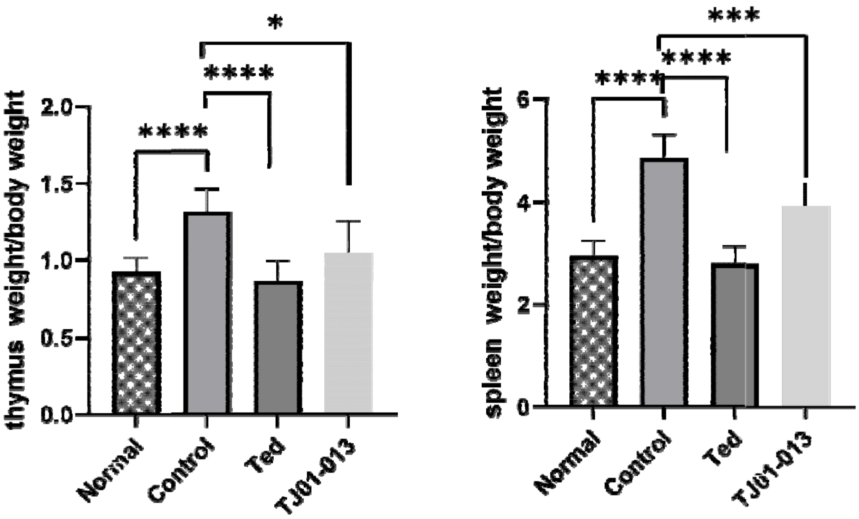


FIG. 28

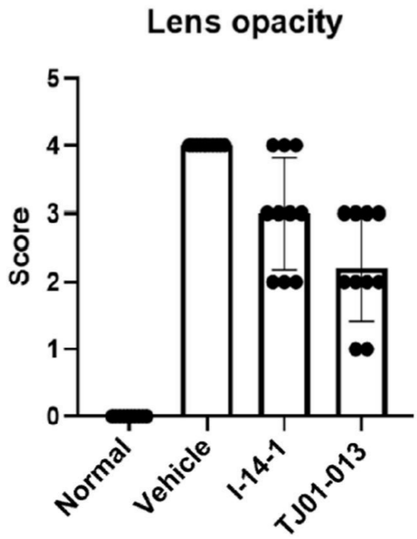


FIG. 29

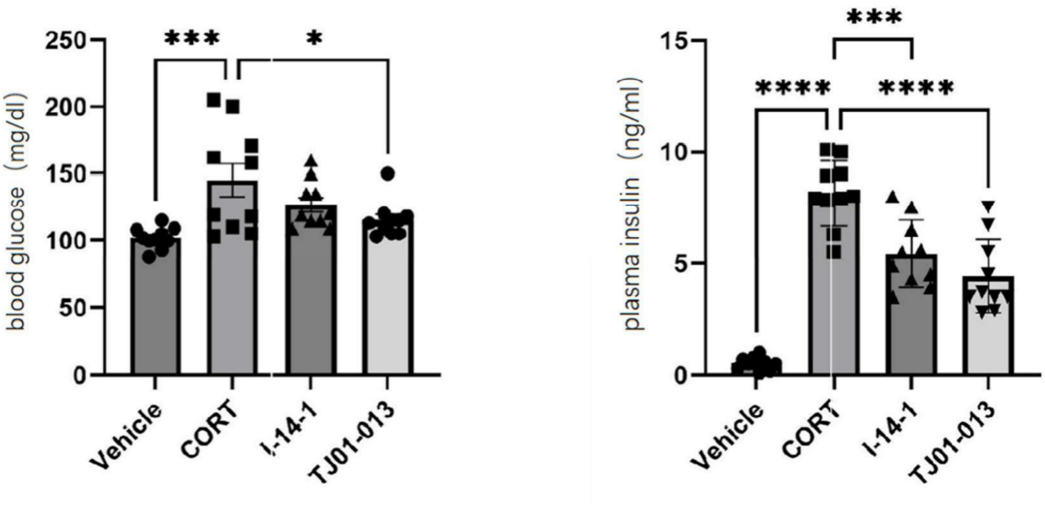


FIG. 30

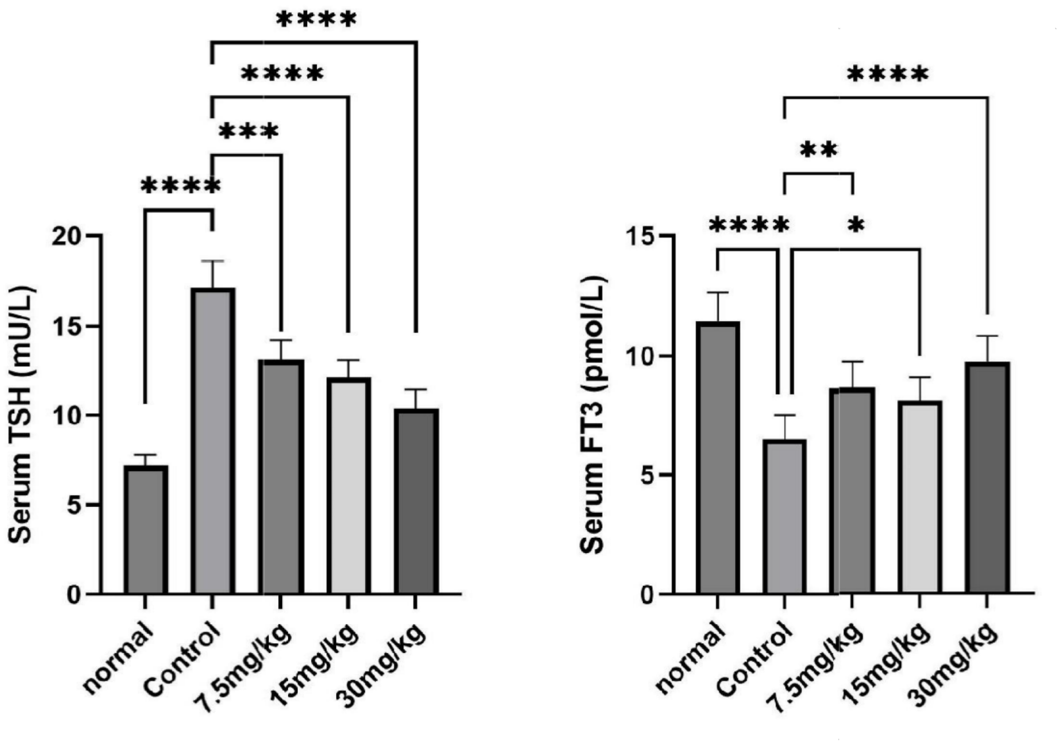
Serum TSH (mU/L)
20
15
10
5
0
normal
Control
7.5mg/kg
15mg/kg
30mg/kg
****
***
****
****
Serum FT3 (pmol/L)
15
10
5
0
normal
Control
7.5mg/kg
15mg/kg
30mg/kg
****
**
*
****

FIG. 31

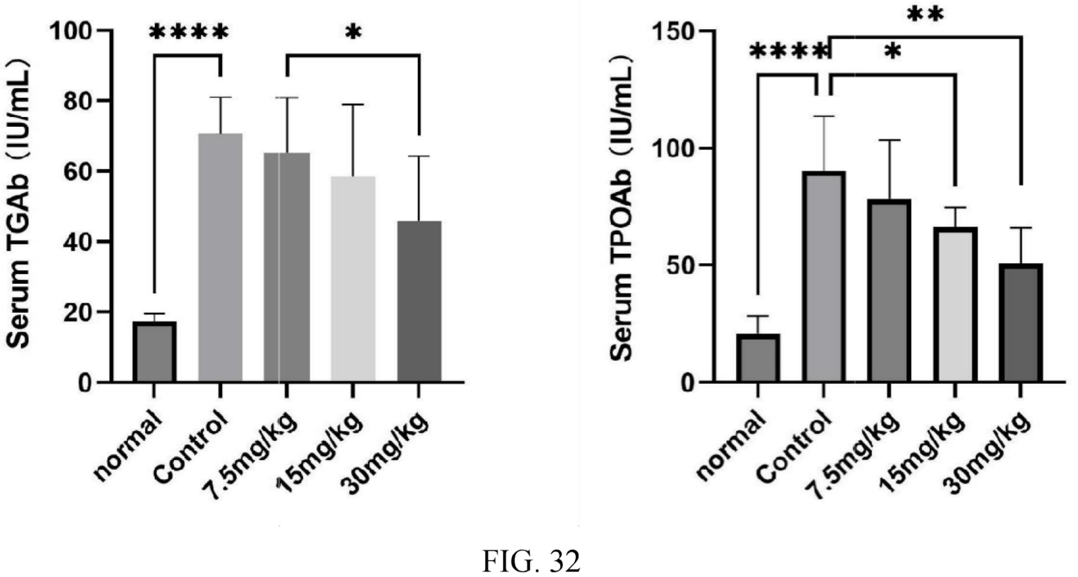
Serum TGAb（IU/mL）
100
80
60
40
20
0
normal
Control
7.5mg/kg
15mg/kg
30mg/kg
****
*
Serum TPOAb（IU/mL）
150
100
50
0
normal
Control
7.5mg/kg
15mg/kg
30mg/kg
****
*
**

FIG. 32

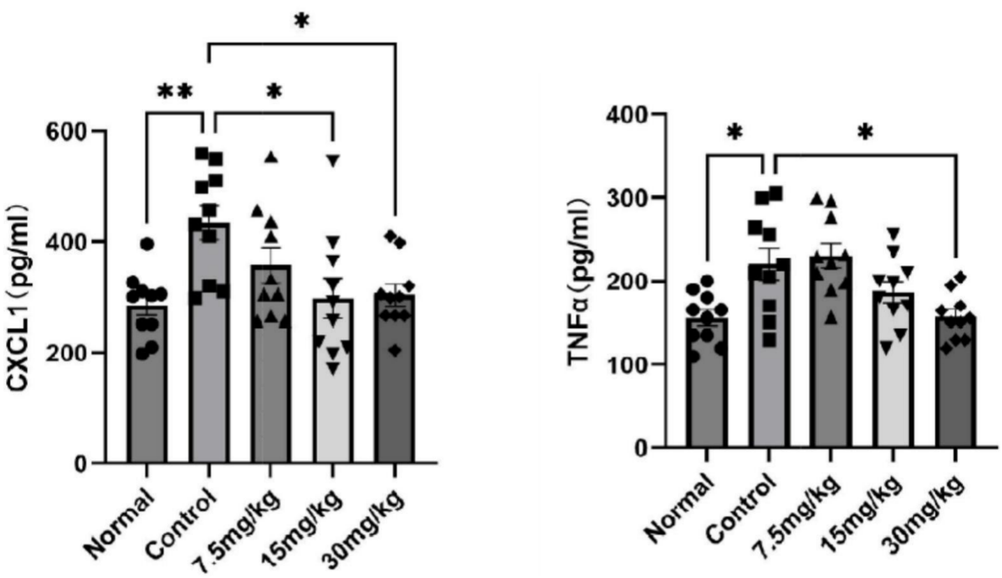
CXCL1 (pg/ml)
600
400
200
0
Normal
Control
7.5mg/kg
15mg/kg
30mg/kg
**
*
*
TNFα (pg/ml)
400
300
200
100
0
*
*

FIG. 33

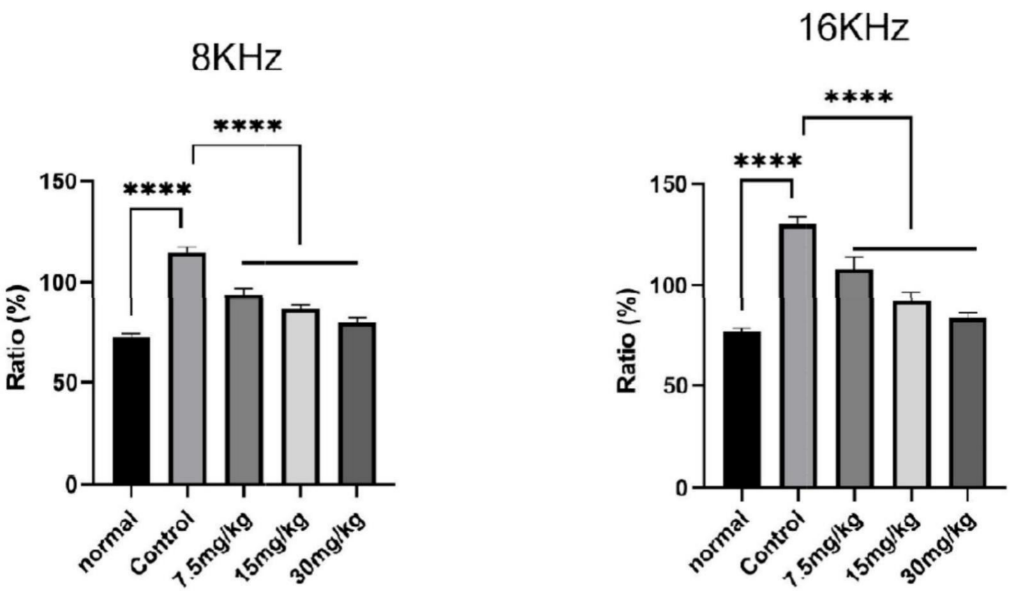
8KHz
16KHz
Ratio (%)
150
100
50
0
normal
Control
7.5mg/kg
15mg/kg
30mg/kg
****
****

FIG. 34

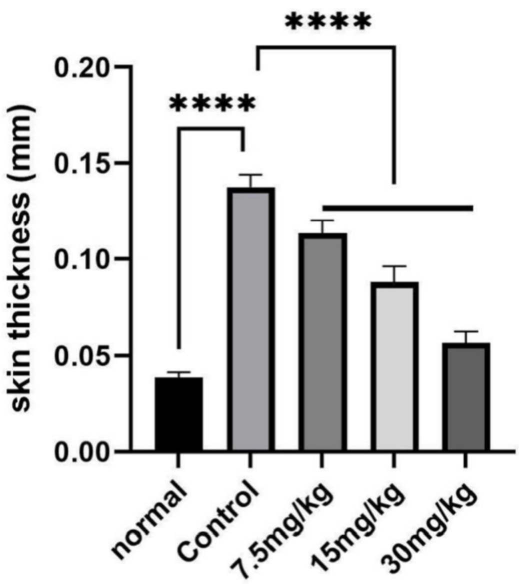
****
****
0.20
0.15
0.10
0.05
0.00
skin thickness (mm)
normal
Control
7.5mg/kg
15mg/kg
30mg/kg

FIG. 35

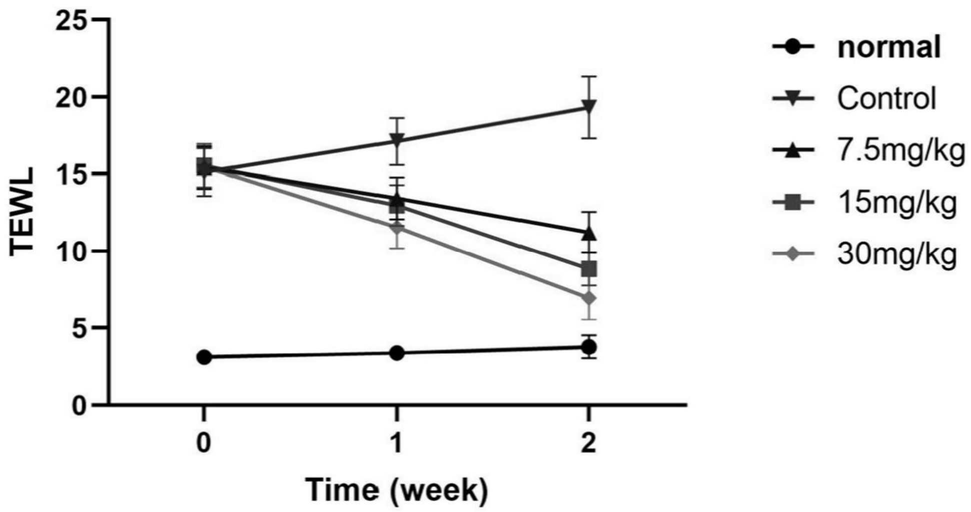
25
20
15
10
5
0
TEWL
0
1
2
Time (week)
normal
Control
7.5mg/kg
15mg/kg
30mg/kg

FIG. 36

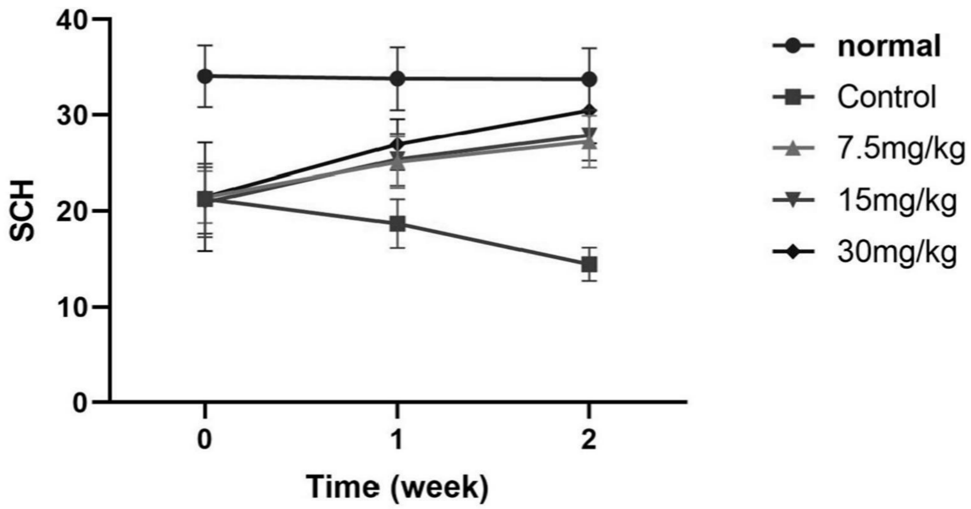

40
30
20
10
0
SCH
0
1
2
Time (week)
normal
Control
7.5mg/kg
15mg/kg
30mg/kg


FIG. 37

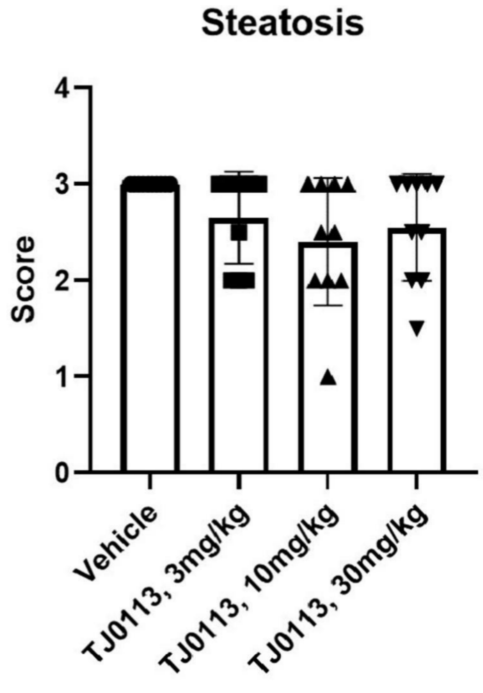

Steatosis
4
3
2
1
0
Score
Vehicle
TJ0113, 3mg/kg
TJ0113, 10mg/kg
TJ0113, 30mg/kg


FIG. 38

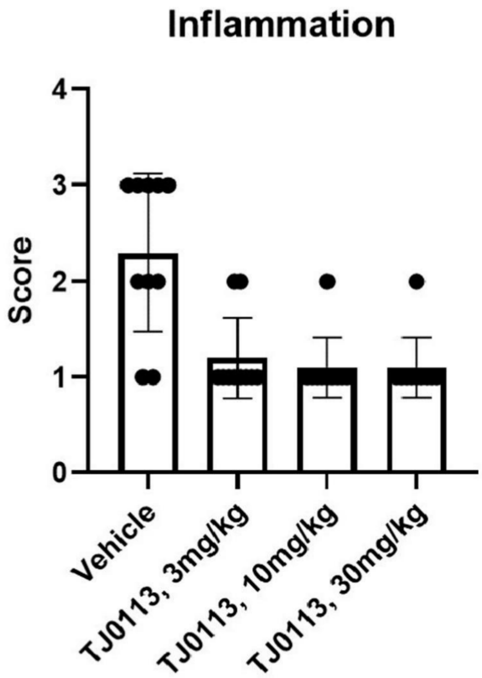
Inflammation
Score
0
1
2
3
4
Vehicle
TJ0113, 3mg/kg
TJ0113, 10mg/kg
TJ0113, 30mg/kg

FIG. 39

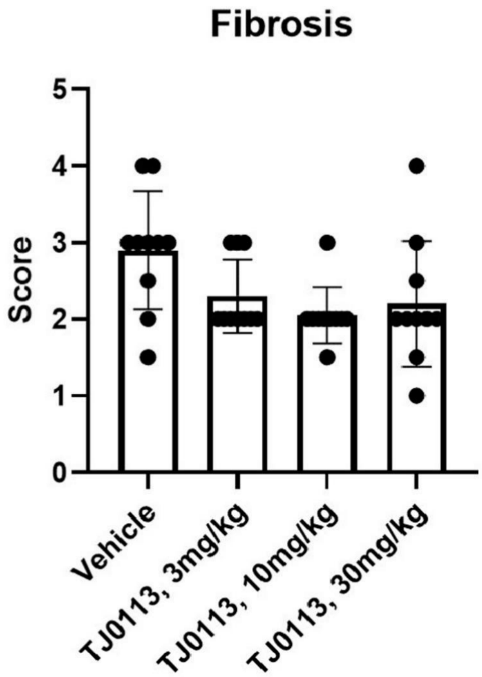
Fibrosis
Score
0
1
2
3
4
5
Vehicle
TJ0113, 3mg/kg
TJ0113, 10mg/kg
TJ0113, 30mg/kg

FIG. 40

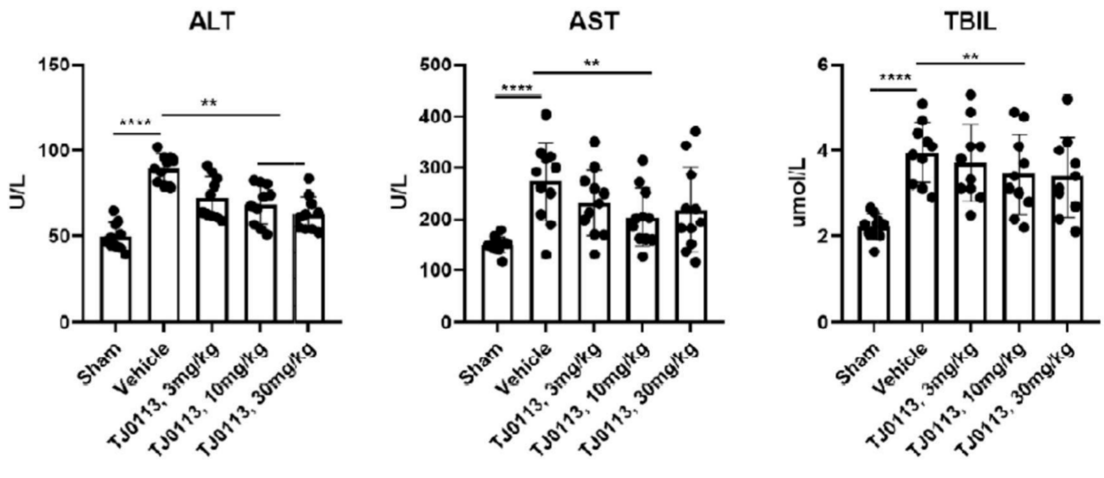


FIG. 41

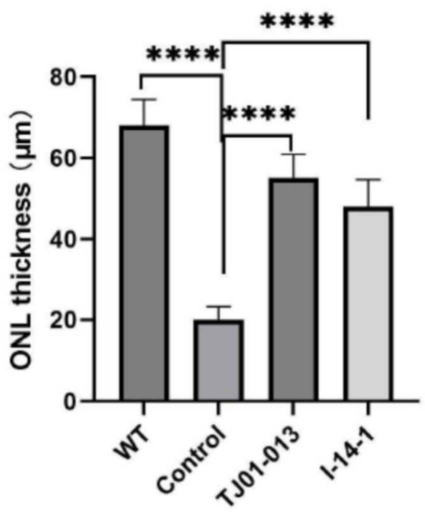


FIG. 42

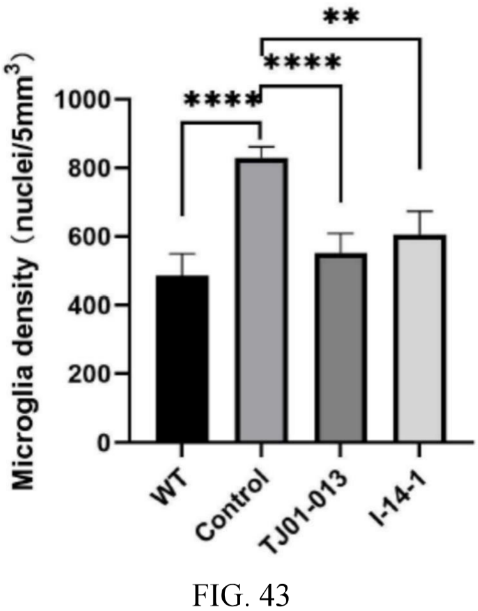


FIG. 43

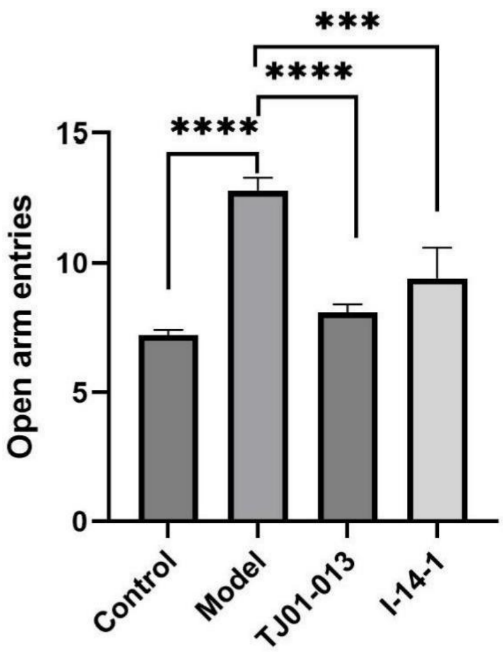
Open arm entries
15
10
5
0
Control
Model
TJ01-013
I-14-1
****
****
***

FIG. 44

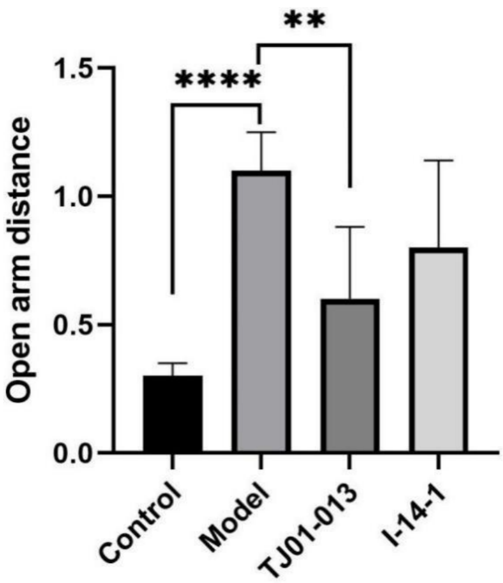
Open arm distance
1.5
1.0
0.5
0.0
Control
Model
TJ01-013
I-14-1
****
**

FIG. 45

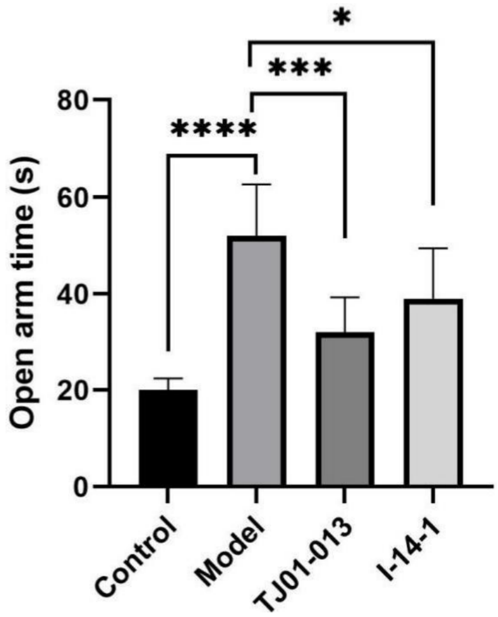
*
***
****
80
60
40
20
0
Open arm time (s)
Control
Model
TJ01-013
I-14-1

FIG. 46

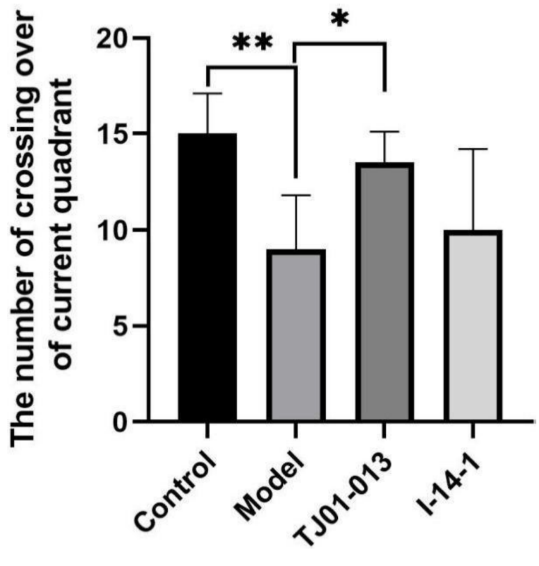
**
*
20
15
10
5
0
The number of crossing over of current quadrant
Control
Model
TJ01-013
I-14-1

FIG. 47

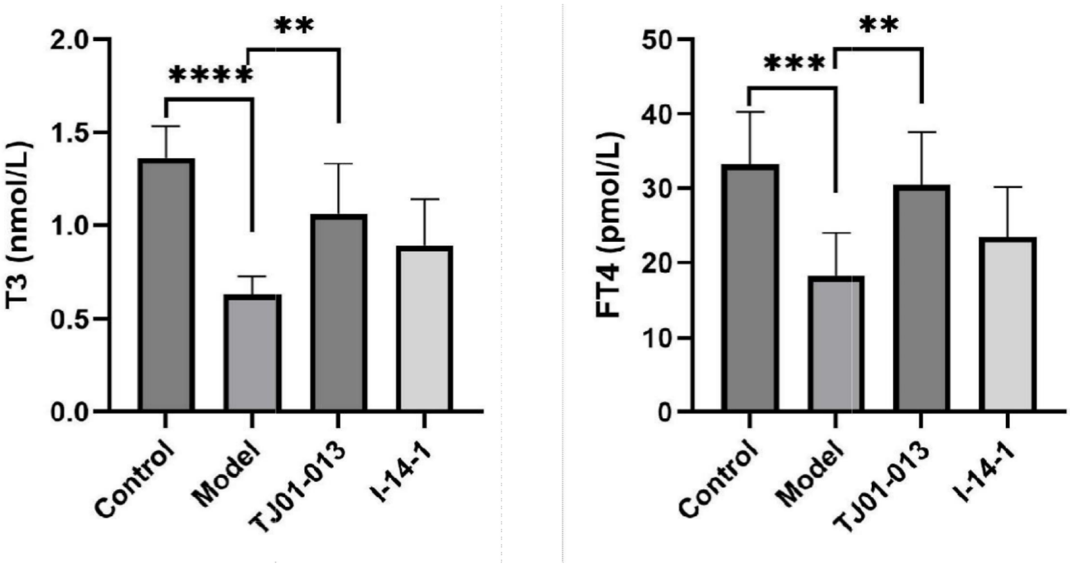
2.0
1.5
1.0
0.5
0.0
T3 (nmol/L)
****
**
Control
Model
TJ01-013
I-14-1
50
40
30
20
10
0
FT4 (pmol/L)
***
**
Control
Model
TJ01-013
I-14-1

FIG. 48

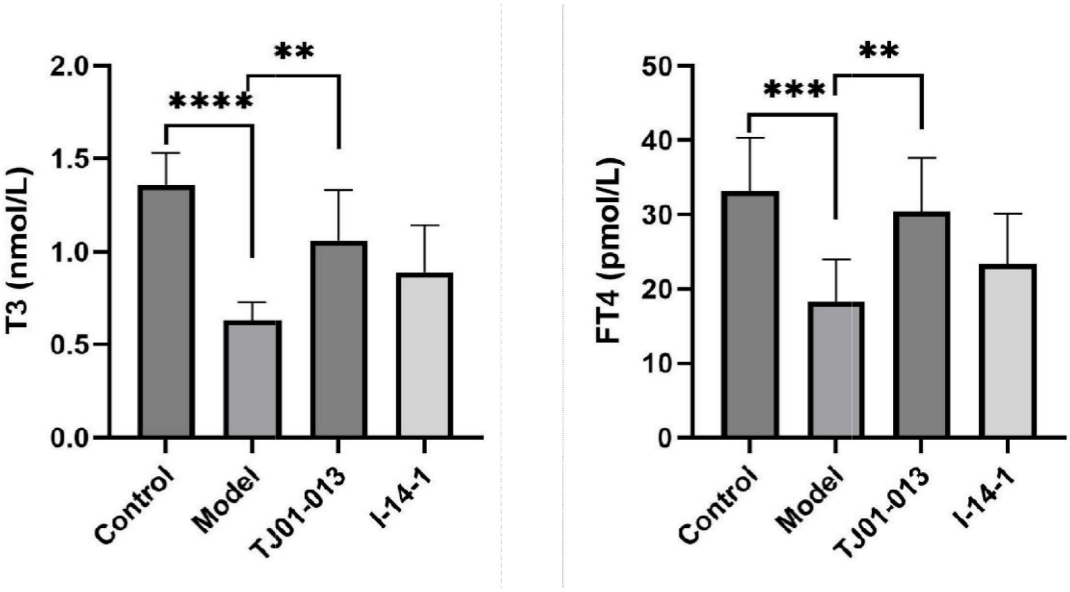
2.0
1.5
1.0
0.5
0.0
T3 (nmol/L)
****
**
Control
Model
TJ01-013
I-14-1
50
40
30
20
10
0
FT4 (pmol/L)
***
**
Control
Model
TJ01-013
I-14-1

FIG. 49

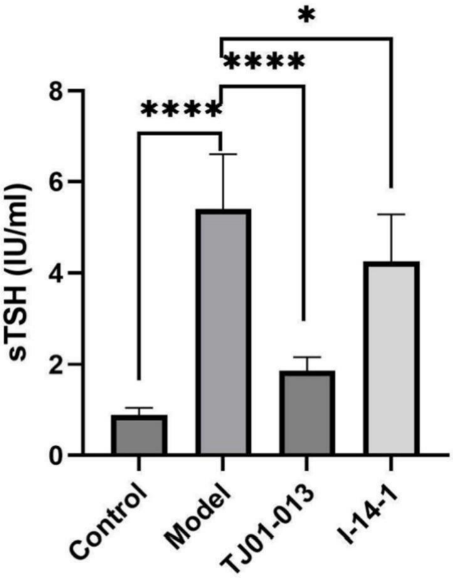


FIG. 50

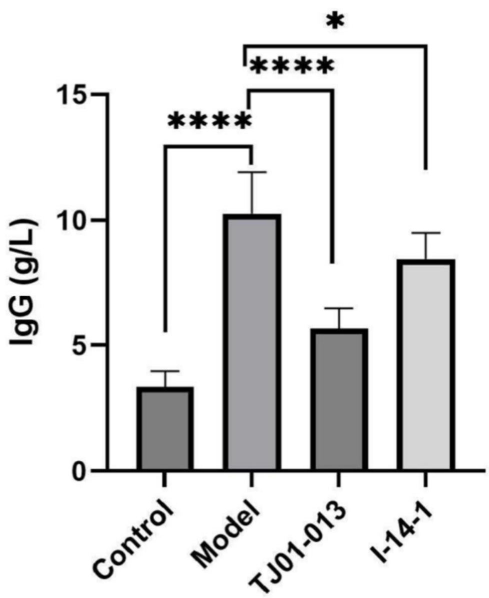


FIG. 51

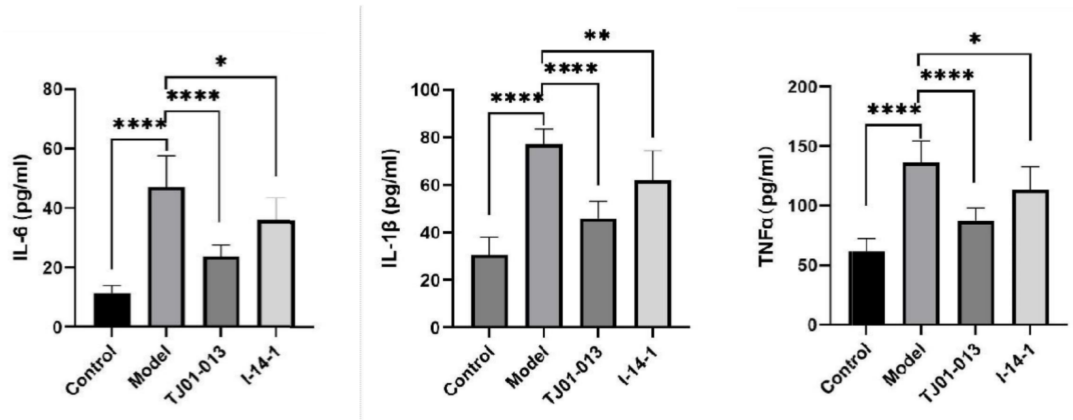


FIG. 52

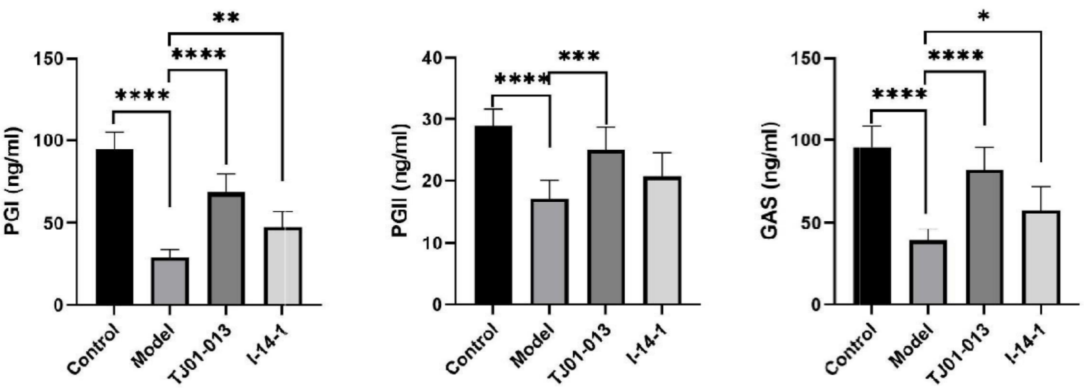
PGI (ng/ml)
150
100
50
0
****
****
**
Control
Model
TJ01-013
I-14-1
PGII (ng/ml)
40
30
20
10
0
****
***
Control
Model
TJ01-013
I-14-1
GAS (ng/ml)
150
100
50
0
****
****
*
Control
Model
TJ01-013
I-14-1

FIG. 53

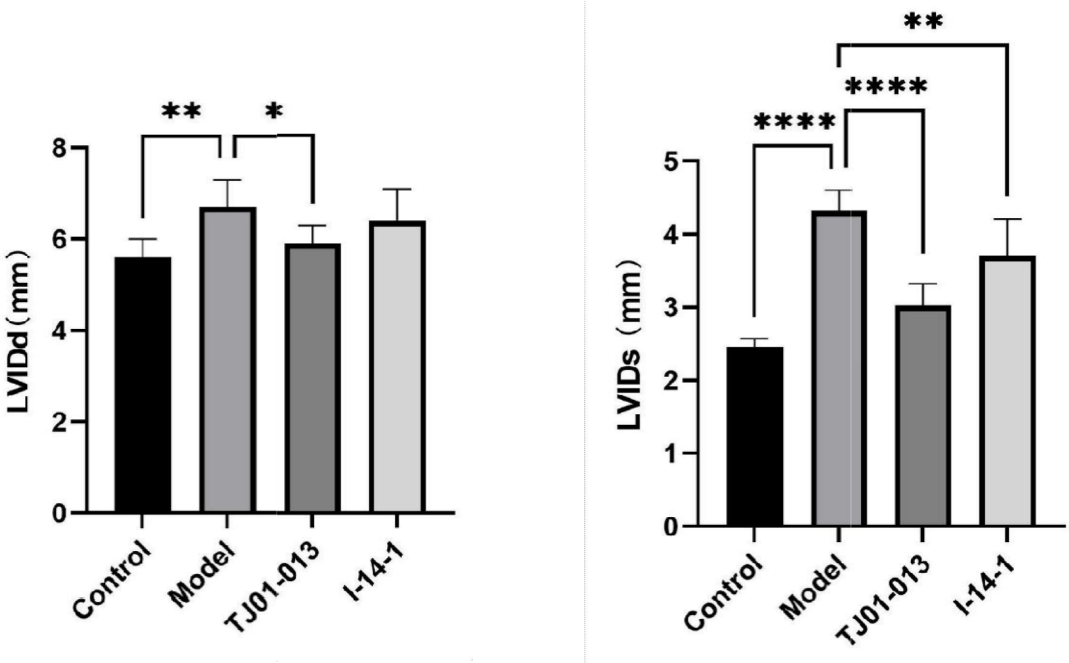
**
*
8
6
4
2
0
LVIDd（mm）
Control
Model
TJ01-013
I-14-1
**
****
****
**
5
4
3
2
1
0
LVIDs（mm）
Control
Model
TJ01-013
I-14-1

FIG. 54

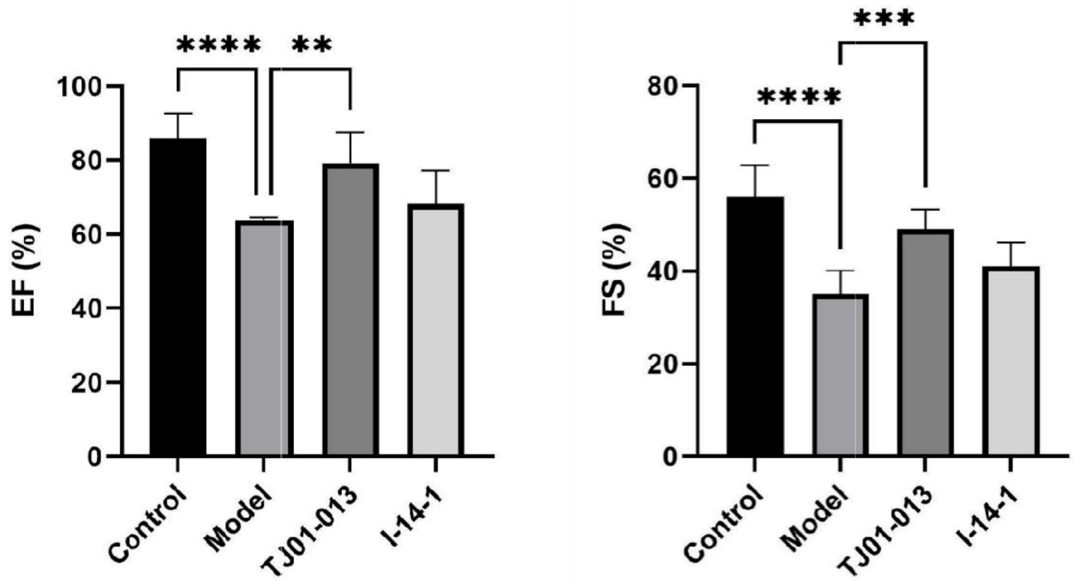
****
**
100
80
60
40
20
0
EF (%)
Control
Model
TJ01-013
I-14-1
***
****
80
60
40
20
0
FS (%)
Control
Model
TJ01-013
I-14-1

FIG. 55

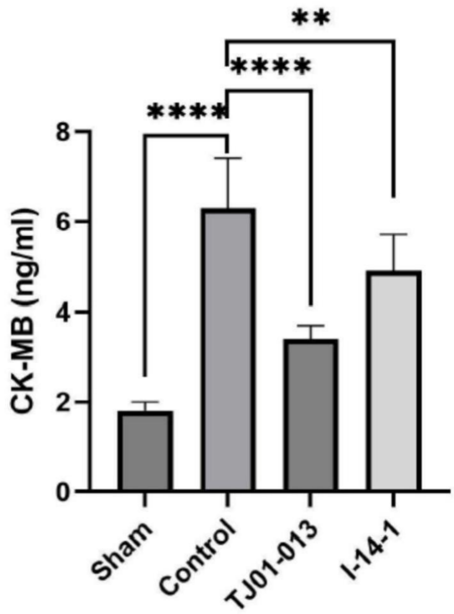
**
****
****
8
6
4
2
0
CK-MB (ng/ml)
Sham
Control
TJ01-013
I-14-1

FIG. 56

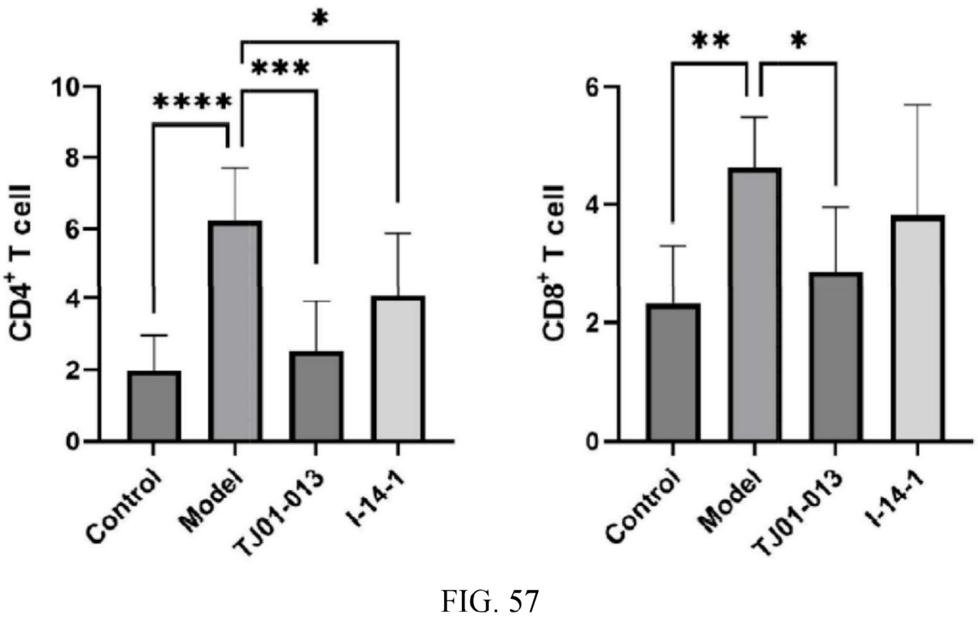
*
***
****
10
8
6
4
2
0
CD4+ T cell
Control
Model
TJ01-013
I-14-1
**
*
6
4
2
0
CD8+ T cell
Control
Model
TJ01-013
I-14-1

FIG. 57

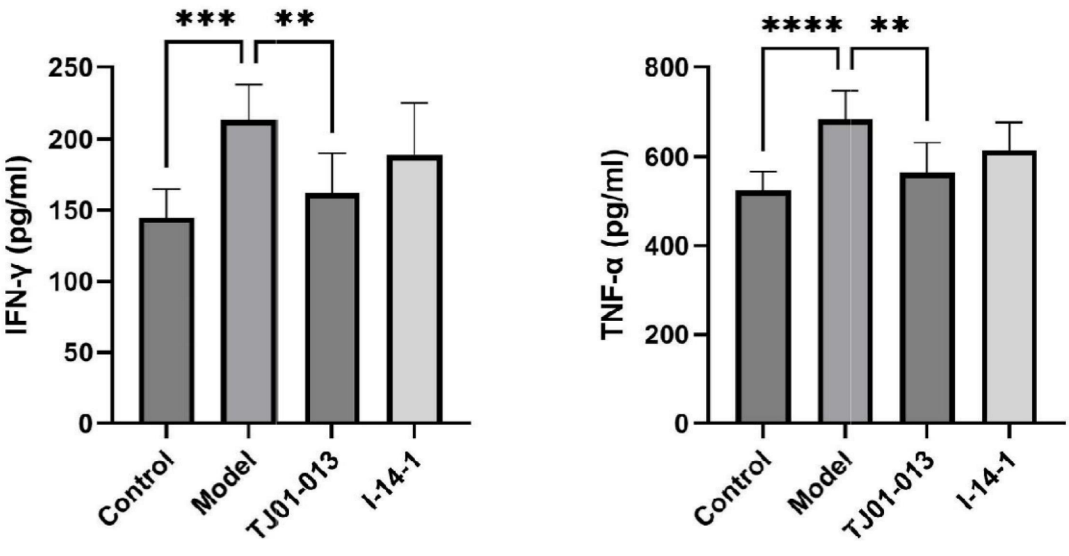
***
**
250
200
150
100
50
0
IFN-γ (pg/ml)
Control
Model
TJ01-013
I-14-1
****
**
800
600
400
200
0
TNF-α (pg/ml)
Control
Model
TJ01-013
I-14-1

FIG. 58

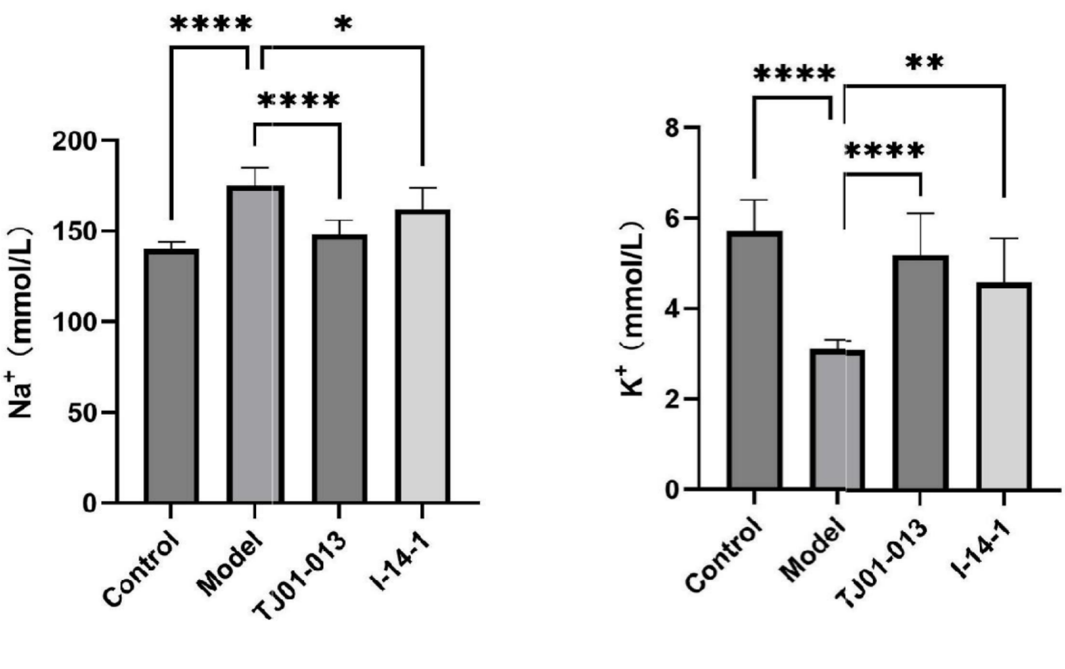
****
*
****
200
150
100
50
0
Na+（mmol/L）
Control
Model
TJ01-013
I-14-1
****
**
****
8
6
4
2
0
K+（mmol/L）
Control
Model
TJ01-013
I-14-1

FIG. 59

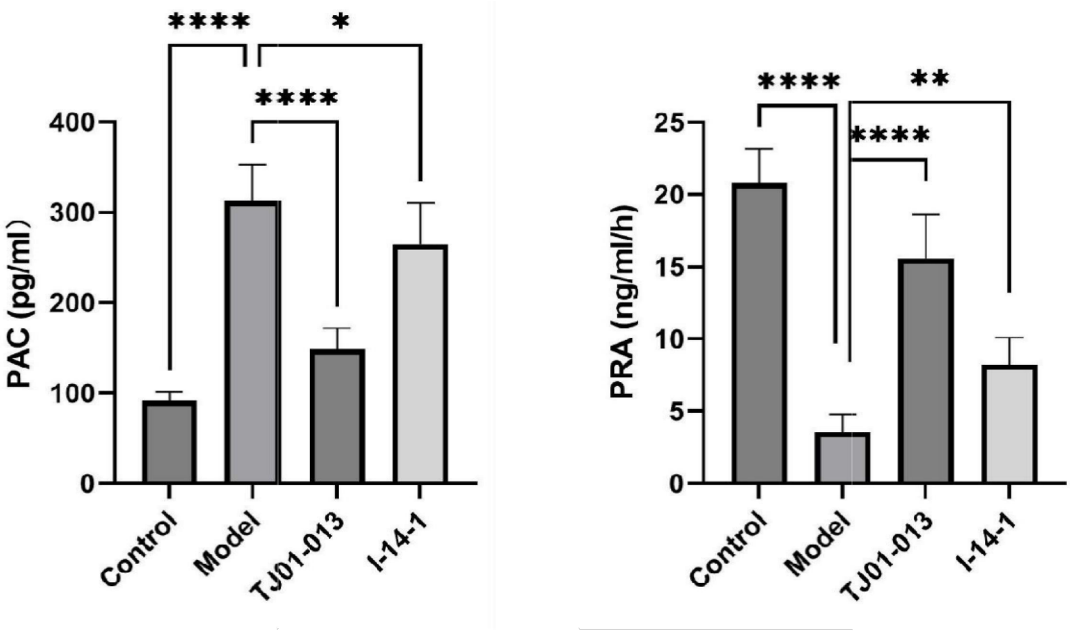
****
*
****
PAC (pg/ml)
400
300
200
100
0
Control
Model
TJ01-013
I-14-1
****
**
****
PRA (ng/ml/h)
25
20
15
10
5
0
Control
Model
TJ01-013
I-14-1

FIG. 60

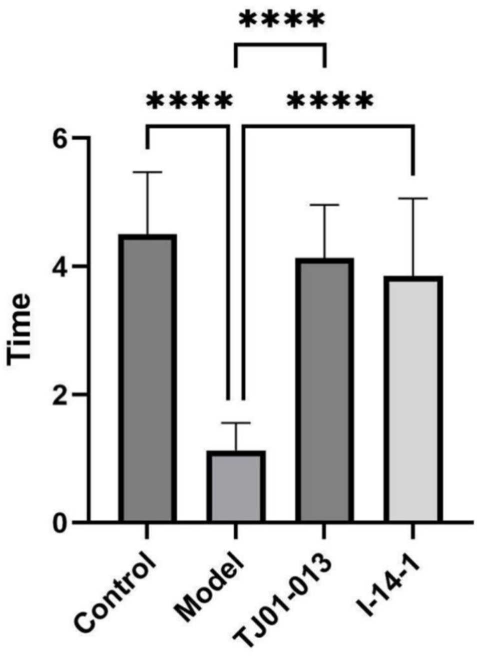
****
****
****
Time
6
4
2
0
Control
Model
TJ01-013
I-14-1

FIG. 61

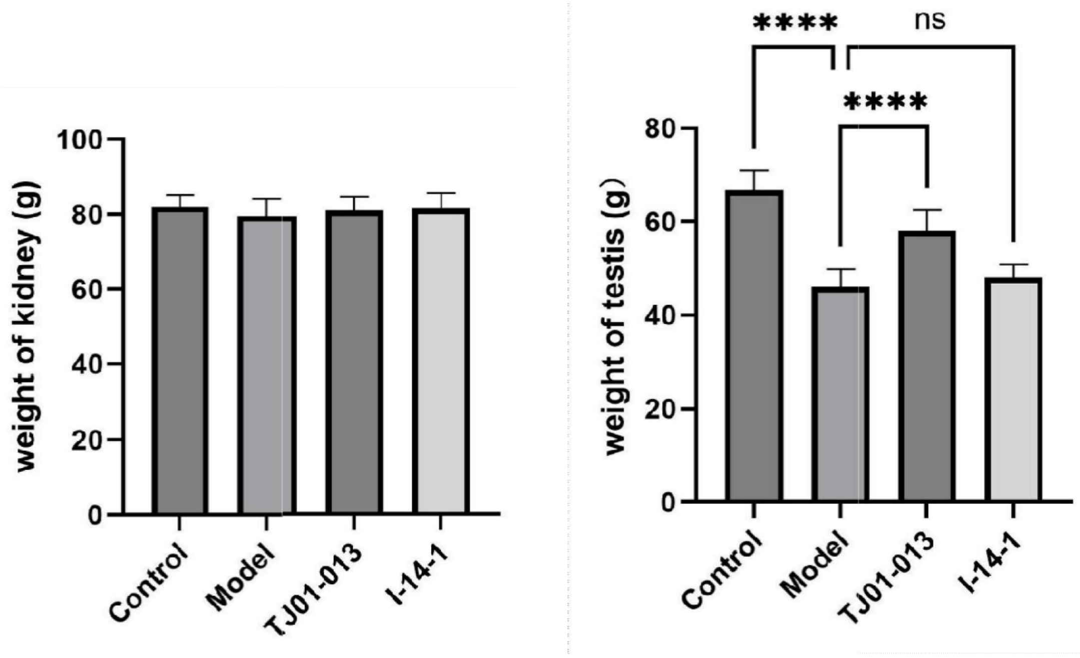
weight of kidney (g)
100
80
60
40
20
0
Control
Model
TJ01-013
I-14-1
****
ns
****
weight of testis (g)
80
60
40
20
0
Control
Model
TJ01-013
I-14-1

FIG. 62

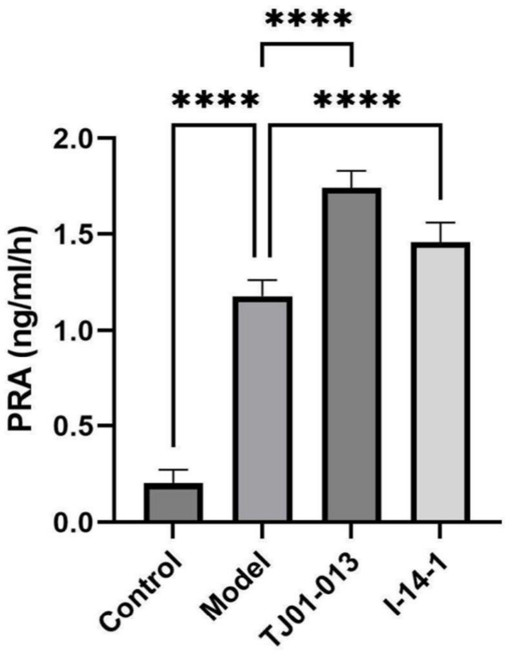
****
****
****
PRA (ng/ml/h)
2.0
1.5
1.0
0.5
0.0
Control
Model
TJ01-013
I-14-1

FIG. 63

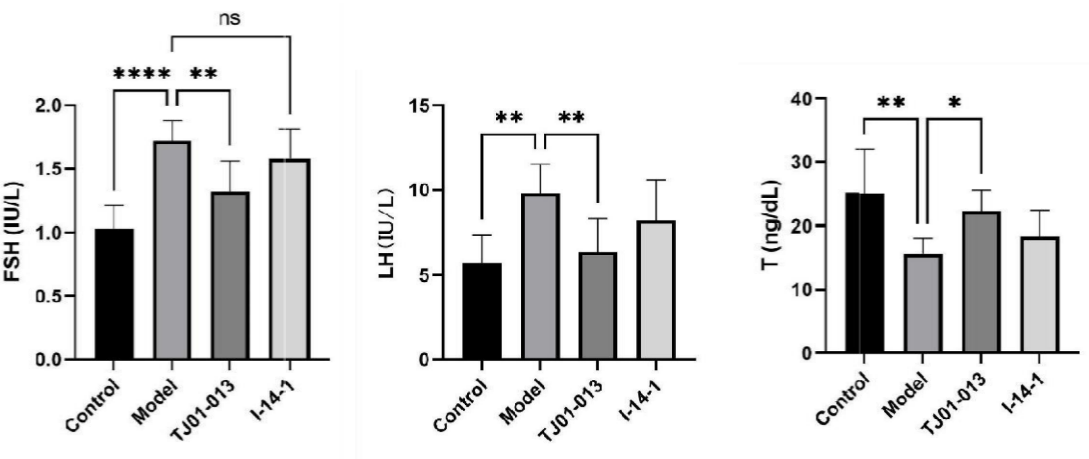
ns
****
**
FSH (IU/L)
2.0
1.5
1.0
0.5
0.0
Control
Model
TJ01-013
I-14-1
**
**
LH (IU/L)
15
10
5
0
Control
Model
TJ01-013
I-14-1
**
*
T (ng/dL)
40
30
20
10
0
Control
Model
TJ01-013
I-14-1

FIG. 64

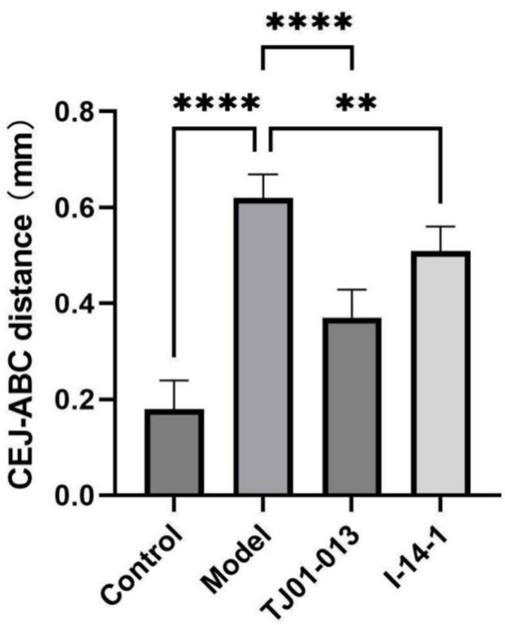
****
****
**
CEJ-ABC distance (mm)
0.8
0.6
0.4
0.2
0.0
Control
Model
TJ01-013
I-14-1

FIG. 65

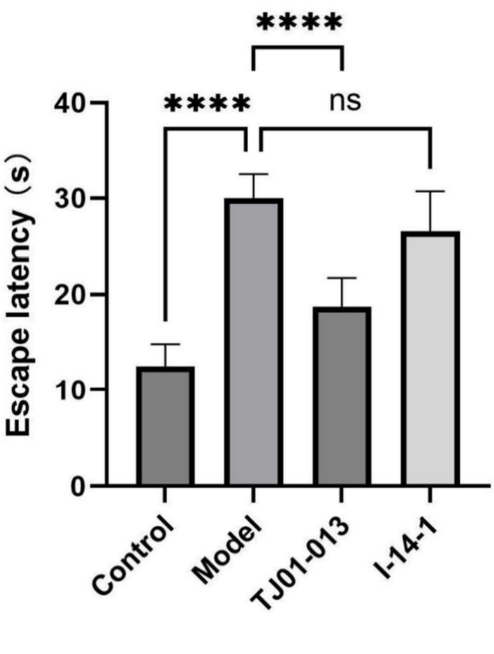
****
****
ns
Escape latency（s）
40
30
20
10
0
Control
Model
TJ01-013
I-14-1

FIG. 66

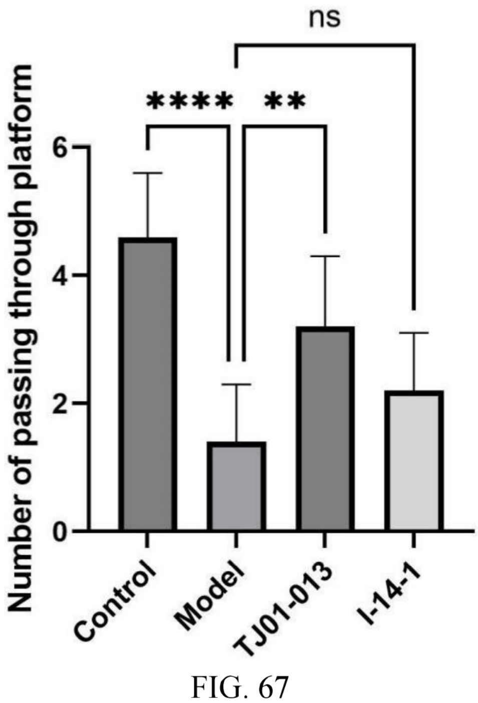
ns
****
**
Number of passing through platform
6
4
2
0
Control
Model
TJ01-013
I-14-1

FIG. 67

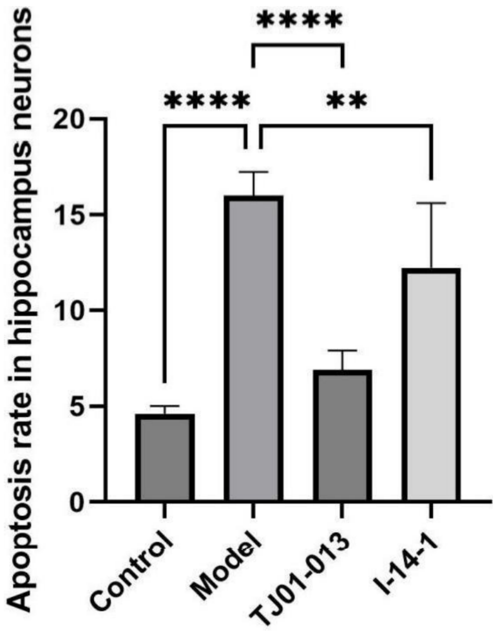
Apoptosis rate in hippocampus neurons
****
****
**
20
15
10
5
0
Control
Model
TJ01-013
I-14-1

FIG. 68

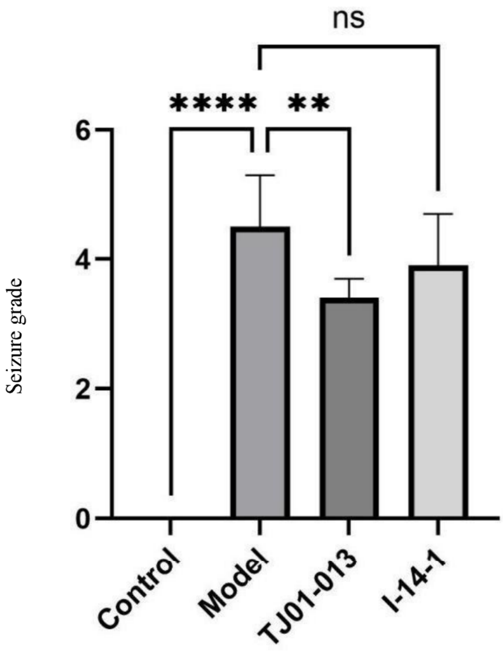
ns
****
**
6
4
2
0
Seizure grade
Control
Model
TJ01-013
I-14-1

FIG. 69

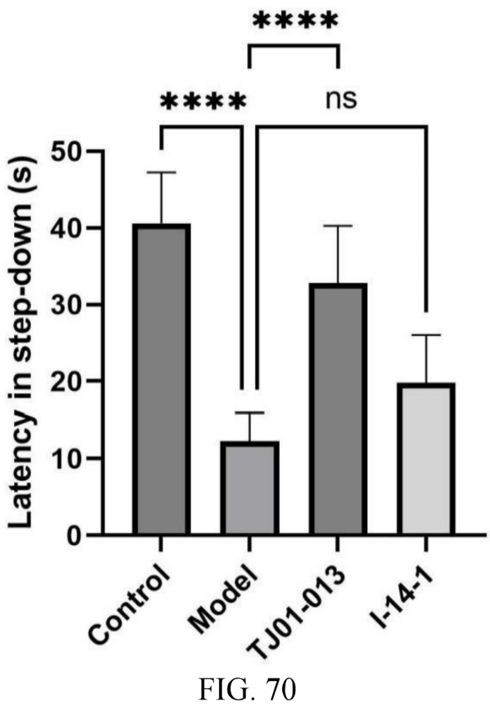
****
****
ns
50
40
30
20
10
0
Latency in step-down (s)
Control
Model
TJ01-013
I-14-1

FIG. 70

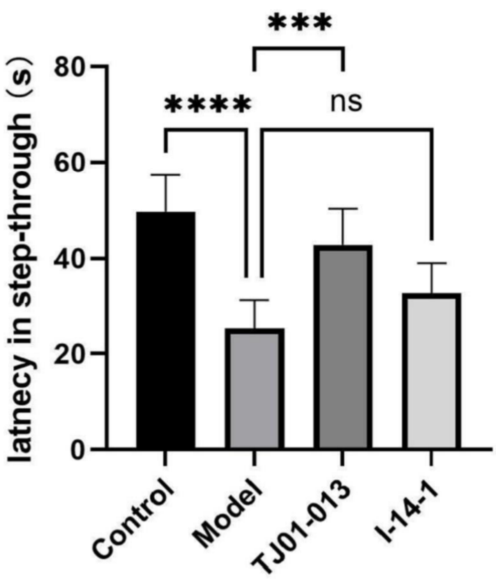
***
****
ns
latnecy in step-through（s）
80
60
40
20
0
Control
Model
TJ01-013
I-14-1

FIG. 71

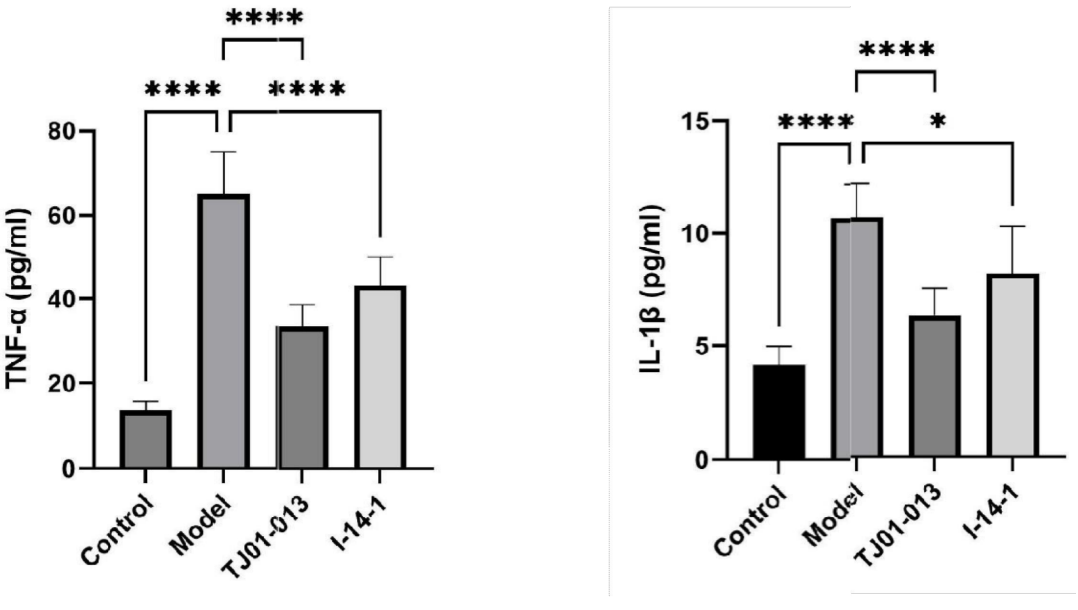
****
****
****
TNF-α (pg/ml)
80
60
40
20
0
Control
Model
TJ01-013
I-14-1
****
****
*
IL-1β (pg/ml)
15
10
5
0
Control
Model
TJ01-013
I-14-1

FIG. 72

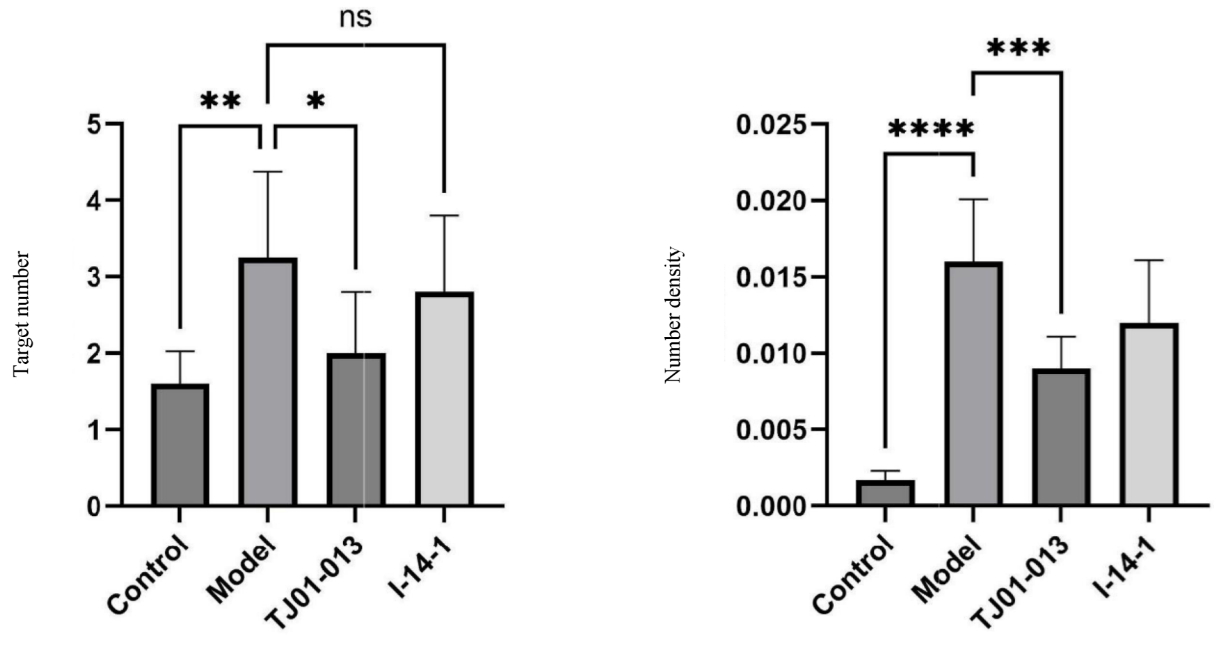
ns
**
*
Target number
0
1
2
3
4
5
Control
Model
TJ01-013
I-14-1
***
****
Number density
0.000
0.005
0.010
0.015
0.020
0.025
Control
Model
TJ01-013
I-14-1

FIG. 73

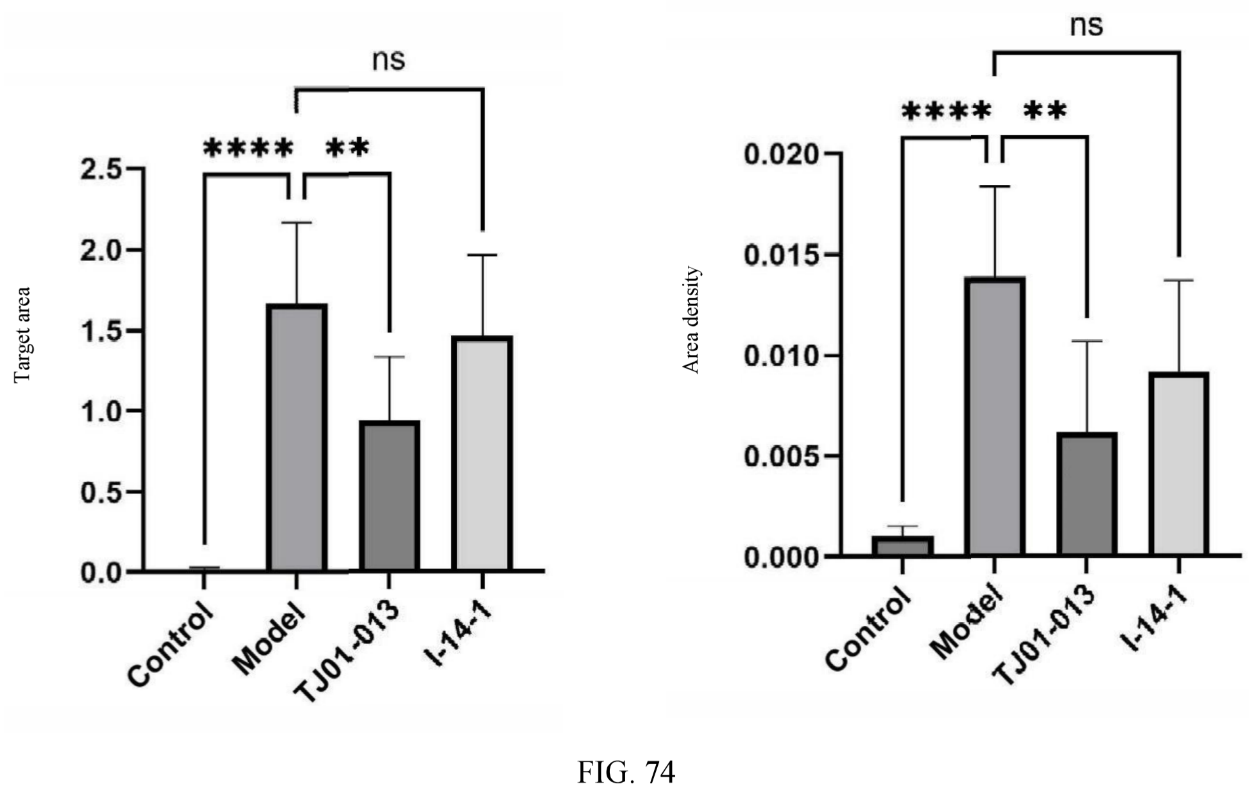
ns
****
**
Target area
0.0
0.5
1.0
1.5
2.0
2.5
Control
Model
TJ01-013
I-14-1
ns
****
**
Area density
0.000
0.005
0.010
0.015
0.020
Control
Model
TJ01-013
I-14-1

FIG. 74

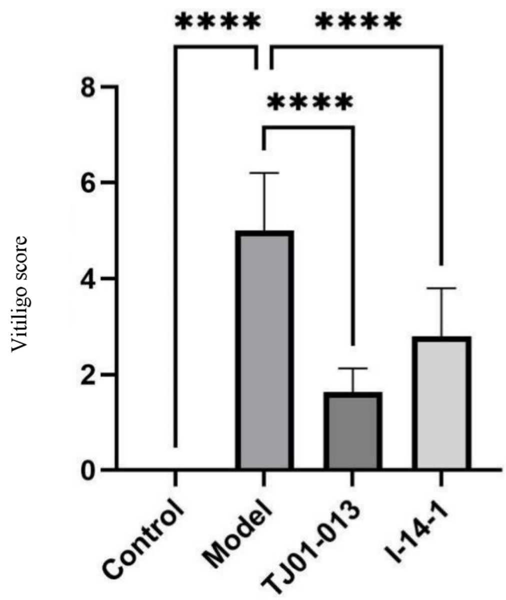
****
****
****
Vitiligo score
8
6
4
2
0
Control
Model
TJ01-013
I-14-1

FIG. 75

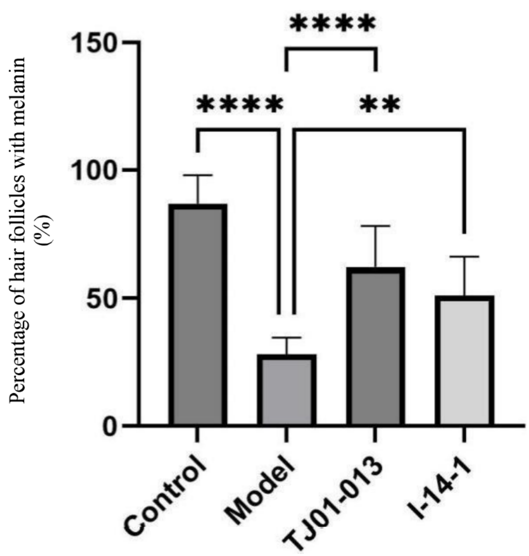
****
****
**
Percentage of hair follicles with melanin (%)
150
100
50
0
Control
Model
TJ01-013
I-14-1

FIG. 76

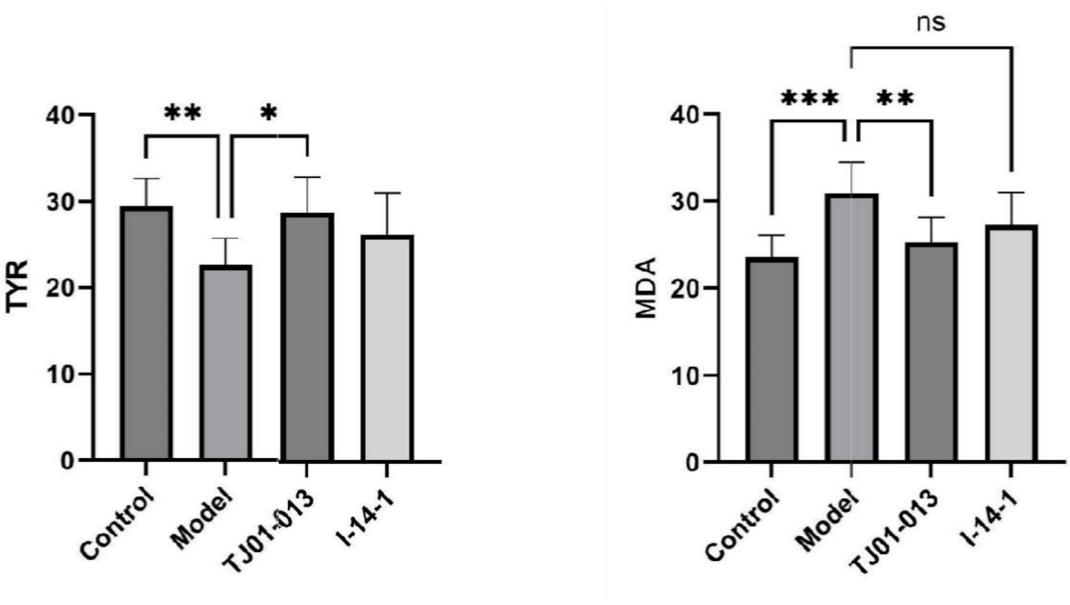
TYR
40
30
20
10
0
**
*
Control
Model
TJ01-013
I-14-1
MDA
40
30
20
10
0
***
**
ns
Control
Model
TJ01-013
I-14-1

FIG. 77

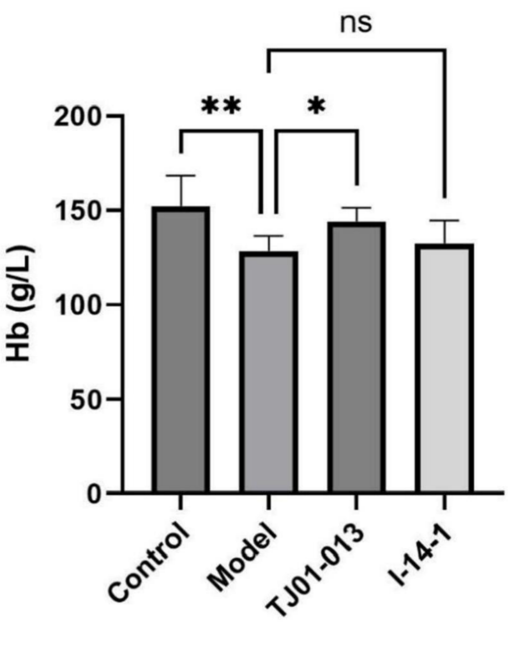
Hb (g/L)
200
150
100
50
0
**
*
ns
Control
Model
TJ01-013
I-14-1

FIG. 78

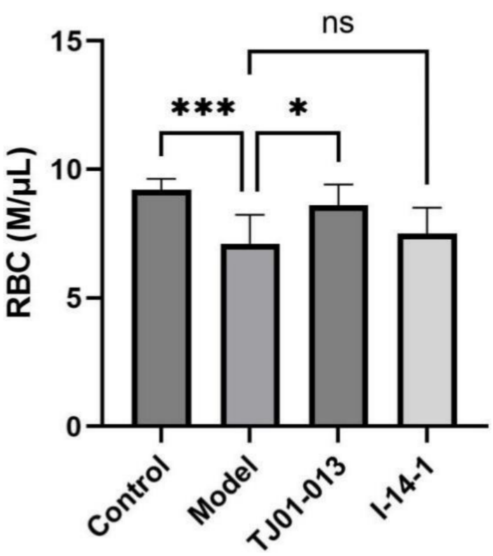
ns
***
*
15
10
5
0
RBC (M/μL)
Control
Model
TJ01-013
I-14-1

FIG. 79

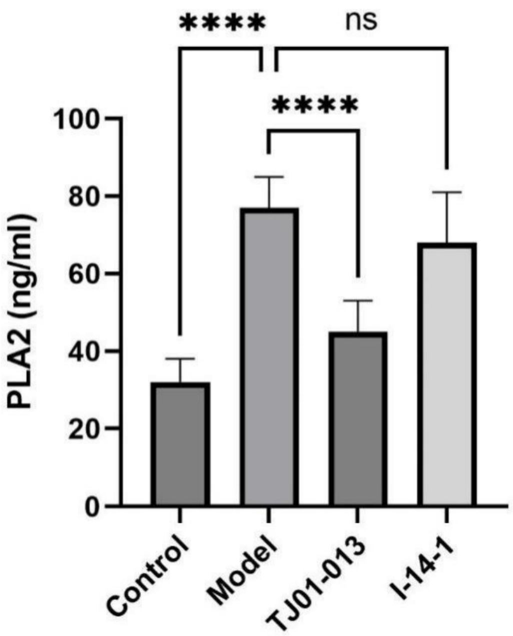
****
ns
****
100
80
60
40
20
0
PLA2 (ng/ml)
Control
Model
TJ01-013
I-14-1

FIG. 80

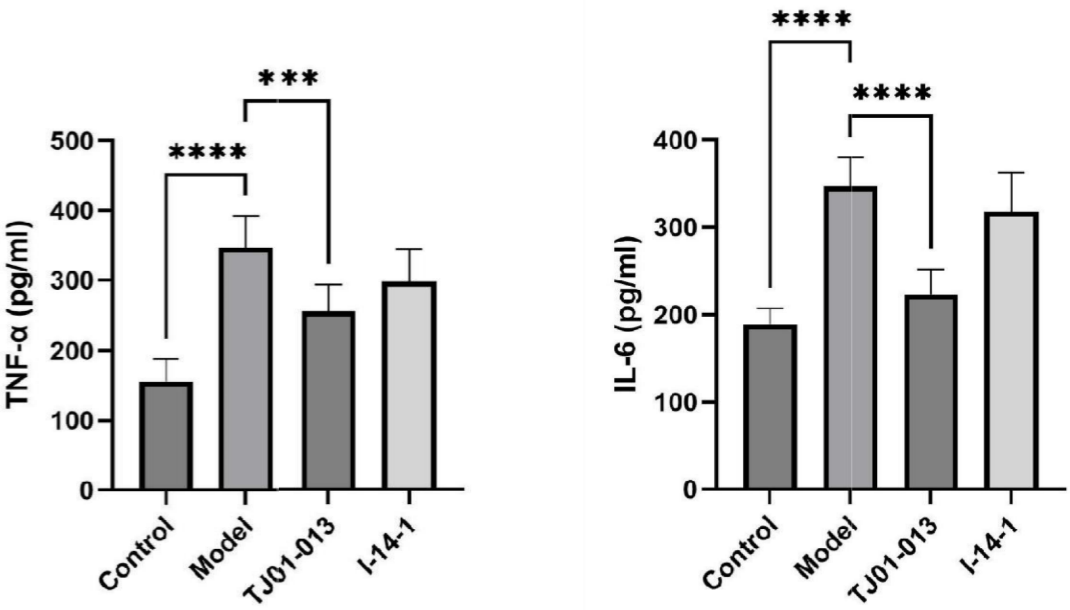
TNF-α (pg/ml)
500
400
300
200
100
0
****
***
Control
Model
TJ01-013
I-14-1
IL-6 (pg/ml)
400
300
200
100
0
****
****
Control
Model
TJ01-013
I-14-1

FIG. 81

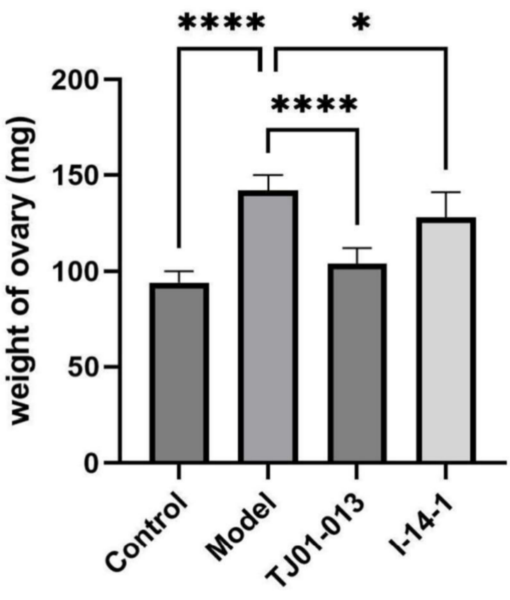
weight of ovary (mg)
200
150
100
50
0
****
*
****
Control
Model
TJ01-013
I-14-1

FIG. 82

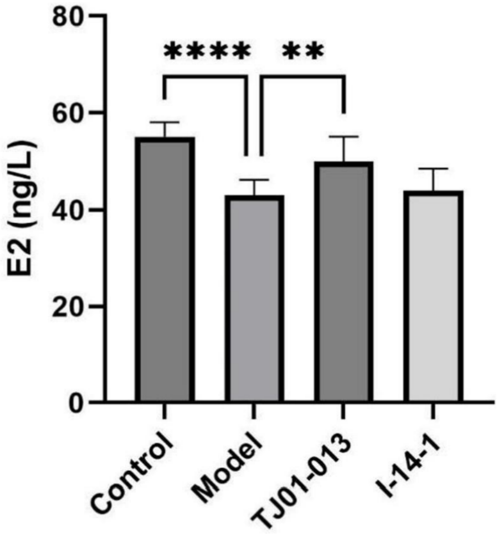
80
60
40
20
0
E2 (ng/L)
****
**
Control
Model
TJ01-013
I-14-1

FIG. 83

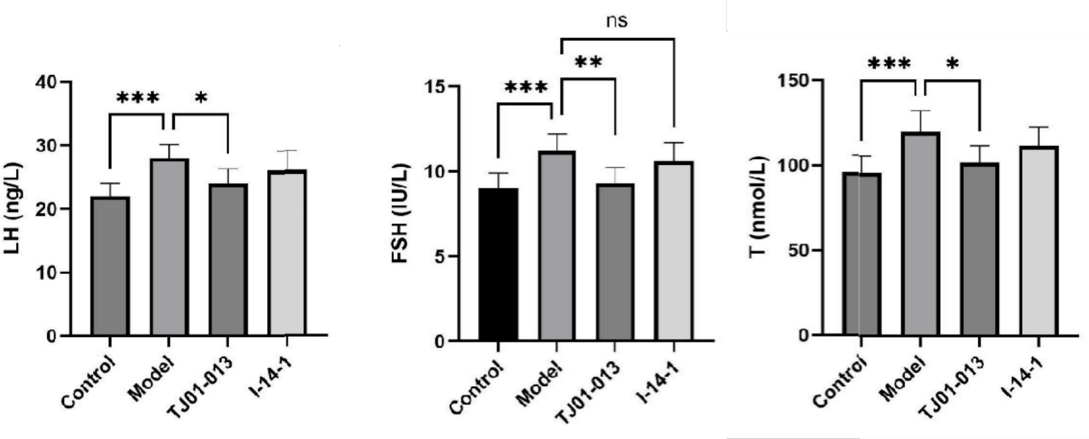
LH (ng/L)
40
30
20
10
0
***
*
Control
Model
TJ01-013
I-14-1
FSH (IU/L)
15
10
5
0
ns
***
**
Control
Model
TJ01-013
I-14-1
T (nmol/L)
150
100
50
0
***
*
Control
Model
TJ01-013
I-14-1

FIG. 84

## INTERNATIONAL SEARCH REPORT

International application No.
**PCT/CN2024/082356**

### A. CLASSIFICATION OF SUBJECT MATTER

A61K 31/145(2006.01)i; A61K31/196(2006.01)i; A61P25/00(2006.01)i; A61P13/12(2006.01)i; A61P1/00(2006.01)i; A61P29/00(2006.01)i; A61P9/00(2006.01)i; A61P1/16(2006.01)i

According to International Patent Classification (IPC) or to both national classification and IPC

### B. FIELDS SEARCHED

Minimum documentation searched (classification system followed by classification symbols)

IPC: A61K; A61P

Documentation searched other than minimum documentation to the extent that such documents are included in the fields searched

Electronic data base consulted during the international search (name of data base and, where practicable, search terms used)

CNABS; CNTXT; ENTXT; DWPI; STNext Registry; STNext Caplus; CNKI: 基于通式(I)的结构检索, structural search according to general formula (1), 萘胺, 线粒体自噬, MCL1, UMI-77, naphthylamine, mitochondrial dysfunction

### C. DOCUMENTS CONSIDERED TO BE RELEVANT

| Category* | Citation of document, with indication, where appropriate, of the relevant passages | Relevant to claim No. |
|---|---|---|
| E | CN 117736124 A (HANGZHOU PHECDAMED CO., LTD.) 22 March 2024 (2024-03-22) claims 1 and 13, and description, paragraphs 0272-285 | 1-8, 10-11 |
| PY | WO 2023045909 A1 (HANGZHOU PHECDAMED CO., LTD.) 30 March 2023 (2023-03-30) claims 1-11 | 1-11 |
| X | WO 2020224364 A1 (ZHEJIANG UNIVERSITY) 12 November 2020 (2020-11-12) claims 1-15 | 1-11 |
| X | WO 2013052943 A2 (THE REGENTS OF THE UNIVERSITY OF MICHGIAN et al.) 11 April 2013 (2013-04-11) claims 1-32, and page 34, line 3-page 35, line 4 | 1-11 |
| Y | WO 2020224364 A1 (ZHEJIANG UNIVERSITY) 12 November 2020 (2020-11-12) claims 1-15 | 1-11 |
| A | GE, Yiyu et al. "Discovery and Synthesis of Hydronaphthoquinones as Novel Proteasome Inhibitors" *Journal of Medicinal Chemistry*, Vol. 55, No. 5, 05 January 2012 (2012-01-05), 1978-1998 compounds 10e and 11e in Scheme 2 | 1-11 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 July 2024** | **05 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/082356**

| Patent document cited in search report | | | Publication date (day/month/year) | Patent family member(s) | | | Publication date (day/month/year) |
|---|---|---|---|---|---|---|---|
| CN | 117736124 | A | 22 March 2024 | None | | | |
| WO | 2023045909 | A1 | 30 March 2023 | IL | 311496 | A | 01 May 2024 |
| | | | | AU | 2022350943 | A1 | 21 March 2024 |
| | | | | KR | 20240076778 | A | 30 May 2024 |
| | | | | CA | 3230925 | A1 | 03 March 2023 |
| WO | 2020224364 | A1 | 12 November 2020 | None | | | |
| WO | 2013052943 | A2 | 11 April 2013 | US | 2014235702 | A1 | 21 August 2014 |
| | | | | US | 9486422 | B2 | 08 November 2016 |
| | | | | WO | 2013052943 | A3 | 20 June 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023102998399
- CN 2023108029717
- CN 2023115501191
- CN 202111108417